(19) Europäisches Patentamt / European Patent Office / Office européen des brevets

(11) **EP 4 221 794 B1**

(12) **EUROPEAN PATENT SPECIFICATION**

(45) Date of publication and mention of the grant of the patent:
**12.11.2025 Bulletin 2025/46**

(21) Application number: **21873737.7**

(22) Date of filing: **30.09.2021**

(51) International Patent Classification (IPC):
***A61M 16/06*** *(2006.01)* ***A61M 16/08*** *(2006.01)*
***A61M 16/20*** *(2006.01)*

(52) Cooperative Patent Classification (CPC):
(C-Sets available)
**A61M 16/06; A61B 5/0826; A61B 5/087; A61B 5/4836; A61B 5/6803; A61M 16/024; A61M 16/0616; A61M 16/0666; A61M 16/0672; A61M 16/0683; A61M 16/0833; A61M 16/0875; A61M 16/16; A61M 16/20; A61M 16/202;** (Cont.)

(86) International application number:
**PCT/AU2021/051149**

(87) International publication number:
**WO 2022/067393 (07.04.2022 Gazette 2022/14)**

(54) **POSITIONING AND STABILIZING STRUCTURE FOR A PATIENT INTERFACE**

POSITIONIERUNGS- UND STABILISIERUNGSSTRUKTUR FÜR EINE PATIENTENSCHNITTSTELLE

STRUCTURE DE POSITIONNEMENT ET DE STABILISATION POUR UNE INTERFACE PATIENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.09.2020 AU 2020903542**
**30.09.2020 AU 2020903543**
**30.09.2020 AU 2020903544**
**02.10.2020 AU 2020903563**

(43) Date of publication of application:
**09.08.2023 Bulletin 2023/32**

(73) Proprietor: **ResMed Pty Ltd**
**Bella Vista, New South Wales 2153 (AU)**

(72) Inventor: **DANTANARAYANA, Muditha, Pradeep**
**North Ryde, New South Wales 2113 (AU)**

(74) Representative: **Vossius & Partner**
**Patentanwälte Rechtsanwälte mbB**
**Siebertstraße 3**
**81675 München (DE)**

(56) References cited:
**EP-B1- 2 964 300** **WO-A1-2012/107858**
**WO-A1-2014/142681** **WO-A1-2018/065867**
**WO-A2-2005/009501** **US-A1- 2002 092 527**
**US-A1- 2014 102 456** **US-A1- 2015 367 092**
**US-A1- 2015 367 092** **US-B2- 8 631 799**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



**(Cont. next page)**

(52) Cooperative Patent Classification (CPC): (Cont.)
**G16H 20/40; G16H 40/63;** A61B 5/0022; A61B 5/0871; A61M 16/0051; A61M 16/0066; A61M 16/0075; A61M 16/0622; A61M 16/0633; A61M 16/0688; A61M 16/0858; A61M 16/0883; A61M 16/101; A61M 16/1055; A61M 16/107; A61M 16/1085; A61M 16/109; A61M 16/1095; A61M 16/12; A61M 16/161; A61M 16/204; A61M 2016/0021; A61M 2016/0024; A61M 2016/0027; A61M 2016/003; A61M 2202/0208; A61M 2202/0225; A61M 2205/0216; A61M 2205/0238; A61M 2205/15; A61M 2205/18; A61M 2205/215; A61M 2205/3317; A61M 2205/3331; A61M 2205/3334; A61M 2205/3348; A61M 2205/3365; A61M 2205/3368; A61M 2205/3379; A61M 2205/3553; A61M 2205/3561; A61M 2205/3584; A61M 2205/3592; A61M 2205/3653; A61M 2205/42; A61M 2205/505; A61M 2205/52; A61M 2205/581; A61M 2205/582; A61M 2205/583; A61M 2205/7518; A61M 2205/8206; A61M 2207/00; A61M 2209/086; A61M 2209/088; A61M 2230/205; A61M 2230/40; A61M 2230/62; A61M 2230/63; B29C 63/42; B29L 2031/753

C-Sets
A61M 2202/0208, A61M 2202/0007;
A61M 2202/0225, A61M 2202/0085;
A61M 2230/205, A61M 2230/005;
A61M 2230/40, A61M 2230/005;
A61M 2230/62, A61M 2230/005;
A61M 2230/63, A61M 2230/005

## Description

1 BACKGROUND OF THE TECHNOLOGY

1.1 FIELD OF THE TECHNOLOGY

The present technology relates to one or more of the screening, diagnosis, monitoring, treatment, prevention and amelioration of respiratory-related disorders. The present technology also relates to medical devices or apparatus, and their use. In forms, the present technology relates to headgear conduit mask systems, treatment system, their component parts and their use.

1.2 DESCRIPTION OF THE RELATED ART

### 1.2.1 Human Respiratory System and its Disorders

The respiratory system of the body facilitates gas exchange. The nose and mouth form the entrance to the airways of a patient.

The nose typically includes two nostrils which provides nasal passages to facilitate entry of respiratory gas to the airways. One of the nostrils is substantially on a left side of the patient and leads to a left nasal passage. The other nostril is located substantially on a right side of the patient leads to a right nasal passage.

Each of the left and right nasal passages are lined with airway surface liquid in a mucociliary clearance/transport. The airway surface liquid has multiple functions including, but not limited to, trapping and removing inhaled particles, keeping the nasal passages hydrated and regulating the temperature of the inhaled respiratory gas.

If the airway surface liquid is dehydrated, it can cause dryness in the nose which may lead to discomfort while breathing and / or nosebleeds in a patient. Moreover, dehydration of the airway surface liquid also interferes with its function of intercepting particles, including pathogens, before they can reach lung tissue/airways.

During the process of breathing, each of the left nasal passage and the right nasal passage may alternately undergo partial congestion followed by decongestion. When the left nasal passage is partially congested, the right nasal passage may not experience congestion and vice versa.

This congestion / decongestion is not a result of physical and / or pathological blocking / unblocking of the nasal passages. The partial congestion of one of the two nasal passages is physiological and it occurs when the hypothalamus selectively activates one half of the autonomic nervous system.

Partial congestion of the left nasal passage and no congestion of the right nasal passage implies that inflow of respiratory gas through the right nasal passage is greater than that of the left nasal passage. After a period of time, the hypothalamus causes the autonomic nervous system to decongest the left nasal passage and partially congest the right nasal passage. Now the left nasal passage conducts a majority of the respiratory gas to the airways as compared to the right nasal passage.

This alternation of partial congestion between the left and right nasal passages is called 'nasal cycle'. It enables the airway surface liquid in the partially congested nasal passage to be replenished with moisture while the other uncongested nasal passage conducts the bulk of the respiratory gas to the airways. When the hypothalamus and the autonomic nervous system switch the partial congestion from one nasal passage to the other, the hydrated nasal passage predominantly facilitates flow of respiratory gas, thereby allowing the other, now congested, nasal passage to recuperate.

The airways include a series of branching tubes, which become narrower, shorter and more numerous as they penetrate deeper into the lung. The prime function of the lung is gas exchange, allowing oxygen to move from the inhaled air into the venous blood and carbon dioxide to move in the opposite direction. The trachea divides into right and left main bronchi, which further divide eventually into terminal bronchioles. The bronchi make up the conducting airways, and do not take part in gas exchange. Further divisions of the airways lead to the respiratory bronchioles, and eventually to the alveoli. The alveolated region of the lung is where the gas exchange takes place, and is referred to as the respiratory zone. See "Respiratory Physiology", by John B. West, Lippincott Williams & Wilkins, 9th edition published 2012.

The human body has natural, physiological processes which aim to protect the airways and ensure that they can continue to perform optimally. Nasal cycling is one of those processes in which there is alternating partial or complete congestion and decongestion of the two nasal passages. Congestion of the nasal conchae or turbinates is caused by selective activation of one half of the automatic nervous system by the hypothalamus. During natural nasal breathing, which is typically asymmetric, one nasal airway is responsible for airflow for a period of time before changing to the other nasal airway. The turbinates in one nasal passage fill up with blood while the turbinates in the other nasal passage decongests by moving blood away. The congested side will suspend cilia motility until it has decongested. The nasal cycle ensures that one nasal passage remains moist which facilitates humidification of the nose. See for instance "Why nasal airways experience drying during nasal-applied continuous positive airway pressure therapy" by D. White et al., Journal of Sleep Research, European Sleep Research Society, JSR 26 (Suppl. 1), first published on 24 October 2017.

A range of respiratory disorders exist. Certain disorders may be characterised by particular events, e.g. apneas, hypopneas, and hyperpneas.

Examples of respiratory disorders include Obstructive Sleep Apnea (OSA), Cheyne-Stokes Respiration (CSR), respiratory insufficiency, Obesity Hyperventilation Syndrome (OHS), Chronic Obstructive Pulmonary Disease (COPD), Neuromuscular Disease (NMD) and Chest wall disorders.

Obstructive Sleep Apnea (OSA), a form of Sleep Disordered Breathing (SDB), is characterised by events including occlusion or obstruction of the upper air passage during sleep. It results from a combination of an abnormally small upper airway and the normal loss of muscle tone in the region of the tongue, soft palate and posterior oropharyngeal wall during sleep. The condition causes the affected patient to stop breathing for periods typically of 30 to 120 seconds in duration, sometimes 200 to 300 times per night. It often causes excessive daytime somnolence, and it may cause cardiovascular disease and brain damage. The syndrome is a common disorder, particularly in middle aged overweight males, although a person affected may have no awareness of the problem. See US Patent No. 4,944,310 (Sullivan).

Cheyne-Stokes Respiration (CSR) is another form of sleep disordered breathing. CSR is a disorder of a patient's respiratory controller in which there are rhythmic alternating periods of waxing and waning ventilation known as CSR cycles. CSR is characterised by repetitive de-oxygenation and re-oxygenation of the arterial blood. It is possible that CSR is harmful because of the repetitive hypoxia. In some patients CSR is associated with repetitive arousal from sleep, which causes severe sleep disruption, increased sympathetic activity, and increased afterload. See US Patent No. 6,532,959 (Berthon-Jones).

Respiratory failure is an umbrella term for respiratory disorders in which the lungs are unable to inspire sufficient oxygen or exhale sufficient $CO_2$ to meet the patient's needs. Respiratory failure may encompass some or all of the following disorders.

A patient with respiratory insufficiency (a form of respiratory failure) may experience abnormal shortness of breath on exercise.

Obesity Hyperventilation Syndrome (OHS) is defined as the combination of severe obesity and awake chronic hypercapnia, in the absence of other known causes for hypoventilation. Symptoms include dyspnea, morning headache and excessive daytime sleepiness.

Chronic Obstructive Pulmonary Disease (COPD) encompasses any of a group of lower airway diseases that have certain characteristics in common. These include increased resistance to air movement, extended expiratory phase of respiration, and loss of the normal elasticity of the lung. Examples of COPD are emphysema and chronic bronchitis. COPD is caused by chronic tobacco smoking (primary risk factor), occupational exposures, air pollution and genetic factors. Symptoms include: dyspnea on exertion, chronic cough and sputum production.

Neuromuscular Disease (NMD) is a broad term that encompasses many diseases and ailments that impair the functioning of the muscles either directly via intrinsic muscle pathology, or indirectly via nerve pathology. Some NMD patients are characterised by progressive muscular impairment leading to loss of ambulation, being wheelchair-bound, swallowing difficulties, respiratory muscle weakness and, eventually, death from respiratory failure. Neuromuscular disorders can be divided into rapidly progressive and slowly progressive: (i) Rapidly progressive disorders: Characterised by muscle impairment that worsens over months and results in death within a few years (e.g. Amyotrophic lateral sclerosis (ALS) and Duchenne muscular dystrophy (DMD) in teenagers); (ii) Variable or slowly progressive disorders: Characterised by muscle impairment that worsens over years and only mildly reduces life expectancy (e.g. Limb girdle, Facioscapulohumeral and Myotonic muscular dystrophy). Symptoms of respiratory failure in NMD include: increasing generalised weakness, dysphagia, dyspnea on exertion and at rest, fatigue, sleepiness, morning headache, and difficulties with concentration and mood changes.

Chest wall disorders are a group of thoracic deformities that result in inefficient coupling between the respiratory muscles and the thoracic cage. The disorders are usually characterised by a restrictive defect and share the potential of long term hypercapnic respiratory failure. Scoliosis and/or kyphoscoliosis may cause severe respiratory failure. Symptoms of respiratory failure include: dyspnea on exertion, peripheral oedema, orthopnea, repeated chest infections, morning headaches, fatigue, poor sleep quality and loss of appetite.

A range of therapies have been used to treat or ameliorate such conditions. Furthermore, otherwise healthy individuals may take advantage of such therapies to prevent respiratory disorders from arising. However, these have a number of shortcomings.

### 1.2.2 Therapies

Various respiratory therapies, such as Continuous Positive Airway Pressure (CPAP) therapy, Non-invasive ventilation (NIV), Invasive ventilation (IV), and High Flow Therapy (HFT) have been used to treat one or more of the above respiratory disorders.

### 1.2.2.1 Respiratory pressure therapies

Respiratory pressure therapy is the application of a supply of air to an entrance to the airways at a controlled target pressure that is nominally positive with respect to atmosphere throughout the patient's breathing cycle (in contrast to negative pressure therapies such as the tank ventilator or cuirass).

Continuous Positive Airway Pressure (CPAP) therapy has been used to treat Obstructive Sleep Apnea (OSA). The mechanism of action is that continuous positive airway pressure acts as a pneumatic splint and may prevent upper airway occlusion, such as by pushing the soft palate and tongue forward and away from the posterior oropharyngeal wall. Treatment of OSA by CPAP therapy may be voluntary, and hence patients may elect not to comply with therapy if they find devices used to provide such therapy one or more of: uncomfortable, difficult to use, expensive and aesthetically unappealing.

Non-invasive ventilation (NIV) provides ventilatory support to a patient through the upper airways to assist the patient breathing and/or maintain adequate oxygen levels in the body by doing some or all of the work of breathing. The ventilatory support is provided via a non-invasive patient interface. NIV has been used to treat CSR and respiratory failure, in forms such as OHS, COPD, NMD and Chest Wall disorders. In some forms, the comfort and effectiveness of these therapies may be improved.

Invasive ventilation (IV) provides ventilatory support to patients that are no longer able to effectively breathe themselves and may be provided using a tracheostomy tube. In some forms, the comfort and effectiveness of these therapies may be improved.

### 1.2.2.2 Flow therapies

Not all respiratory therapies aim to deliver a prescribed therapeutic pressure. Some respiratory therapies aim to deliver a prescribed respiratory volume, by delivering an inspiratory flow rate profile over a targeted duration, possibly superimposed on a positive baseline pressure. In other cases, the interface to the patient's airways is 'open' (unsealed) and the respiratory therapy may only supplement the patient's own spontaneous breathing with a flow of conditioned or enriched gas. In one example, High Flow therapy (HFT) is the provision of a continuous, heated, humidified flow of air to an entrance to the airway through an unsealed or open patient interface at a "treatment flow rate" that is held approximately constant throughout the respiratory cycle. The treatment flow rate is nominally set to exceed the patient's peak inspiratory flow rate. HFT has been used to treat OSA, CSR, respiratory failure, COPD, and other respiratory disorders. One mechanism of action is that the high flow rate of air at the airway entrance improves ventilation efficiency by flushing, or washing out, expired $CO_2$ from the patient's anatomical deadspace. Hence, HFT is thus sometimes referred to as a deadspace therapy (DST). Other benefits may include the elevated warmth and humidification (possibly of benefit in secretion management) and the potential for modest elevation of airway pressures. As an alternative to constant flow rate, the treatment flow rate may follow a profile that varies over the respiratory cycle.

Another form of flow therapy is long-term oxygen therapy (LTOT) or supplemental oxygen therapy. Doctors may prescribe a continuous flow of oxygen enriched gas at a specified oxygen concentration (from 21%, the oxygen fraction in ambient air, to 100%) at a specified flow rate (e.g., 1 litre per minute (LPM), 2 LPM, 3 LPM, etc.) to be delivered to the patient's airway.

### 1.2.2.3 Supplementary oxygen

For certain patients, oxygen therapy may be combined with a respiratory pressure therapy or HFT by adding supplementary oxygen to the pressurised flow of air. When oxygen is added to respiratory pressure therapy, this is referred to as RPT with supplementary oxygen. When oxygen is added to HFT, the resulting therapy is referred to as HFT with supplementary oxygen.

### 1.2.3 Respiratory Treatment Systems

These respiratory therapies may be provided by a respiratory treatment system or device. Such systems and devices may also be used to screen, diagnose, or monitor a condition without treating it.

A treatment respiratory therapy system may comprise a Respiratory Pressure Therapy Device (RPT device), an air circuit, a humidifier, a patient interface, an oxygen source, and data management.

Another form of treatment therapy system is a mandibular repositioning device.

### 1.2.3.1 Patient Interface

A patient interface may be used to interface respiratory equipment to its wearer, for example by providing a flow of air to an entrance to the airways. The flow of air may be provided via a mask to one or more of the patient's airways, e.g. the nose and/or mouth, a tube to the mouth or a tracheostomy tube to the trachea of a patient. Depending upon the therapy to be applied, the patient interface may form a seal, e.g., with a region of the patient's face, to facilitate the delivery of gas at a pressure at sufficient variance with ambient pressure to effect therapy, e.g., at a positive pressure of about 10 $cmH_2O$ relative to ambient pressure. For other forms of therapy, such as the delivery of oxygen, the patient interface may not include a seal sufficient to facilitate delivery to the airways of a supply of gas at a positive pressure of about 10 $cmH_2O$. For flow therapies such as nasal HFT, the patient interface is configured to insufflate the nares but specifically to avoid a complete seal. One example of such a patient interface is a nasal cannula.

Certain other mask systems may be functionally unsuitable for the present field. For example, purely ornamental masks may be unable to maintain a suitable pressure. Mask systems used for underwater swimming or diving may be configured to guard against ingress of water from an external higher pressure, but not to maintain air internally at a higher pressure than ambient.

Certain masks may be clinically unfavourable for the present technology e.g. if they block airflow via the nose and only allow it via the mouth.

Certain masks may be uncomfortable or impractical for the present technology if they require a patient to insert a portion of a mask structure in their mouth to create and maintain a seal via their lips.

Certain masks may be impractical for use while sleeping, e.g. for sleeping while lying on one's side in bed with a head on a pillow.

The design of a patient interface presents a number of challenges. The face has a complex three-dimensional shape. The size and shape of noses and heads varies considerably between individuals. Since the head includes bone, cartilage and soft tissue, different regions of the face respond differently to mechanical forces. The jaw or mandible may move relative to other bones of the skull. The whole head may move during the course of a period of respiratory therapy.

As a consequence of these challenges, some masks suffer from being one or more of obtrusive, aesthetically undesirable, costly, poorly fitting, difficult to use, and uncomfortable especially when worn for long periods of time or when a patient is unfamiliar with a system. Wrongly sized masks can give rise to reduced compliance, reduced comfort and poorer patient outcomes. Masks designed solely for aviators, masks designed as part of personal protection equipment (e.g. filter masks), SCUBA masks, or for the administration of anaesthetics may be tolerable for their original application, but nevertheless such masks may be undesirably uncomfortable to be worn for extended periods of time, e.g., several hours. This discomfort may lead to a reduction in patient compliance with therapy. This is even more so if the mask is to be worn during sleep.

Respiratory therapy, such as nasal CPAP therapy or high-flow therapy, may reduce or prevent natural nasal cycling and its benefits. For instance, nasal CPAP therapy forces air which is pressurised above ambient into nasal flow paths of a mask and into the nares of the patient, which reduces the effectiveness of natural nasal cycling. As a result, nasal CPAP mask users commonly experience symptoms associated with dryness of the nasal passage. These symptoms reduce therapy compliance. The most common approach to relieving these symptoms is supplementary humidification; see for example "Why nasal airways experience drying during nasal-applied continuous positive airway pressure therapy" by D. White et al., Journal of Sleep Research, European Sleep Research Society, JSR 26 (Suppl. 1), first published on 24 October 2017. However, humidifying respiratory gases introduces additional challenges and can be difficult to implement, and requires additional components.

CPAP therapy is highly effective to treat certain respiratory disorders, provided patients comply with therapy. If a mask is uncomfortable, or use of a mask causes discomfort, e.g. dryness of nasal passages, or a mask is difficult to use, a patient may not comply with therapy. Since it is often recommended that a patient regularly wash their mask, if a mask is difficult to clean (e.g., difficult to assemble or disassemble), patients may not clean their mask and this may impact on patient compliance.

While a mask for other applications (e.g. aviators) may not be suitable for use in treating sleep disordered breathing, a mask designed for use in treating sleep disordered breathing may be suitable for other applications.

For these reasons, patient interfaces for delivery of CPAP during sleep form a distinct field.

#### *1.2.3.1.1 Seal-forming structure*

Patient interfaces may include a seal-forming structure. Since it is in direct contact with the patient's face, the shape and configuration of the seal-forming structure can have a direct impact the effectiveness and comfort of the patient interface.

A patient interface may be partly characterised according to the design intent of where the seal-forming structure is to engage with the face in use. In one form of patient interface, a seal-forming structure may comprise a first sub-portion to form a seal around the left naris and a second sub-portion to form a seal around the right naris. In one form of patient interface, a seal-forming structure may comprise a single element that surrounds the nares in use. Such single element may be designed to for example overlay an upper lip region and a nasal bridge region of a face. In one form of patient interface a seal-forming structure may comprise an element that surrounds a mouth region in use, e.g. by forming a seal on a lower lip region of a face. In one form of patient interface, a seal-forming structure may comprise a single element that surrounds the nares and a mouth region in use. These different types of patient interfaces may be known by a variety of names by their manufacturer including nasal masks, full-face masks, nasal pillows, nasal puffs and oro-nasal masks.

A seal-forming structure that may be effective in one region of a patient's face may be inappropriate in another region, e.g. because of the different shape, structure, variability and sensitivity regions of the patient's face. For example, a seal on swimming goggles that overlays a patient's forehead may not be appropriate to use on a patient's nose.

Certain seal-forming structures may be designed for mass manufacture such that one design fit and be comfortable and effective for a wide range of different face shapes and sizes. To the extent to which there is a mismatch between the shape of the patient's face, and the seal-forming structure of the mass-manufactured patient interface, one or both must adapt in order for a seal to form.

One type of seal-forming structure extends around the periphery of the patient interface, and is intended to seal against the patient's face when force is applied to the patient interface with the seal-forming structure in confronting engagement with the patient's face. The seal-forming structure may include an air or fluid filled cushion, or a moulded or formed surface of a resilient seal element made of an elastomer such as a rubber. With this type of seal-forming structure, if the fit is not adequate, there will be gaps between the seal-forming structure and the face, and additional force will be required to force the patient interface against the face in order to achieve a seal.

Another type of seal-forming structure incorporates a flap seal of thin material positioned about the periphery of the mask so as to provide a self-sealing action against the face of the patient when positive pressure is applied within the mask. Like the previous style of seal forming portion, if the match between the face and the mask is not good, additional force may be required to achieve a seal, or the mask may leak. Furthermore, if the shape of the seal-forming structure does not match that of the patient, it may crease or buckle in use, giving rise to leaks.

Another type of seal-forming structure may comprise a friction-fit element, e.g. for insertion into a naris, however some patients find these uncomfortable.

Another form of seal-forming structure may use adhesive to achieve a seal. Some patients may find it inconvenient to constantly apply and remove an adhesive to their face.

A range of patient interface seal-forming structure technologies are disclosed in the following patent applications, assigned to ResMed Limited: WO 1998/004,310; WO 2006/074,513; WO 2010/135,785.

One form of nasal pillow is found in the Adam Circuit manufactured by Puritan Bennett. Another nasal pillow, or nasal puff is the subject of US Patent 4,782,832 (Trimble et al.), assigned to Puritan-Bennett Corporation.

ResMed Limited has manufactured the following products that incorporate nasal pillows: SWIFTTM nasal pillows mask, SWIFTTM II nasal pillows mask, SWIFTTM LT nasal pillows mask, SWIFTTM FX nasal pillows mask and MIRAGE LIBERTYTM full-face mask. The following patent applications, assigned to ResMed Limited, describe examples of nasal pillows masks: International Patent Application WO2004/073,778 (describing amongst other things aspects of the ResMed Limited SWIFTTM nasal pillows), US Patent Application 2009/0044808 (describing amongst other things aspects of the ResMed Limited SWIFTTM LT nasal pillows); International Patent Applications WO 2005/063,328 and WO 2006/130,903 (describing amongst other things aspects of the ResMed Limited MIRAGE LIBERTYTM full-face mask); International Patent Application WO 2009/052,560 (describing amongst other things aspects of the ResMed Limited SWIFTTM FX nasal pillows).

### *1.2.3.1.2 Positioning and stabilising*

A seal-forming structure of a patient interface used for positive air pressure therapy is subject to the corresponding force of the air pressure to disrupt a seal. Thus a variety of techniques have been used to position the seal-forming structure, and to maintain it in sealing relation with the appropriate portion of the face.

One technique is the use of adhesives. See for example US Patent Application Publication No. US 2010/0000534. However, the use of adhesives may be uncomfortable for some.

Another technique is the use of one or more straps and/or stabilising harnesses. Many such harnesses suffer from being one or more of ill-fitting, bulky, uncomfortable and awkward to use.

The straps and/or stabilising harnesses may form a positioning and stabilising structure that secures the seal-forming structure against one or more portions of a patient's face.

Another technique is to use conduits which deliver a supply of breathable gas to the patient interface as part of the positioning and stabilising structure i.e. a conduit headgear. In these arrangements, the headgear performs two functions, being to hold the seal-forming structure in a position relative to the patient's airways, and to convey the breathable gas from the air circuit to the patient interface.

It is common for patients to sleep on their sides e.g. either their left or right hand side. When they do so, they apply pressure to the conduits in a conduit headgear treatment system. Many conduits have rigid reinforcing structures which prevent them collapsing under the pressure of the patient's weight. However, the rigid nature of the reinforcing structures can cause patient discomfort due to rigid reinforcing structure pressing into the patient's head and / or face.

It is therefore known to provide flexible conduits that can completely of partially collapse under a patient's weight. This reduces patient discomfort. However, the tendency to collapse causes complete or partial occlusions in the conduit(s), which reduces the total available flow area. The reduction in flow area creates a significant impedance in the respiratory treatment system. That impedance can be a significant limitation on effective provision of respiratory therapy and treating respiratory disorders.

Some existing conduits, such as those made from silicone, are prone to collapse and can become occluded when compressed by a patient's head while side sleeping. This can partially or completely obstruct gas flow through the conduit affecting therapy efficacy. To overcome this, the conduits may be stiffened or rigidised to limit or prevent this collapse. However, this may increase the size and weight of the conduit. This may also compromise the flexibility of the conduit because of the stiffening or rigidisation. Furthermore, this may increase turbulence and noise in the conduit. These are not desirable from a patient comfort and fit perspective and can result in reduced respiratory therapy compliance by a patient. In addition, such conduits may require more material for manufacture which may increase manufacturing costs.

Occlusion of the conduits can also create other issues such as jetting and inconsistent air flow. This can further add to patient discomfort and reduce compliance with therapy.

### 1.2.3.2 Respiratory Pressure Therapy (RPT) Device

A respiratory pressure therapy (RPT) device may be used individually or as part of a system to deliver one or more of a number of therapies described above, such as by operating the device to generate a flow of respiratory gas for delivery to an interface to the airways. The flow of air may be pressure-controlled (for respiratory pressure therapies) or flow-controlled (for flow therapies such as HFT). Thus, RPT devices may also act as flow therapy devices. Examples of RPT devices include a CPAP device and a ventilator.

### 1.2.3.3 Gas or air circuit

A gas or air circuit is a conduit or a tube constructed and arranged to allow, in use, a flow of air to travel between two components of a respiratory treatment system such as the RPT device and the patient interface. In some cases, there may be separate limbs of the air circuit for inhalation and exhalation. In other cases, a single limb air circuit is used for both inhalation and exhalation.

The conduits forming the breathable gas or air circuit may be removably or permanently connected to the patient interface. However, the conduits themselves, or the attachment components, can be bulky or obtrusive, and can affect comfort or seal. In addition, they can restrict patient movement which further decreases patient comfort.

It is also known to adjust the properties of a flow of respiratory gas to customise the properties of the gas delivered to each of the nares i.e. to provide different properties to each of the nares. For instance, two discrete flows of gas may be created which are each delivered to a respective one of the patient's nares, and the humidity, flow rate, temperate or other property of each flow of gas may be changed independently of the other flow.

Such a respiratory system is described in PCT/AU2009/000671. Parameters like the flow rate, pressure, temperature and humidity of the respiratory gas delivered to each of the patient's nares can be adjusted to optimise patient comfort or therapy efficacy.

However, systems which control parameters of flow to the nares of the patient may have a range of failings. For instance, they may be cumbersome, expensive, energy inefficient, complex or require a large number of components.

Therefore, there is a need for a simple solution which may resolves flow of breathable gas in a mask system comprising conduit headgear.

### 1.2.3.4 Humidifier

Delivery of a flow of air without humidification may cause drying of airways and / or the airway surface liquid. The use of a humidifier with an RPT device and the patient interface produces humidified gas that may minimize drying of the nasal mucosa and increase patient airway comfort.

However, supplementary humidification to treat dryness of nasal passages is not a perfect solution in many situations or many patients.

The drying of airway surface liquid is particularly acute in case of nasal CPAP therapy, although it may occur in other artificial respiratory systems as well.

Therefore, there is a need for a simple and/or an improved solution that prevents dehydration of airway surface liquid, particularly in artificial respiratory systems.

### 1.2.3.5 Vent technologies

Some forms of treatment systems may include a vent to allow the washout of exhaled carbon dioxide. The vent may allow a flow of gas from an interior space of a patient interface, e.g., the plenum chamber, to an exterior of the patient interface, e.g., to ambient.

The vent may comprise an orifice and gas may flow through the orifice in use of the mask. Many such vents are noisy. Others may become blocked in use and thus provide insufficient washout. Some vents may be disruptive of the sleep of a bed partner of the patient, e.g. through noise or focussed airflow.

US 2015/367092 A1 relates to positive airway pressure (PAP) systems and methods with various mechanisms for altering the air pressure based in part on the head position of the user. This can be achieved actively or passively. Passively, pressure is altered when head position is altered, as gravity acts to open or close venting elements.

WO 2018/065867 A1 relates to a conduit for conducting a flow of breathing gas from a pressure generating device to a cushion of a patient interface device that includes a housing that defines a passage therethrough. The conduit further includes a first magnetic element disposed on a first side of the passage and a second magnetic element disposed on a second side of the passage opposite the first side. The first and second magnetic elements are positioned such that the magnetic fields produced by each magnetic element interact in a manner such that the first magnetic element and the second magnetic element are repelled away from each other in a manner which resists collapse of the passage.

WO 2012/107858 A1 relates to a patient interface device that includes a mask including a interface element structured to engage a portion of the face of a patient and a headgear assembly coupled to the mask. The headgear assembly has a top surface member and a bottom surface member, the top surface member being coupled to the bottom surface member to form a fluid delivery housing having an airtight internal chamber. The fluid delivery housing is structured to receive a flow of breathing gas from a pressure generating device and is fluidly coupled to the interface element. Also, the headgear assembly has a plurality of spaced support members provided within the internal chamber to define a plurality of flow paths through the internal chamber around the support members, the support members being structured to support the top surface member relative to bottom surface member and prevent the internal chamber from collapsing.

EP 2 964 300 A1 relates to a respiratory interface device that includes a manifold assembly including a manifold body and a rotational coupling. The manifold body includes an elastic portion. The rotational coupling includes a rigid conduit. The rigid conduit is rotatably coupled to the manifold body elastic portion. The rotational coupling and the elastic portion decouple the remaining portions of the manifold body from external forces applied to the rigid conduit.

## 2 SUMMARY

### 2.1 SUMMARY OF THE INVENTION

The present invention provides a positioning and stabilizing structure as defined in claim 1. Further preferred embodiments of the present invention are defined in the dependent claims.

### 2.2 SUMMARY OF THE TECHNOLOGY

Reference will be made in the following description to various examples or aspects of the present technology. Examples or aspects disclosed herein which do not fall under the scope of the appended claims do not form part of the present invention, but are useful for understanding the principles of the invention. The scope of the present invention is defined by the appended claims.

The present technology is directed towards providing medical devices used in the treatment or prevention of respiratory disorders having one or more of improved comfort, cost, efficacy, ease of use and manufacturability.

A first aspect of the present technology relates to apparatus used in the treatment or prevention of a respiratory disorder.

Another aspect of the present technology relates to methods used in the treatment or prevention of a respiratory disorder.

An aspect of certain forms of the present technology is to provide methods and/or apparatus that improve the compliance of patients with respiratory therapy.

Another aspect of certain forms of the present technology is to reduce impedance to flow in a respiratory treatment system.

Another aspect of certain forms of the present technology is to reduce jetting in the headgear tube(s) of a positioning and stabilizing structure.

Another aspect of certain forms of the present technology is to provide a respiratory treatment system and components therefor that improve patient compliance during respiratory therapy.

Another aspect of certain forms of the present technology is to provide a treatment system including conduit headgear which may be one or more of less cumbersome, less obtrusive and less unsightly.

Another aspect of certain forms of the present technology is to provide a respiratory treatment system and components therefor which are easy to use, e.g. by a person who does not have medical training, by a person who has limited dexterity, vision or by a person with limited experience in using this type of medical device.

An aspect of one form of the present technology is a respiratory system and components therefor that may be washed in a home of a patient, e.g., in soapy water, without requiring specialised cleaning equipment.

Another aspect of certain forms of the present technology is to provide a respiratory treatment system and components therefor, and a method of treating or ameliorating a respiratory disorder, which may reduce dryness and irritation of nasal airways.

Another aspect of certain forms of the present technology relates to a method and / or respiratory treatment system and components therefor, wherein the respiratory treatment system comprises a positioning and stabilising system having a first headgear tube and a second headgear tube, which minimises the effect of occlusion of the first headgear tube on impedance in the treatment system.

Another aspect of certain forms of the present technology relates to a method and / or respiratory treatment system and components therefor, wherein the respiratory treatment system comprises a positioning and stabilising system having a first headgear tube and a second headgear tube, wherein the system is configured to direct a substantial portion of a flow of respiratory gas into the second headgear tube when the first headgear tube is partially or completed occluded.

Another aspect of certain forms of the present technology relates to a method and / or respiratory treatment system and components therefor which mimic natural native cycling.

Another aspect of one form of the present technology relates to a flow regulator.

Another aspect of one form of the present technology relates to a respiratory therapy treatment system having a flow regulator as described herein.

Another aspect of one form of the present technology relates to a respiratory treatment system comprising:

a patient interface configured to interface with one or more of the patient's airways; and

a positioning and stabilizing structure as described herein.

Another aspect of one form of the present technology is a flow regulator, wherein the flow regulator is configured for use with a respiratory therapy treatment system having at least a first headgear tube and a second headgear tube, wherein the flow regulator is configured to adjust the proportion of a flow of respiratory gas flowing into the first headgear tube and the second headgear tube.

Another aspect of one form the present technology comprises a positioning and stabilizing structure including one or more headgear tubes, wherein the positioning and stabilizing structure has a flow regulator configured to adjust the proportion of the flow of respiratory gas flowing into the one or more headgear tubes.

Another aspect of certain forms of the present technology is to provide a respiratory treatment system and components therefor to proportion a flow of respiratory gas into a relatively greater flow portion and a lesser flow portion, and to deliver the relatively greater flow portion to a patient's first nasal airway for a first period of time and then subsequently to deliver the relatively greater flow portion to the patient's second nasal airway.

Another aspect of certain forms of the present technology relates to a method of providing respiratory therapy to a patient, the method including the steps of:

proportioning a flow of respiratory gas into a first, relatively greater flow portion and a second, relatively less flow portion;

directing the first, relatively greater portion into a first pathway, and a second, relatively less flow portion and directing the second relatively less flow portion into a second flow pathway; and

subsequently conveying the first, relatively greater flow portion to the second flow path and the second relatively less flow portion into the first flow path.

Another aspect of one form of the present technology relates to a positioning and stabilizing structure comprising:

a first headgear tube and a second headgear tube,

wherein the positioning and stabilizing structure is configured in use to maintain a patient interface in a position relative to a patient's airway(s), and

a flow regulator configured to adjust the proportion of the flow of respiratory gas flowing into the first headgear tube and the second headgear tube.

According to an aspect of the present technology, the patient interface may be configured to convey the respiratory gas to one or more of the patient's nasal airways and the patient's mouth.
According to an aspect of the present technology, the patient interface may include a sealing component which is configured to sealingly engage at least a portion of the patient's face.
According to an aspect of the present technology, the sealing component may be configured to seal around at least one of the patient's nasal airways and the patient's mouth.
According to an aspect of the present technology, the sealing component may be a cushion.
According to an aspect of the present technology, the cushion may include a full-face cushion.
According to an aspect of the present technology, the cushion may comprise one or more nasal pillow or nasal prong.
According to an aspect of the present technology, the first headgear tube comprises a first connector configured to connect to a patient interface and the second headgear tube comprises a second connector configured to connect to a patient interface.
According to an aspect of the present technology, the flow regulator may be configured to provide the first flow portion to the first headgear tube for a first period of time and then subsequently to provide the first flow portion to the second headgear tube for a period of time.
According to an aspect of the present technology, the first headgear tube may be located substantially on the first side of the patient, and the second headgear tube may be located substantially on the second side of the patient.
According to an aspect of the present technology, the first headgear tube may be configured to lie against a first cheek region of the patient, and the second headgear tube may be configured to lie against a second cheek region of the patient.
According to an aspect of the present technology, the first headgear tube may be configured to extend above a transverse plane passing through a first ear of the patient and the second headgear tube may be configured to extend above a transverse plane passing through of a second ear of the patient.
According to an aspect of the present technology, the first headgear tube and the second headgear tube may be configured to be connected to each other at the top of the patient's head.
According to an aspect of the present technology, the flow of respiratory gas may be substantially or completely directed to the first headgear tube if/when the second headgear tube is partially or completely occluded and the flow of respiratory gas may be substantially or completely directed to the second headgear tube if/when the first headgear tube is partially or completely occluded.
According to an aspect of the present technology, the flow of respiratory gas may be substantially or completely directed to the first headgear tube if/when the patient's head is tilted substantially to the left of the sagittal plane and the flow of respiratory gas may be predominantly or completely directed to the second headgear tube if/when the patient's head is tilted substantially to the right of the sagittal plane.
According to an aspect of the present technology, the ratio of the first flow portion and the second flow portion may depend on the angle at which the patient's head is tilted with respect to the sagittal plane.
According to an aspect of the present technology, one or more of the first headgear tube and the second headgear tube may be partially or completely compressible.
According to an aspect of the present technology, the flow regulator may be configured to be connected to the first headgear tube and the second headgear tube.
According to an aspect of the present technology, the flow regulator may include a barrier arrangement configured to adjust the proportion of the flow of respiratory gas flowing into the first headgear tube and the second headgear tube.
According to an aspect of the present technology, the barrier arrangement may be movable between a first configuration and a second configuration. When in the first configuration, the barrier arrangement directs the first flow portion to the first headgear tube. When in the second configuration, the barrier arrangement may be configured to direct the first flow portion to the second headgear tube.
According to an aspect of the present technology, the barrier arrangement may be configured to direct the second flow portion to the second headgear tube when in the first configuration.
According to an aspect of the present technology, the barrier arrangement is configured to direct the second flow portion to the first headgear tube when in the second configuration.
According to an aspect of the present technology, the barrier arrangement may substantially or completely prevent the flow of respiratory gas flowing into the second headgear tube when the barrier arrangement is in the first configuration.
According to an aspect of the present technology, the barrier arrangement may substantially or completely prevent the flow of respiratory gas flowing into the first headgear tube when the barrier arrangement is in the second configuration.

According to an aspect of the present technology, the barrier arrangement may be configured to be moved between the first configuration and the second configuration by gravity.

According to another aspect of the present technology, the positioning and stabilizing structure may include an actuator configured to move the barrier arrangement between the first configuration and the second configuration.

According to an aspect of the present technology, the actuator may include at least one of, a servomotor, a spring actuator, an electric actuator, a magnetic actuator, a pneumatic actuator and a hydraulic actuator.

According to another aspect of the present technology, the barrier arrangement may be moved by a combination of gravity and the actuator.

According to an aspect of the present technology, the barrier arrangement may be a piston or a spherically shaped ball component.

According to an aspect of the present technology, when in the first configuration the barrier arrangement completely or substantially blocks the second outlet.

According to an aspect of the present technology, when in the second configuration the barrier arrangement may completely or substantially block the first outlet.

According to an aspect of the present technology, when in the first configuration the first outlet may be substantially or completely open e.g. unblocked.

According to an aspect of the present technology, when in the second configuration the second outlet may be substantially or completely open e.g. unblocked.

According to an aspect of the present technology, the positioning and stabilizing structure includes a flow controller configured to engage the actuator to move the barrier arrangement between the first configuration and the second configuration according to the signal.

According to an aspect of the present technology, the positioning and stabilizing structure may include a sensor configured to in use determine a property related to one or more of an orientation of the patient's head and movement of the patient's head.

According to an aspect of the present technology, the sensor may be configured to send a signal to the flow controller indicative of the property related to the orientation of the patient's head and movement of the patient's head.

According to an aspect of the present technology, the flow regulator may comprise a body having an inlet, a first outlet and a second outlet.

According to an aspect of the present technology, the first outlet may be configured to attach to the first headgear tube and the second headgear tube is configured to attach to the second headgear tube.

According to an aspect of the present technology the connector may be an elbow.

According to an aspect of the present technology, the flow regulator further comprises a decoupling component between the inlet and a / the duct.

According to a preferred aspect of the present technology, the duct may be configured to rotate and/or swivel with respect to the flow regulator.

According to an aspect of the present technology, the positioning and stabilizing structure may include one or more vents which is/are configured to expel gases exhaled by the patient. The vent(s) may be configured to prevent rebreathing exhaled gases which may be pressured to enter the first headgear tube and/or the second headgear tube.

According to an aspect of the present technology, the vent(s) may be provided on the patient interface.

According to an aspect of the present technology, the respiratory treatment system may include a flow generator.

According to an aspect of the present technology, the duct is configured to convey the flow of respiratory gas from the flow generator to the positioning and stabilizing structure.

According to an aspect of the present technology, the positioning and stabilizing structure includes one or more headgear straps.

According to an aspect of the present technology, the positioning and stabilizing structure includes at least one rear strap configured to be located at a substantially rear portion of the patient's head.

Another aspect of form of the present technology relates to a patient interface system having a first headgear tube and a second headgear tube, wherein the treatment system minimises the effect of occlusion of the first headgear tube on impedance in the treatment system.

According to an aspect of the present technology relates to a patient interface system which comprises a positioning and stabilising system having a first headgear tube and a second headgear tube, wherein the respiratory treatment system is configured to direct a substantial portion of a flow of respiratory gas into the second headgear tube when the first headgear tube is partially or completed occluded.

According to an aspect of the present technology, the patient interface system may be configured to proportion a flow of respiratory gas into a relatively greater flow portion and a relatively lesser flow portion, and to direct the relatively greater flow portion to a patient's first nasal airway for a first period of time and then subsequently to direct the relatively greater flow portion to the patient's second nasal airway.

Another aspect of one form of the present technology relates to a method of providing respiratory therapy to a patient, the method including the steps of:

creating a flow of respiratory gas;

directing a relatively greater portion of the flow into a first headgear tube and a relatively lesser portion of the flow into a second headgear tube for a first period of time; and

directing a relatively greater portion of the flow into the second headgear tube and a relatively lesser portion of the flow into the first headgear tube for a second period of time. According to an aspect of the present technology

According to another aspect of the present technology, the respiratory therapy treatment system includes at least a first headgear tube and a second headgear tube, wherein the flow regulator is configured to in use receive a flow of respiratory gas and to adjust the proportion of a flow of respiratory gas which flows from the flow regulator into the first headgear tube and the second headgear tube.

According to another aspect of the present technology, the flow regulator is configured to direct a relatively greater portion of the flow of respiratory gas into the first headgear tube and a relatively less portion of the flow of respiratory gas into the second headgear tube.

According to another aspect of the present technology, the flow regulator is configured to direct a relatively greater portion of the flow of respiratory gas to the second headgear tube and a relatively lesser portion of the flow of respiratory gas to the first headgear tube.

According to another aspect of the present technology, the relatively greater portion of the flow of respiratory is all, or a substantial portion of, the flow.

According to another aspect of the present technology, the flow regulator is configured to, in use, direct all, or a substantial portion of, the flow of respiratory gas to the first headgear tube if/when the patient's head is tilted substantially to a first side.

According to another aspect of the present technology, the flow regulator is configured to, in use, direct all, or a substantial portion of, the flow of respiratory gas to the second headgear tube if/when the patient's head is tilted to a second side.

According to another aspect of the present technology, wherein the ratio of the portion of the flow directed to first headgear tube and the second headgear tube is dependent on the orientation of the patient's head.

According to another aspect of the present technology, wherein the flow regulator comprises a body which defines a chamber.

According to another aspect of the present technology, the chamber includes an inlet configured to facilitate a flow of respiratory gas flowing into the chamber.

According to another aspect of the present technology, the flow regulator includes an inlet connector that is configured to in use connect a duct to the inlet.

According to another aspect of the present technology, the inlet connector is an elbow.

According to another aspect of the present technology, wherein the flow regulator further comprises a decoupling component between the inlet and a / the duct.

According to another aspect of the present technology, wherein the chamber includes a first outlet configured to in use be in flow communication with the first headgear tube and a second outlet configured to in use be in flow communication with the second headgear tube.

According to another aspect of the present technology, the flow regulator includes a first outlet connector configured to facilitate connecting the flow regulator to the first headgear tube.

According to another aspect of the present technology, wherein the flow regulator includes a second outlet connector configured to facilitate connecting the flow regulator to the second headgear tube.

According to another aspect of the present technology, the flow regulator includes a barrier arrangement which is moveable between a first configuration and a second configuration.

According to another aspect of the present technology, when in the first configuration the barrier arrangement completely or substantially blocks the second outlet.

According to another aspect of the present technology, wherein the barrier arrangement substantially or completely prevents the flow of respiratory gas flowing into the second headgear tube when the barrier arrangement is in the first configuration.

According to another aspect of the present technology, wherein when in the first configuration the first outlet is substantially or completely open.

According to another aspect of the present technology, wherein when in the second configuration the barrier arrangement completely or substantially blocks the first outlet.

According to another aspect of the present technology, the barrier arrangement substantially or completely prevents the flow of respiratory gas flowing into the first headgear tube when the barrier arrangement is in the second configuration.

According to another aspect of the present technology, when in the second configuration the second outlet is substantially or completely open.

According to another aspect of the present technology, the barrier arrangement is a piston or a spherically shaped ball component.

According to another aspect of the present technology, the barrier arrangement is provided in a / the chamber.

According to another aspect of the present technology, wherein the barrier arrangement is configured to be moved between the first configuration and the second configuration by gravity.

According to another aspect of the present technology, including an actuator configured to move the barrier arrangement between the first configuration and the second configuration.

Another aspect of certain forms of the present technology relates to a positioning and stabilizing structure, comprising:

a first headgear tube and a second headgear tube,

wherein the positioning and stabilizing structure is configured in use to maintain a patient interface in a position relative to a patient's airway(s), and

a flow regulator as described herein.

According to another aspect of the present technology, the first headgear tube comprises a first connector configured to connect to the patient interface and the second headgear tube comprises a second connector configured to connect to the patient interface.

According to another aspect of the present technology, the first headgear tube is configured to in use lie against a first cheek region of the patient, and the second headgear tube is configured to in use lie against a second cheek region of the patient.

According to another aspect of the present technology, one or more of the first headgear tube and the second headgear tube may be partially or completely compressible.

Another aspect of certain forms of the present technology relates to a respiratory treatment system, including

the positioning and stabilising structure as described herein, and

a patient interface.

According to another aspect of the present technology, the patient interface is configured to convey the respiratory gas to one or more of the patient's nasal airways and the patient's mouth.

According to another aspect of the present technology, the patient interface includes a sealing component which is configured to sealingly engage at least a portion of the patient's face.

According to another aspect of the present technology, the sealing component is configured to seal around at least one of the patient's nasal airways and the patient's mouth.

According to another aspect of the present technology, the sealing component is a cushion.

According to another aspect of the present technology, the cushion is a full-face cushion.

According to another aspect of the present technology, the cushion includes one or more nasal pillow or nasal prong.

Another aspect of certain forms of the present technology relates to a flow regulator, which includes

a body which defines a chamber,

wherein the chamber includes an inlet configured to in use facilitate a flow of respiratory gas flowing into the chamber, a first outlet and a second outlet,

a barrier arrangement which is moveable between a first configuration and a second configuration, wherein

in the first configuration the barrier arrangement substantially or completely blocks the first outlet, and

further wherein in the second configuration the barrier arrangement substantially or completely blocks the second outlet.

According to another aspect of the present technology, the flow regulator includes a first outlet connector configured to facilitate attaching the flow regulator to a first headgear tube.
According to another aspect of the present technology, the flow regulator includes a second outlet connector configured to facilitate attaching the flow regulator to a second headgear tube.
According to another aspect of the present technology, in the first configuration the first outlet has a first outlet flow area and the inlet has an inlet flow area, and further wherein in the first configuration the first outlet flow area is substantially equal to the inlet flow area.
According to another aspect of the present technology, when in the first configuration the second outlet has a second outlet flow area that is substantially zero.
According to another aspect of the present technology, when in the second configuration the first outlet has a first outlet flow area that is substantially zero.
According to another aspect of the present technology, the barrier arrangement comprises at least one barrier element.
According to another aspect of the present technology, the at least one barrier element comprises a piston or a spherical ball component in the chamber.
Another aspect of certain forms of the present technology relates to a flow regulator, which includes

a body which defines a chamber,

wherein the chamber includes an inlet configured to in use facilitate a flow of respiratory gas flowing into the chamber, a first outlet and a second outlet,

a barrier arrangement which is configured to be moved by gravity between a first configuration and a second configuration.

Another aspect of certain forms of the present technology relates to a positioning and stabilizing structure, comprising:

a first headgear tube and a second headgear tube,

wherein the positioning and stabilizing structure is configured in use to maintain a patient interface in a position relative to a patient's airway(s), and

the flow regulator as described herein.

Another aspect of certain forms of the present technology relates to a respiratory treatment system, including

a positioning and stabilising structure as described herein, and

a patient interface.

According to another aspect of the present technology, the at least one rear strap is configured to be secured to one or more of the first headgear tube and the second headgear tube.
Another aspect of the present technology relates to a patient interface configured to deliver a supply of breathable gas to a patient during respiratory therapy, including:

a first flow path configured to be, in use, in flow communication with a first naris of the patient,

a second flow path configured to be, in use, in flow communication with a second naris of the patient, and

a flow regulator configured to selectively adjust the proportion of the supply of breathable gas flowing from the first flow path to the first naris and from the second flow path to the second naris.

Another aspect of the present technology relates to a system to provide respiratory therapy, including

a patient interface as described herein; and

a positioning and stabilising structure.

According to an aspect of the present technology, the flow regulator comprises a barrier arrangement which selectively limits or prevents the supply of breathable gas flowing from one of the first flow path to the first naris or from the second flow path to the second naris to thereby adjust the proportion of the supply of breathable gas flowing from the first flow path to the first naris and from the second flow path to the second naris.

According to an aspect of the present technology, the barrier arrangement is configured to move between a first configuration in which it limits or substantially prevents the supply of breathable gas flowing from the first flow path to the first naris, and a second configuration in which it limits or prevents the supply of breathable gas flowing from the second flow path to the second naris.

According to an aspect of the present technology, the barrier arrangement is configured to substantially or completely prevent the supply of breathable gas flowing from the first flow path to the first naris in the first configuration, and substantially or completely prevent the supply of breathable gas flowing from the second flow path to the second naris in the second configuration.

According to an aspect of the present technology, the barrier arrangement is configured to move to the first configuration when the patient interface is in a first orientation and move to the second position when the patient interface is in a second orientation.

According to an aspect of the present technology, the barrier arrangement is further configured to move to a third configuration which permits a sufficient proportion of the supply of breathable gas to flow from each of the first flow path and the second flow path to the respective naris for normal breathing e.g. the proportion is substantially equal.

According to an aspect of the present technology, the barrier arrangement is configured to move to the third configuration when the patient interface is in a third orientation. For instance, in preferred forms the third orientation may be substantially horizontal.

According to an aspect of the present technology, the barrier arrangement comprises at least one barrier element.

According to an aspect of the present technology, the at least one barrier element may be a piston e.g. which has a cylindrical shape. It is also envisaged that the barrier element may have other shapes. For instance, the barrier element(s) may be a spherical component such as a ball bearing, or component having another shape.

According to an aspect of the present technology, the barrier element may comprise one or more flaps or gates which are moveable to selectively permit flow of the supply of breathable gas from the first flow path and the second flow path e.g. hinges are provided to allow the flaps to selectively bock or allow the supply of breathable gas to flow from the first flow path to the first naris and from the second flow path to the second naris.

According to an aspect of the present technology, in use, the barrier element(s) can move to a first position when the barrier arrangement moves to the first configuration, and a second position when the barrier arrangement moves to the second orientation. When in the first position the at least one barrier element limits or prevents the supply of breathable gas flowing from the first flow path to the first naris and does not limit or substantially prevent the supply of breathable gas flowing from the second flow path to the second naris. When in the second position, the at least one barrier element limits or prevents the supply of breathable gas flowing from the second flow path to the second naris and does not limit or substantially prevents the supply of breathable gas flowing from the first flow path to the second naris.

In addition, in this embodiment the at least one barrier element moves to a third position when the barrier arrangement is in the third configuration. When in the third position, the at least one barrier element does not limit or substantially prevent the supply of breathable gas flowing from the first flow path and the second flow path e.g. there is substantially equal flow from the first flow path and the second flow path.

It is also envisaged that the barrier arrangement may comprise two or more barrier elements. For instance, the barrier arrangement may comprise a first barrier element associated with the first flow path and a second barrier element associated with the second flow path. When the barrier arrangement is in the first configuration the first barrier element limits or prevents the supply of breathable gas flowing from the first flow path to the first naris and the second barrier element does not limit or prevent the supply of breathable gas flowing from the second flow path to the second naris. When the barrier arrangement is in the second configuration the first barrier element does not limit or prevent the supply of breathable gas flowing from the first flow path to the first naris and the second barrier element limits or prevents the supply of breathable gas flowing from the second flow path to the second naris.

The provision of a flow regulator having the barrier arrangement according to the present technology may provide a range of advantages for therapy such as the alternation of a flow of breathable gas between the patient’s first naris and second naris to mimic nasal cycling.

According to an aspect of the present technology, the barrier arrangement is configured to be moved to one or more of the first configuration, the second configuration and the third configuration by gravity. For instance, as a patient in use move's their head such as when rolling onto their side, gravity vectors relative to the barrier arrangement change and therefore move the barrier arrangement to another configuration.

It should also be understood that the present technology may comprise at least one actuator e.g. a servomotor or pneumatic actuator. In use, the actuator (s) can move the barrier arrangement to one or more of the first configuration, the second configuration and the third configuration. In these embodiments, the provision of an actuator may be beneficial as it could allow a patient to benefit from respiratory therapy which can mimic native nasal cycling irrespective of the orientation of their head e.g. how they sleep.

According to an aspect of the present technology, the patient interface comprises a cushion which provides the first flow path and the second flow path.

According to an aspect of the present technology, the cushion comprises a body portion.

According to an aspect of the present technology, the cushion comprises at least one nasal pillow, cannula or nozzle which provides the first flow path, and at least one nasal pillow, cannula or nozzle which provides the second flow path.

According to an aspect of the present technology, the nasal pillow(s), cannula(e) or nozzle(s) may be integrally moulded with, or to, the body portion.

According to an aspect of the present technology, the patient interface comprises a first cushion which provides the first flow path and a second cushion which provides the second flow path.

According to an aspect of the present technology, the cushion is constructed from a biocompatible, soft, flexible and/or resilient material. In these embodiments, the cushion may be constructed from silicone, thermoplastic elastomer

(TPE), or foam, or similar. For instance, the cushion may be constructed from a liquid silicone rubber (LSR), or a biocompatible TPE.

According to an aspect of the present technology, the cushion comprises a first seal-forming structure which is constructed and arranged to create a seal with the patient's face e.g. at or around the patient's first naris, and a second seal-forming structure which is constructed and arranged to create a seal with the patient's face e.g. at or around the patient's second naris.

According to an aspect of the present technology, the first seal-forming structure is configured to contact and form a seal with the alar of the patient's first naris.

According to an aspect of the present technology, the second seal-forming structure is configured to contact and form a seal with the alar of the patient's second naris.

According to an aspect of the present technology, the first cannula and the second cannula do not create a seal at the patient's first naris and second naris. For instance, the cannulae are configured for use in a high-flow therapy system for providing respiratory therapy to a patient.

According to an aspect of the present technology, the patient interface comprises a frame. In these embodiments, the cushion(s) is / are attachable to the frame. For instance, the cushion(s) is / are releasably attachable to the frame.

According to an aspect of the present technology, the frame may be constructed from a more rigid material relative to the material from which the cushion is constructed. In these embodiments, the frame may be constructed from a relatively rigid material. For instance, the frame may be constructed from a polycarbonate material or a flexible material having a greater stiffness or durometer hardness greater than the cushion material. In some embodiments, the frame may comprise areas having a relatively higher stiffness provided by greater thickness and/or reinforcing structure(s).

According to an aspect of the present technology, the patient interface may comprise a connection mechanism to facilitate attachment of the frame to the cushion.

According to an aspect of the present technology, the connection mechanism may comprise one or more tabs configured to interlock with one or more corresponding slots or components. For instance, the one or more tabs may be provided on the cushion and the one or more corresponding slot(s) or component(s) may be provided on the frame.

According to an aspect of the present technology, the connection mechanism may comprise a snap-fit, interference fit or other suitable connecting or locking arrangement.

According to an aspect of the present technology, the cushion may comprise a cushion clip configured to facilitate attaching the cushion and the frame together. The cushion clip may be overmoulded to the cushion to form a one-piece component.

According to an aspect of the present technology, the cushion clip may be constructed from a more rigid material relative to the rest of the cushion. For instance, the cushion clip may be constructed from a polycarbonate or a flexible material having a greater stiffness or durometer hardness greater than the rest of the cushion. In yet further alternate examples, the cushion may be stiffened by areas of relatively greater thickness and/or reinforcing structure(s).

According to an aspect of the present technology, the patient interface may be structured to connect to the supply of breathable gas. For instance, in embodiments, the patient interface comprises at least one opening configured to facilitate delivery of the supply of breathable gas into the patient interface.

According to an aspect of the present technology, the at least one opening is provided to the frame. In alternative embodiments, the at least one opening may be provided to the cushion.

According to an aspect of the present technology, the patient interface further comprises an air conduit.

According to an aspect of the present technology, the air conduit may be relatively short.

According to an aspect of the present technology, the air conduit may be extensible.

According to an aspect of the present technology, the patient interface may comprise one or more decoupling mechanisms. In these embodiments, the one or more decoupling mechanisms may be provided between the air conduit and the cushion and/or frame. For instance, the decoupling mechanism may comprise a ball and socket-type joint. In other embodiments, the decoupling mechanism may comprise one or more of a swivel ring or a swivel cuff. In other embodiments, the decoupling mechanism may comprise one or more of a gusset, a fold, a concertina section, an area of relatively lower stiffness, and an area of reduced thickness.

According to an aspect of the present technology, the barrier arrangement may be provided in a chamber.

According to an aspect of the present technology, the chamber comprises one or more walls.

According to an aspect of the present technology, the chamber may comprise at least one inlet, a first outlet and a second outlet.

According to an aspect of the present technology, the first outlet is configured to be, in use, in flow communication with the first flow path, and the second outlet is configured to be, in use, in flow communication with the second flow path.

According to an aspect of the present technology, the at least one inlet is configured to be associated, or align, in use with the at least one opening configured to facilitate delivery of the supply of breathable gas to the patient interface. For instance, the at least one inlet is configured to be associated, or align, in use with the opening formed in the frame.

According to an aspect of the present technology, at least a portion of the one or more chamber walls is relatively rigid. For instance, the relatively rigid portion may be constructed from a relatively rigid material that is sufficiently rigid to hold the shape of the chamber constant in use. In embodiments, the relatively rigid material is a polycarbonate.

According to an aspect of the present technology, the one or more chamber walls may be constructed from a relatively softer, flexible material which is reinforced by structural features to ensure the shape of the chamber is relatively constant in use.

According to an aspect of the present technology, the barrier arrangement is removable from the patient interface. For instance, in embodiments, the at least one barrier element is removable from the chamber, e.g. the one or more chamber walls may comprise at least one opening structured and arranged to facilitate insertion of the at least one barrier element into, and its removal from, the chamber.

According to an aspect of the present technology, the at least one opening is structured and arranged to receive a retaining member which is removably attached to a portion of the one or more chamber walls. In embodiments, the retaining member does not seal the at least one opening. In these embodiments, the at least one opening forms the at least one inlet.

According to an aspect of the present technology, the flow regulator may be removably attached to the cushion, e.g. the chamber is configured to be removably positioned in the cushion to removably attach the flow regulator to the cushion.

According to an aspect of the present technology, the patient interface may comprise a connection mechanism to facilitate attachment of the flow regulator to the patient interface.

According to an aspect of the present technology, the flow regulator may be a separate component which is provided to the patient interface. For instance, the chamber with the barrier arrangement positioned in it may be a separate component which may be in use attached to the patient interface.

According to an aspect of the present technology, the first and second outlets are configured to create a seal with the first and second flow paths. In these embodiments, the first outlet and the second outlet comprise first and second outlet sealing-forming structures configured to contact and form a seal with the first and second flow paths.

According to an aspect of the present technology, the chamber is provided by a plenum chamber defined by one or more of the cushion and the frame.

According to an aspect of the present technology, the patient interface may comprise one or more connectors to facilitate releasable attachment of the patient interface to the positioning and stabilising structure. For instance, the frame may comprise the one or more connectors.

According to an aspect of the present technology, the frame comprises two connectors, one on each lateral side of the frame. In yet further embodiments, the frame may comprise four connectors, a pair of upper connectors and a pair of lower connectors.

According to an aspect of the present technology, the cushion may comprise the one or more connectors. For instance, the body portion may comprise the one or more connectors.

According to an aspect of the present technology, the patient interface may comprise a vent arrangement constructed and arranged to facilitate washout of exhaled gases. For instance, the vent arrangement may be provided by one or more vent holes which may be provided on one or more of the frame and the cushion.

According to an aspect of the present technology, the flow regulator comprises a vent structure which may be configured to be in flow communication with the vent arrangement. For instance, in preferred forms the vent structure may comprise a first vent pathway on a first lateral side of the flow regulator and a second vent pathway on a second lateral side of the flow regulator. In these embodiments, the first vent pathway may comprise at least one vent hole and the second vent pathway may comprise at least one vent hole.

Another aspect of the present technology relates to a flow regulator for a patient interface, wherein the flow regulator is configured to in use selectively adjust the proportion of a supply of breathable gas that flows from a first flow path of the patient interface to a first naris and from a second flow path of the patient interface to a second naris of the patient interface.

Another aspect of the present technology relates to a method of providing respiratory therapy to a patient using a supply of breathable gas, the method including the steps of:

generating the supply of breathable gas;

diverting a relatively larger proportion of the supply of breathable gas to a first flow path and into the patient's first naris and diverting a relatively smaller proportion of the supply of breathable gas to a second flow path and into the patient's second naris; and

subsequently diverting a relatively higher proportion of the supply of breathable gas to the second flow path and into the patient's second naris and a relatively lower proportion of the supply of breathable gas to the first flow path and into the first naris.

Another aspect of the present technology relates to a system to deliver a supply of breathable gas to a patient, including:

a patient interface which comprises:

a first flow path configured to be, in use, in flow communication with the patient's first naris, and
a second flow path configured to be, in use, in flow communication with the patient's second naris;

an apparatus to generate a supply of breathable gas and provide the supply to the patient interface; and

a flow regulator configured to adjust the proportion of the supply of breathable gas that flows from the first flow path to the patient's first naris and from the second flow path to the patient's second naris.

According to an aspect of the present technology, the system may comprise an actuator configured to control the flow regulator and selectively adjust the proportion of the supply of breathable gas that flows from the first flow path to the first naris and from the second flow path to the second naris to mimic nasal cycling.

According to an aspect of the present technology, the actuator may be configured to move the barrier arrangement between a first configuration in which it limits or prevents the supply of breathable gas flowing from the first flow path to the first naris, and a second configuration in which it limits or prevents the supply of breathable gas flowing from the second flow path to the second naris.

According to an aspect of the present technology, the treatment system may comprise one or more sensors each configured to generate one or more signals indicative of a sensed parameter. For instance, the sensed parameter may be indicative of a predetermined condition. In embodiments, the one or more sensors may be provided to the patient interface.

According to an aspect of the present technology, the system may comprise a controller configured to receive and process the one or more sensor signals and generate one or more control signals in response to the one or more sensor signals.

According to an aspect of the present technology, the actuator may be configured to receive the one or more control signals and move the barrier arrangement between the first and second configuration in response the one or more control signals.

According to an aspect of the present technology, the one or more sensors may comprise one or more of a flow rate sensor, a pressure sensor and a temperature sensor. For instance, the signal(s) generated by the flow rate, pressure and/or temperature sensor may be indicative of a resistance to a supply of breathable gas flowing from one of the first and second flow path to the first and second nares. In these embodiments, an indication of resistance to the flow of breathable gas in one of the first or second nares may indicate the start of a natural congestion occurrence in one of the first or second nares which may be indicative of the start of a natural alternation of the flow of breathable gas to one of the first and second nares to the other one of the first and the second nares.

According to an aspect of the present technology, a first sensor may be configured to generate the sensor signal(s) indicative of one or more of the flow rate, pressure and temperature of the supply of breathable gas flowing from the first flow path to the first naris, and a second sensor may be configured to generate the sensor signal(s) indicative one or more of the flow rate, pressure and temperature of the supply of breathable gas flowing from the second flow path to the second naris.

According to an aspect of the present technology, the first sensor signal(s) and the second sensor signals may be correlated to the sensor signal(s) generated by the other sensor(s) to determine resistance to the supply of breathable gas, which may be used to indicate a sleep event, e.g. one or more of natural alteration as described above, a partially / entirely blocked naris or nares, or proneness to apneas. In some embodiments, data related to the sleep event(s) may be recorded and used for screening, diagnosis, monitoring, amelioration, treatment or prevention of a respiratory disorder, or to improve the compliance of patients with respiratory therapy.

Another aspect of the present technology relates to a conduit for a respiratory therapy system, wherein the conduit comprises:

a support structure, and

a casing,

wherein the support structure has a spine having a length and a plurality of ribs spaced apart from each other along the length of the spine,

and wherein the casing is substantially gas impermeable.

Another aspect of the present technology relates to a headgear conduit system for a respiratory therapy system which comprises:

at least one conduit which comprises:

a support structure,

a casing, and

an inlet configured to in use receive a supply of pressurised respiratory gas,

wherein the support structure has a spine having a length and a plurality of ribs spaced apart from each other along the length of the spine,

and wherein the casing is substantially gas impermeable.

According to an aspect of the present technology, the casing may comprise at least one layer.

According to an aspect of the present technology, at least one of the layers may be substantially gas impermeable.

According to an aspect of the present technology, at least one of the layers may be soft.

According to an aspect of the present technology, at least one of the layers may be stretchable, e.g. stretchable in one or more directions.

According to an aspect of the present technology, the casing may be formed by at least a first layer and a second layer.

According to an aspect of the present technology, the first layer may be a textile layer.

According to an aspect of the present technology, the second layer may be a coating layer. For instance, the coating layer may be substantially gas impermeable. The coating layer may comprise a relatively soft, flexible material, e.g. a thermoplastic material such as polyurethane (TPU), or a rubber material.

According to an aspect of the present technology, the second layer may be attached to the first layer. For instance, the coating layer may be laminated onto the textile layer.

According to an aspect of the present technology, the casing may be substantially fluid impermeable, e.g. waterproof.

According to an aspect of the present technology, the casing may be releasably attached or permanently attached to the support structure.

According to an aspect of the present technology, the ribs may be integrally formed with the spine.

According to an aspect of the present technology, the ribs and the spine may be formed separately from each other and attached together.

According to an aspect of the present technology, one or more ribs may be positioned on a first side of the spine, and one or more ribs may be positioned on a second side of the spine.

According to an aspect of the present technology, the support structure may comprise one or more partial hoop structures.

According to an aspect of the present technology, two or more ribs may form a partial hoop structure.

According to an aspect of the present technology, the ribs may be provided in pairs. For instance, a first rib positioned on the first side of the spine may be aligned with a second rib positioned on the second side of the spine.

According to an aspect of the present technology, an end of the first rib may be spaced apart from an end of the second rib to define a gap therebetween.

According to an aspect of the present technology, one or more ribs may form a complete hoop structure.

According to an aspect of the present technology, at least a portion of a first rib may overlap with at least a portion of a second rib to form the complete hoop structure.

According to an aspect of the present technology, each rib may be a complete hoop structure, e.g. a ring-like member. For instance, one rib may extend from the first of the spine to the second side of the spine to form the complete hoop structure.

According to an aspect of the present technology, the support structure may be configured to limit or prevent compression, for example when weight of the patient's head presses down on the support structure during side sleeping. This may be achieved by the structure, materials and arrangements for the support structure as described herein.

According to an aspect of the present technology, at least one of the spine and the ribs may be substantially flexible. For instance, at least one of the spine and the ribs may be constructed from a flexible material.

According to an aspect of the present technology, the casing may be more flexible than the support structure. For instance, the casing may be formed from a first material or arrangement, and the support structure may be formed from a second material or arrangement.

According to an aspect of the present technology, at least one of the ribs and the spine may flex. For instance, a pair of ribs e.g. first rib and second rib, may be constructed and / or arranged to flex away from or towards each other.

According to an aspect of the present technology, at least one of the ribs and the spine may be structured and/or arranged to be resilient.

According to an aspect of the present technology, at least one of the spine and the ribs may be constructed from a resilient material.

According to an aspect of the present technology, at least one of the spine and the ribs may be constructed from an elastomeric material.

According to an aspect of the present technology, at least one of the spine and the ribs may be constructed from a thermoset material, e.g. silicone.

According to an aspect of the present technology, at least one of the spine and the ribs may be constructed from a material selected from the list of a thermoplastic material, e.g. at least one Hytrel, a thermoplastic polyester (TPE) material, and a thermoplastic polyurethane (TPU) material.

According to an aspect of the present technology, the conduit may have a non-circular cross-section when viewed in a plane substantially parallel to its length. The cross-section may be oval shaped.

According to an aspect of the present technology, the conduit may have a substantially circular cross-section when viewed in a plane substantially parallel to its length.

According to an aspect of the present technology, the ribs may be substantially curved. For instance, the ribs may be substantially C-shaped.

According to an aspect of the present technology, the conduit comprises a patient contacting surface configured to in use lie against a surface of the patient's head and/or face, and a distal surface.

According to an aspect of the present technology, the patient contacting surface may be relatively flat. For instance, the spine may be substantially flat.

According to an aspect of the present technology, a plurality of conduits may be attached or formed together to provide a portion of a/the headgear conduit system.

According to an aspect of the present technology, the support structure may be substantially flexible and resilient, but may provide enough stiffness to limit or prevent occlusion or collapse of the conduit in use.

According to an aspect of the present technology, the support structure may be formed from a substantially flexible, resilient, but relatively stiff material.

According to an aspect of the present technology, the support structure may be shaped and/or dimensioned to provide a substantially flexible, resilient, but relatively stiff support structure.

According to an aspect of the present technology, the support structure may be structured and / or arranged to provide regions which have different stiffness to each other.

According to an aspect of the present technology, at least one of the ribs and the spine are structured and / or arranged to provide regions which have different stiffness to each other e.g. a first region which has a first stiffness, and a second region which has a second stiffness. For instance, the rib(s) and/or the spine in the first region may be relatively thicker, wider and/or constructed from a stiffer material than the rib(s) and/or the spine in the second region.

According to an aspect of the present technology, the conduit may comprise a first region and a second region. For instance, the first region may be configured to contact in use a side of the patient's head, and the second region may be configured to contact in use on a top region of the patient's head.

According to an aspect of the present technology, the first region may be relatively stiffer than the second region. For instance, the side of head region may be stiffer than the top of the head region because the side of head region is more prone to compression during side sleeping e.g. when the patient is lying on the side of their head.

According to an aspect of the present technology, the spacing between ribs located adjacent to each other along the spine may be different e.g. a first pair of ribs may have a first separation between them and a second pair of ribs may have a second separation between them. The spacing of the ribs may affect flexibility of the conduit. For instance, having ribs further separated from each other may provide the first region with a relatively higher flexibility than the second region.

According to an aspect of the present technology, the support structure may be structured and / or arranged to provide regions which have a different cross-sectional flow path area to each other.

According to an aspect of the present technology, the conduit may comprise a cross-section which varies along the length of conduit. For instance, the conduit may be configured to provide a larger cross-sectional area of the flow path through the second region compared to a cross-sectional area of the flow path through the first region.

According to an aspect of the present technology, the cross-sectional area of the conduit may taper along the length of the conduit e.g. the flow area of the conduit decreases from one end of the conduit towards another end.

According to an aspect of the present technology, a/the first region of the conduit may be narrower than the a/ the second region of the conduit.

According to an aspect of the present technology, the conduit may flex to conform to the shape of the patient's face and/or head. For instance, the spine may flex to conform to the shape of the patient's face and/or head

According to an aspect of the present technology, the conduit may be shaped to lie against one or more of the patient's face and head in use. For instance, the conduit may comprise a predefined shape.

According to an aspect of the present technology, the conduit may be relatively more flexible in a first directions and relatively less flexible in a second direction. For instance, the spine may be structured to allow the conduit to flex towards or away from the patient's head and/or face in use. In addition, the spine may be relatively less flexibility to restrict or prevent the conduit flexing across the side of the patient's head and/or face.

According to an aspect of the present technology, at least one dimension of the spine may be different along the length of the spine. For instance, a portion of the spine in the first region may be relatively wider and/or thicker than a portion of the spine in the second region.

According to an aspect of the present technology, the conduit may comprise a substantially smooth inner surface.

According to an aspect of the present technology, the support structure may comprise at least one connector configured to facilitate the attachment of the support structure and the casing to each other. For instance, the at least one connector may comprise at least one aperture.

According to an aspect of the present technology, the at least one connector may be located on the spine, on one or more of the ribs, or both of the spine and the rib(s).

According to an aspect of the present technology, a portion of the casing can enter into the aperture(s) when attaching the casing to the support structure, e.g. by thermo-processing or moulding the components together. For instance, the portion may extend through the aperture(s) when the casing is attached to the support structure.

According to an aspect of the present technology, the casing may comprise at least one connector to facilitate attachment of the support structure and the casing to each other. For instance, the at least one connector may comprise at least one preformed projecting members, e.g. a press stud or a push-button.

According to an aspect of the present technology, the casing connector(s) can engage with the support structure connector(s) to facilitate attachment of the support structure and the casing to each other.

According to an aspect of the present technology, the preformed projecting members may be formed on an inner surface of the casing.

According to an aspect of the present technology, the preformed projecting members may be inserted into the aperture(s) to facilitate attachment of the casing to the support structure. For instance, each preformed projecting member may be structured to engage with a portion of the support structure.

According to an aspect of the present technology, the portion of the support structure may be a portion surrounding the aperture(s). For instance, each preformed projecting member may comprise a lip or ridge configured to engage with the portion of the support structure.

Another aspect of the present technology relates to a patient interface system which comprises:

a patient interface,

a positioning and stabilising structure, and

a conduit or a headgear conduit system as substantially described herein.

According to an aspect of the present technology, the conduit or headgear conduit system may comprise part of a/the positioning and stabilising structure.

According to an aspect of the present technology, the positioning and stabilising structure may comprise a pair of headgear tubes, wherein the at least one of the headgear tubes comprises a/the conduit as described herein.

According to an aspect of the present technology, the headgear tubes may be configured to fluidly connect to the patient interface and supply the flow of pressurised breathable gas to the patient interface in use. For instance, an inferior end region of each headgear tube may connect to the patient interface. Alternatively, an inferior end region of each headgear tube may connect to a connector such as a T-piece connector, which in turn fluidly connects to the patient interface.

According to an aspect of the present technology, the headgear tubes may be configured to be releasably attached or permanently attached to the patient interface. For instance, each headgear tube may comprise a connector configured to connect to the patient interface.

According to an aspect of the present technology, the headgear tubes may be attached or formed together. For instance, superior end regions of the headgear tubes may be attached or formed together to provide the headgear conduit system described herein.

According to an aspect of the present technology, the patient interface system may comprise a connection port which is configured to connect to a supply of pressurised respiratory gas in use, e.g. provided by an air delivery conduit. For instance, an inlet may be provided to the conduit or headgear conduit system.

According to an aspect of the present technology, superior end regions of the headgear tubes may be configured to connect to a connector configured to connect to a supply of pressurised respiratory gas in use. For instance, the connector may include the connection port.

According to an aspect of the present technology, the patient interface system comprises a compact full-face or oro-nasal patient interface having a seal forming structure configured to create a seal with or around a patient's nose and mouth.

According to an aspect of the present technology, the positioning and stabilising structure may comprise one or more headgear straps.

According to an aspect of the present technology, one or more headgear straps may be attached to the patient interface.

According to an aspect of the present technology, one or more headgear straps may be attached to a respective headgear tube.

Another aspect of the present technology relates to a method of manufacturing a conduit or headgear conduit system as described herein, wherein the method comprises one or more of the following steps, occurring on any order:

(a) forming at least one casing as described herein;

(b) forming at least one support structure as described herein; and

(c) attaching the casing and the support structure(s) together to form the conduit.

According to an aspect of the present technology, step (a) may comprise one or more of:

- forming a flat sheet of material e.g. forming a multi-layer sheet comprising a first layer and a second layer;
- forming the casing as a sleeve.

According to an aspect of the present technology, step (b) may comprise one or more of:

- forming a plurality of ribs as described herein and a spine as described herein;
- integrally forming the ribs and spine together;
- forming the ribs and spine separately and attaching the ribs and spine together.

According to an aspect of the present technology, step (c) may comprise one or more of:

- positioning the support structure(s) on a flat sheet of material;
- wrapping the flat sheet of material around the support structure(s);

- securing the casing(s) in position on the support structure(s), e.g. using adhesive, tape or any other method that can form a gas-tight seal necessary for the conduit to function as a pathway in a/the respiratory therapy system;
- positioning the sleeve over the support structure(s);
- attaching the sleeve and support structure(s) together, e.g. from a thermoformable material and heat may be applied to heat shrink the sleeve onto the support structure(s).

According to an aspect of the present technology, the support structure(s) and the casing(s) may be formed together. Therefore, steps (a) and (b) may be performed together. For instance, a material may be applied onto a flat sheet of material to form the support structure(s) integrally to the flat sheet of material. In one form, an additive layer manufacturing process may be used to apply bead(s) of a material onto the flat sheet of material.

According to an aspect of the present technology, step (c) may comprise deforming the sheet of material to wrap around the spine and ribs.

Another aspect of the present technology relates to a method of manufacturing a headgear conduit system as described herein, the method comprising one or more of the following steps in any order:

(a) to (c); and

(d) attaching a plurality of the conduits together to provide the headgear conduit system.

According to an aspect of the present technology, the method(s) may comprise the step of forming an inlet as described herein, in the conduit or the headgear conduit system.

Another aspect of one form of the present technology is a patient interface that is moulded or otherwise constructed with a perimeter shape which is complementary to that of an intended wearer.

An aspect of certain forms of the present technology is a medical device that is easy to use, e.g. by a person who does not have medical training, by a person who has limited dexterity, vision or by a person with limited experience in using this type of medical device.

An aspect of one form of the present technology is a portable RPT device that may be carried by a person, e.g., around the home of the person.

An aspect of one form of the present technology is a patient interface that may be washed in a home of a patient, e.g., in soapy water, without requiring specialised cleaning equipment. An aspect of one form of the present technology is a humidifier tank that may be washed in a home of a patient, e.g., in soapy water, without requiring specialised cleaning equipment.

The methods, systems, devices and apparatus described may be implemented so as to improve the functionality of a processor, such as a processor of a specific purpose computer, respiratory monitor and/or a respiratory therapy apparatus. Moreover, the described methods, systems, devices and apparatus can provide improvements in the technological field of automated management, monitoring and/or treatment of respiratory conditions, including, for example, sleep disordered breathing.

Of course, portions of the aspects may form sub-aspects of the present technology. Also, various ones of the sub-aspects and/or aspects may be combined in various manners and also constitute additional aspects or sub-aspects of the present technology.

Other features of the technology should become apparent from consideration of the information contained in the following detailed description, abstract, drawings and claims.

## 3 BRIEF DESCRIPTION OF THE DRAWINGS

The present technology is illustrated by way of example, and not by way of limitation, in the Figs of the accompanying drawings, in which like reference numerals refer to similar elements including:

### 3.1 RESPIRATORY TREATMENT SYSTEM

Fig. 1A shows a respiratory treatment system in use, the respiratory treatment system including a patient interface which receives a supply of respiratory at positive pressure from a RPT device. The patient interface is worn by a patient. Respiratory gas from the RPT device is passed along an air circuit to the patient interface and from thereon to the patient.

Fig. 1B shows a system including a patient wearing a patient interface, in the form of a nasal mask, receiving a supply of air at positive pressure from an RPT device. Air from the RPT device is humidified in a humidifier, and passes along an air circuit to the patient.

Fig. 1C shows a system including a patient wearing a patient interface, in the form of a full-face mask, receiving a supply of air at positive pressure from an RPT device. Air from the RPT device is humidified in a humidifier, and passes along an air circuit to the patient. The patient is sleeping in a side sleeping position.

3.2 RESPIRATORY SYSTEM AND FACIAL ANATOMY

Fig. 2A shows an overview of a human respiratory system including the nasal and oral cavities, the larynx, vocal folds, oesophagus, trachea, bronchus, lung, alveolar sacs, heart and diaphragm.

Fig. 2B shows a view of a human upper airway including the nasal cavity, nasal bone, lateral nasal cartilage, greater alar cartilage, nostril, lip superior, lip inferior, larynx, hard palate, soft palate, oropharynx, tongue, epiglottis, vocal folds, oesophagus and trachea.

Fig. 2C is a front view of a face with several features of surface anatomy identified including the lip superior, upper vermilion, lower vermilion, lip inferior, mouth width, endocanthion, a nasal ala, nasolabial sulcus and cheilion. Also indicated are the directions superior, inferior, radially inward and radially outward.

Fig. 2D is a side view of a head with several features of surface anatomy identified including glabella, sellion, pronasale, subnasale, lip superior, lip inferior, supramenton, nasal ridge, alar crest point, otobasion superior and otobasion inferior. Also indicated are the directions superior & inferior, and anterior & posterior.

Fig. 2E is a further side view of a head. The approximate locations of the Frankfort horizontal and nasolabial angle are indicated. The coronal plane is also indicated.

Fig. 2F shows a base view of a nose with several features identified including naso-labial sulcus, lip inferior, upper Vermilion, naris, subnasale, columella, pronasale, the major axis of a naris and the midsagittal plane.

Fig. 2G shows a side view of the superficial features of a nose.

Fig. 2H shows subcutaneal structures of the nose, including lateral cartilage, septum cartilage, greater alar cartilage, lesser alar cartilage, sesamoid cartilage, nasal bone, epidermis, adipose tissue, frontal process of the maxilla and fibrofatty tissue.

Fig. 2I shows a medial dissection of a nose, approximately several millimeters from the midsagittal plane, amongst other things showing the septum cartilage and medial crus of greater alar cartilage.

Fig. 2J shows a front view of the bones of a skull including the frontal, nasal and zygomatic bones. Nasal concha are indicated, as are the maxilla, and mandible.

Fig. 2K shows a lateral view of a skull with the outline of the surface of a head, as well as several muscles. The following bones are shown: frontal, sphenoid, nasal, zygomatic, maxilla, mandible, parietal, temporal and occipital. The mental protuberance is indicated. The following muscles are shown: digastricus, masseter, sternocleidomastoid and trapezius.

Fig. 2L shows an anterolateral view of a nose.

3.3 PATIENT INTERFACE SYSTEM

Fig. 3A shows a front view of a patient interface system including an unsealed patient interface and a positioning and stabilizing structure. The unsealed patient interface includes a nasal cannula and nasal prongs.

Fig. 3B shows a patient interface in the form of a nasal mask in accordance with one form of the present technology.

Fig. 3C shows a seal-forming structure that includes two pillows. An exterior surface of the seal-forming structure is indicated. An edge of the surface is indicated. Dome and saddle regions are indicated.

Fig. 3D shows a seal-forming structure. An exterior surface of the seal-forming structure is indicated. An edge of the surface is indicated. A path on the surface between points A and B is indicated. A straight line distance between A and B is indicated. Two saddle regions and a dome region are indicated.

3.4 EXAMPLES OF THE PRESENT TECHNOLOGY

Fig. 4A shows a perspective view of the positioning and stabilizing structure including a first headgear tube, a second headgear tube and a flow regulator.

Fig. 4B shows a front view of the positioning and stabilizing structure including a first headgear tube, a second headgear tube and a flow regulator.

Fig. 4C shows a top view of the positioning and stabilizing structure including a first headgear tube, a second headgear tube and a flow regulator.

Fig. 4D shows a front view of the positioning and stabilizing structure 3000 including a first headgear tube, a second headgear tube and a flow regulator.

Fig. 5 shows a top view of the body of the flow regulator having an inlet, a first outlet and a second outlet.

Fig. 6 shows a perspective view of a junction at which the flow regulator is connected to a duct which conveys the respiratory gas from the RPT device to the flow regulator. The duct is connected to the inlet.

Fig. 7 shows a perspective view of the junction at which the first headgear tube 3302, the second headgear tube and the duct are connected to the flow regulator. The first outlet is connected to the first headgear tube, the second outlet is connected to the second headgear tube and the duct is connected to the inlet.

Fig. 8 shows a cross-sectional view of the flow regulator including a piston and a chamber, the piston being movable within the chamber. The piston is in a first configuration where the piston blocks entry of the respiratory gas to the second outlet and allows the respiratory gas to flow through the first outlet.

Fig. 9 shows a cross-sectional view of the flow regulator including the piston and the chamber, the piston being movable within the chamber. The piston is in a second configuration where the piston blocks entry of the respiratory gas to the first outlet and allows the respiratory gas to flow through the second outlet.

Fig. 10A shows a patient lying on one side in a foetus position with their head oriented towards the first side.

Fig. 10B shows a patient lying on one side in a log position with their head oriented towards the second side.

Fig.10C shows a patient lying on one side in a yearner position with their head oriented towards the second side.

Fig.10D shows a patient lying supine in a soldier position with their head oriented towards the first side.

Fig. 10E shows a patient lying supine in a starfish position with their head oriented towards the second side.

Fig. 10F shows a patient lying face down in a free fall position with their head oriented towards the first side.

Fig. 11 shows a schematic diagram of a flow regulator having an actuator, a flow controller and one or more sensors.

Fig. 12 shows a first perspective view of a flow regulator in accordance with one form of the present technology.

Fig. 13 shows a second perspective view of the flow regulator according to Fig. 12.

Fig. 14 shows a cross-sectional view of the flow regulator of Figs. 12 and 13, through section A-A shown in Fig. 12.

Fig. 15 shows a cross-sectional view of the flow regulator of Figs. 12 and 13 through section B-B shown in Fig. 12.

Fig. 16 shows a cross-sectional view of the flow regulator through section B-B in a first orientation.

Fig. 17 shows a cross-sectional view through section B-B with the flow regulator in a second orientation.

Fig. 18 shows a comparison of pressure drop across a commercially available conduit headgear interface with and without a flow regulator according to one form of the present technology.

Fig. 19A shows a graphical representation of flow of respiratory gas through an available conduit headgear interface when one tube is occluded.

Fig 19B shows a graphical representation of flow of respiratory gas through the conduit headgear interface of Fig. 19A fitted with a flow regulator according to one form of the present technology.

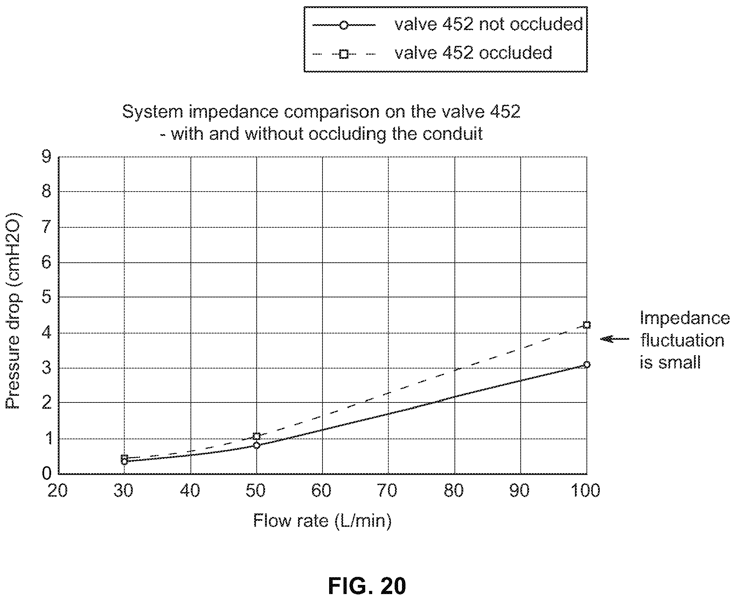

Fig. 20 is a graph comparing pressure drop in an available conduit headgear interface with and without a flow regulator according to one form of the present technology.

3.5 OTHER EXAMPLES OF PRESENT TECHNOLOGY

Fig. 21A shows a patient interface in the form of a nasal mask in accordance with one form of the present technology.

Fig. 21B shows a close up view of a portion of the nasal mask of Fig. 21A.

Fig. 22A shows a view of a cushion component according to one form of the present technology.

Fig. 22B shows an exploded view of the cushion component of Fig. 22A.

Fig. 22C shows a view through a cross-section of the cushion component of Fig. 22A in a first orientation.

Fig. 22D shows a view through a cross-section of the cushion component of Fig. 22A in a second orientation.

Fig. 22E shows a view through a cross-section of the cushion component of Fig. 22A in a third orientation.

Fig. 22F shows a view through a cross-section of the cushion component of Fig. 22A.

Fig. 23A shows a perspective view of a barrier arrangement in accordance with one form of the present technology.

Fig. 23B shows an exploded view of the barrier arrangement of Fig. 23A.

Fig. 23C shows a view through a cross-section of the barrier arrangement in Fig. 23A in a second configuration.

Fig. 24A is a first lateral side view of a user wearing the patient interface of Fig. 21A lying in a supine position.

Fig. 24B is a second lateral side view of a user wearing the patient interface of Fig. 21A lying on their left hand side.

Fig. 24C is a third lateral side view of a user wearing the patient interface of Fig. 21A lying on their right hand side.

Fig. 25A shows a patient interface in the form of a nasal mask in accordance with another form of the present technology.

Fig. 25B shows a close up view of a portion of the nasal mask of Fig. 25A.

Fig. 25C shows an exploded view of the nasal mask of Fig. 25A.

Fig. 25D shows a perspective view of the cushion component of the patient interface of Fig. 25A.

Fig. 25E shows a perspective view of the frame component of the patient interface of Fig. 25A.

Fig. 25F shows a view through a cross-section of the patient interface of Fig. 25A.

Fig. 25G shows a view through a cross-section of the patient interface of Fig. 25A showing a user wearing the patient interface in a supine position.

Fig. 25H shows a view through a cross-section of the patient interface of Fig. 25A showing a user wearing the patient interface lying on their left hand side.

Fig. 25I shows a view through a cross-section of the patient interface of Fig. 25A showing a user wearing the patient interface lying on their right hand side.

Fig. 25J shows a view through a cross-section of the patient interface of Fig. 25A showing flow directions of a supply of breathable gas and exhaled gases.

Fig. 25K shows a patient interface including a positioning and stabilising structure in accordance with one form of the present technology.

Fig. 26A is a schematic diagram showing features of a system for controlling the flow of breathable gas when a patient uses a patient interface to deliver a supply of breathable gas to the patient in accordance with another form of the present technology.

Fig. 26B is a schematic illustration of a flow regulator including an actuator in accordance with one form of the present technology.

3.6 OTHER EXAMPLES OF THE PRESENT TECHNOLOGY

Fig. 27A shows a perspective view of a conduit according to one form of the present technology.

Fig. 27B shows a perspective view of a portion of the conduit of Fig. 27A.

Fig. 27C shows a first cross-sectional view through the length of the conduit shown in Fig. 27A.

Fig. 27D shows a second cross-sectional view through the length of the conduit shown in Fig. 27A.

Fig. 28A shows a perspective view of a support structure according to one form of the present technology.

Fig. 28B shows a first side view of the support structure of Fig. 28A.

Fig. 28C shows a second side view of the support structure of Fig. 28A.

Fig. 28D shows an end on view of the support structure of Fig. 29A.

Fig. 29A shows a perspective view of a headgear conduit system according to one form of the present technology positioned on a patient’s head.

Fig. 29B shows a cross-sectional view through the length of the headgear conduit system of Fig. 29A.

Fig. 29C shows a cross-sectional view through the width of a conduit of the headgear conduit system of Fig. 29A.

Fig. 30 shows a perspective view of the headgear conduit system of Fig. 29A comprising an inlet.

Fig. 31 shows a perspective view of a support structure according to one form of the present technology.

Fig. 32 shows a perspective view of a support structure according to another form of the present technology.

Fig. 33 shows a perspective view of a support structure according to another form of the present technology.

Fig. 34A shows a perspective view of a pair of support structures according to another form of the present technology.

Fig. 34B shows a perspective view of the support structures of Fig. 34A positioned on a patient's head.

Fig. 35A shows a perspective view of a pair of support structures according to another form of the present technology.

Fig. 35B shows an enlarged view of a section of one of the support structures of Fig. 35A.

Fig. 36A shows a perspective view of a pair of support structures according to another form of the present technology.

Fig. 36B shows another perspective view of the support structures of Fig. 36A.

Fig. 37A shows a perspective view of a headgear conduit system according to another form of the present technology.

Fig. 37B a cross-sectional view of the headgear conduit of Fig. 37A.

Fig. 38A shows a perspective view of a support structure according to another form of the present technology.

Fig. 38B is a close-up end perspective view of a conduit according to another form of the present technology.

Fig. 39A is a close-up end perspective view of a section of a conduit according to another form of the present technology.

Fig. 39B is a perspective view of the casing shown in Fig. 39A.

Fig. 40 shows a first view of a patient interface system including a conduit or headgear conduit system according to one form of the present technology.

Fig. 41 shows a second view of the patient interface system of Fig. 40.

3.7 RPT DEVICE

Fig. 42 shows an RPT device in accordance with one form of the present technology.

Fig. 43 is a schematic diagram of the pneumatic path of an RPT device in accordance with one form of the present technology. The directions of upstream and downstream are indicated with reference to the blower and the patient interface. The blower is defined to be upstream of the patient interface and the patient interface is defined to be downstream of the blower, regardless of the actual flow direction at any particular moment. Items which are located within the pneumatic path between the blower and the patient interface are downstream of the blower and upstream of the patient interface.

Fig. 44 is a schematic diagram of the electrical components of an RPT device in accordance with one form of the present technology.

Fig. 45 is a schematic diagram of the electrical components of an RPT device in accordance with one form of the present technology.

Fig. 46 is a schematic diagram of the algorithms implemented in an RPT device in accordance with one form of the present technology.

Fig. 47 is a flow chart illustrating a method carried out by the therapy engine module of Fig. 46 in accordance with one form of the present technology.

3.8 HUMIDIFIER

Fig. 48 shows an isometric view of a humidifier in accordance with one form of the present technology.

Fig. 49 shows an isometric view of a humidifier in accordance with one form of the present technology, showing a humidifier reservoir removed from the humidifier reservoir dock.

Fig. 50 shows a schematic of a humidifier in accordance with one form of the present technology.

4 DETAILED DESCRIPTION OF EXAMPLES OF THE TECHNOLOGY

Before the present technology is described in further detail, it is to be understood that the technology is not limited to the particular examples described herein, which may vary. It is also to be understood that the terminology used in this disclosure is for the purpose of describing only the particular examples discussed herein, and is not intended to be limiting.

The following description is provided in relation to various examples which may share one or more common characteristics and/or features. It is to be understood that one or more features of any one example may be combinable with one or more features of another example or other examples. In addition, any single feature or combination of features in any of the examples may constitute a further example.

4.1 THERAPY

In one form, the present technology comprises a method for treating a respiratory disorder comprising applying positive pressure to the entrance of the airways of a patient 9000.

In certain examples of the present technology, a supply of air at positive pressure is provided to the nasal passages of the patient via one or both nares.

In certain examples of the present technology, mouth breathing is limited, restricted or prevented.

4.2 RESPIRATORY TREATMENT SYSTEM

In one form, the present technology comprises a respiratory treatment system 500 for treating a respiratory disorder. The respiratory treatment system 500 may comprise an RPT device 4000 for supplying a flow of air to the patient 9000 via an air circuit 4170 as described herein, and a patient interface system 3000.

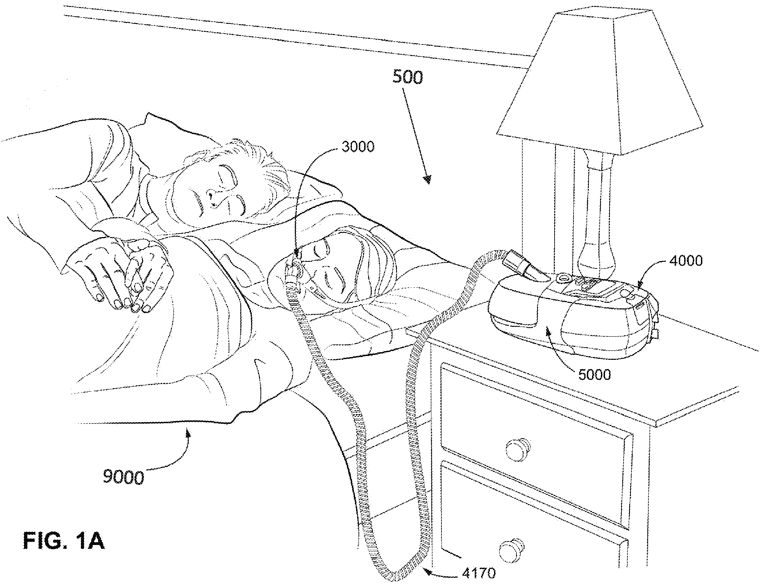

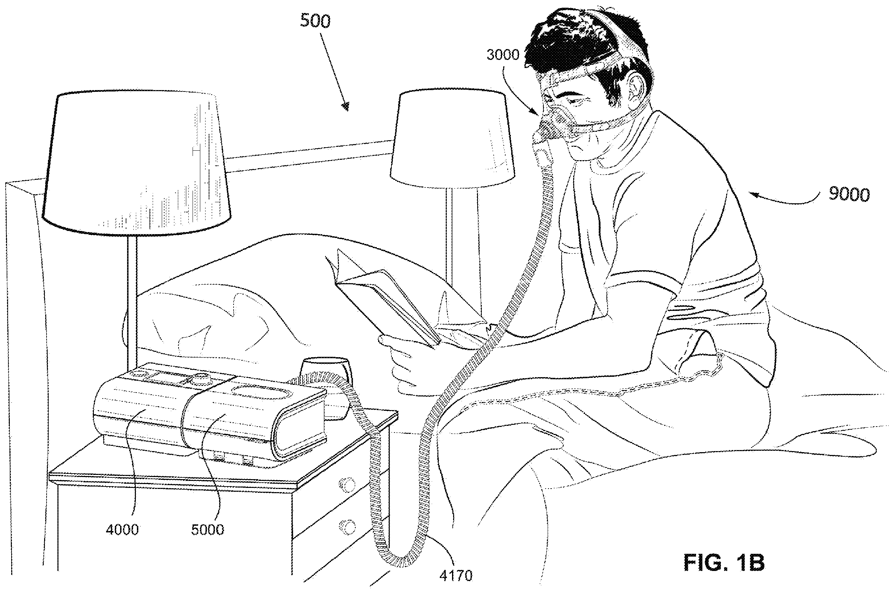

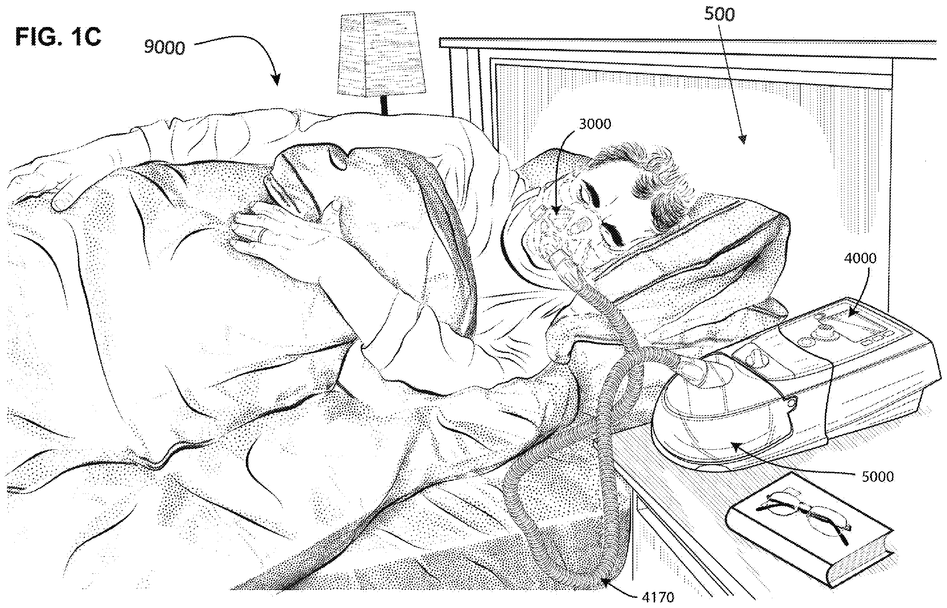

Figs. 1A to 1C illustrate embodiments of a respiratory treatment system 500 in use by a patient 9000.

The RPT device 4000 is configured to generate a flow of respiratory gas, preferably at a positive pressure.

4.3 PATIENT INTERFACE SYSTEM

**4.3.1 Unsealed Patient Interface**

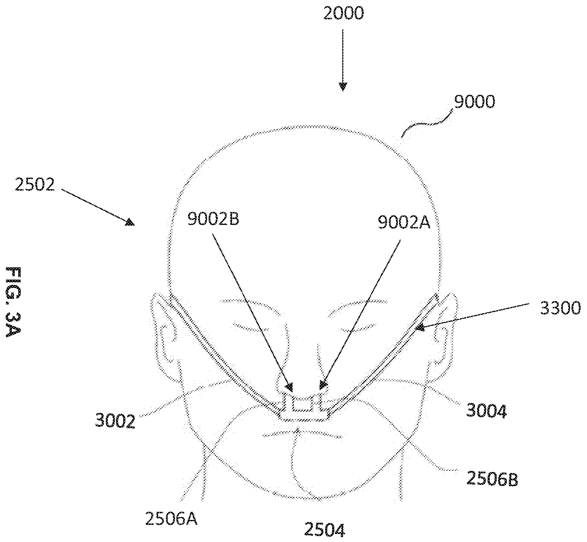

Referring to Fig. 3A which illustrates an aspect of the present technology in which a patient interface system 2000 comprises an unsealed patient interface, e.g. patient interface 2502, and a positioning and stabilizing structure 3300.

The unsealed patient interface 2502 is preferably in the form of nasal cannula 2504 that includes nasal prongs 2506 which can deliver the respiratory gas to a respective one of the nares 9002A, 9002B of the patient 9000 via respective orifices in their tips. Such nasal prongs 2506 do not generally form a seal with the inner or outer skin surface of the nares 9002A, 9002B.

According to one form of the present technology, which is illustrated in Fig. 3A, the nasal prongs 2506 include a first nasal prong 2506A and a second nasal prong 2506B. The first nasal prong 2506A is configured to convey the respiratory gas to a first naris 9002A, and the second nasal prong 2506B is configured to convey the respiratory gas to a second naris 9002B.

The respiratory gas to the nasal prongs 2506 may be delivered by the positioning and stabilizing structure 3300 which may include one or more headgear tubes 3302, 3304 that are coupled with the nasal cannula 2504. The headgear tubes 3302, 3304 lead from the nasal cannula 2504 to a RPT device 4000 via a duct 4050.

The unsealed patient interface 2502 is particularly suitable for delivery of flow therapies, in which the RPT device 4000 generates the flow of air at controlled flow rates rather than controlled pressures.

#### 4.3.2 Sealed Patient Interface

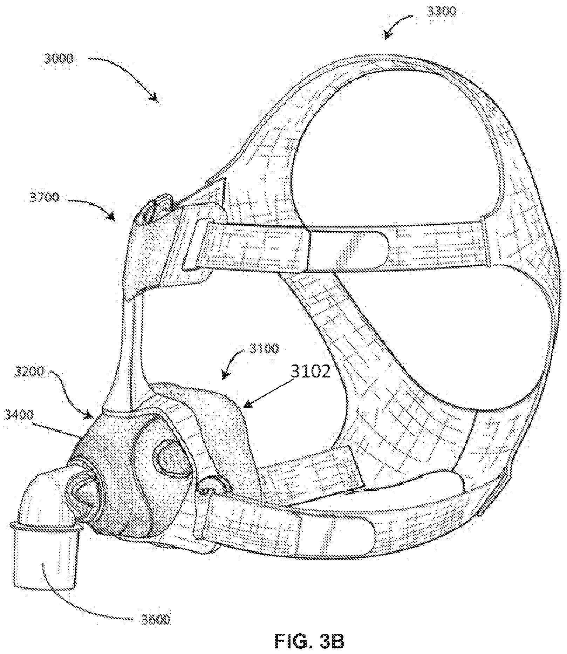

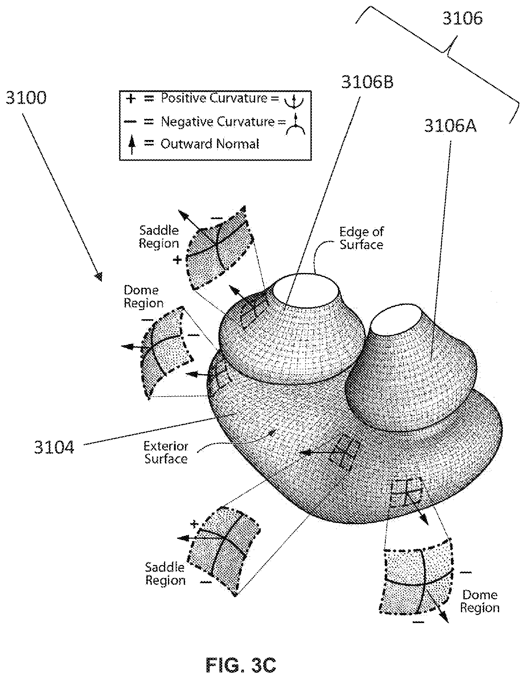

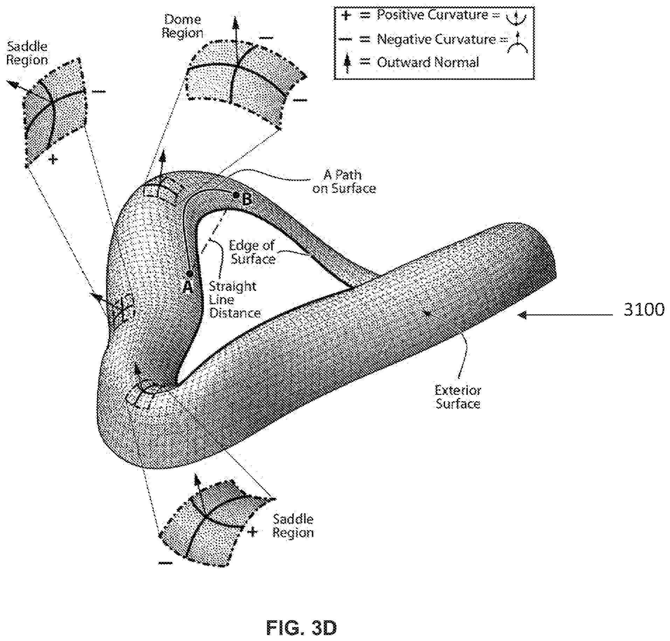

Referring now to Fig. 3B which illustrates an exemplary sealed patient interface system, e.g. patient interface system 3000 in accordance with one aspect of the present technology comprises the following functional aspects: a seal-forming structure 3100, a plenum chamber 3200, a positioning and stabilising structure 3300, a vent 3400, and one form of connection port 3600 for connection to an air circuit 4170. The seal-forming structure 3100 and the plenum chamber 3200 together form a sealed patient interface, e.g. patient interface 3102.
In some forms a functional aspect may be provided by one or more physical components. In some forms, one physical component may provide one or more functional aspects.
In use the seal-forming structure 3100 is arranged to surround an entrance to the airway(s) of the patient 9000 so as to maintain positive pressure at the entrance(s) to the airway(s) of the patient 9000. The sealed patient interface system 3000 is therefore suitable for delivery of positive pressure therapy.
According to an aspect of the present technology illustrated by Fig 3B and 3C, the seal-forming structure 3100 comprises a nasal cushion 3104 which is configured to convey the respiratory gas to the nasal airways of the patient 9000. As shown in Fig. 3C, the nasal cushion may include nasal puffs or pillows 3106 to form a seal with the patient's nares 9002A, 9002B in order to convey the respiratory gas to the nasal airways of the patient 9000.
According to an aspect of the present technology illustrated by Fig. 3D, the seal-forming structure 3100 comprises a full-face cushion which is configured to convey the respiratory gas to the patient's nares and mouth (not shown in Fig. 3D).
The patient interface 3102 in accordance with one form of the present technology is constructed and arranged to be able to provide a supply of air at a positive pressure of at least 6 $cmH_2O$ with respect to ambient.
The patient interface 3102 in accordance with one form of the present technology is constructed and arranged to be able to provide a supply of air at a positive pressure of at least 10 $cmH_2O$ with respect to ambient.
The patient interface 3102 in accordance with one form of the present technology is constructed and arranged to be able to provide a supply of air at a positive pressure of at least 20 $cmH_2O$ with respect to ambient.
Further aspects of the seal-forming structure 3100 and the plenum chamber 3200 of the sealed patient interface 3102 are discussed in the following paragraphs.

#### 4.3.3 Seal-forming structure

In one form of the present technology, as shown in Figs. 3B to 3D, the seal-forming structure 3100 provides a target seal-forming region, and may additionally provide a cushioning function. The target seal-forming region is a region on the seal-forming structure 3100 where sealing may occur. The region where sealing actually occurs- the actual sealing surface- may change within a given treatment session, from day to day, and from patient to patient, depending on a range of factors including for example, where the patient interface was placed on the face, tension in the positioning and stabilising structure 3300 and the shape of a patient's face.
In one form the target seal-forming region is located on an outside surface of the seal-forming structure 3100.
In certain forms of the present technology, the seal-forming structure 3100 is constructed from a biocompatible material, e.g. silicone rubber.
A seal-forming structure 3100 in accordance with the present technology may be constructed from a soft, flexible, resilient material such as silicone.
In certain forms of the present technology, the patient interface system 3000 comprises more than one seal-forming structures 3100, each being configured to correspond to a different size and/or shape range. For example, the patient interface system 3000 may comprise one form of a seal-forming structure 3100 suitable for a large sized head, but not a small sized head and another suitable for a small sized head, but not a large sized head.

#### 4.3.3.1.1 Sealing mechanisms

In one form, the seal-forming structure 3100 includes a sealing flange utilizing a pressure assisted sealing mechanism. In use, the sealing flange can readily respond to a system positive pressure in the interior of the plenum chamber 2200 acting on its underside to urge it into tight sealing engagement with the face. The pressure assisted mechanism may act in conjunction with elastic tension in the positioning and stabilising structure 3300.
In one form, the seal-forming structure 3100 comprises a sealing flange and a support flange. The sealing flange comprises a relatively thin member with a thickness of less than about 1mm, for example about 0.25mm to about 0.45mm, which extends around the perimeter of the plenum chamber 3200. The support flange may be relatively thicker than the sealing flange. The support flange is disposed between the sealing flange and the marginal edge of the plenum chamber 3200, and extends at least part of the way around the perimeter. The support flange is or includes a spring-like element and functions to support the sealing flange from buckling in use.

In one form, the seal-forming structure 3100 may comprise a compression sealing portion or a gasket sealing portion. In use the compression sealing portion, or the gasket sealing portion is constructed and arranged to be in compression, e.g. as a result of elastic tension in the positioning and stabilising structure 3300.

In one form, the seal-forming structure 3100 comprises a tension portion. In use, the tension portion is held in tension, e.g. by adjacent regions of the sealing flange.

In one form, the seal-forming structure 3100 comprises a region having a tacky or adhesive surface.

In certain forms of the present technology, a seal-forming structure 3100 may comprise one or more of a pressure-assisted sealing flange, a compression sealing portion, a gasket sealing portion, a tension portion, and a portion having a tacky or adhesive surface.

**4.3.3.1.1.1** Nasal **pillows**

In one form, as shown in Figs 3C, the seal-forming structure 3100 of the non-invasive patient interface 3102 comprises a pair of nasal puffs, or nasal pillows 2106, each nasal puff or nasal pillow 2106 being constructed and arranged to form a seal with a respective naris 9002A, 9002B of the nose of a patient 9000. For instance, a first nasal pillow 3106A is configured to form a seal with a first naris 9002A, and a second nasal pillow 3106B is configured to form a seal with a second naris 9002B.

The nasal pillows 3106A, 3106B in accordance with an aspect of the present technology include: a frusto-cone, at least a portion of which forms a seal on an underside of the patient's nose, a stalk, a flexible region on the underside of the frusto-cone and connecting the frusto-cone to the stalk. In addition, the structure to which the nasal pillow 2106 of the present technology is connected includes a flexible region adjacent the base of the stalk. The flexible regions can act in concert to facilitate a universal joint structure that is accommodating of relative movement both displacement and angular of the frusto-cone and the structure to which the nasal pillow 2106 is connected. For example, the frusto-cone may be axially displaced towards the structure to which the stalk is connected.

**4.3.3.1.1.2** Nose **bridge or nose ridge region**

In one form, as shown in Fig 3B, the non-invasive patient interface 3102 comprises a seal-forming structure 3100 that forms a seal in use on a nose bridge region or on a nose-ridge region of the patient's face.

In one form, as shown in Fig 3D, the seal-forming structure 3100 includes a saddle-shaped region constructed to form a seal in use on a nose bridge region or on a nose-ridge region of the patient's face.

**4.3.3.1.1.3 Upper lip region**

In one form, as shown in Fig. 3B, the non-invasive patient interface 3102 comprises a seal-forming structure 3100 that forms a seal in use on an upper lip region (that is, the lip superior) of the patient's face.

In one form, as shown in Fig. 3C, the seal-forming structure 3100 includes a saddle-shaped region constructed to form a seal in use on an upper lip region of the patient's face.

**4.3.3.1.1.4 Chin-region**

In one form as shown in Fig. 3B, the non-invasive patient interface 3102 comprises a seal-forming structure 3100 that forms a seal in use on a chin-region of the patient's face.

In one form, as shown in Fig. 3D, the seal-forming structure 3100 includes a saddle-shaped region constructed to form a seal in use on a chin-region of the patient's face.

**4.3.3.1.1.5 Forehead region**

In one form (not shown in the Figs), the seal-forming structure 3100 forms a seal in use on a forehead region of the patient's face. In such a form, the plenum chamber 2200 may cover the eyes in use.

**4.3.4 Plenum chamber**

As shown in Fig. 3B, the plenum chamber 3200 has a perimeter that is shaped to be complementary to the surface contour of the face of an average person in the region where a seal will form in use. In use, a marginal edge of the plenum chamber 3200 is positioned in close proximity to an adjacent surface of the face. Actual contact with the face is provided by the seal-forming structure 3100.

The seal-forming structure 3100 may extend in use about the entire perimeter of the plenum chamber 2200. In some forms, the plenum chamber 2200 and the seal-forming structure 3100 are formed from a single homogeneous piece of material.

In certain forms of the present technology, the plenum chamber 3200 does not cover the eyes of the patient in use. In other words, the eyes are outside the pressurised volume defined by the plenum chamber 3200. Such forms tend to be less obtrusive and / or more comfortable for the wearer, which can improve compliance with therapy.

In certain forms of the present technology, the plenum chamber 3200 is constructed from a transparent material, e.g. a transparent polycarbonate. The use of a transparent material can reduce the obtrusiveness of the patient interface 3102, and help improve compliance with therapy. The use of a transparent material can aid a clinician to observe how the patient interface is located and functioning.

In certain forms of the present technology, the plenum chamber 3200 is constructed from a translucent material. The use of a translucent material can reduce the obtrusiveness of the patient interface 3102 and help improve compliance with therapy.

#### 4.3.5 Positioning and stabilising structure

The patient interface system 3000 may comprise the patient interface 3102 and a positioning and stabilizing structure 3300.

The patient interface 3102 is configured to interface with one or more of the patient's airways. The positioning and stabilizing structure 3300 is configured to maintain the patient interface 3102 in a position relative to the patient's airways.

#### 4.3.6 Vent

In one form, the patient interface system 3000 includes a vent 3400 constructed and arranged to allow for the washout of exhaled gases, e.g. carbon dioxide.

In certain forms the vent 3400 is configured to allow a continuous vent flow from an interior of the plenum chamber 3200 to ambient whilst the pressure within the plenum chamber is positive with respect to ambient. The vent 3400 is configured such that the vent flow rate has a magnitude sufficient to reduce rebreathing of exhaled $CO_2$ by the patient while maintaining the therapeutic pressure in the plenum chamber in use.

One form of vent 3400 in accordance with the present technology comprises a plurality of holes, for example, about 20 to about 80 holes, or about 40 to about 60 holes, or about 45 to about 55 holes.

The vent 3400 may be located in the plenum chamber 2200. Alternatively, the vent 3400 is located on another component of the patient interface system 3000.

In an alternative form of the present technology which is not shown in the Figures, the positioning and stabilizing structure 3300 may include separate headgear tubes for inhalation and exhalation. The exhaled gases may be expelled out through the headgear tubes configured to carry out the exhaled gases.

#### 4.3.7 Decoupling structure(s)

In one form, the patient interface system 3000 includes at least one decoupling structure, for example, a swivel or a ball and socket.

#### 4.3.8 Connection port

In one form, the patient interface system 3000 includes at least one connection port 3600. The connection port 3600 allows for connection to the air circuit 4170.

#### 4.3.9 Forehead support

In one form, the patient interface system 3000 includes a forehead support 3700.

#### 4.3.10 Anti-asphyxia valve

In one form, the patient interface system 3000 includes an anti-asphyxia valve.

In another form, the positioning and stabilizing structure 3300 includes an anti-asphyxia valve.

#### 4.3.11 Ports

In one form of the present technology, a patient interface system 3000 includes one or more ports that allow access to the volume within the plenum chamber 3200. In one form this allows a clinician to supply supplementary oxygen. In one form, this allows for the direct measurement of a property of gases within the plenum chamber 3200, such as the pressure.

## 4.4 EXAMPLES OF THE PRESENT TECHNOLOGY

In one form, the present technology comprises a respiratory treatment system for treating a respiratory disorder. The respiratory treatment system may comprise a RPT device 4000 for supplying a flow of respiratory gas to the patient 9000 via a positioning and stabilizing structure 3300 and a patient interface 3102.

The flow of respiratory gas is conveyed to the positioning and stabilizing structure 3300, preferably through a duct 4050. The positioning and stabilizing structure 3300 conveys the flow of respiratory gas further on to the patient interface 3102.

The patient interface 3102 is configured to convey the flow of respiratory gas to one or more of the patient's airways. The patient's airways include nasal airways and buccal airway. Air or the respiratory gas enters the nasal airways through the patient's nares 9002A, 9002B. Similarly, entrance to the buccal airway is through the patient's mouth.

The positioning and stabilizing structure 3300 has a dual function of maintaining the patient interface 3102 in a position relative to the patient's airways and conveying the respiratory gas to the patient interface 3102.

In certain forms of the present technology, a respiratory treatment system is provided comprising more than one patient interface system 3000, each being configured to provide a retaining force to correspond to a different size and/or shape range. For example, the respiratory treatment system may comprise one form of patient interface system 3000 suitable for a large sized head, but not a small sized head, and another suitable for a small sized head, but not a large sized head.

In certain forms of the present technology, a respiratory treatment system is provided comprising more than one positioning and stabilizing structure 3300, each being configured to correspond to a different size and/or shape range. For example, the respiratory treatment system may comprise one form of positioning and stabilizing structure 3300 suitable for a large sized head, but not a small sized head, and another suitable for a small sized head, but not a large sized head.

In other forms of the present technology, the respiratory treatment system may include one or more embodiments of the positioning and stabilizing structure 3300 described in the following description. In other forms of the present technology, the respiratory treatment system may include one or more embodiments of a patient interface system 3000 including the patient interface 3102, as described in the following description.

In one form, the present technology comprises a patient interface system 3000. The patient interface system 3000 may comprise the patient interface 3102 and a positioning and stabilizing structure 3300.

In one form of the present technology, the positioning and stabilizing structure 3300 also conveys the respiratory gas to the patient interface 3102.

In one form of the present technology, the patient interface may be an unsealed patient interface 2502. In an alternative form of the present technology, the patient interface may be a sealed patient interface 3102.

### 4.4.1 Positioning and stabilising structure

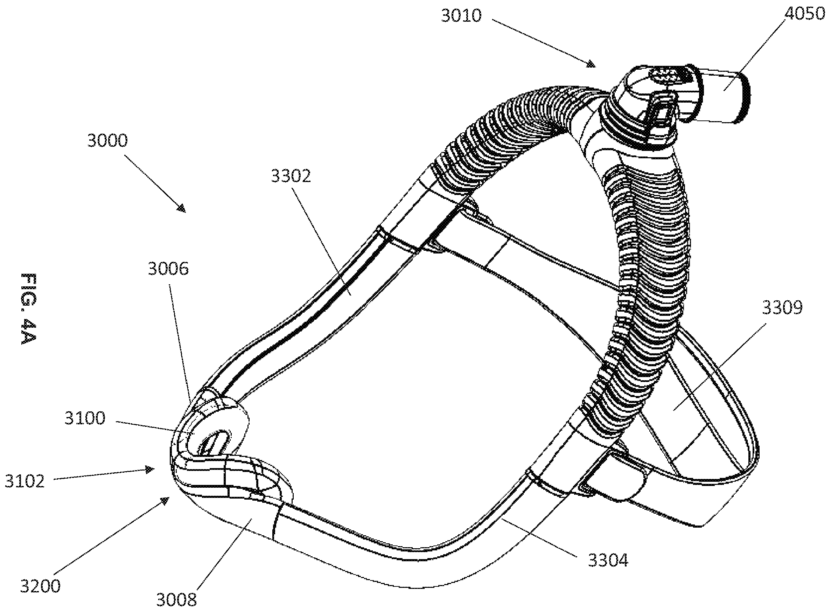

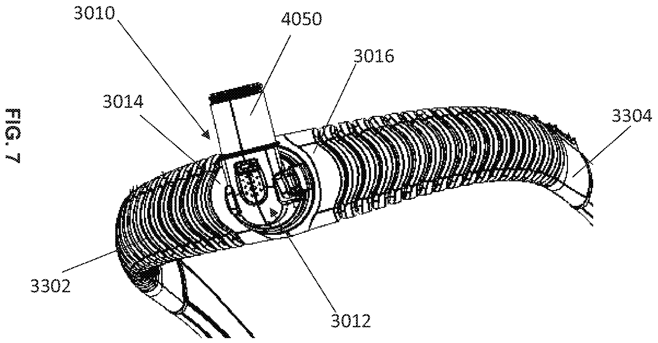

According to an aspect of the present technology, illustrated by Figs. 4A, 4B, 4C, 4D, 5, 6, 7, 8 and 9 the positioning and stabilizing structure 3300 comprises a first headgear tube 3302, a second headgear tube 3304, and a flow regulator 3010.

The positioning and stabilizing structure 3300 is configured in use to convey the respiratory gas to the patient interface 3102 as well as to maintain the patient interface 3102 in a position relative to the patient's airways.

The patient interface 3102 of the present technology may be held in position with respect to the patient's airways in use by the positioning and stabilising structure 3300.

In one form of the present technology, as shown in Fig. 3A, the unsealed patient interface 2502, including the nasal cannula 2504 and the nasal prongs 2506, may be held in position in use by the positioning and stabilising structure 3300.

In one form of the present technology, as shown in Fig. 3B, the seal-forming structure 3100 of the sealed patient interface 3102 may be held in sealing position in use by the positioning and stabilising structure 3300.

In one form the positioning and stabilising structure 3300 provides a retention force at least sufficient to overcome the effect of the positive pressure in the plenum chamber 3200 to lift off the patient's face.

In one form the positioning and stabilising structure 3300 provides a retention force to overcome the effect of the gravitational force on the patient interface 3102.

In one form the positioning and stabilising structure 3300 provides a retention force as a safety margin to overcome the potential effect of disrupting forces on the patient interface 3102, such as from tube drag, or accidental interference with the patient interface.

In one form of the present technology, a positioning and stabilising structure 3300 is provided that is configured in a manner consistent with being worn by a patient while sleeping. In one example the positioning and stabilising structure 3300 has a low profile, or cross-sectional thickness, to reduce the perceived or actual bulk. In one example, the positioning and stabilising structure 3300 comprises at least one headgear tube 3302, 3304 having a rectangular cross-section.

In one form of the present technology, a positioning and stabilising structure 3300 is provided that is configured so as not to be too large and bulky to prevent the patient from lying in a supine sleeping position with a back region of the patient's head on a pillow.

In one form of the present technology, a positioning and stabilising structure 3300 is provided that is configured so as not to be too large and bulky to prevent the patient from lying in a side sleeping position with a side region of the patient's head on a pillow.

In one form of the present technology, a positioning and stabilising structure 3300 is provided with a decoupling portion located between an anterior portion of the positioning and stabilising structure 3300, and a posterior portion of the positioning and stabilising structure 3300. The decoupling portion does not resist compression and may be, e.g. a flexible or floppy strap. The decoupling portion is constructed and arranged so that when the patient lies with their head on a pillow, the presence of the decoupling portion prevents a force on the posterior portion from being transmitted along the positioning and stabilising structure 3300 and disrupting the seal.

In one form of the present technology, the positioning and the stabilizing structure 3300 is constructed and arranged to in use lie over at least a portion of the zygomatic bone regions of the patient's face.

In one form of the present technology, the positioning and the stabilizing structure 3300 is constructed and arranged to in use lie over the frontal and/or parietal bone regions of the patient's head.

In one form of the present technology, the positioning and the stabilizing structure 3300 is constructed and arranged to in use to lie over at least a portion of the patient's temple region.

In one form of the present technology, the positioning and the stabilizing structure 3300 is constructed and arranged to in use to extend across at least a portion of the portion of the cheek regions of the patient's face.

In one form of the present technology, the positioning and stabilising structure 3300 is constructed and arranged so that in use at least a portion of an inferior edge thereof passes superior to an otobasion superior of the patient's head and overlays a portion of a parietal bone without overlaying the occipital bone.

In one form of the present technology suitable for a nasal-only mask or for a full-face mask, the positioning and stabilising structure 3300 is constructed and arranged so that in use at least a portion of a superior edge thereof passes inferior to an otobasion inferior of the patient's head and overlays or lies inferior to the occipital bone of the patient's head.

#### 4.4.1.1 Headgear Tubes

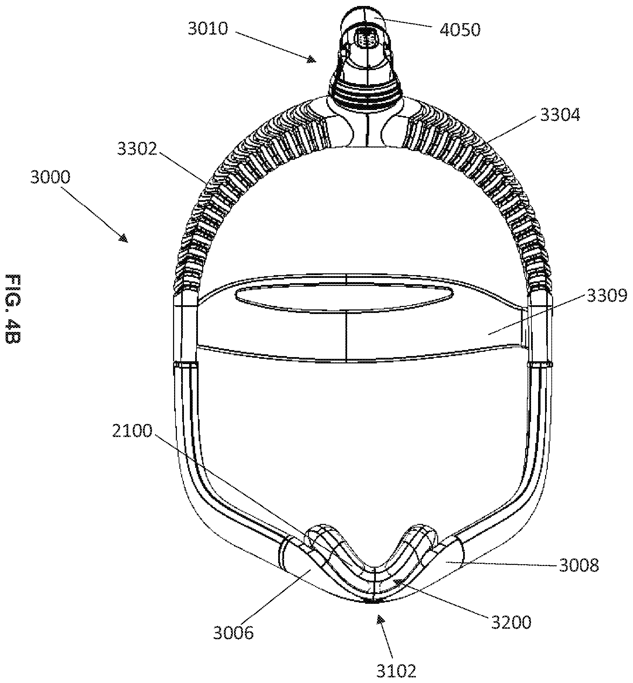

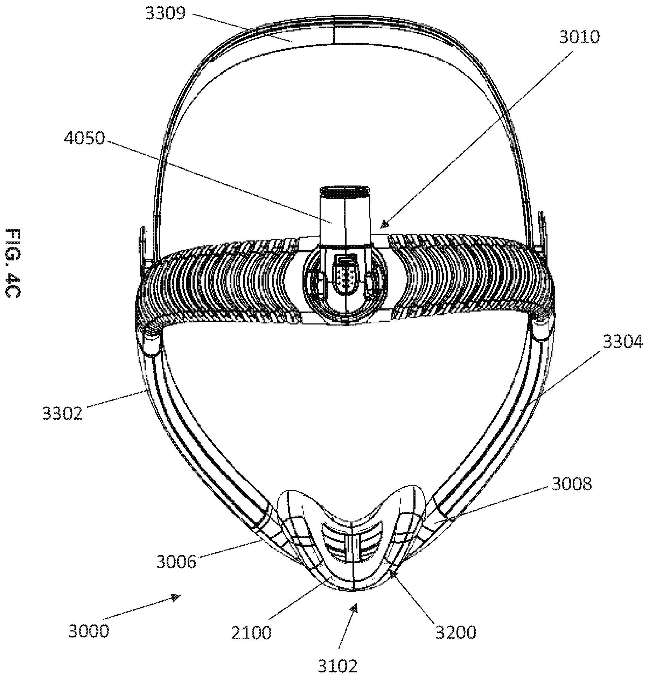

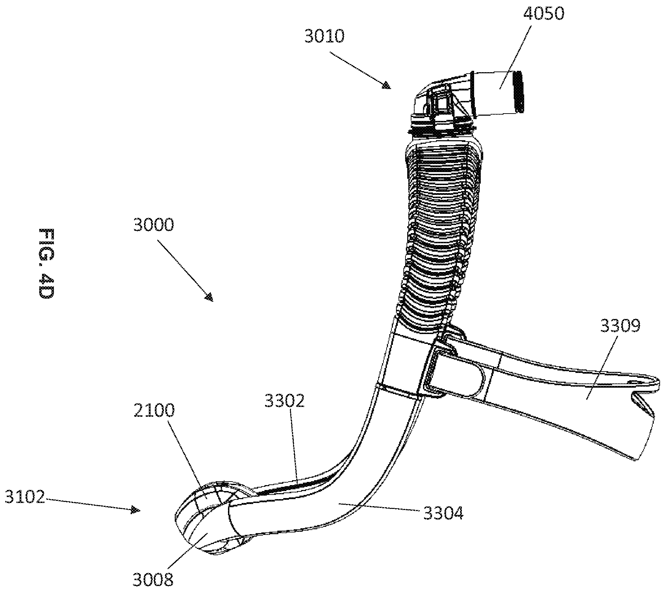

Referring now to Figs 4A, 4B, 4C and 4D which shows the positioning and stabilizing structure 3300 having a first headgear tube 3302, a second headgear tube 3304 and a flow regulator 3010.

In certain forms of the present technology, a positioning and stabilising structure 3300 comprises one or more headgear tubes 3302, 3304 that are bendable and e.g. non-rigid.

In certain forms of the present technology, a positioning and stabilising structure 3300 comprises one or more headgear tubes 3302, 3304 that is extensible, e.g. resiliently extensible. For example, the headgear tubes 3302, 3304 may be configured in use to be in tension, and to direct a force to draw a seal-forming structure 3100 into sealing contact with a portion of a patient's face.

In one form of the present technology, a positioning and stabilising structure 3300 comprises headgear tubes 3302, 3304 configured to convey the respiratory gas to the patient interface 3102, wherein the headgear tubes 3302, 3304 are constructed from a laminate of a fabric patient-contacting layer, a foam inner layer and a fabric outer layer.

In one form of the present technology, one or more of the headgear tubes 3302, 3304 are made of (a) compressible material(s).

In an alternative form of the present technology, one or more of the headgear tubes 3302, 3304 are made of (a) compression-resistant material(s).

In another form of the present technology, at least a portion of the first headgear tube 3302 and the second headgear tube 3304 may be made of (a) compressible material(s), and at least a portion of the first headgear tube 3302 and the second headgear tube 3304 may be made of (a) compression-resistant material(s).

In certain alternative forms of the present technology, not shown in the Figs, a positioning and stabilising structure 3300 comprises headgear tubes 3302, 3304 formed from straps attached to conduit tubes. The straps may preferably be in contact with the patient's face. Further, the straps may be constructed to be breathable to allow moisture vapour to be transmitted through the straps. Alternatively, or in addition, the straps and/or the conduit tubes may be flexible.

In certain forms of the present technology, as shown in Figs 4A, 4B, 4C and 4D, the positioning and stabilizing structure 3300 may include a rear strap 3309 which in use is located substantially over a rear portion of the patient's head. The rear strap 3309 may be attached to the first headgear tube 3302 and the second headgear tube 3304.

In alternative forms of the present technology (not shown in the Figs), the positioning and stabilizing structure 3300 may include multiple straps to secure the headgear tube(s) 3302, 3304 and/or the patient interface 3102 to the patient's head. The multiple straps may include multiple rear straps.

In one form of the present technology, the first headgear tube 3302 may be located substantially on a first side of the patient 9000, and the second headgear tube 3304 may be located substantially on a second side of the patient 9000.

In one form of the present technology, the first headgear tube 3302 may be configured in use to be located substantially over a first cheek of the patient 9000, and the second headgear tube 3304 may be located substantially over a second cheek of the patient 9000.

In one form of the present technology, the first headgear tube 3302 and the second headgear tube 3304 may be connected to each other.

In a preferred form of the present technology, the first headgear tube 3302 and the second headgear tube 3304 may be connected at a substantially top portion of the patient's head.

In an alternative form of the present technology, the first headgear tube 3302 and the second headgear tube 3304 may be connected substantially proximal to and/or substantially over the patient interface 3102.

In an alternative form of the present technology, the first headgear tube 3302 and the second headgear tube 3304 may be connected substantially proximal to and/or over the nose and/or mouth regions of the patient 9000.

In one form of the present technology, the first headgear tube 3302 and the second headgear tube 3304 may be connected at a region which is substantially perpendicular to the central sagittal plane of the patient 9000.

In one form of the present technology, the first headgear tube 3302 may be connected to a patient interface 3102 by a first connector 3006, and the second headgear tube 3304 may be connected to a patient interface 3102 by a second connector 3008.

In a preferred form of the present technology, respiratory gas flowing through the first headgear tube 3302 may be conveyed to the first nare 9002A, and respiratory gas flowing through the second headgear tube 3304 may be conveyed to the second nare 9002B. For instance, the first connector 3006 may connect the first headgear tube 3302 to the first nasal prong 2506A or the first nasal pillow 3106A, and the second connector 3008 may connect the first headgear tube 3304 to the second nasal prong 2506B or the second nasal pillow 3106A.

#### 4.4.1.2 Flow Regulator

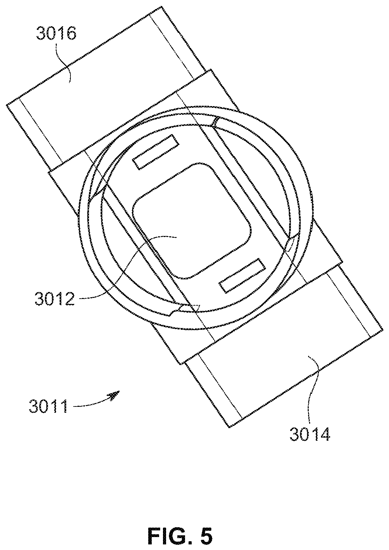

Referring now to Figs. 5 to 9 showing a flow regulator 3010 according to one form of the present technology.

In the embodiment of Figs. 5 to 9, the flow regulator 3010 is configured to adjust the proportion of the flow of respiratory gas flowing into the first headgear tube 3302 and the second headgear tube 3304 as is discussed in more detail below.

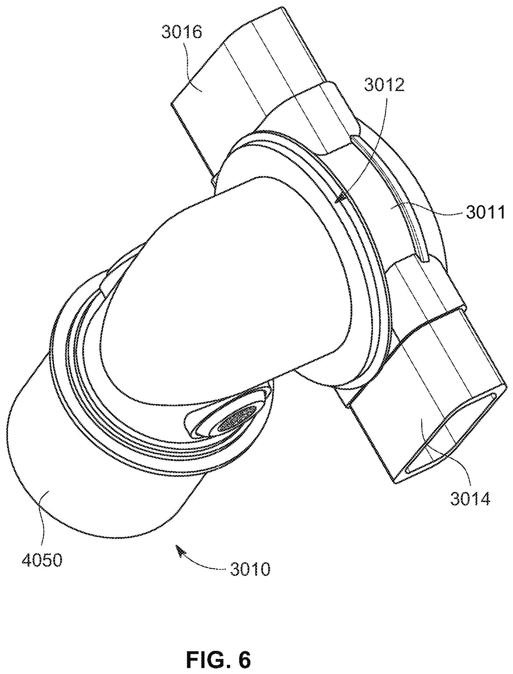

As shown in Figs. 5 and 6, the flow regulator 3010 includes a body 3010A having an inlet 3012, a first outlet 3014 and a second outlet 3016.

The flow regulator 3010 may form a junction to connect the duct 4050 and the headgear tubes 3302, 3304. In use, the duct 4050 can convey the flow of respiratory gas to the positioning and stabilizing structure 3300. The headgear tubes 3302, 3304 may be configured to convey the flow of respiratory gas to the patient interface 3102.

The inlet 3012 is located generally on the top of the patient's head and in a region through which the central sagittal plane (not illustrated in the Figures) of the patient 9000 passes through.

The first outlet 3014 is orientated substantially towards the first side of the patient 9000, and the second outlet 3016 is orientated substantially towards the second side of the patient 9000.

An elbow (not shown) is rotatably attached to the body and in fluid communication with the first inlet.

The first outlet 3014 is configured to connect to the first headgear tube 3302 and the second outlet 3016 is configured to connect to the second headgear tube 3304.

In an alternative form of the present technology, the junction may be located substantially adjacent to and/or substantially proximal to the nose and/or mouth regions of the patient 9000.

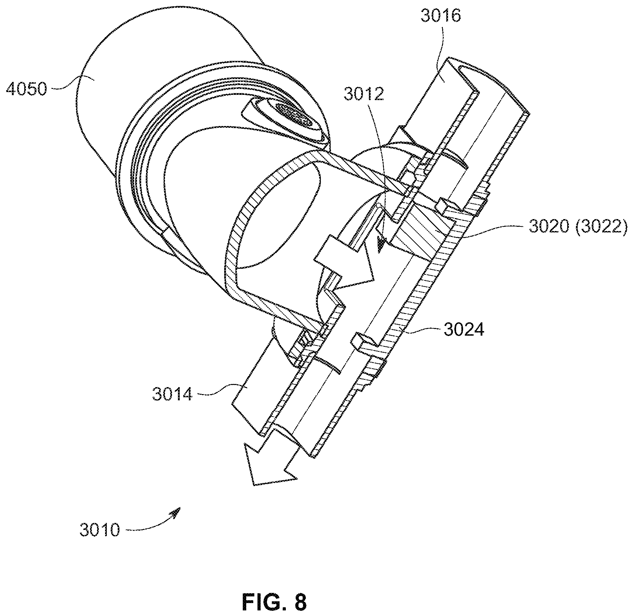

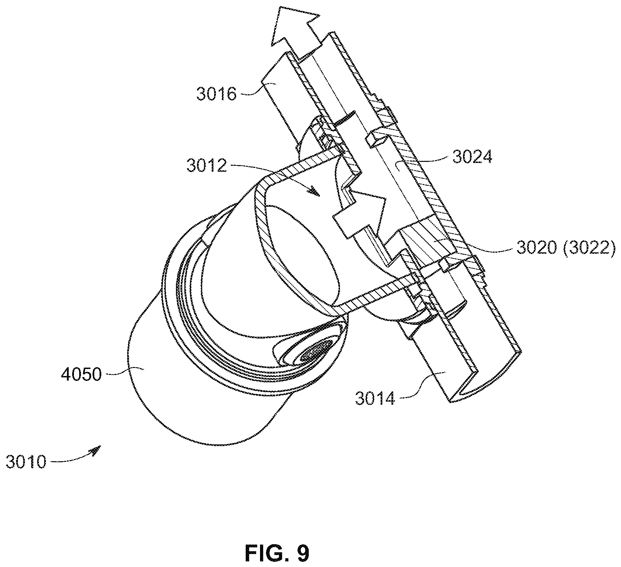

The flow regulator 3010 includes a barrier arrangement indicated generally as 3020. The barrier arrangement 3020 is movable from a first configuration (as shown in Fig 8) to a second configuration (as shown in Fig 9).

As illustrated in Figs. 8 and 9, the barrier arrangement 3020 includes a piston 3022 located in a chamber 3024 defined by the body.

In one form of the present technology, as shown in Fig. 8, the piston 3022 is configured to block passage of the flow of respiratory gas to the second headgear tube 3304 and diverts the flow of respiratory gas to the first headgear tube 3302.

In one form of the present technology, as shown in Fig. 9, the piston 3022 is configured to block passage of the flow of respiratory gas to the first headgear tube 3302 and diverts the flow of respiratory gas to the second headgear tube 3304.

In one form of the present technology, the piston 3022 may assume any of have multiple positions between the first configuration and the second configuration.

In one form of the present technology, when the piston 3022 is positioned substantially in a central region of the chamber 3024, the piston 3022 is configured to divert the flow of respiratory gas substantially equally between the first headgear tube 3302 and the second headgear tube 3304.

In one form of the present technology, the first flow portion and the second flow portion are dependent on the ratio of displacement of the piston 3022 from the location which is substantially in the central region of the chamber 3024. When the piston 3022 is displaced towards the first headgear tube 3302, then first flow portion is lesser than the second flow portion. When the piston 3022 is displaced towards the second headgear tube 3304, the first flow portion is greater than the second flow portion.

In a preferred form of the present technology, the piston 3022 is configured to partially or completely prevent passage of the flow of respiratory gas to the second outlet 3016 in the first configuration (as shown in Fig. 8), and the piston is configured to partially or completely prevent passage of the flow of respiratory gas to the first outlet 3014 in the second configuration (as shown in Fig. 9).

In one form of the present technology (as shown in Figs. 8 and 9), the piston 3022 has a substantially quadrilateral cross-sectional area. Preferably, the piston 3022 may have a rectangular or square cross-sectional area.

In a preferred form of the present technology, one or more of the inlet 3012, the first outlet 3014 and the second outlet 3016 may have a substantially quadrilateral cross-section. Preferably, the quadrilateral may be a rectangular and/or a square.

In one form of the present technology, the piston 3022 may be configured to engage in an air-tight fashion with the second outlet 3016 in the first configuration, and the piston 3022 may be configured to engage in an air-tight fashion with the first outlet 3014 in the second configuration.

In one form of the present technology, the piston 3022 may be rigid and incompressible in use. For instance, the piston 3022 may be made of a material that is incompressible under air pressure or force exerted by the walls of the chamber 3024 when the piston 3022 moves inside the chamber 3024.

In the embodiment of Figs. 5 to 9 the flow regulator 3010 is a gravity-actuated component and the barrier arrangements moves between the first configuration and the second configuration under gravitational forces e.g. the piston 3022 is moved in the chamber 3024 by gravity.

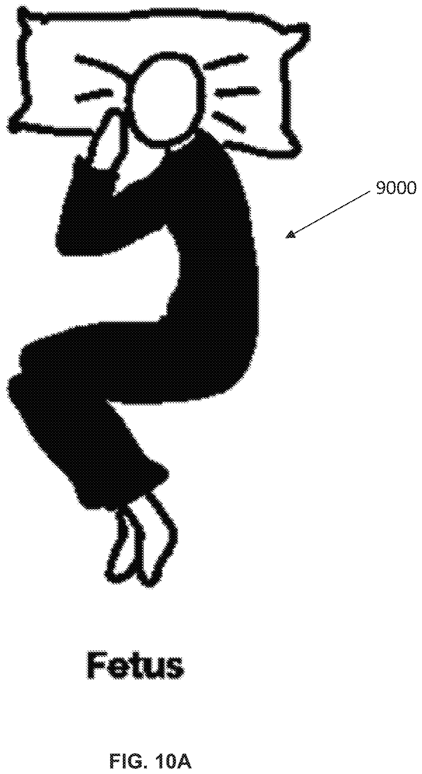

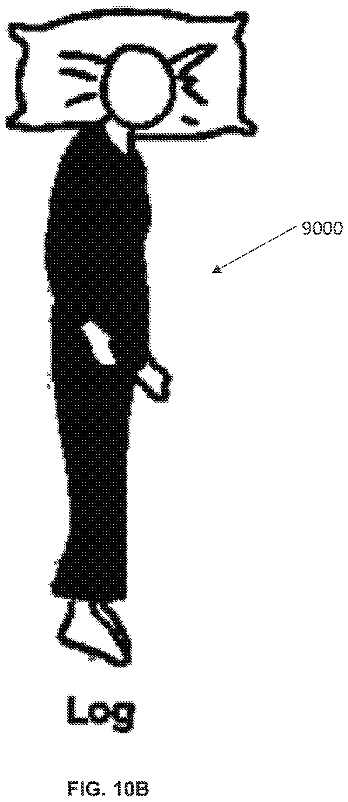

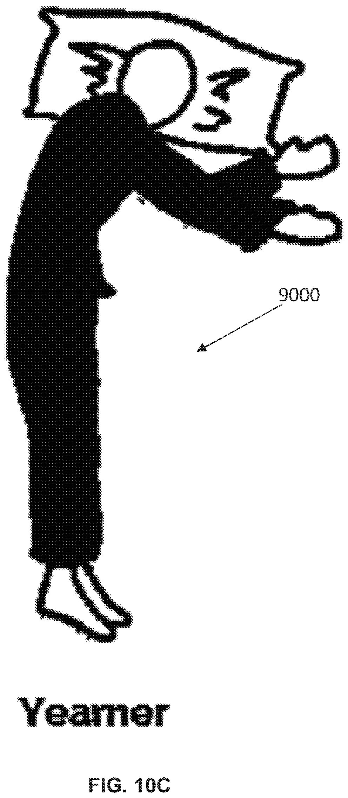

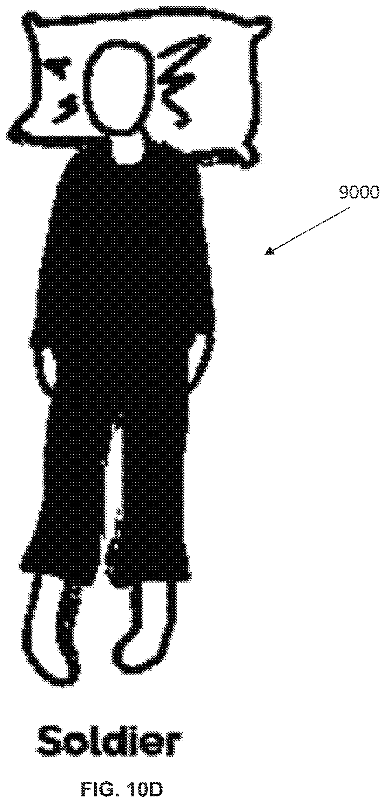

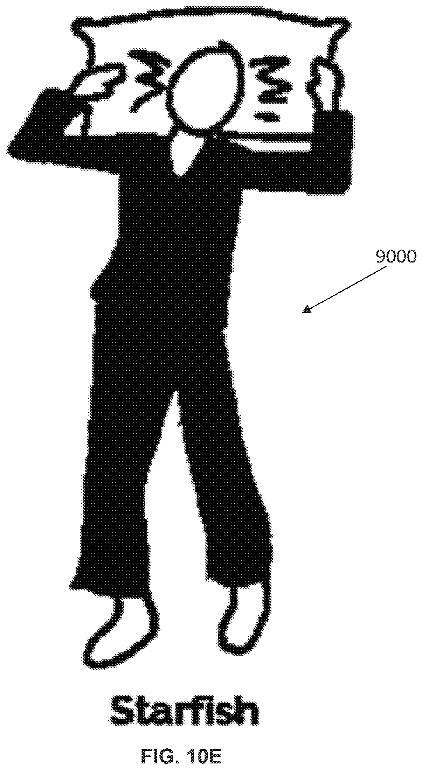

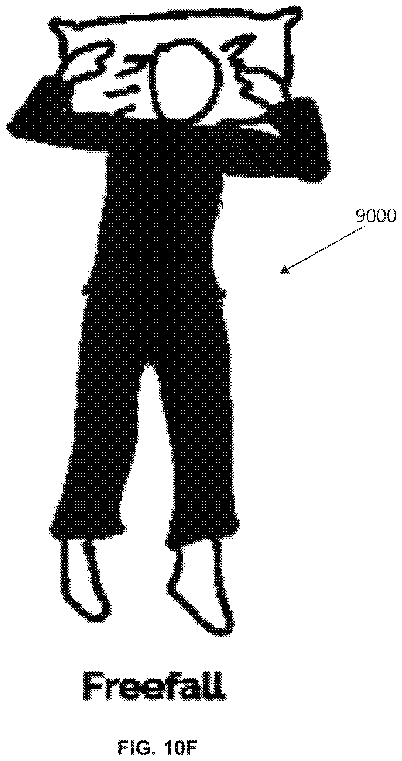

Referring now to Figs. 10A to 10F which show some positions in which a patient 9000 may sleep.

The orientation of the patient's head may be determined by the side to which the patient's head is tilted towards. If the patient's head is tilted slightly or substantially towards their left or right side, the barrier arrangement is moved to the first or the second configuration.

In certain forms of the present technology, the piston 3022 is configured to be moved by gravity towards the patient's first side when the patient's head is oriented towards the first side, and the piston 3022 is configured to be moved by gravity towards the patient's second side when the patient's head is oriented towards the second side.

In certain forms of the present technology, the piston 3022 is configured to slide freely under gravity in the chamber 3024.

In one form of the present technology, the piston 3022 is configured to be moved to the first configuration when the patient's head is oriented towards the second side, and the piston 3022 is configured to be moved to the second configuration when the patient's head is oriented towards the first side.

In one form of the present technology, the extent of displacement of the piston 3022 from the centre of the chamber 3024 may be dependent on the angle at which the patient's head is oriented towards either the first side or the second side.

In one form of the present technology, the piston 3022 is configured to be moved to the first configuration only when the patient's head is oriented substantially to the second side, and the piston 3022 is configured to be moved to the second configuration only when the patient's head is oriented substantially to the first side.

In one form of the present technology, the piston 3022 is located in the central region of the chamber 3024 when the patient's head is not tilted towards the first side or the second side e.g. when they are lying on their back.

The barrier arrangement 3020 is configured to proportion the flow of respiratory gas into a first flow portion and a second flow portion based on the extent of displacement of the piston 3022 from the central portion of the chamber 3024.

The entire flow of respiratory gas is directed to the first headgear tube 3302 when the piston 3022 is in the first configuration and it blocks the second outlet, and the entire flow of respiratory gas is directed to the second headgear tube 3304 when the piston 3022 is in the second configuration and it blocks the first outlet.

The barrier arrangement 3020 can proportion the flow of respiratory gas substantially equally between the headgear tubes 3302, 3304 so that substantially equal portions at deliver to the first naris 9002A and the second naris 9002B when the piston 3022 is located substantially in the central region of the chamber 3024.

When the patient's head is oriented towards one side, the headgear tube on that side is likely to be compressed due to the weight of the patient's head and either partly or completely occluded. The barrier arrangement 3020 diverts a substantially or entire portion of the flow of respiratory gas to the non-occluded headgear tube.

If the respiratory gas flows through constricted areas of lesser cross-sectional area, it causes an increase in velocity of the flow of respiratory gas. Such an increase in velocity can lead to an effect called jetting in which the respiratory gas is delivered to the nares 9002A, 9002B at a high speed. Jetting can be extremely uncomfortable for a patient 9000. It can cause a patient 9000 to wake up in sleep, thereby reducing sleep quality which in turn discourages the patient 9000 from continuing respiratory therapy. Therefore, the flow regulator 3025 may prevent jetting.
In addition, the flow regulator 3025 may reduce impedance in the respiratory treatment system and minimise or reduce pressure differences between the inlet and the patient interface.
In addition, the flow regulator 3025 may assist in mimicking the native nasal cycle and allow the nasal passages to recover and hydrate.
In addition, the gravity actuated flow regulator 3025 does not require components like sensors, actuators and processing systems to determine when the air flow should be adjusted, or an additional power supply.
Therefore, the gravity actuated flow regulator 3025 is a simple component that can be easily manufactured and used.
The flow regulator can also be retrofitted to existing conduit headgear treatment system. For instance, the flow regulator can replace an existing conduit in the conduit headgear treatment system.

#### 4.4.1.3 Actuator- Based Flow Regulator

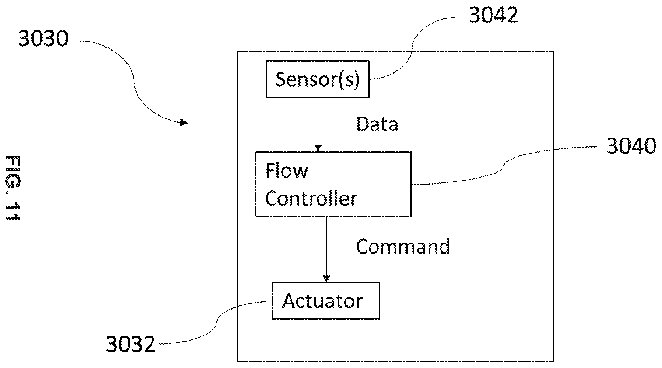

Referring now to Fig. 11 which is a schematic diagram showing the components of an actuator-based flow regulator 3030.
In certain alternative forms of the present technology, the flow regulator 3010 may be an actuator -based flow regulator 3030 which includes an actuator 3032 which is configured to move the piston 3022 between the first configuration, the second configuration and/or to any position between the first configuration and the second configuration.
In one form of the present technology, the actuator 3032 may be one or more of a hydraulic, pneumatic, mechanical, electrical and/or magnetic actuator.
In certain forms of the present technology, the actuator-based flow regulator 3030 further includes a flow controller 3040 which is configured to control the position of the piston 3022 within the chamber 3024.
In one form of the present technology, the flow controller 3040 is configured to transmit command(s) to the actuator 3032 which moves the piston 3022.
In certain forms of the present technology, the actuator-based flow regulator 3030 further includes at least one sensor 3042 which may be configured to detect properties which constitute data related to control of the position of the piston 3022 within the chamber 3024.
In one form of the present technology, the sensor(s) 3042 may include an orientation sensor 3044 which may be configured to detect the orientation of the patient's head. The data provided by the orientation sensor 3044 may be used to determine the position of the piston 3022 to ensure appropriate flow of respiratory gas through the headgear tube 3302, 3304 which is not occluded.
In one form of the present technology, the sensor(s) 3042 may include an occlusion sensor 3046 which may be configured to detect if the first headgear tube 3302 and/or the second headgear tube 3304 is/are occluded. The occlusion sensor 3046 may also be configured to detect the extent of occlusion of the first headgear tube 3302 and/or the second headgear tube 3304. The flow controller 3040 may be configured to command the actuator 3032 to displace the piston 3022 to an extent which is proportional to the extent of occlusion in the first headgear tube 3302 and the second headgear tube 3304.
In one form of the present technology, the sensor(s) 3042 may include a dryness sensor 3048 which may be configured to detect dryness in a nasal passage. For instance, if the dryness sensor 3048 detects that the nasal passage on the first side is dry, the flow controller 3040 may command the actuator 3032 to move the piston towards the second side and/or to the second configuration so as to facilitate a majority or the entirety of the flow of respective gas to be conveyed to the second nare 9002B.
In one form of the present technology, the sensor(s) 3042 may include a feedback sensor 3050 which may be configured to detect the location of the piston 3022 within the chamber 3024. Data provided by the feedback sensor 3050 may be useful to determine the initial position of the piston 3022 as well as to obtain feedback over the accuracy of the actuator 3032.

#### 4.4.1.4 Second Embodiment of a Flow Regulator

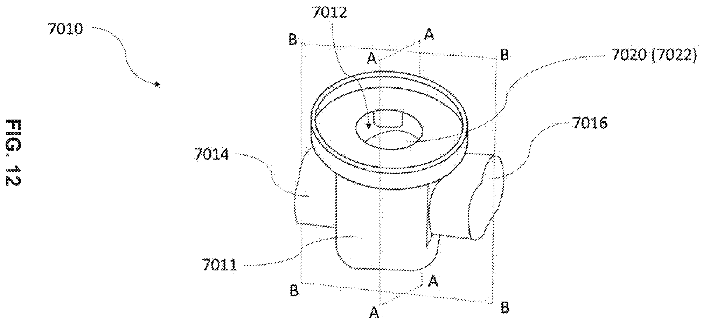

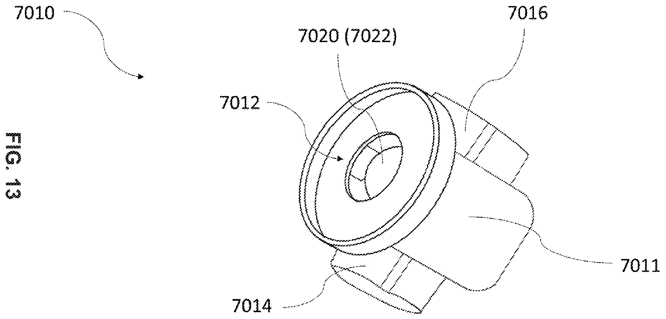

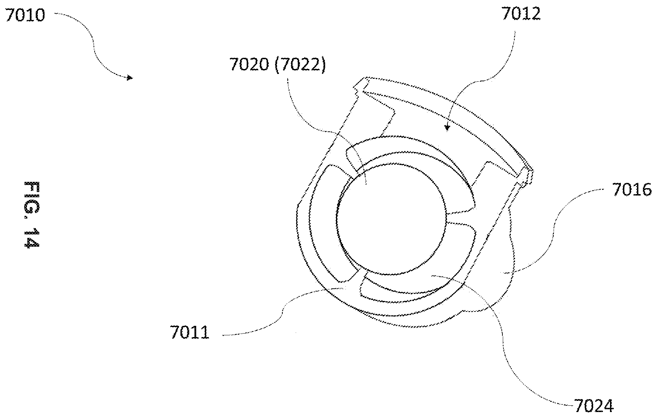

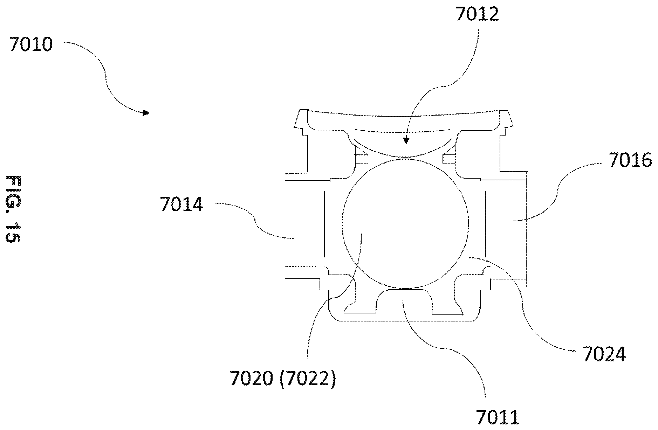

Referring now to Figs. 12 to 17 which show a second embodiment of a flow regulator 7010 in accordance with one form of the technology,
**[0532]** The flow regulator 7010 includes a body 7011 which defines a chamber 7024.
The body 7011 also includes an inlet indicated generally as 7012, a first outlet indicated generally as 7014, and a second outlet indicated generally as 7016.

The flow regulator 7010 includes an inlet connector 7013 which is in flow communication with the inlet 7012. The inlet connector 7013 is configured to facilitate attaching the flow regulator 7010 to a duct e.g. 4050 which connects to a supply of pressurised respiratory gas e.g. RPT device 4000.

The flow regulator 7010 includes a first outlet connector 7015 which is in flow communication with the first outlet 7014, and a second outlet connector 7017 which is in flow communication with the second outlet 7016. The first outlet connector 7015 and the second outlet connector 7017 are shaped to facilitate connecting to a first headgear tube 3302 and a second headgear tube 3304 respectively (neither shown in the Figs.). For instance, the first outlet connector 7015 and the second outlet connector 7017 may be shaped to facilitate a press fit of corresponding connector 7015, 7017 on a headgear tube 3302, 3304 as described herein.

The inlet 7012 is configured to facilitate a flow of respiratory gas flowing into the chamber 7024. The first outlet 7014 and the second outlet 7016 are configured to facilitate the flow of respiratory gas flowing out of the chamber 7024.

The flow regulator 7010 includes a barrier arrangement 7020 configured to selectively block the first outlet 7014 and the second outlet 7016. In the illustrated embodiment, the barrier arrangement 7020 is provided by a spherical shaped ball component 7022 which is provided in the chamber 7024. However, the barrier arrangement 7020 could have other forms e.g. it may be one or more flaps which are pivotally mounted to the flow regulator, a rotatable plate or a piston 3022.

The barrier arrangement 7020 is movable between a first configuration and a second configuration. In the first configuration the barrier arrangement 7020 substantially or completely blocks the second outlet 7016 while also leaving the first outlet 7014 sufficiently unblocked to allow the flow of respiratory gas to flow through the first outlet 7014.

In the second configuration, the barrier arrangement 7020 substantially or completely blocks the first outlet 7014 while also leaving the second outlet 7016 sufficiently unblocked to allow the flow of respiratory gas to flow through the second outlet 7016.

In the illustrated embodiment, the barrier arrangement 7020 (spherically shaped ball component 7022), the chamber 7024, the inlet 7012, the first outlet 7014 and the second outlet 7016 are shaped and dimensioned to facilitate control of the flow of respiratory gas through the flow regulator 7010 and also subsequently through headgear tubes 3302, 3304 of a positioning and stabilising structure 3300. For instance, the relative shape and dimensions of the components of the flow regulator 7010 may be selected to reduce impedance of flow through the headgear tubes 3302, 3304 e.g. if one of the headgear tubes 3302 or 3304 is partially or completely occluded in use.

The distance between the barrier arrangement 7020 and the inlet 7012 defines an inlet flow area. The distance between the barrier arrangement 7020 and the first outlet 7014 defines a first outlet flow area, and the distance between the barrier arrangement and the second outlet 7016 defines a second outlet flow area. In the first configuration the first outlet flow area is substantially equal to the inlet flow area while the second outlet flow area is substantially zero e.g. the second outlet 7016 is substantially or completely blocked. In the second configuration, the first outlet flow area is substantially zero e.g. the first outlet 7014 is substantially or completely blocked, while the second outlet area is substantially equal to the inlet flow area.

The headgear tubes 3302, 3304 can become blocked if a patient's orientation changes e.g. they roll onto, and sleep on, their side such as the foetus position shown in Fig. 10A. In the prior art, occlusion of the headgear tubes 3302, 3304 reduces the total area through which a flow of respiratory gas can flow. This occlusion can cause several problems, including a drop in pressure in a respiratory therapy treatment system between the patient interface 3102 and a RPT device 4000 e.g. through the inlet 7012. However, a flow regulator 7010 according to the present technology can mitigate or reduce these problems. For instance, the barrier arrangement 7020 of Figs. 12 to 17 is configured to ensure that the flow regulator 7010 has a substantially constant total flow area through the first outlet 7014 and second outlet 7016 i.e. the sum of the first outlet flow area and the second outlet flow area is substantially constant irrespective of the position of the barrier arrangement 7020 relative to the first inlet 7014 and the second inlet 7016. In addition, the sum of the first outlet flow area and the second outlet flow areas is substantially equal to the inlet flow area for any position of the barrier arrangement.

In addition, the barrier arrangement 7020 is movable to a third configuration, in which the first outlet 7014 and the second outlet 7016 are both partially blocked. In the third configuration, the sum of the first outlet flow area and the second outlet flow area is substantially equal to the inlet flow area i.e. the flow of respiratory gas is split into two equal portions, one of which flows through the first outlet 7014 and one of which flows through the second outlet 7016.

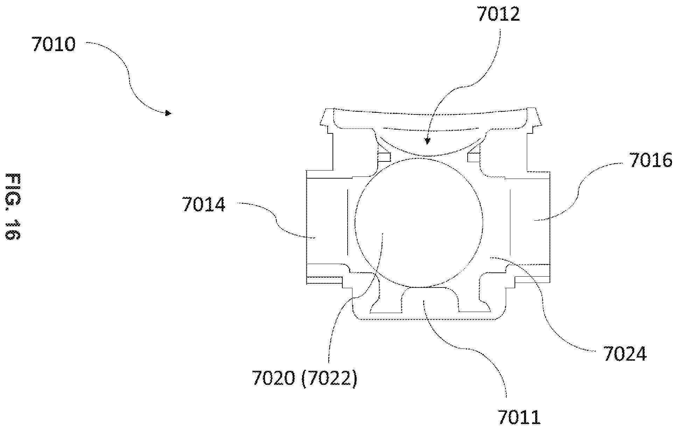

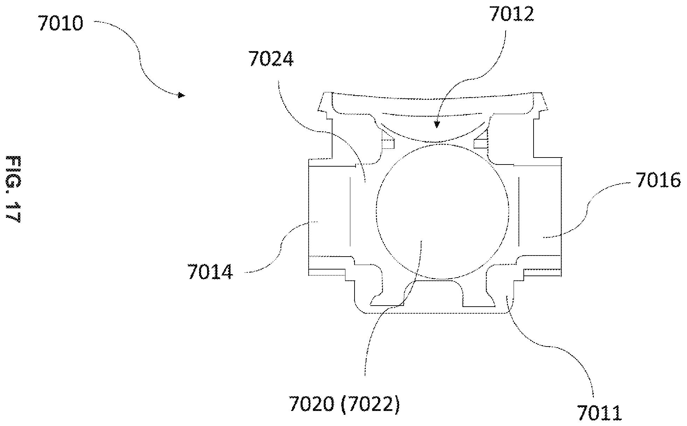

In the illustrated embodiment, the barrier arrangement 7020 is moved between the first configuration and the second configuration by gravity. For instance, as the flow regulator 7010 is tilted in a first direction to assume a first orientation the spherical ball component 7022 can move in the chamber 7024 to block the first outlet 7014; this configuration is shown in Fig. 16. When the flow regulator 7010 is tilted in a second direction to assume a second orientation the spherical ball component 7022 can move in the chamber 7024 to block the second outlet 7016; this position is shown in Fig. 17.

It should also be understood that other arrangements and mechanisms for movement of the barrier arrangement 7020 between the first configuration and the second configuration are envisaged. For instance, an actuator (not illustrated) may be provided to move the barrier arrangement 7020 between the first configuration and the second configuration.

It should be understood that the size of the first inlet flow area and the second inlet flow area changes depending on the position of the barrier arrangement 7020 in the chamber 7024.

The flow regulator 7010 of Figs. 12 to 17 may be connected to a patient interface system 3000 as illustrated in Figs. 4A to 4D.

As can be seen from Fig. 17, in the first configuration, at least a portion of the spherical ball component 7022 extends past the second outlet 7016. Therefore, the second outlet 7016 is not completely open or unblocked. However, the first outlet flow area in the configuration illustrated in Fig. 17 is substantially equal to the inlet flow area. As a result, the flow regulator 7010 can reduce or limit impedance in the respiratory treatment system 500 on occlusion of one of the headgear tube 3302, 3304.

Similarly, in the second configuration as illustrated in Fig. 16, the spherically shaped ball component 7022 substantially blocks the first outlet 7014. In addition, at least a portion of the spherically shaped ball component 7022 extends past the first outlet 7014. Therefore, the first outlet 7014 is not completed open or unblocked. However, the second outlet flow area is substantially equal to the inlet flow area. Again, as a result, the flow regulator 7010 can reduce or limit impedance in the respiratory treatment system 500 on occlusion of one of the headgear tube 3302, 3304.

The shape of the spherical ball component 7022 provides a gradual and relatively smooth transition as the barrier arrangement 7020 is moved from the first configuration to the second configuration. By selecting the shape of the barrier arrangement 7020 e.g. the radius of curvature of the spherical ball component 7022, the flow through the first outlet 7014 and the second outlet 7016 can be controlled as the flow regulator 7010 moves from the first configuration to the second configuration.

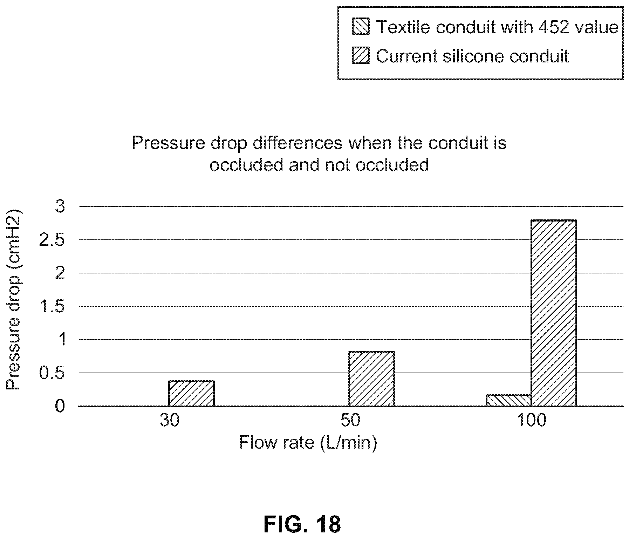

The flow regulator 7010 of the present technology can provide a number of advantages. For instance, the flow regulator 7010 can reduce pressure drop between the inlet 7012 to the flow regulator 7010 and a patient interface 3102 on occlusion of one of the headgear tubes 3302, 3304. As illustrated in Fig. 18, the pressure drop in the respiratory therapy treatment system 500 between the inlet 7012 and the patient interface 3102 at three flow rates across a range of flow rates used to provide respiratory therapy.

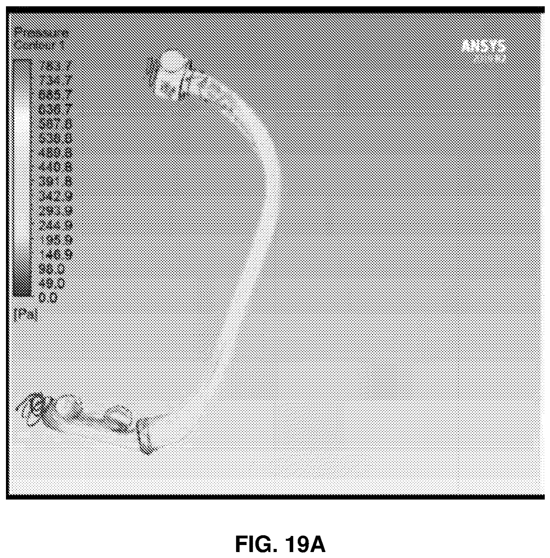

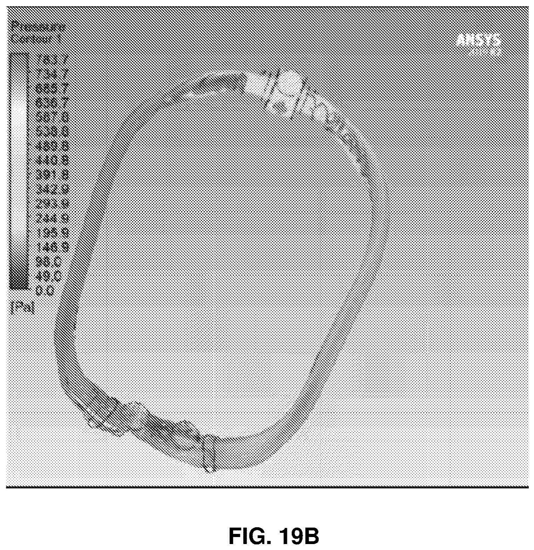

Referring now to Figs. 19A and 19B which show models of pressures through a respiratory treatment system 500 with a flow regulator 7010 when one of the headgear tubes 3302 or 3304 is not occluded. Fig. 19A shows that the pressure at the patient interface 3102 is not significantly different to the pressure at the inlet 7012 when one of the headgear tubes (not shown in Fig. 19A) is occluded. By comparison, Fig. 19B shows that the pressure in the patient interface 3102 is not significantly different to the pressure at the inlet 7012 when neither headgear tube 3302 or 3304 is occluded. It can be seen that the flow regulator 7010 reduces effective pressure differences between the inlet 7012 and the patient interface 3102 on occlusion of a headgear tube 3302 or 3304.

The present technology may provide other advantages. For instance, reducing pressure drop in the respiratory therapy treatment system 500 can improve detection of sleep apeneas experienced by a patient. This may be because the flow regulator 7010 reduces or substantially removes impedance in the respiratory therapy treatment system 500 due to occlusion of a headgear tube 3302 or 3304. However, the total flow rate from the inlet 7012 to the patient interface 3102 is not substantially reduced by occlusion. Therefore, the flow rate to the patient interface 3102 from the RPT device 4000 remains substantially constant even when one of the headgear tubes 3302 or 3304 is occluded.

In addition, the flow regulator 7010 may simplify determining impedance for a given conduit headgear treatment system 500, and reduce the number of impedance curves for masks. This could simplify determination of the correct therapy settings and therefore provisions of effective therapy to patients 9000.

Furthermore, in preferred forms, the use of gravity to move the flow regulator between configurations may provide a simple and cost-effective component to manufacture. This embodiment does not require actuators or sensors to determine when a headgear tube 3302 or 3304 is (or likely is) occluded.

### 4.4.2 Method to provide a flow of respiratory gas

In certain forms, the present technology comprises a method of providing a flow of respiratory gas to a patient 9000 through a positioning and stabilizing structure 3300 having a first headgear tube 3302 and a second headgear tube 3304.

In certain forms, the present technology comprises a method of providing a uniform flow of respiratory gas to a patient 9000.

In certain forms, the present technology comprises a method of mimicking the natural nasal cycle when providing a flow of respiratory gas to a patient 9000.

In one form of the present technology, the method comprises a step of proportioning the flow of respiratory gas into a first flow portion and a second flow portion.

In one form of the present technology, the method comprises a step of providing the first flow portion to the first nare 9002A and providing the second flow portion to the second nare 9002B.

In one form of the present technology, the method comprises a step of providing the first flow portion to the first headgear tube 3302 and providing the second flow portion to the second headgear tube 3304.

In one form of the present technology, the method comprises a step of locating the first headgear tube 3302 on a first side of the patient 9000 and the second headgear tube 3304 on a second side of the patient 9000 in use, wherein the first side and the second side are opposite to each other with respect to the central sagittal plane of the patient 9000.

In one form of the present technology, the method comprises a step of directing a majority of the flow of respiratory gas through the first headgear tube 3302 if the second headgear tube 3304 is compressed and/or otherwise occluded, and directing a majority of the flow of respiratory gas through the second headgear tube 3304 if the first headgear tube 3302 is compressed and/or otherwise occluded.

In one form of the present technology, the method comprises a step of conveying the entire flow of respiratory gas through the first headgear tube 3302 if the second headgear tube 3304 is compressed and/or otherwise occluded, and conveying the entire the flow of respiratory gas through the second headgear tube 3304 if the first headgear tube 3302 is compressed and/or otherwise occluded.

In one form of the present technology, the method comprises a step of alternately conveying a majority of and/or the entirety of the flow of respiratory gas between the first headgear tube 3302 and the second headgear tube 3304.

In one form of the present technology, the method comprises a step of alternately conveying a majority of and/or the entirety of the flow of respiratory gas between the first nare 9002A and the second nare 9002B.

In one form of the present technology, the method comprises a step of sensing orientation of the patient's head.

In one form of the present technology, the method comprises a step of proportioning the flow of respiratory gas into the first flow portion and the second flow portion based on the orientation of the patient's head. For instance, if the patient's head is tilted to and/or if the patient is lying on the first side, then the second flow portion may be adjusted to be greater than the first flow portion. Similarly, if the patient's head is tilted to and/or if the patient is lying on the second side, then the first flow portion may be adjusted to be greater than the second flow portion.

In one form of the present technology, the method comprises a step of sensing if the first headgear tube 3302 and/or second headgear tube 3304 is/are occluded and/or the extent of occlusion.

In one form of the present technology, the method comprises a step of proportioning the flow of respiratory gas into the first flow portion and the second flow portion based on the extent of occlusion of the first headgear tube 3302 and/or the second headgear tube 3304. For instance, if the first headgear tube 3302 is occluded, then the second flow portion may be adjusted to be greater than the first flow portion. The greater the occlusion of the first headgear tube 3302, the greater the second flow portion may be in comparison to the first flow portion.

In one form of the present technology, the method comprises a step of sensing if the first nasal passage and/or the second nasal passage are dry.

In one form of the present technology, the method comprises a step of proportioning the flow of respiratory gas into the first flow portion and the second flow portion based on detection of dryness in the first nasal passage and/or the second nasal passage. For instance, if the first nasal passage is detected as being dry, then the second flow portion may be adjusted to be greater than the first flow portion.

In one form of the present technology, the method comprises a step of sensing the position of a piston 3022 of a barrier arrangement 3020 which is configured to proportion the flow of respiratory gas into the first flow portion and the second flow portion.

In one form of the present technology, the method comprises a step of moving the piston 3022 within a chamber 3024 to adjust the proportion of the first flow portion and the second flow portion.

In one form of the present technology, the method comprises a step of moving the piston 3022 using gravity.

In an alternative form of the present technology, the method comprises a step of moving the piston 3022 using an actuator 3032.

In one form of the present technology, the method comprises a step of generating the flow of respiratory gas. The generated flow of respiratory gas preferably includes parameters like positive velocity and positive pressure.

In one form of the present technology, the method comprises a step of conveying the generated flow of respiratory gas to at least one of a flow regulator 3010 and the positioning and stabilizing structure 3300.

In one form, the present technology comprises a method for treating a respiratory disorder comprising applying positive pressure to the entrance of the airways of a patient 9000.

In certain examples of the present technology, a supply of respiratory gas at positive pressure is provided to the nasal passages of the patient 9000 via one or both nares 9002A, 9002B.

In certain examples of the present technology, mouth breathing is limited, restricted or prevented.

## 4.5 OTHER EXAMPLES OF THE PRESENT TECHNOLOGY

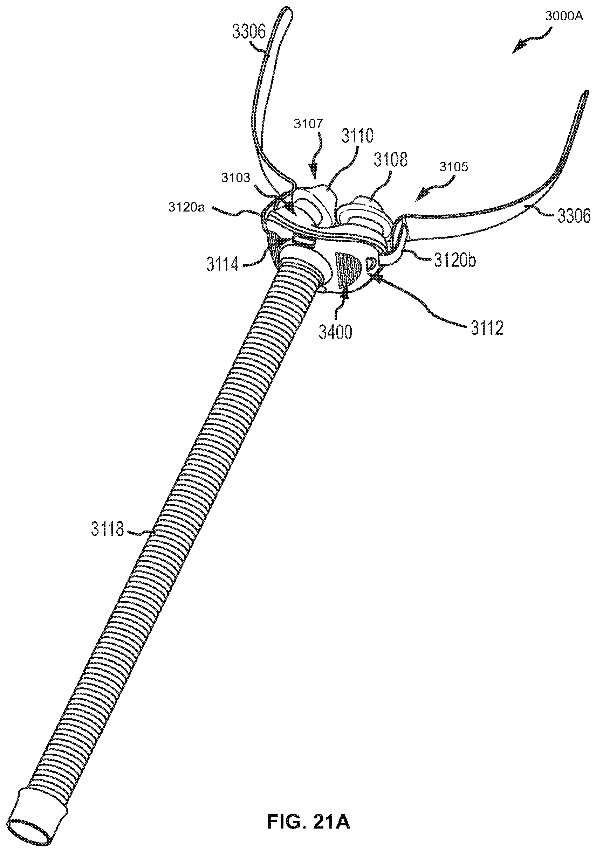

Referring now to Figures 21A to 24C which illustrate an embodiment of a patient interface 3000A according to another aspect of the present technology which is configured to deliver a supply of breathable gas to a patient 9000 during respiratory therapy.

In the illustrated examples, the patient interface 3000A comprises a first flow path 3105 and second flow path 3107, a cushion component 3103, a frame 3112, an air conduit 3118, a positioning and stabilising structure, and a vent arrangement.

### 4.5.1 Patient interface with flow regulator

In the illustrated examples, the patient interface 3000A is configured to selectively adjust the proportion of the supply of breathable gas that flows to the patient's first naris 9002A and to the patient's second naris 9002B. As illustrated, the patient interface 3000A comprises a flow regulator 3122.

#### 4.5.1.1 First and second flow paths

The patient interface 3000A comprises a first flow path 3105 configured to be, in use, in flow communication with a first naris 9002A of the patient 9000, and a second flow path 3107 configured to be, in use, in flow communication with a second naris 9002B of the patient.

In the illustrated examples, the patient interface 3000A is configured to selectively adjust the proportion of the supply of breathable gas that flows from the first flow path 3105 to the first naris 9002A and from the second flow path 3107 to the second naris 9002B.

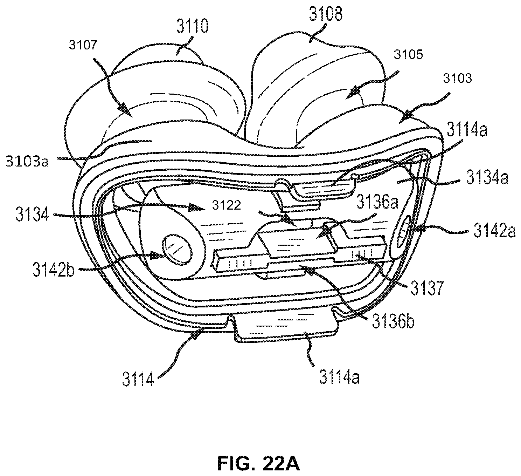

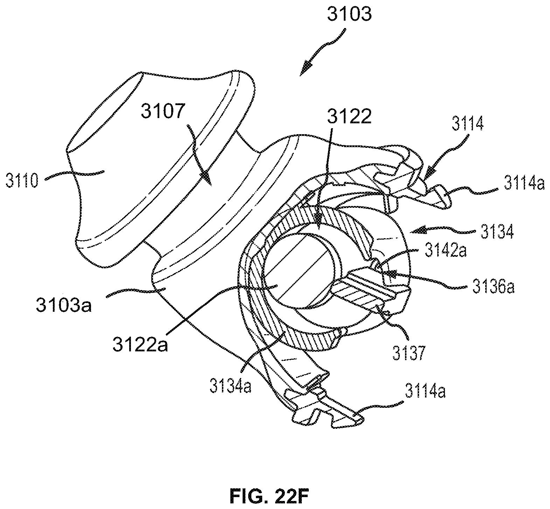

As illustrated, the first flow path 3105 is provided in a first conduit and the second flow path 3107 is provided in a second conduit. In the forms illustrated in Figs. 22A to 22C, the first and second conduits are nasal pillows. In an alternate form, the first and second conduits may each be a cannula or nozzle. Therefore, it is to be appreciated that the first and second conduits may either be sealed or unsealed with the patient's nasal airways. In yet another form, first and second flow paths may be provided as a pair of apertures formed in a seal-forming structure, e.g. a membrane, which align with a respective of the first naris 9002A and the second naris 9002B.

#### 4.5.1.2 Cushion component

The first flow path 3105 and the second flow path 3107 are preferably provided in one or more cushion components.

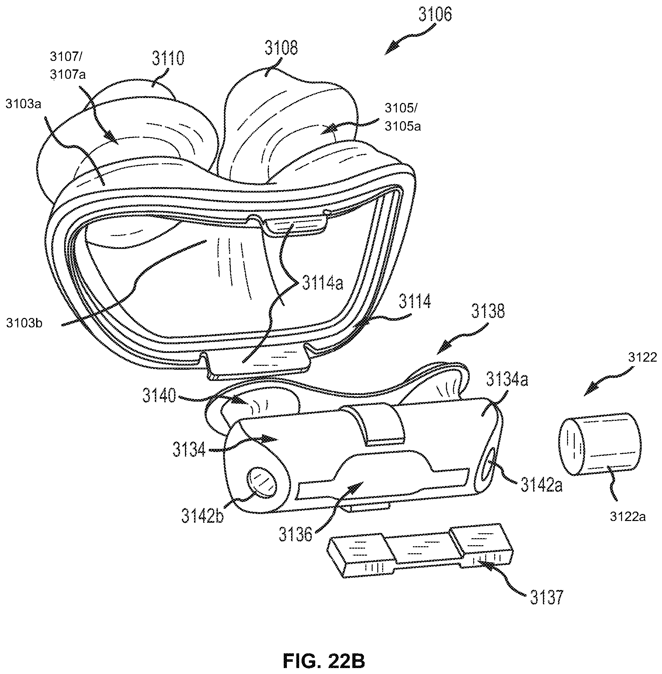

In the example illustrated in Figures 22A and 22B, the patient interface 3000A comprises a cushion 3103 which provides both of the first flow path 3105 and the second flow path 3107. In examples, the cushion 3103 comprises a body portion 3103a. In the example shown in Figs. 22A and 22B, the first conduit 3105 and the second conduit 3107, e.g. nasal pillows, are integrally moulded with, or to, the body portion 3103a.

However, in an alternate form, the patient interface 3000A may comprise a first discrete cushion (not illustrated) which provides a first conduit as described herein, and a second discrete cushion (not illustrated) which provides a second conduit as described herein.

In the illustrated examples, the cushion 3103 may be constructed from a biocompatible, soft, flexible and/or resilient material. For example, the cushion 3103 may be constructed from silicone, thermoplastic elastomer (TPE), or foam, or similar, e.g. liquid silicone rubber (LSR), or a biocompatible TPE.

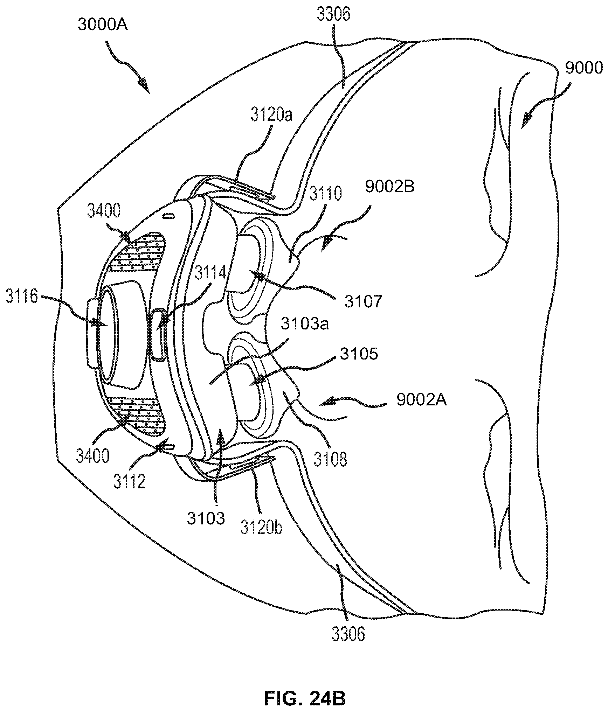

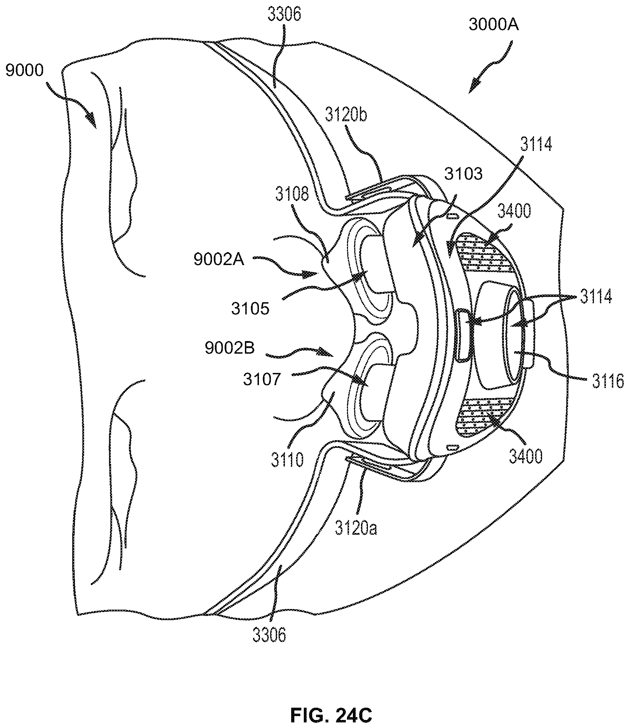

The first conduit 3105 comprises a first seal-forming structure 3108 and the second conduit 3107 comprises a second seal-forming structure 3110, which are each constructed and arranged to create a seal at or around the patient's first and second nares 9002A, 9002B. In use, the first and second seal-forming structures 3108, 3110 are configured to contact and form a seal with the alar of the patient's first and second nares 9002A, 9002B e.g. as illustrated in Fig. 24B and 24C.

In an alternate form, the first and second conduits 3105, 3107, do not create a seal at the patient's first naris 9002A and second naris 9002B, e.g. the first and second conduits 3100, 3102 are formed by a first and second cannula.

#### 4.5.1.3 Frame

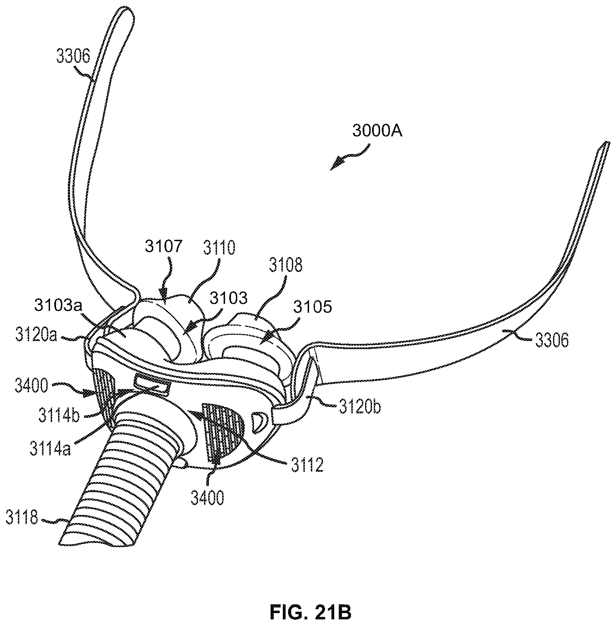

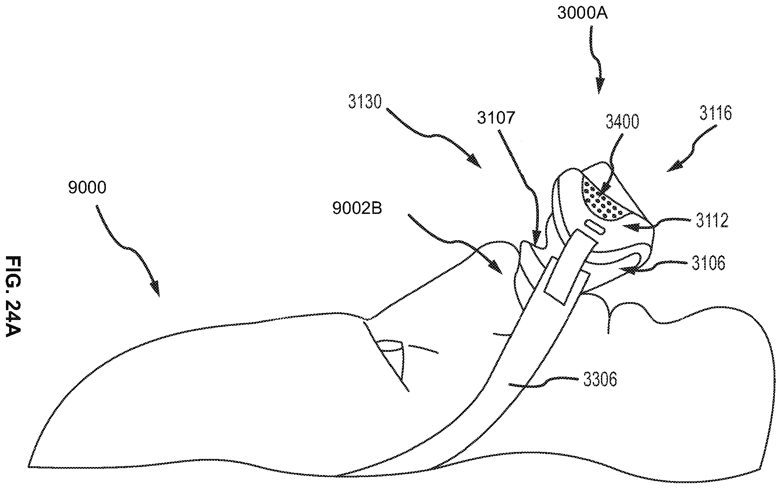

Referring to the example shown in Figs. 21A and 21B and 24A to 24C, the patient interface 3000A comprises a frame 3112. In examples, the cushion(s) 3103 is / are attached in use to the frame 3112. For example, the cushion 3103 is releasably attachable to the frame 3112. In other forms, the cushion 3103 and frame 3112 may be integrally formed, e.g. co-moulded or formed as a single unit. For example, the cushion 3103 and frame 3112 may be integrally formed from the same material.

In the illustrated examples, the frame 3112 is constructed from a more rigid material relative to the cushion material, e.g. a relatively rigid material such as polycarbonate or a flexible material having a greater stiffness or durometer hardness greater than the cushion material. In examples, the frame 3112 may be stiffened by areas of greater thickness and/or reinforcing structure(s).

### 4.5.1.4 Connection mechanism

In examples, the patient interface 3000A comprises a connection mechanism 3114 to facilitate attachment of the frame 3112 to the cushion 3103.

As shown in the example of Fig. 22A and 22B, the connection mechanism 3114 comprises one or more tabs 3114a configured to interlock with one or more corresponding slots or components 3114b on the frame 3112, e.g. the one or more tabs 3114a are provided on the cushion 3103 and the one or more corresponding slots or components 3114b are provided on the frame 3112. It should be appreciated that the tab(s) 3114a may alternatively be provided to the frame and the corresponding slot(s)/component(s) 3114b provided to the cushion 3103.

As illustrated, the connection mechanism 3114 is in the form of a cushion clip 3114 which provides the tabs 3114a. In the illustrated examples, the cushion clip 3114 is provided to the cushion 3103, e.g. to the body portion 3103a. The cushion 3103 is overmoulded to the cushion clip 3114 to form a one-piece component. It should be appreciated that the cushion clip 3114 may be provided to the cushion 3103 in other suitable ways or forms, e.g. it may be releasable attachable to the cushion 3103.

In the illustrated examples, the cushion clip 3114 is constructed from a material which is relatively more rigid than the material from which the rest of the cushion 3103 is constructed e.g. a polycarbonate or a flexible material having a greater stiffness or durometer hardness greater. In examples, the cushion 3103 may be stiffened by areas of greater thickness and/or reinforcing structure(s).

In forms, the connection mechanism 3114 may provide a snap-fit, interference fit or other suitable connecting or locking arrangement to facilitate attachment of the frame 3112 and the cushion 3106 together.

### 4.5.1.5 Air conduit

In embodiments of the present technology, the patient interface 3000A is structured to connect to the supply of breathable gas, as illustrated in Figs. 21A and 21B.

In examples, the patient interface 3000A comprises at least one opening 3116 configured to facilitate delivery of the supply of breathable gas into a breathing chamber formed by the frame 3112 and /or the cushion 3103. As shown in the example of Figs. 24A to 24C, the frame 3112 comprises the at least one opening 3116. In other forms, the cushion 3103 may comprise the at least one opening 3116.

In embodiments of the present technology, the patient interface 3000A comprises at least one air conduit 3118 configured to facilitate delivery of the supply of breathable gas into the breathing chamber. In the example illustrated in Figs. 21A and 21B, the at least one air conduit 3118 is provided to the frame 3112, e.g. the air conduit 3118 is in use attached to a portion of the frame which is structured about opening 3116.

In an alternate form, the at least one air conduit 3118 may be provided to the cushion 3103, e.g. the air conduit 3118 may be in use attached to a portion(s) of the cushion which is structured about the opening formed in the cushion 3103.

In examples, the at least one air conduit 3118 may be relatively short. In examples, the at least one air conduit 3118 may be extensible. In examples, the at least one air conduit 3118 may be configured to twist.

In embodiments of the present technology, the patient interface 3000A may comprise one or more decoupling mechanisms, e.g. a ball and socket-type joint, one of a swivel ring or swivel cuff , a gusset, a fold, a concertina section, an area of relatively lower stiffness, and an area of reduced thickness. The decoupling mechanism(s) may be provided to one or more of the cushion 3103, the frame 3112, and the at least one air conduit 3118. In examples, the decoupling mechanisms may be provided to the patient interface 3000A between the at least one air conduit 3118 and the cushion 3103 and/or frame 3112.

### 4.5.1.6 Positioning and stabilising structure

In embodiments of the present technology, the patient interface 3000A comprises a positioning and stabilising structure. In examples, the positioning and stabilising structure comprises a headgear assembly, which may comprise one or more headgear straps. As shown in Figs. 21A and 21B, the positioning and stabilising structure comprises headgear rigidisers 3306.

In embodiments of the present technology, the patient interface 3000A is configured to facilitate releasable attachment of the patient interface 3000A to a part of a positioning and stabilising structure, e.g. to the headgear rigidisers 3306 and/or headgear straps.

### 4.5.1.7 Headgear connector(s)

In the example illustrated in Figs. 21A, 21B and Figs. 24A to 24C, the patient interface 3000A comprises one or more headgear connectors, e.g. the frame 3112 comprises two connectors 3120a, 3120b, one on each lateral side of the frame 3112.

In an alternative form, the patient interface 3000A may comprise four headgear connectors, which may comprise a pair of upper connectors and a pair of lower connectors. In these alternate forms, the frame may comprise all four headgear connectors, or it may comprise two of the four connectors, and the cushion may comprise the other two connectors.

### 4.5.1.8 Vent arrangement

In embodiments of the present technology, the patient interface 3000A comprises a vent arrangement configured to facilitate washout of exhaled gases. In the embodiment illustrated, the vent arrangement may be formed by one or more vent holes 3400, preferably a plurality of vent holes 3400. The vent arrangement is provided on the frame 3112 in preferred forms, e.g. vent holes 3400 are located on each lateral side of frame 3112 as illustrated in Figs. 21A, 21B and 24A to 24C. It is to be appreciated that in alternate forms the vent arrangement may be provided on the cushion 3103, or an elbow assembly or other component of the patient interface 3000A.

### 4.5.1.9 Flow regulator

In embodiments of the present technology, the patient interface 3000A comprises a flow regulator 3122 configured to adjust the proportion of the supply of breathable gas that flows to the patient's first naris 9002A and to the patient's second naris 9002B.

In embodiments of the present technology, the flow regulator 3122 is configured to adjust airflow from the first flow path 3105 and the second flow path 3017 based on the orientation of the patient interface 3000A. For instance, the flow regulator 3122 is a gravity-assisted device in which gravity moves a barrier arrangement between configurations.

In examples, the barrier arrangement is provided to a chamber 3134, e.g. positioned in the chamber 3134.

#### *4.5.1.9.1 Barrier arrangement*

Referring to the example shown in Figs. 22A to 23C, the flow regulator 3122 comprises a barrier arrangement which selectively limits or prevents the supply of breathable gas flowing from one of the first flow path 3105 to the first naris 9002A or the second flow path 3107 to the second naris 9002B to thereby adjust the proportion of the flow of breathable gas flowing from the first flow path 3105 to the first naris 9002A and from the second flow path 3102 to the second naris 9002B. In the example shown in Figs 22A to 23C, the barrier arrangement comprises a barrier element 3122a, e.g. in the form of a piston.

The piston can have various shapes. However in the embodiment of Figs. 21A to 23C the piston has a cylindrical shape.

In an alternate form, the barrier arrangement may comprise more than one barrier element 3122a.

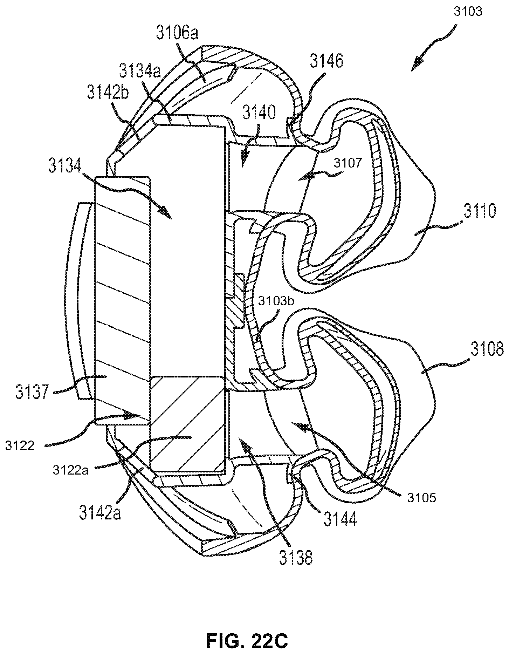

In examples, the barrier element 3122a is configured to move to a first position as shown in Fig. 22C in which it limits or prevents, e.g. substantially or completely prevents, the supply of breathable gas flowing from the first flow path 3105 to the first naris 9002A.

In examples, the barrier element 3122a is configured to move to the first position when the patient interface 3000A is in a first orientation. In these examples, gravity moves the barrier element 3122a to the first position e.g. when the patient is lying on their left-hand side, as shown in Fig. 24B.

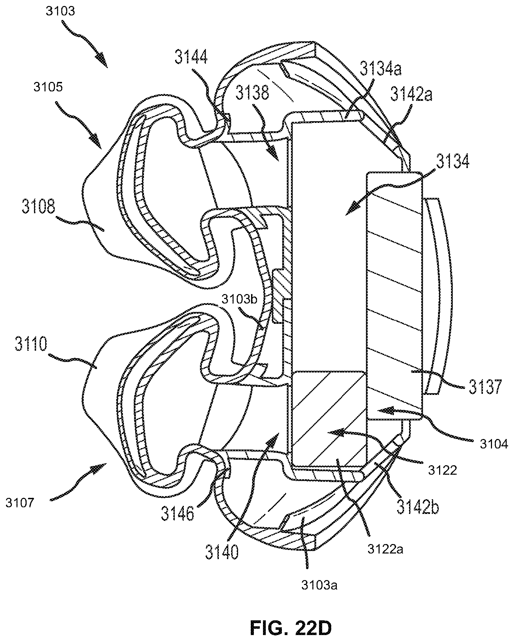

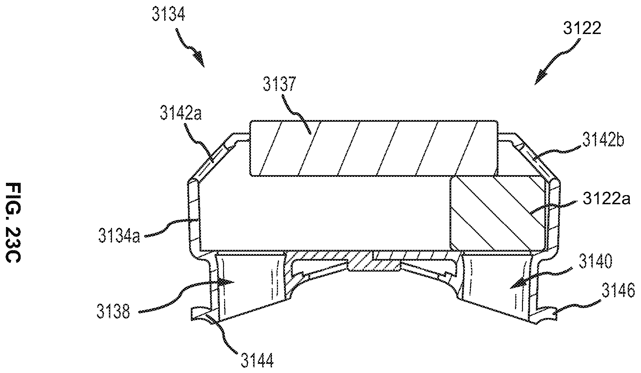

In examples, the barrier element 3104a is configured to move to a second position which is shown in Figs. 22D and 23C in which it limits or prevents, e.g. substantially or completely prevents, the supply of breathable gas flowing from the second flow path 3107 to the second naris 9002B.

In examples, the barrier element 3122a is configured to move to the second position when the patient interface 3000A is in a second orientation. In these examples, gravity moves the barrier element 3122a to the second position e.g. when the patient is lying on their right-hand side as shown in Fig. 24C.

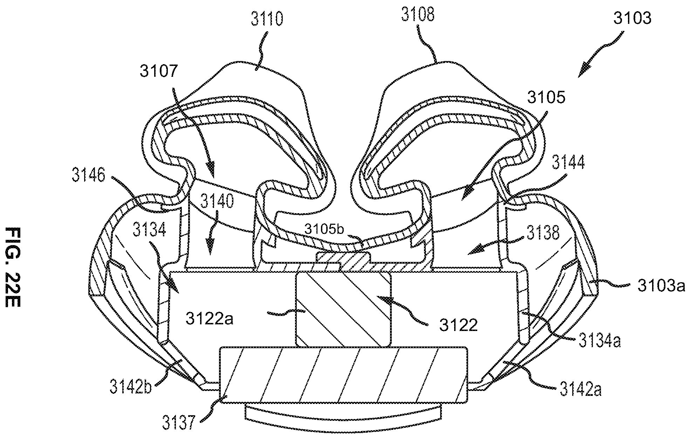

In examples, the barrier element 3122a is configured to move to a third position which is shown in Fig. 22E in which it substantially equal proportions of the supply of breathable gas can flow from each of the first flow path 3105 to the first naris 9002A and the second flow path 3107 to the second naris 9002B.

In examples, the barrier element 3122a is configured to move to the third position when the patient interface 3000A is in a third orientation. In these examples, gravity moves the barrier element 3122a to the third position. In preferred forms, the third position is when the patient may be lying in a supine position as shown in Fig. 24A.

#### *4.5.1.9.2 Chamber*

The chamber 3134 is formed by one or more walls 3134a. As illustrated in Figs. 22C to 22F and Fig. 23C, the barrier element 3104a is positioned in the chamber 3134.

In the illustrated examples, at least a portion of the one or more chamber walls 3134a is relatively rigid, e.g. at least a portion is made from a relatively rigid material, such as polycarbonate. In an alternate form, the chamber wall(s) 3134a may be constructed from a relatively softer, flexible material which is reinforced by structural features to ensure the shape of the chamber 3134 is relatively constant in use.

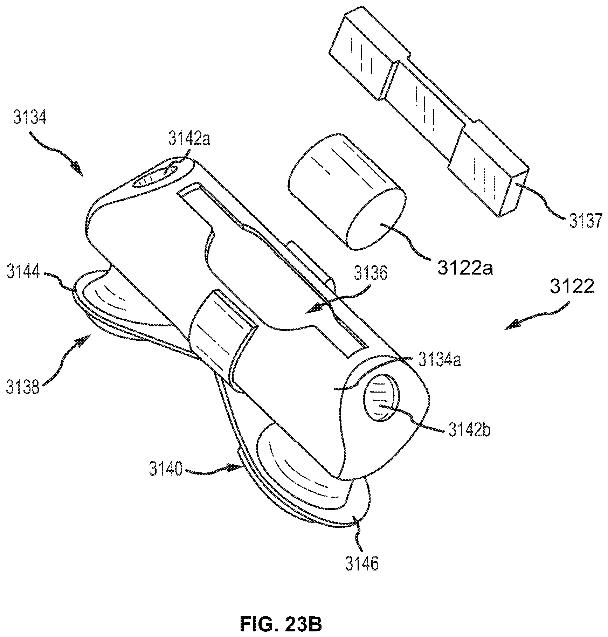

As can be seen in Figs. 22B and 23B, the chamber 3134 comprises at least one inlet 3136, a first outlet 3138 and a second outlet 3140. As illustrated in Fig. 22C, the first outlet 3138 is configured to be, in use, in flow communication with the first flow path 3105, and the second outlet 3140 is configured to be, in use, in flow communication with the second flow path 3107.

In examples, the chamber 3134 may be structured and arranged to permit removal of the barrier elements(s) 3122a from the chamber 3134. In examples, the at least one inlet 3136 is dimensioned to allow the barrier element(s) 3122a to be inserted into, and removed from, the chamber 3134. A retaining member 3317 can be provided to at least partially block the outlet 3136 to retain the barrier element(s) 3122a in the chamber 3134. In examples, the retaining member 3137 also assists with guiding movement of the barrier element(s) 3122a.

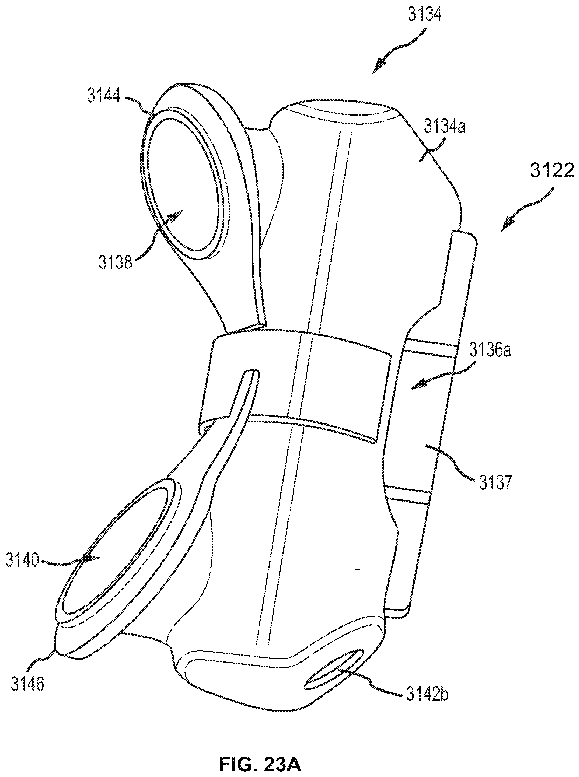

In examples, the retaining member 3137 is substantially rectangular shaped, but may be formed in any other suitable shape. As shown in the example of Figs. 22A, 22B, and Figs. 23A, 23B, the retaining member 3137 may comprise a narrowed region which may form part of, or increase the size of, the at least one inlet 3136, e.g. a narrowed central region which increases the size of inlets 3136a, 3136b.

In examples, as shown in Figs. 22A to 22E and Figs. 23A to 23C, the chamber 3134 comprises a vent structure constructed and arranged to facilitate washout of exhaled gases from the chamber 3134. In examples, the vent structure is in the form of a first vent pathway 3142a and a second vent pathway 3142b, e.g. one on each lateral side of the chamber 3134. The first vent pathway 3142a is positioned and configured to be in flow communication with the vent holes 3400 while second vent pathway 3142b is positioned and configured to be in flow communication with vent holes 3400.

In the illustrated examples, the barrier element 3122a is configured to move in the chamber 3134 to the first configuration, the second configuration and the third configuration.

In preferred forms, the chamber 3134 is cylindrical-shaped. In other examples, the chamber 3134 may be any other suitable shape which permits movement of the barrier element 3122a between the first, second and third configurations.

#### 4.5.1.10 Attachment of flow regulator to patient interface

In embodiments of the present technology, the flow regulator 3122 is provided to the patient interface 3000A.

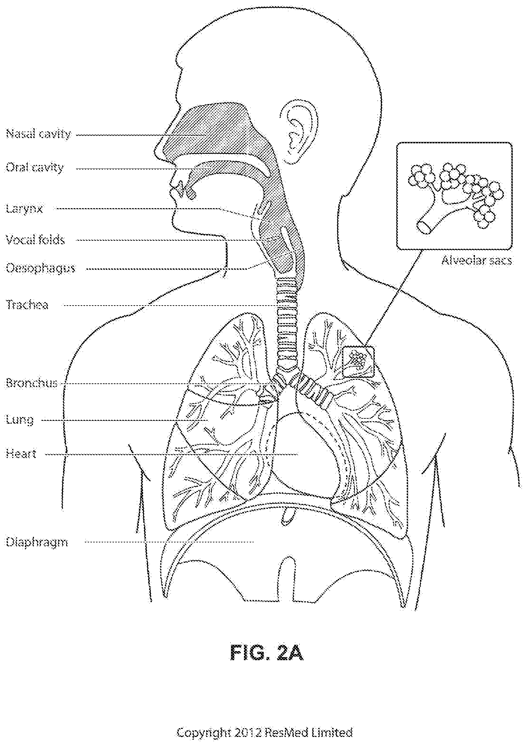

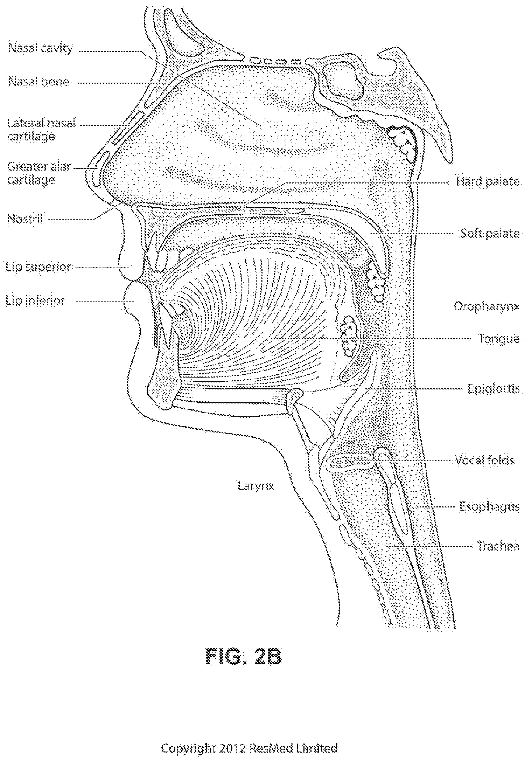

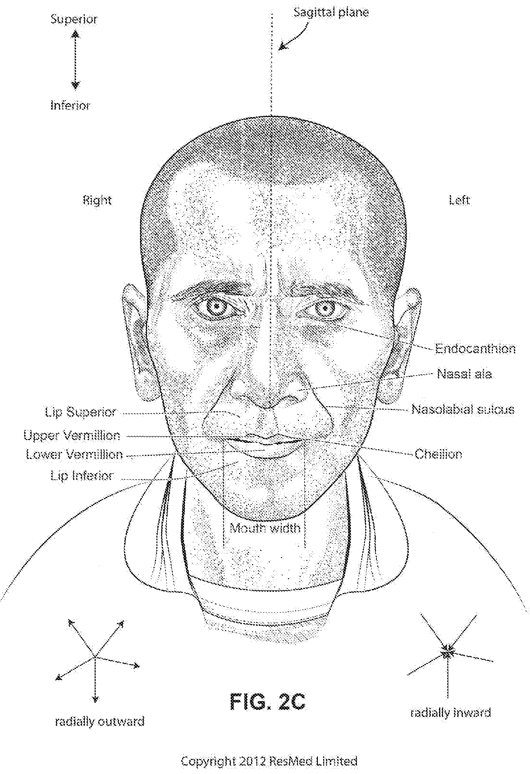

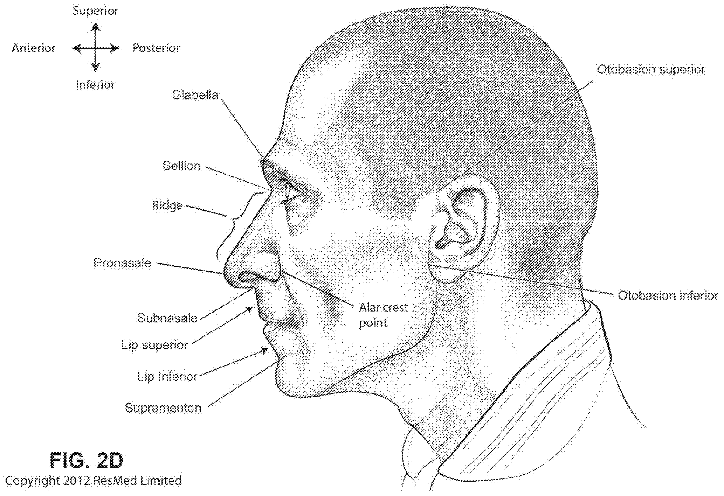

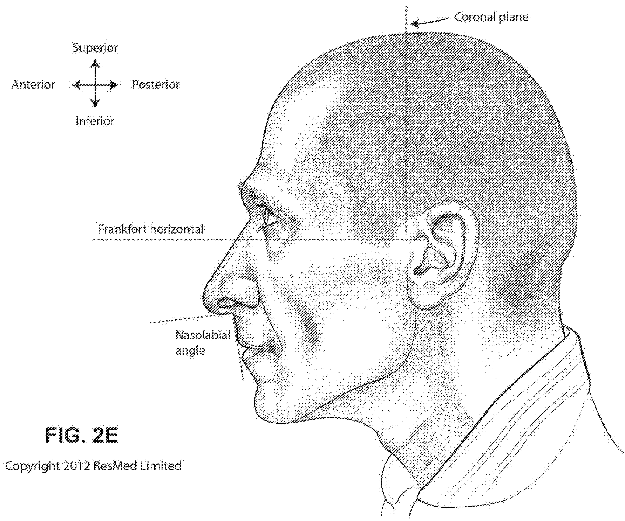

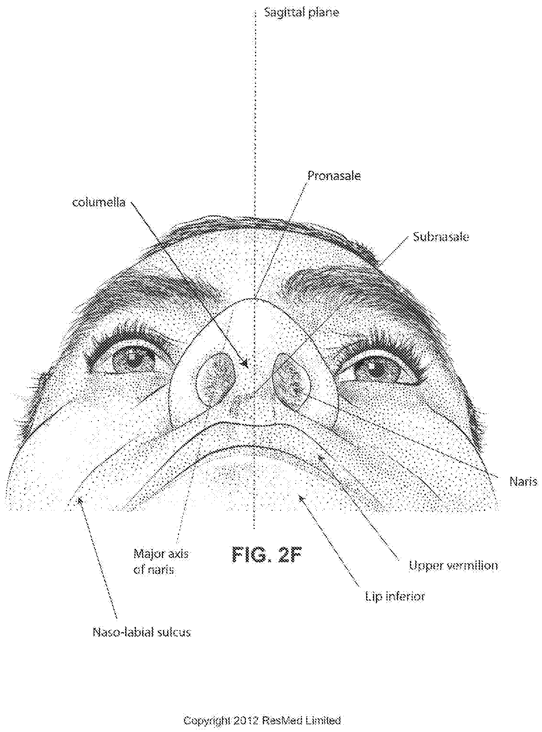

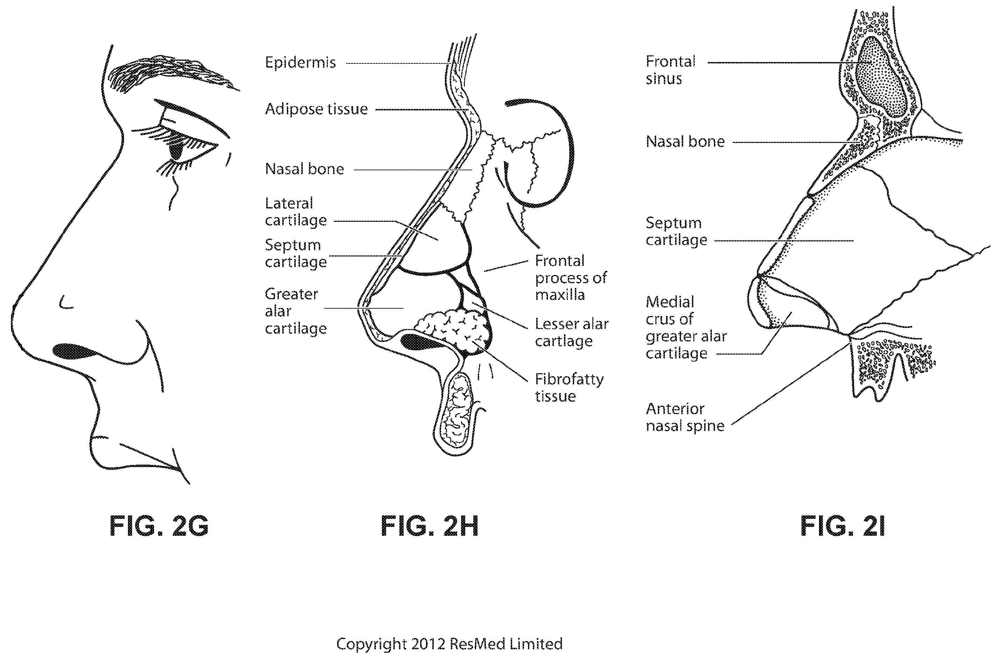

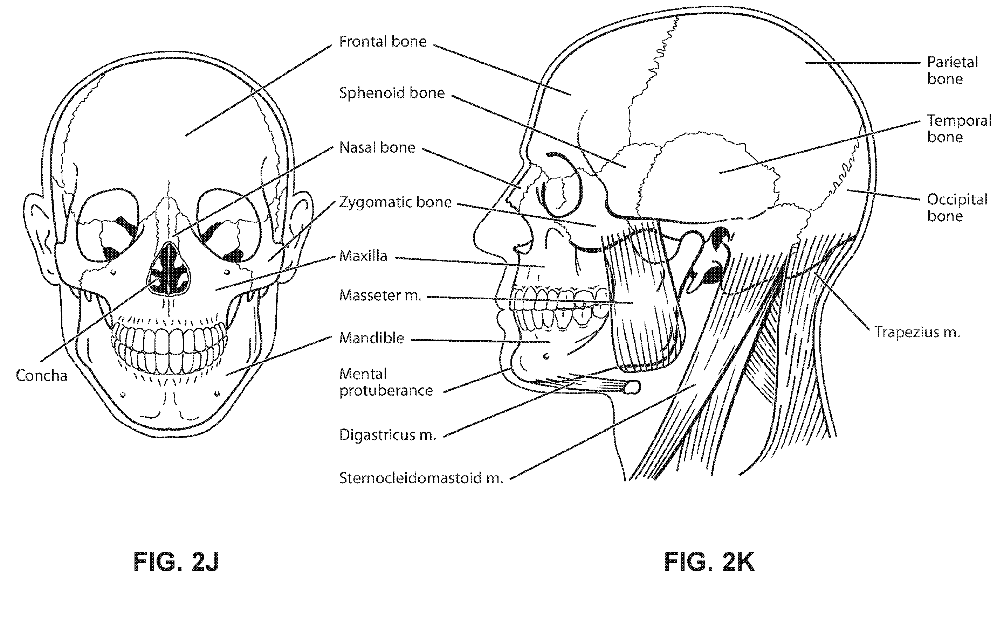

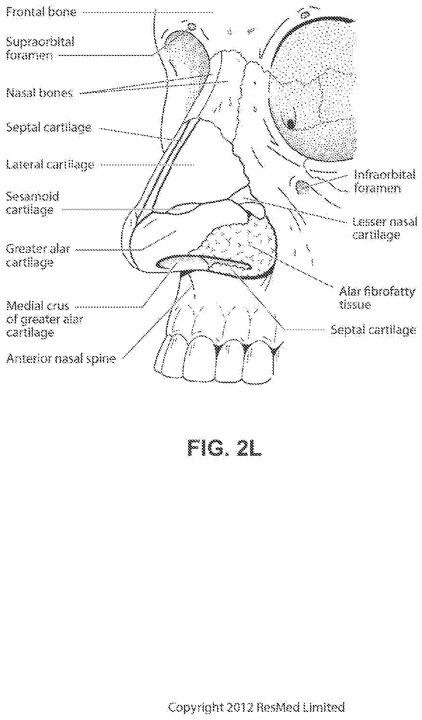

In the preferred form shown in Figs. 22A and 2B, the flow regulator 3122 is configured to be removably attached to the patient interface 3000A, e.g. the chamber 3134 is removably attached to the cushion 3103. It is to be appreciated that the patient interface 3000A may be configured to be suitable for use without the flow regulator 3104 during respiratory therapy.

In examples, the patient interface 3000A comprises a connection mechanism to facilitate attachment of the flow regulator 3122 to the patient interface 3000A, e.g. attachment of the chamber 3134 to a posterior interior surface 3103b of the cushion 3103 or attachment of the chamber 3134 to an interior surface of frame 3112. In examples, the connection mechanism may include locking tab(s) and corresponding slot(s) or component(s), a clip, a snap-fit, interference fit or other suitable connecting or locking arrangement.

In the example shown in Figs. 22A and 2B, the flow regulator 3122 is manufactured as a separate component which is attached in use to the patient interface 3000A. In this embodiment, the barrier arrangement is positioned in the chamber 3134 which is then provided to the cushion 3103. In other examples, the chamber 3134 may be provided to the frame 3112.

In examples, the first and second outlets 3138, 3140 are configured to in use create a seal with the first and second flow paths 3105, 3107. In the example shown in Figs. 22C to 22E, the first outlet 3138 comprises a first outlet seal-forming structure 3144 configured to contact and form a seal with an inner surface of the first flow path 3105, and the second outlet 3140 comprises a second outlet seal-forming structure 3146 configured to contact and form a seal with an inner surface of the second flow path 3107.

It should be appreciated that in other examples the first and second outlets 3138, 3140 may not create a seal with the first and second flow paths 3105, 3107. Therefore, a seal between the components may not be created, but the first and second outlets 3138, 3140 are still in flow communication with each of the first and second flow paths 3105, 3107.

In examples, the first and second outlets 3138, 3140 may protrude from the chamber wall(s) 3134a. As shown in the preferred forms of Figs. 22C to 22E and 24A to 24C, the first and second outlets 3138, 3140 protrude outwardly of the chamber.

In examples, the at least one inlet 3136 is configured to be in flow communication with the opening 3116. For example, inlet openings 3136a, 3136b illustrated in Fig. 22A, are configured to be associated or aligned in use with the frame opening 3116 as is illustrated in Fig. 24B. In examples, the at least one inlet opening 3136 and the frame opening 3116 may in use be located centrally on the patient interface 3000A.

#### 4.5.1.11 Alternate embodiment of a patient interface with a flow regulator

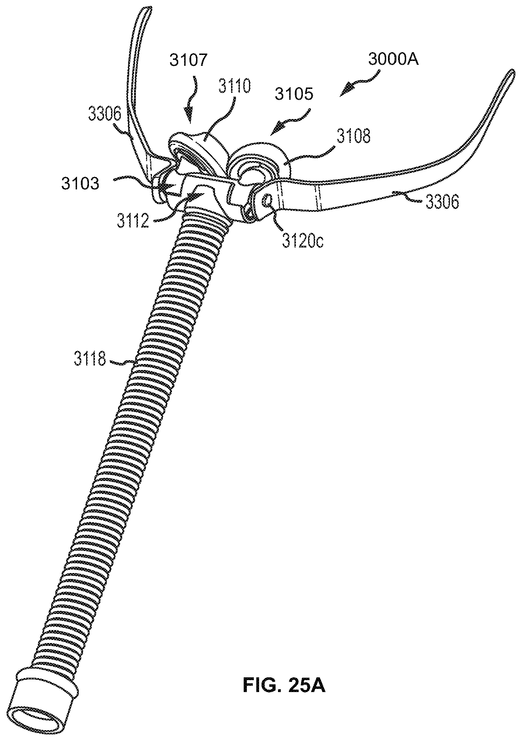

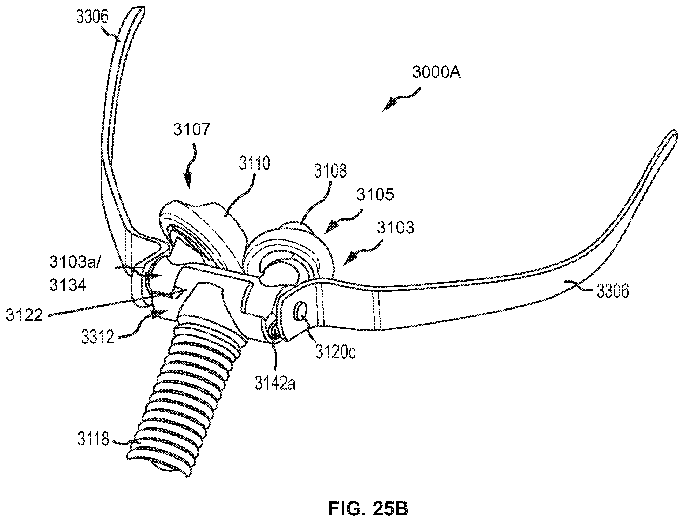

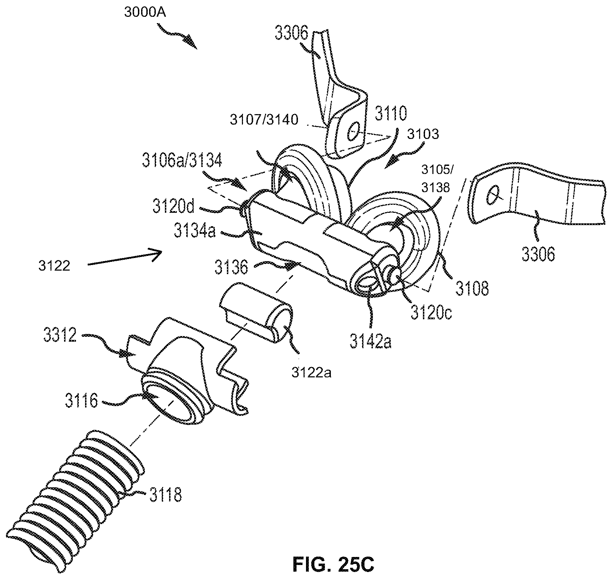

Referring now to Figs. 25A to 25J - which show an alternate embodiment of the present technology. Like references refer to like components.

In the embodiment of Figs. 25A to 25J, the chamber 3134 is formed in the cushion 3103, e.g. the chamber 3134 is provided by a cavity structure formed in the cushion 3103.

It should also be appreciated that in an alternate form, the chamber 3134 may be formed in the frame 3112, e.g. the chamber 3134 may be provided by a cavity structure formed in the frame 3112.

In examples, at least a portion of the cushion 3103, e.g. the cavity structure, is relatively rigid, or it is constructed from a relatively softer, flexible material and reinforced by structural features to ensure the shape is relatively constant in use.

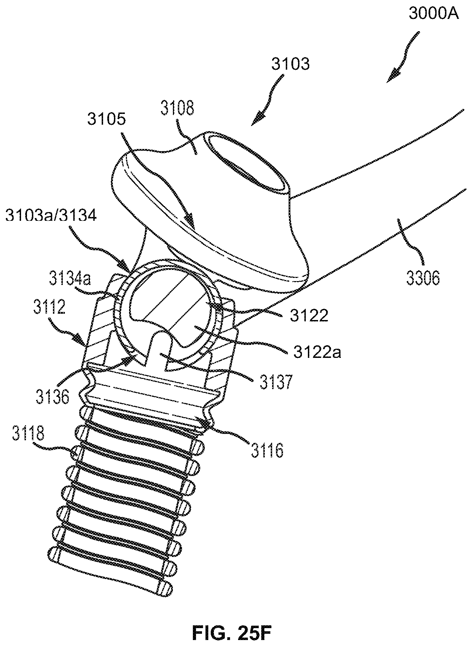

As shown in Figs. 25F to 25J, the barrier arrangement is provided in the cushion's body portion 3103a.

In the illustrated examples, the first and second outlets 3138, 3140 of the chamber 3134 are provided by the first and second flow paths 3105, 3107, e.g. nasal pillows.

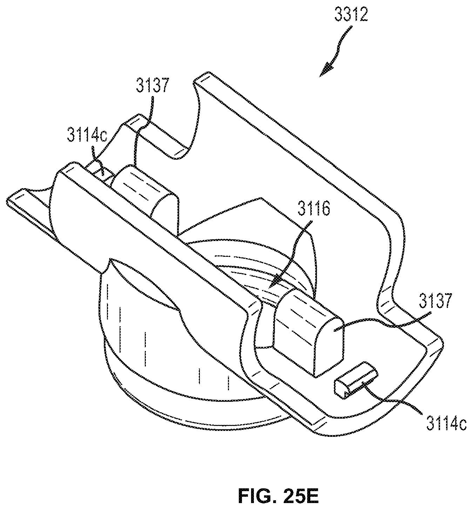

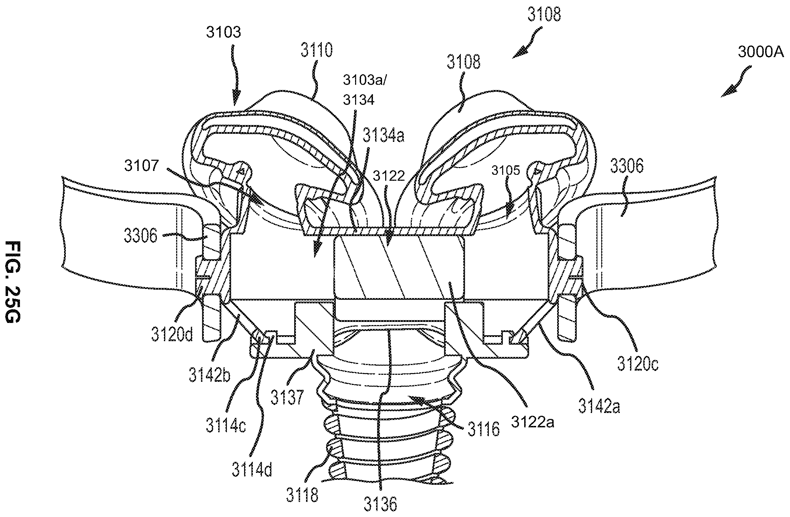

Referring to the example shown in Figs. 25E and 25G, the connection mechanism 3114 is in the form of one or more tabs 3114c configured on the frame 3112 which interlock with one or more corresponding components 3114d configured on the body portion 3103a.

In the example of Fig. 25E, the retaining member 3137 may be formed in the frame 3112.

It is to be appreciated that in an alternate form, the retaining member 3137 may be structured and arranged to form an additional or alternative connection mechanism to connection mechanism 3114.

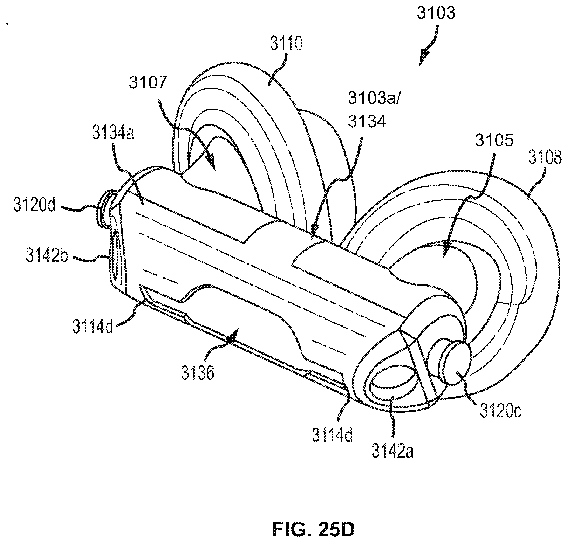

Referring to the example shown in Fig. 25D, the cushion 3103 may comprise the one or more headgear connectors, e.g. the body portion 3103a may comprise two headgear connectors 3120c, 3120d, one on each lateral side of the cushion 3103.

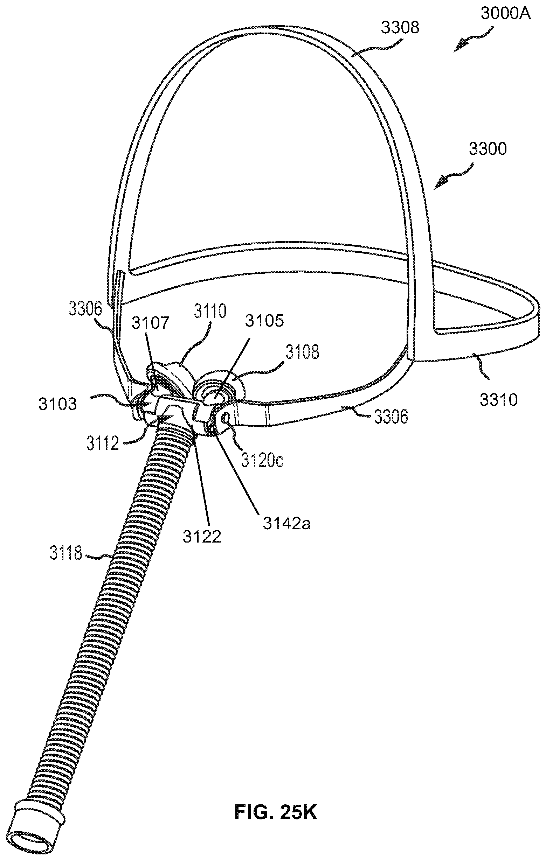

Fig. 25K illustrates an example of patient interface 3000A comprising positioning and stabilising structure 3300, e.g. a headgear assembly. In the example shown, the positioning and stabilising structure 3300 comprises a top headgear strap 3008 and a rear headgear strap 3310, and a pair of headgear rigidisers 3306.

In examples, the vent structure described herein may form the vent arrangement. In the example illustrated, the first vent pathway 3142a and the second vent pathway 3142b act as the vent holes 3400.

It may also be appreciated that in an alternate form, the cushion 3103 of the embodiment illustrated in Figs. 25A to 25J may be used or adapted for use with the cushion 3103 of the embodiment illustrated in Figs. 21A to 24C.

#### 4.5.2 Respiratory therapy treatment

Embodiments of the present technology comprise a method of mimicking native nasal cycling when administering respiratory therapy to a patient using a supply of breathable gas.

In examples, the method comprises the steps of:

(a) diverting a relatively larger proportion of the supply of breathable gas flowing from a first flow path 3105 to a first naris 9002A of the patient and diverting a relatively smaller proportion of the supply of breathable gas flowing from a second flow path 3107 to a second naris 9002B of the patient; and

(b) subsequently diverting a relatively higher proportion of the supply of breathable gas flowing from the second flow path 3107 to the patient's second naris 9002A and a relatively lower proportion of the supply of breathable gas flowing from the first flow path 3105 to the first naris 9002B.

In examples, steps (a) and (b) are provided by the flow regulator 3122.

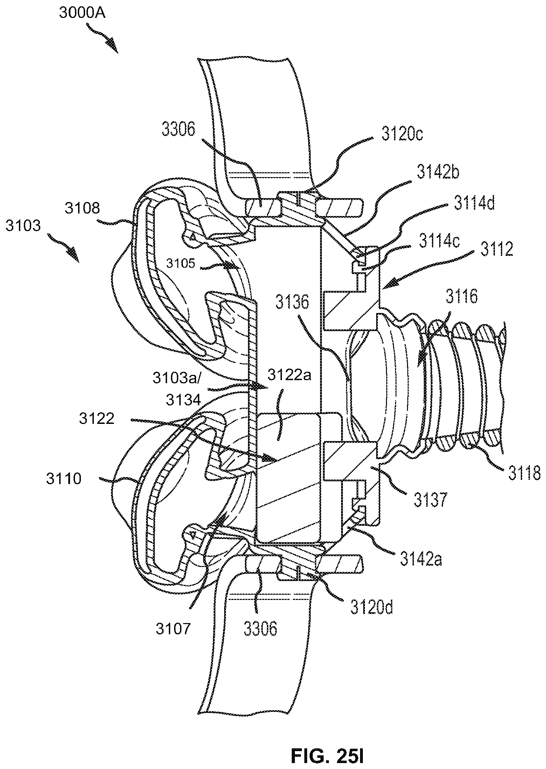

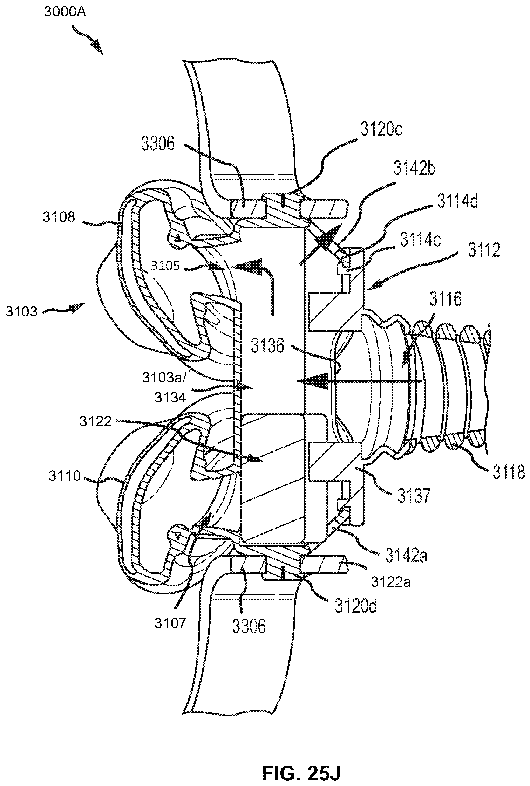

In examples, step (a) is provided when the barrier arrangement is in the second configuration as shown in the examples of Figs. 22D and Figs. 25I and 25J.

In preferred forms, step (a) occurs when the patient interface 3000A is in the second orientation as shown in the examples of Figs. 24C and Figs. 25I and 25J.

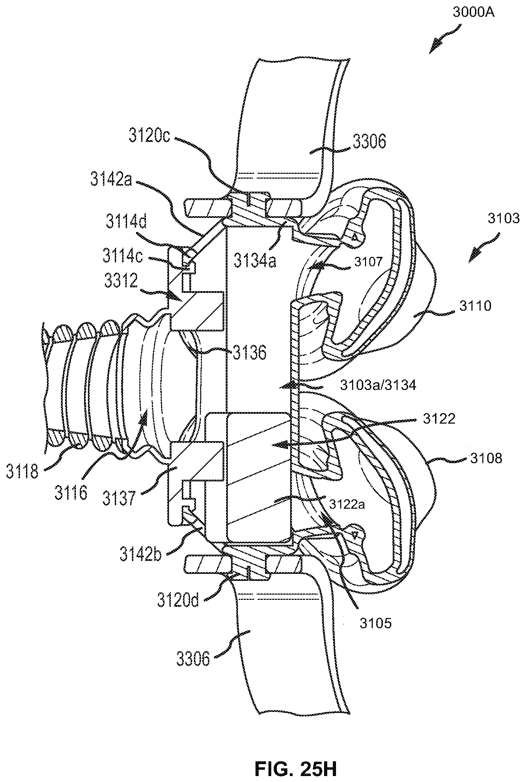

In examples, step (b) is provided when the barrier arrangement is in the first configuration as shown in the examples of Figs. 22C and 25H.

In preferred forms, step (b) occurs when the patient interface 3000A is in the first orientation as shown in the examples of Figs. 24B and 25H.

**4.5.3 System for controlling the flow of breathable gas**

Embodiments of the present technology comprise a system 6000 for controlling the flow of breathable gas when a patient uses a patient interface to deliver a supply of breathable gas to the patient. In examples, the system 6000 may be configured to mimic native nasal cycling.

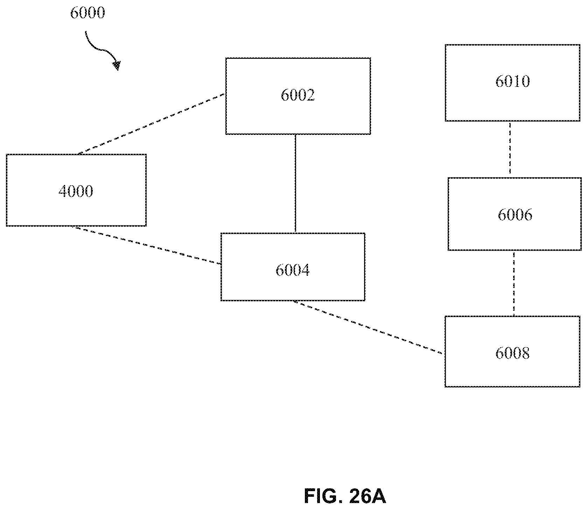

Referring to the example shown in Fig. 26A, the system 6000 comprises a patient interface 6002 which comprises a first flow path 6002a configured to be, in use, in flow communication with a first naris of the patient and a second flow path 6002b configured to be, in use, in flow communication with a second naris of the patient. In preferred forms, the patient interface 6002 may be the patient interface 3000A.

As shown in example of Fig. 26A, the system 6000 may comprise an apparatus configured to provide a supply of breathable gas to the patient interface 6002, e.g. the apparatus may be in the form of RTP device 4000 described herein.

In the form illustrated in Fig. 26A, the system 6000 comprises a flow regulator 6004 configured to adjust the proportion of the supply of breathable gas that flows from the first flow path 6002a to the patient's first naris and from the second flow path 6002b to the patient's second naris.

In examples, the flow regulator 6004 may comprise a barrier arrangement 6004a. In these examples, the barrier arrangement 6004a may comprise at least one barrier element, e.g. in the form of a flap 6004a as illustrated in the example of Fig. 8B.

In examples, the flow regulator 6004 is provided to the patient interface 6002. In an alternate form, the flow regulator 6004 may be provided to any other suitable part of the apparatus 4000.

**4.5.3.1** Actuator(s)

Referring to the example shown in Fig. 26A, the system 6000 may comprise at least one actuator 6008 configured to control the flow regulator 6004.

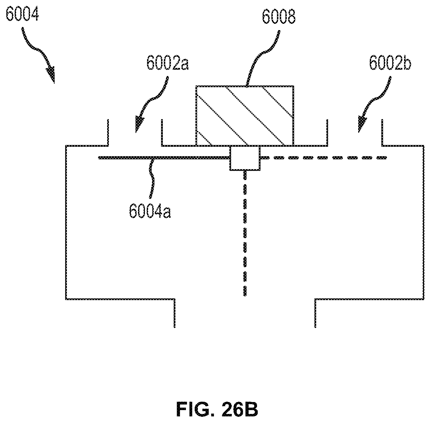

In the example illustrated in Fig. 26B, the actuator 6008 is configured to move the barrier arrangement 6004a to a first configuration as illustrated in Fig. 8B, in which it limits or prevents the supply of breathable gas flowing from the first flow path 6002a to the first naris.

In examples, the actuator 6008 is configured to move the barrier arrangement 6004a to a second configuration (which is illustrated by the right broken line in Fig. 26B) in which it limits or prevents the supply of breathable gas flowing from the second flow path 6002b to the second naris.

In examples, the actuator 6008 is configured to move the barrier arrangement 6004a to a third configuration (which is illustrated by the central broken line in Fig. 26B) in which it permits substantially equal proportions of the supply of breathable gas flowing from each of the first flow path 6002a to the first naris and the second flow path 6002b to the second naris.

**4.5.3.2 Sensor(s)**

In the example of Fig. 26A, the system 6000 may comprises one or more sensors 6010. In examples, the sensor(s) 6010 may be remotely connected to the system 6000. In other examples, the sensor(s) 6010 may be provided to the patient interface 6002.

In examples, the sensor(s) 6010 are configured to generate one or more signals indicative of one or more predetermined conditions, e.g. one or more of time, flow rate, pressure, and temperature.

In examples, the system comprises a controller 6006 configured to generate one or more control signals in response to the sensor signal(s). In examples, the actuator 6008 is configured to move the barrier arrangement 6004a between configurations in response to the control signal(s).

In examples, the controller 6006 comprises a processor configured to receive and process the sensor signal(s) and generate the control signal(s)

In examples, the sensor(s) 6010 comprise one or more of a flow rate sensor and a pressure sensor 6010. In examples, the sensor signal(s) generated by the flow rate and/or pressure sensor 6010 is indicative of a resistance to a supply of breathable gas flowing from one of the first and second flow path 6002a, 6002b to the first and second nares, which is indicative of the start of a natural congestion occurrence in one of the first or second nares, which is indicative of the start of a natural alternation of the flow of breathable gas through one of the first and second nares to the other one of the first and second nares.

In examples, a first sensor is provided to the patient interface proximate the first naris, and is configured to generate a sensor signal(s) indicative of one or more of the flow rate, pressure and temperature of the supply of breathable gas flowing from the first flow path to the first naris. A second sensor is provided to the patient interface proximate the second naris and configured to generate a sensor signal(s) indicative one or more of the flow rate, pressure and temperature of the supply of breathable gas flowing from the second flow path to the second naris.

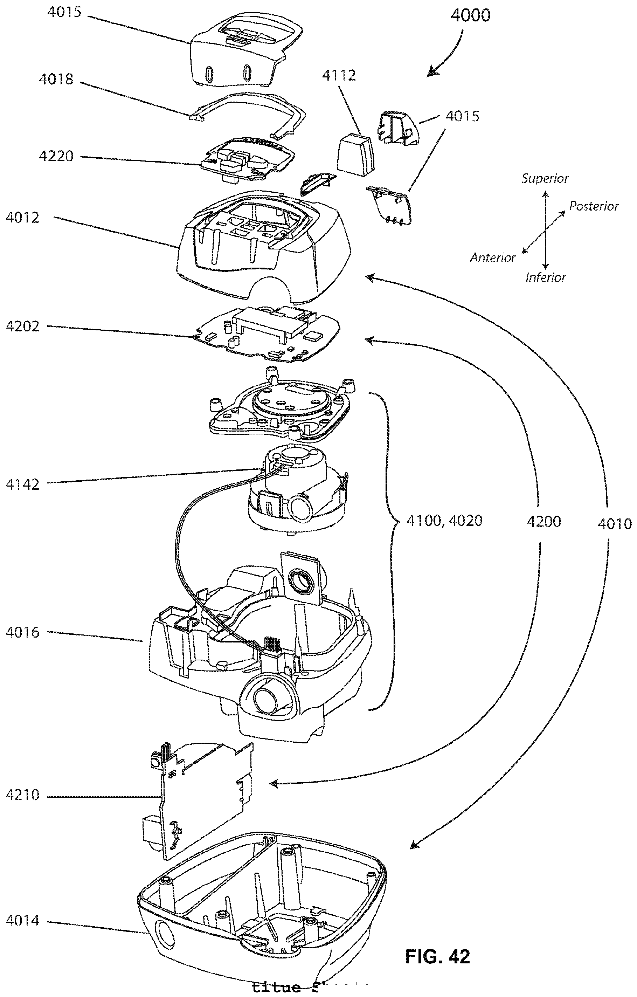

In examples, the first sensor and second sensor may send the sensor signal(s) to RPT device which comprises a pressure generator e.g. RPT device 4000 illustrated in Fig. 42. The RPT device 4000 may be configured to correlate the sensor signal(s) generated by the other sensor(s) to determine resistance to the supply of breathable gas to determine parameters of a patient's sleep profile e.g. to identify a sleep event such as one or more of natural alteration as described above, a partially / entirely blocked naris or nares, or proneness to apneas.

In examples, data related to the sleep event(s) may be generated and recorded and which may be used for screening, diagnosis, monitoring, amelioration, treatment or prevention of a respiratory disorder, or to improve the compliance of patients with respiratory therapy.

4.6 OTHER EXAMPLES OF THE PRESENT TECHNOLOGY

## 4.6.1 Conduit

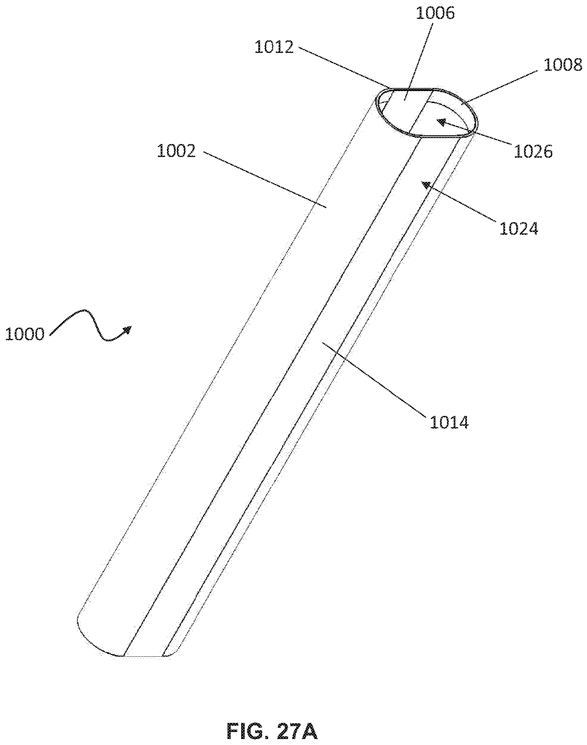

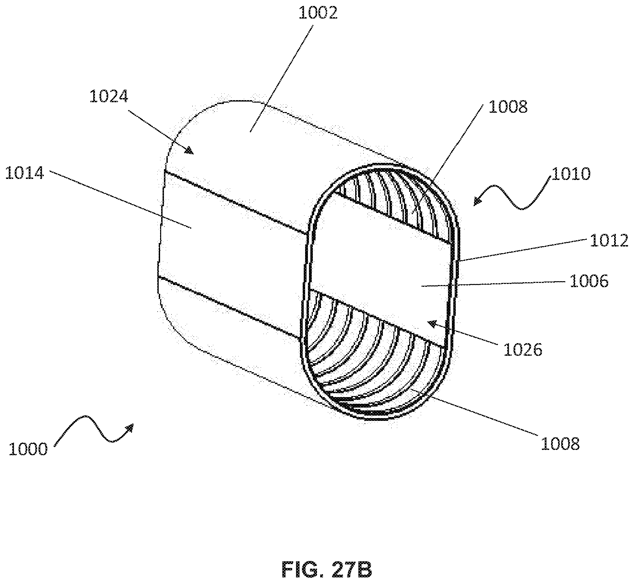

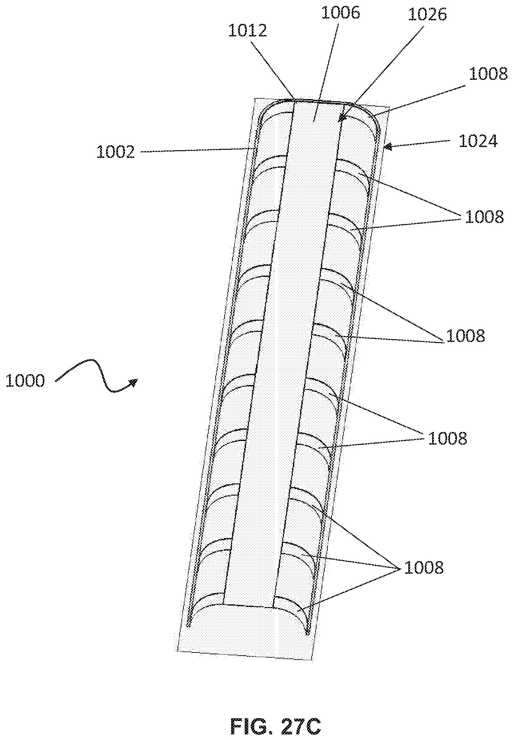

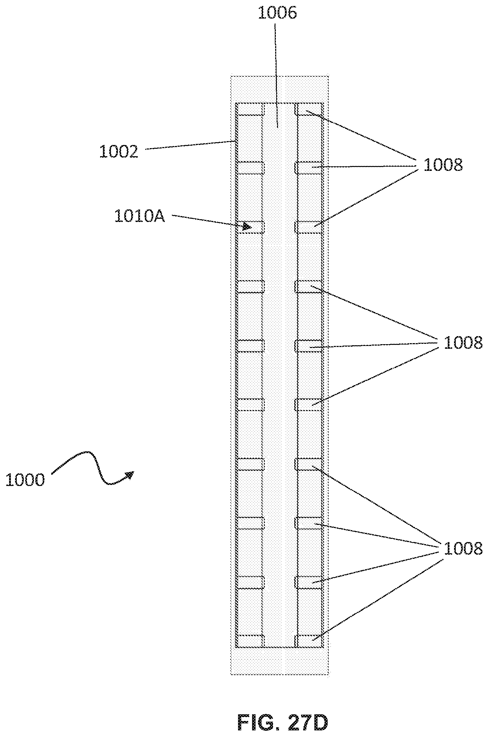

Referring to Figs. 27A to 27D and Figs. 29A to 30 which show various views of a conduit 1000 according to other aspects of the present technology. The conduit 1000 includes a casing 1002 and a support structure 1004.

### 4.6.1.1 Casing

The casing 1002 may be gas impermeable and defines a pathway to deliver a flow of respiratory gas between two components of a respiratory therapy system.

The casing 1002 may comprise at least one layer. For example, the casing 1002 may be formed from a first layer of material, e.g. an outer layer.

The casing 1002 may be gas and/or liquid impermeable. At least one of the layers may be gas and/or liquid impermeable. As a result, the casing 1002 provides a pathway through which in use pressurised breathable gases can flow.

The casing 1002 may be soft, flexible. At least one of the layers may be soft, flexible e.g. the layer(s) comprise a substantially soft, flexible material. For example, the outer layer may comprise a textile material that may be a woven or nonwoven textile, a knitted textile or other material. This may provide a soft feel to the conduit 1000 which can improve patient comfort when the conduit 1000 is positioned on the patient's head and/or face.

The casing 1002 may be cushioned or padded. At least one of the layers may comprise a cushioning material. This may improve patient comfort when the conduit 1000 is positioned on the patient's head and/or face.

The casing 1002 may be breathable. At least one of the layers may be breathable. For example, the textile material may be breathable. This may help with moisture wicking.

The casing 1002 may stretch in one or more directions. At least one of the layers may be stretchable in one or more directions, e.g. the layer(s) may comprise an elastic material. For example, the outer layer may comprise an elastic textile material. This may allow the casing 1002 to stretch in one or more directions. The benefits of this arrangement will be expanded on further below.

The casing 1002 may comprise a multi-layer structure. The casing 1002 may comprise the first layer and at least one other layer, e.g. a second layer. For example, the outer layer may be coated with a layer of coating material to create the multi-layer structure.

The second layer can make the conduit substantially gas impermeable e.g. to air or other respiratory gas used in provision of respiratory therapy. For example, the coating material may be substantially gas impermeable.

The second layer may be provided to an inner surface of the first layer. For example, the layer of coating material may be applied onto the inner surface of the outer layer which may be formed from a textile material. This can help provide a conduit 1000 with a soft feel that is comfortable for a patient to wear.

The coating material can be a relatively soft, flexible material, e.g. the coating material may comprise a thermoplastic material such as polyurethane (TPU), or a rubber material.

In some forms, the casing 1002 may be formed from, or include a coating layer of, an impermeable material, e.g. at least one of silicone, a polyurethane laminate, GORE-TEX®, knitted polyester or nylon fabric.

The casing 1002 may be releasably or permanently attached to the support structure 1004. In one example, the casing 1002 may be overmoulded or co-moulded with the support structure 1004. In another example, the casing 1002 may be formed as a sleeve, e.g. a textile sleeve or a sleeve made from an elastic material such as rubber.

The casing 1002 may be attached to the support structure 1004, e.g. using one or more bonding methods well known to one skilled in the art. For example, the casing 1002 may be made of a thermoformable material, e.g. a thermoformable plastic or rubber, and may be positioned over the support structure 1004 and heat applied to shrink the casing 1002 onto the support structure 1004 to secure the two components together.

The casing 1002 may be configured so that it can be releasably attached to the support structure 1004. For example, the casing 1002 can be positioned over the support structure 1004 and removed as desired. This may facilitate cleaning of conduit 1000 and its components, which may improve patient hygiene.

In other forms (not illustrated in the figures), the conduit may comprise a vent structure, e.g. a plurality of vent holes. to allow the washout of gas from inside the conduit to ambient. The casing may comprise the vent structure. For example, the casing may comprise one or more through holes to function as the vent holes.

### 4.6.1.2 Support structure

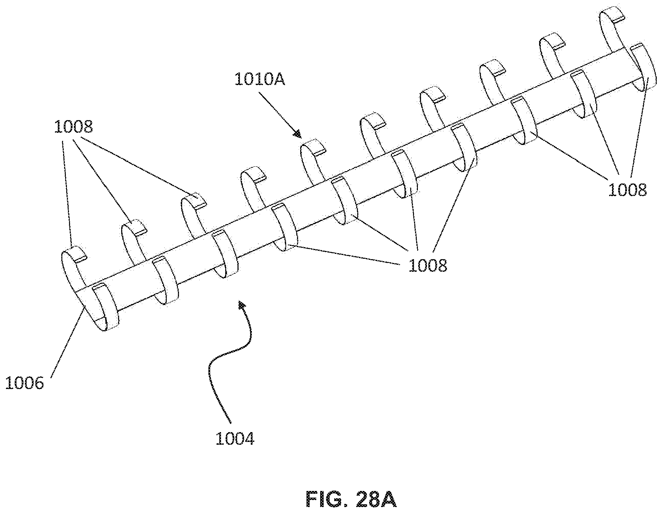

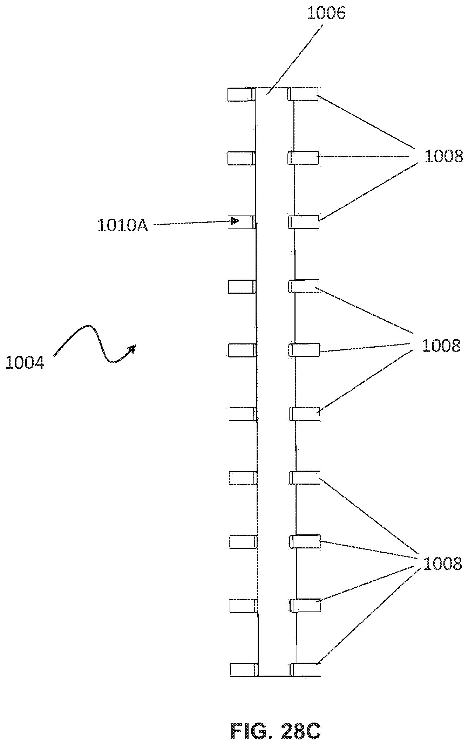

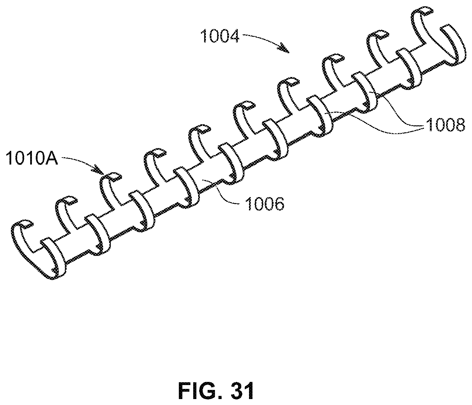

The support structure 1004 is better seen in Figs. 28A to 28D, and Figs. 31 to 33.

The support structure 1004 includes at least one spine 1006. For example, the spine 1006 may be a substantially thin, narrow and elongate element.

A plurality of support members may be provided along at least a portion of the length of the spine 1006, preferably along substantially the entire length of the spine 1006. For example, in the illustrated embodiments, the support members may be in the form of ribs 1008 and reference herein shall be made as such.

One or more ribs 1008 may be positioned on one side of the spine 1006 and one or more ribs 1008 may be positioned on the other side of the spine 1006. The ribs 1008 may comprise relatively thin, narrow and elongate elements. The ribs 1008 are arcuate and extend away from the spine 1006. Each rib 1008 has a pair and together a pair of ribs 1008 define a partial hoop structure 1010A or complete hoop structures 1010B. The hoop structure 1010A and 1010 are described in more detail below.

At least one of the spine 1006 and the ribs 1008 may be constructed from a resilient material.

One or more of the spine 1006 and the ribs 1008 may be constructed from one or more of a thermoplastic material e.g. Hytrel, a thermoplastic polyester (TPE) material, and/or a thermoplastic polyurethane (TPU) material.

The casing 1002 may be softer, and more flexible than, the support structure 1004. The casing 1002 may be formed from a first material, e.g. one or more of the materials described above. The support structure 1004 may be formed from a second material, e.g. one of more of the materials described above. The second material is a stiffer or harder, less flexible material than the first material. Therefore, the casing 1002 may be made from a relatively flexible and soft material, and the support structure 1004 may be made from a relatively harder material.

The support structure 1004 may be more resilient than the casing 1002. The second material may be more resilient than the first material. This may allow the support structure 1004, particularly the ribs 1008, for example, to resiliently support the casing 1002.

However, as will be become apparent further below, the support structure 1004 may be structured so that it does not hinder or prevent the conduit from flexing, but has sufficient stiffness to limit or prevent compression forces that may be applied to the conduit 1000 during use, e.g. when the patient lies on the side of their head. As a result, the conduit 1000 may be prevented from, or has reduced instances of, from occlusion or collapsing. For example, the ribs 1008 are preferably made from a material that provides the ribs 1008 with a spring property or ability to resist flex and store mechanical energy, e.g. a resilient material. Therefore, when the ribs 1008 are compressed from a neutral configuration or resting position, the ribs 1008 are configured to exert an opposing force, e.g. a spring force, which is approximately proportional to its change in deflection. This aspect of the present technology is described in more detail below.

In some alternative embodiments, the spine and/or the ribs may also be made from a thermoset material such as silicone. The silicone spine and/or the silicone ribs may be stiffened so that the support structure is relatively stiffer and less flexible than the casing. However, the silicone spine and/or ribs may be heavier and/or thicker than the ribs 1008 and the spine 1006 constructed from materials other than silicone, as described above. The conduit 1000 comprising a non-silicone spine and/or ribs may have a weight to external surface area ratio which is lower than a weight to external surface area ratio of a conduit comprising silicone spine and/or ribs.

The support structure 1004 may be constructed from a relatively hard but lightweight material.

The support structure 1004 material may have a hardness above 40 Shore A, and more preferably a hardness above 55 Shore A. The support structure 1004 may be made form a material which is relatively harder than silicone.

The support structure 1004 made from a material, e.g. thermoplastic material, may be stiffer or harder than a support structure formed from a different material, e.g. silicone, that has the same dimensions, e.g. thickness, as the support structure 1004.

At least one of the spine 1006 and the ribs 1008 may comprise a multiple-layer structure, e.g. a combination of materials and/or layers.

Making at least the ribs 1008 from a relatively hard material allows them to be relatively resistant to deformation. As a result, the ribs 1008 can provide resistance to collapsing of the conduit 1000 in use. However, the relatively low weight of the material reduces the overall weight of the conduit 1000 compared to a similar structure formed from materials such as silicone and rubber.

In the illustrated embodiment, the ribs 1008 may be integrally formed with the spine 1006. However, in other forms (not illustrated in the figures), ribs and a spine may be separately formed and attached together to form a support structure. In yet other forms not shown, ribs and a spine may not be fixed together. For example, the spine and the ribs may each be fixed to a casing, but not to each other.

As illustrated, the ribs 1008 may be arranged in pairs, which are spaced apart along the length of the spine 1006. One rib of a pair is located one side of the spine 1006 and the other one of the pair of ribs 1008 is located on the other side of the spine 1006.

Each of the pair of ribs 1008 may be located directly opposite the other. However, other arrangements are envisaged. For example, each of the pair of ribs 1008 may be located indirectly opposite the other, e.g. each of the pair of ribs 1008 are diagonally opposed to the other.

As is illustrated, the support structure 1004 includes multiple ribs 1008 spaced apart from each other along the length of the spine 1006.

The spacing between the ribs 1008 may be constant along the whole length of the conduit. However, in other forms, the spacing between the ribs 1008 may not be different at different points along the length of the conduit 1000.

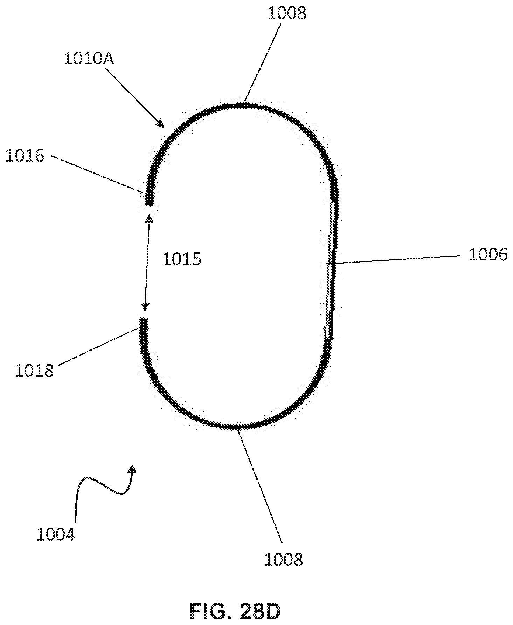

As is perhaps best shown in Fig. 28D, a pair of ribs 1008 may have a gap 1015, which is defined by a space between a distal end 1016 of one rib 1008, and the distal end 1018 of the other rib 1008.

In other forms (not illustrated), ribs can be provided to one side of the spine and not to the other. In these, there may be a gap between an end of the rib and the other side of the spine.

At least one of the ribs 1008 and the spine 1006 may be configured to limit or prevent compression forces. For example, at least one of the spine 1006 and the ribs 1008 may be substantially stiff, e.g. made from a substantially stiff material.

At least one of the ribs 1008 and the spine 1006 may be configured to flex, bend and/or be compressed. For example, at least one of the ribs 1008 and the spine 1006 may be substantially flexible, e.g. made from a substantially flexible material.

The ribs 1008 may flex with respect to each other, and/or the spine 1006 and the ribs 1008 may flex with respect to each other. For example, the ribs 1008 can flex or bend when they are compressed by the patient lying on the side of their head. When compressed, the ribs 1008 positioned on either side of the conduit 1000 flex towards each other and/or inwards towards spine 1006.

At least one of the ribs 1008 and the spine 1006 may be resilient, e.g. formed from a resilient material. The resilient nature of the ribs 1008 may allow the ribs 1008 to flex or bend, and then return to the neutral configuration on release of force(s). This can help limit or prevent the conduit 1000 from collapsing or becoming occluded by compression forces and/or other forces.

In addition, the ribs 1008 may be configured and / or arranged to flex outwardly. This may allow the ribs 1008 to flex or bend to increase the cross-sectional area of the flow path through the conduit 1000. For example, the ribs 1008 positioned on either side of the conduit 1000 can flex apart from each other and/or away from the spine 1006.

The ribs 1008 may be structured and arranged to expand and contract. As the pressure inside the conduit 1000 changes, the ribs 1008 can expand and contract. For example, as the pressure is increase, the ribs 1008 may expand, e.g. flex outwardly and out of their neutral configuration, and when the pressure is lowered, the ribs 1008 may contract, e.g. move to return to their neutral configuration.

However, in other forms of the present technology (not shown), ribs and/or a spine of a support structure for a conduit may be inflexible, i.e. configured not to flex. For example, the ribs may be rigid. In some forms, the support structure may comprise one or more hinged regions. This may facilitate the expansion and contraction of the ribs, as described above, without the ribs having to flex. The hinged region(s) may be provided by a region(s) of the spine and/or ribs which is/are more flexible than other regions of the ribs and/or spine, e.g. a thinned region and/or a region made from a relatively more flexible material. In these embodiments, the more flexible region(s) may act as a hinge. The more flexible region(s) may be provided on at least one of the spine and the ribs, e.g. at a joint between a respective rib and side of the spine.

In addition, the casing 1002 may stretch to facilitate the ribs 1008 flexing. At least a region of the casing 1002 positioned between the gap 1015 may stretch. For example, as described above, the casing 1002 or regions of it may be made from an elastic material which may stretch to permit this stretching.

In an alternative embodiment which is not shown, a casing may have a fold or gusset located in a gap between first and second ends of the ribs, as described herein.

This arrangement may reduce impedance to gas flow and / or decrease turbulence. As a result, noise created during use of the conduit can be reduced and improve compliance with therapy.

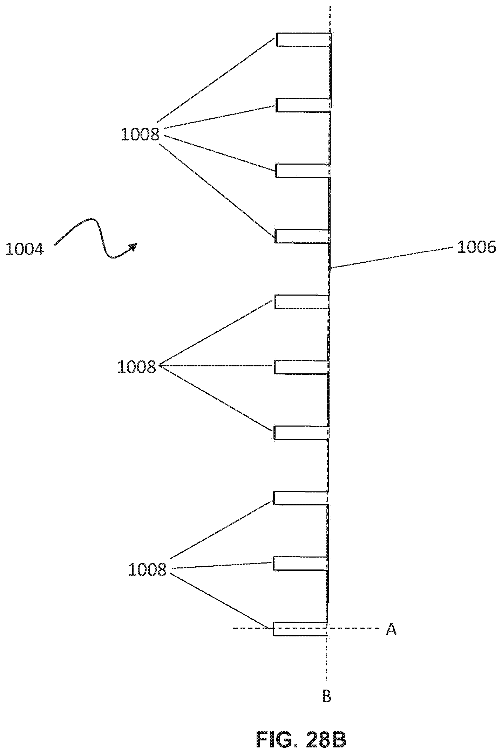

The longitudinal axis A of the rib(s) 1008 may be substantially perpendicular to a plane B defined by the spine 1006 as shown in Fig. 28B. For example, both of a pair of ribs 1008 can lie in a substantially flat plane. However, in other forms (not illustrated in the figures), plane B may be orientated at an acute or obtuse angle with respect to the longitudinal axis A.

In other forms (not illustrated in the figures), ribs may be positioned along the spine so that the ribs on one side of the spine are not located directly opposite the ribs located on the other side of the spine, i.e. they are offset from each other. In yet other forms, ribs may be provided on only one side of the spine. For example, at least a section of the spine comprises ribs extending from one side of the spine and no ribs extending from the other side of the spine.

The cross-section of the conduit 1000 may be substantially oval when in a neutral state e.g. not in use. However, in other forms (not shown), the cross-section of a conduit may be any other suitable shape, e.g. circular, obround or a polygonal shape such as rectangular.

The cross-section of the conduit 1000 may have a substantially flattened shape surface. For example, the oval shaped cross-section illustrated may have a substantially flattened surface when compared to a circular cross-section.

In the illustrated embodiment, the ribs 1008 may be substantially curved. For example, the ribs 1008 may be substantially C-shaped. However, other shaped ribs are envisaged. For example, ribs may be shaped to form a support structure that has a cross-section with one or more of the shapes described above.

The ribs 1008 may be shaped to provide sufficient spring force and to define a cross-sectional area for the flow path that improves or optimises flow dynamics. For example, ribs 1008 shaped to create a circular or oval shaped cross-sectional area in the conduit 1000 may provide better spring force compared to ribs 1008 with other shapes.

The conduit 1000 has a patient contacting surface 1012 which is configured to in use lie against a surface of a patient's head and/or face. The patient contacting surface 1012 may be relatively flat.

For example, the spine 1006 may comprise a substantially flat portion when in a neutral state.

The relatively flat patient contacting surface 1012 can help improve comfort and fit of the conduit on the patient's head and/or face because there are no protruding areas or edges that may cause irritation or discomfort. This may improve treatment compliance.

In addition, the conduit 1000 may have a distal surface 1014 which is relatively flat. The relatively flat distal surface 1014 may be created at least in part by a region of the casing 1002 which extends over the gaps 1015 between the ribs 1008. The conduit 1000 may therefore have a relatively low-profile appearance, which may minimize its size and bulk on a patient in use. This can improve comfort and fit, and consequently may improve treatment compliance.

The relatively flat patient contacting surface 1012 and the relatively flat distal surface 1014 may provide a conduit 1000 with the flattened shape described above.

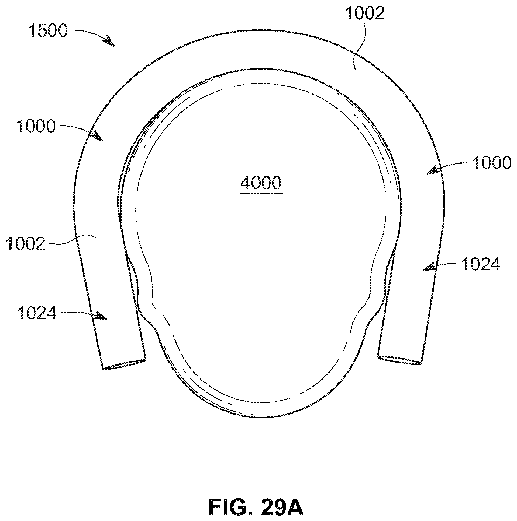

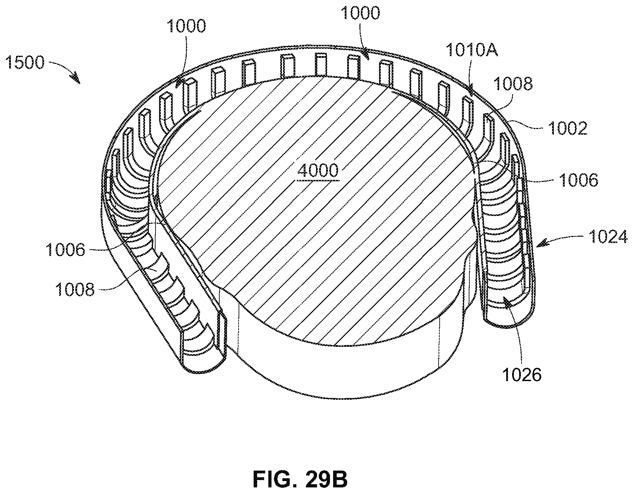

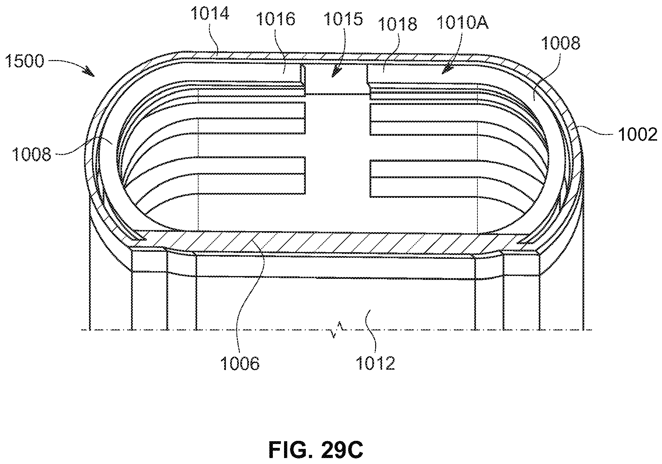

One or more conduits 1000 may provide a headgear conduit system 1500 which is perhaps best illustrated in Figs. 29A to 30. For example, the headgear conduit system 1500 may be provided by a plurality of conduits or conduit sections, e.g. a pair of conduits 1000, which are formed or attached together.

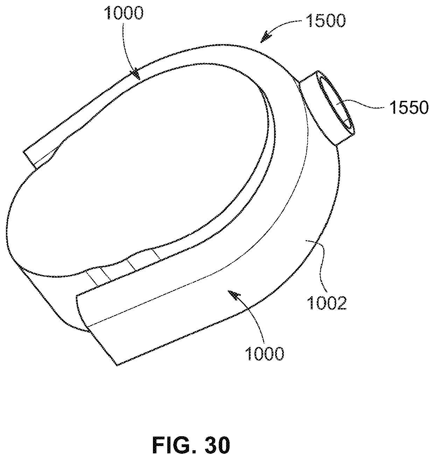

In some forms, as is perhaps best illustrated in Fig. 30, the headgear conduit system 1500 may comprise an inlet 1550 configured to in use receive a supply of pressurised respiratory gas into the interior of the conduit(s) 1000. The inlet 1550 may be configured to fluidly connect to a supply of pressurised respiratory gas in use, e.g. an air delivery conduit (not shown). As illustrated in Fig. 30, the inlet 1550 may be located on the headgear conduit system 1500 such that the inlet 1550 is positioned in a top region of the patient's head in use, wherein the patient's head is represented by 4000. For example, the inlet 1550 may be located in a substantially central region of the headgear conduit system 1500. However, other suitable positions are envisaged in other forms.

#### 4.6.1.3 Alternative support structures

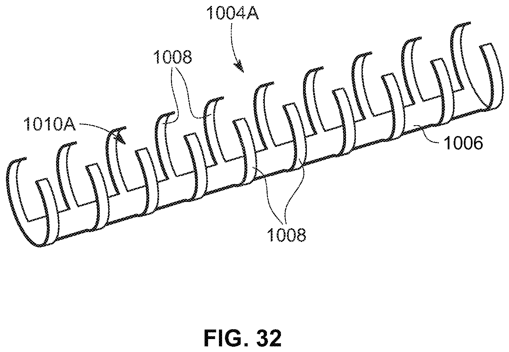

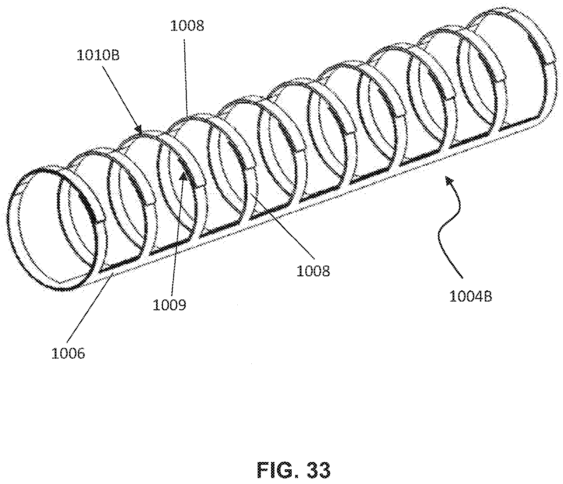

Referring now to Figs. 32 and 33 which show views of alternative support structures 1004A, 1004B according to aspects of the present technology.

The support structure 1004A, 1004B may be shaped to provide a conduit (not shown in the figures) which has a substantially circular cross-section.

As is illustrated, one of each pair of ribs 1008 may abut against or overlap with another of a pair of ribs 1008 when in a neutral configuration (i.e. not compressed, flexed, and/or bent). The ribs 1008 therefore define a complete hoop structure. In addition, as is perhaps best illustrated in Fig. 33, at least a portion of each of a pair of ribs 1008 may overlap each other to create an overlap region 1009.

The overlapping ribs 1008 may not be joined together. As a result, the ribs can slide overlap relative to each other.

However, the distal ends 1016, 1018 of respective ribs 1008 may be attached together e.g. fixedly or slidably.

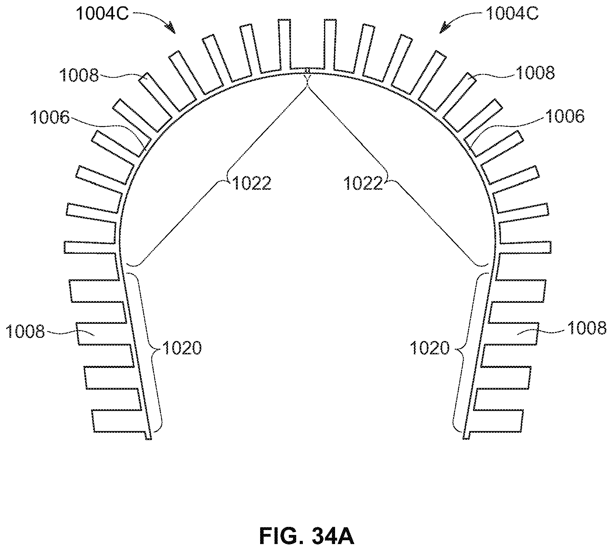

Referring now to Figs. 34A to 39A which show various views of alternative support structures 1004C, 1004D, 1004E, 1004F according to aspects of the present technology.

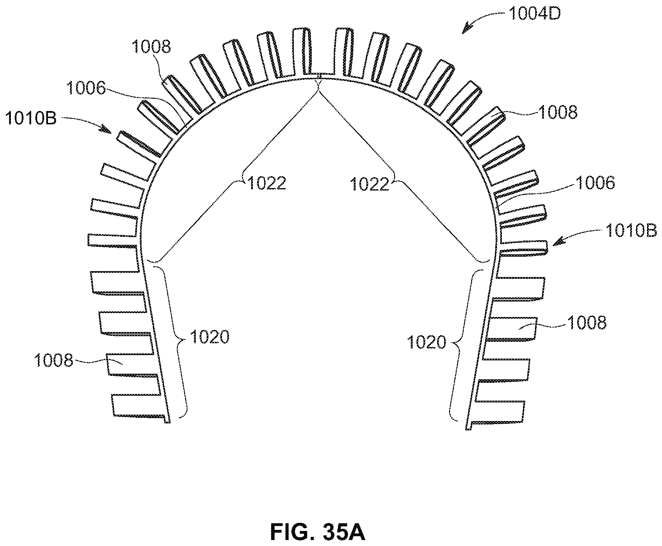

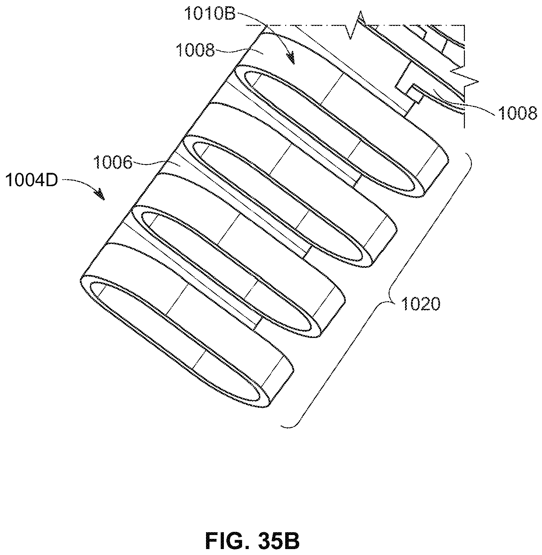

As is perhaps best shown in Figs. 35B to 36B, each rib 1008 may be a complete hoop structure 1010B. For example, one rib may form a complete hoop structure 1010B, e.g. a ring-like member.

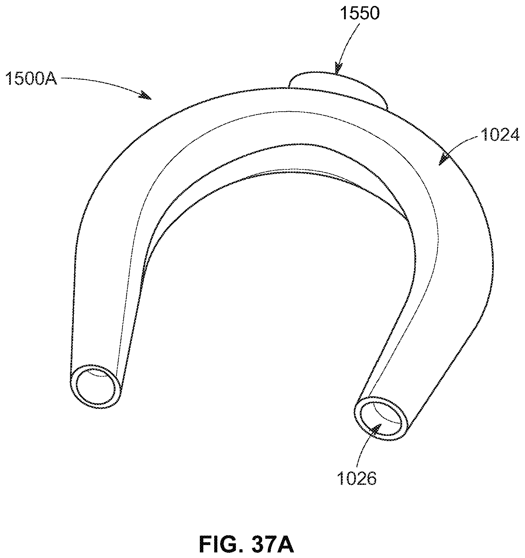

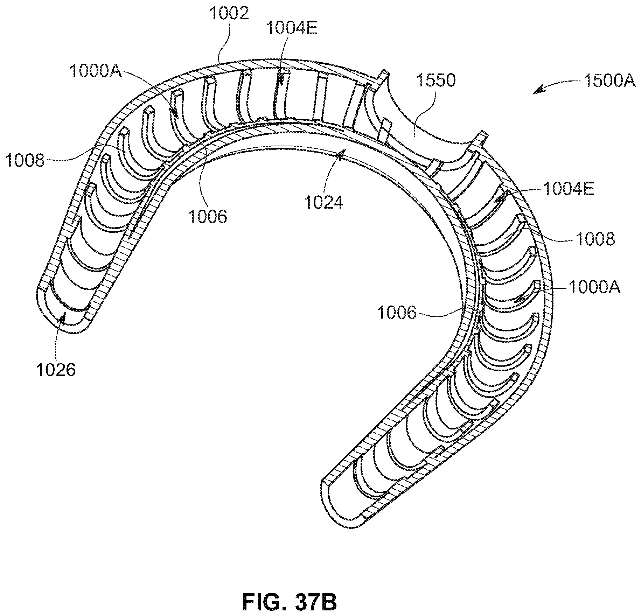

A plurality of support structures or support structure sections may be attached or formed together to form a headgear conduit system. Pairs of support structures 1004, 1004A, 1004B, 1004C, 1004D, 1004F incorporated into conduits 1000, 1000B may be attached or formed together to form headgear conduit system 1500. In another example, a pair of support structures 1004E incorporated into conduits 1000A may be attached or formed together to provide an alternative headgear conduit system 1500A which is shown in Figs. 37A and 37B.

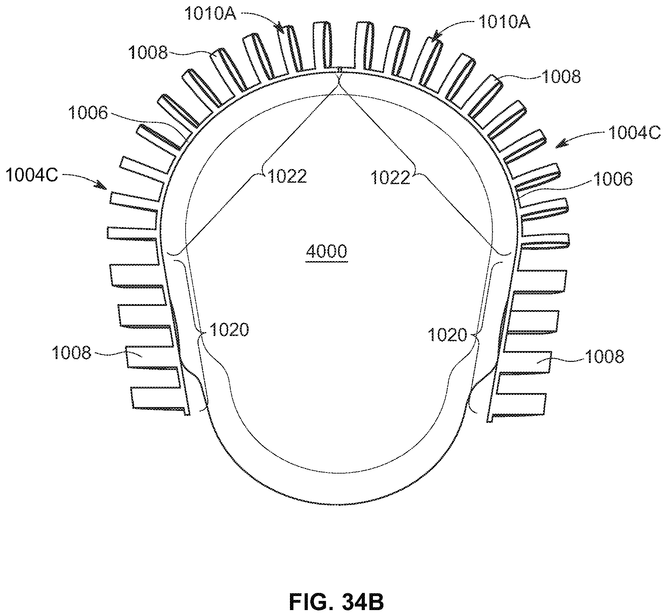

One of the pair of support structures 1004, 1004A, 1004B, 1004C, 1004D, 1004E, 1004F may be positioned in use on a first side of the patient's head 4000 and the other one of the pair of support structures 1004, 1004A, 1004B, 1004C, 1004D, 1004E, 1004F may positioned in use on the other side of the patient's head 4000, for example as is perhaps best shown in Fig. 34B.

One or more of the following features may be selected to provide a conduit 1000, 1000A, 1000B with a desired configuration:

- the material(s) used to form one or more of the casing 1002 and the support structure 1004, 1004A, 1004B, 1004C, 1004D, 1004E, 1004F; and
- the structure of one or more of the casing 1002 and the support structure 1004, 1004A, 1004B, 1004C, 1004D, 1004E, 1004F.

For example, one or more of the above features may be selected to provide a conduit 1000, 1000A, 1000B or parts thereof with one or more of a desired stiffness, flexibility, size, and weight. These and other aspects of the present technology will become apparent from the discussion below.

The support structure 1004, 1004A, 1004B, 1004C, 1004D, 1004E, 1004F may be configured to be substantially flexible and resilient, but may be configured to provide enough stiffness to limit or prevent occlusion or collapse of the conduit 1000, 1000A, 1000B in use.

The material used to form the support structure 1004, 1004A, 1004B, 1004C, 1004D, 1004E, 1004F may be a substantially flexible and resilient, but relatively stiff material, as described above.

Alternatively, or in addition to this, at least one of the spine 1006 and the ribs 1008 may be shaped and/or dimensioned to provide a support structure 1004, 1004A, 1004B, 1004C, 1004D, 1004E, 1004F that is substantially flexible and resilient, but has a desired stiffness. For example, the shape, length, width, and/or thickness of one or more of the ribs 1008 and/or the spine 1006 can be varied so that one or more regions of the conduit 1000, 1000A, 1000B have a stiffness which is greater than one or more other regions of the conduit 1000, 1000A, 1000B.

In other forms (not illustrated in the figures), the shape, width, and/or thickness along the length of each rib can be varied, e.g. each rib may have narrowed regions and/or thinned regions with respect to other regions of the rib. This may help create regions with differing stiffnesses on the ribs, which may create regions with differing flexibility.

The conduit 1000, 1000A, 1000B may comprise a first region and a second region. The first region may be a side of head region 1020, i.e. a region of the conduit 1000, 1000A, 1000B which can be positioned in use on a side region of the patient's head. The second region may be a top of the head region 1022, i.e. a region of the conduit 1000, 1000A, 1000B which can be positioned in use on a top region of the patient's head.

The first region may be stiffer than the second region. For example, the side of head region 1020 may be relatively stiffer compared the top of the head region 1022 because the side of head region 1020 is more susceptible to being compressed when the patient sleeps on the side of their head.

As described, the ribs 1008 may have variable widths and/or thicknesses. The ribs 1008 located in the first region may be wider and/or thicker than the ribs 1008 located in the second region. This can provide the first region which is stiffer than the second region. For example, as is perhaps best shown in Figs. 34A to 36B, one or more ribs 1008 located in the side of head region 1020 are wider than one or more ribs 1008 located in the top of the head region 1022.

At least one of the spacing between the ribs 1008, and the placement of the ribs 1008 along the length spine 1006 may provide a support structure 1004, 1004A, 1004B, 1004C, 1004D, 1004E, 1004F that is substantially flexible and resilient, but relatively stiff.

The number of ribs 1008 along the length of the spine 1006 may be varied to adapt the flexibility of the support structure 1004, 1004A, 1004B, 1004C, 1004D, 1004E, 1004F.

The spacing between ribs 1008 along the length of the spine 1006 may be varied to adapt the flexibility of the support structure 1004, 1004A, 1004B, 1004C, 1004D, 1004E, 1004F. In one example, ribs 1008 that are spaced further apart along the length of the spine 1006 may increase the flexibility of the support structure 1004, 1004A, 1004B, 1004C, 1004D, 1004E. In another example, ribs 1008 that are spaced closer together along the length of the spine 1006 may increase the stiffness of the support structure 1004, 1004A, 1004B, 1004C, 1004D, 1004E.

The spacing between ribs 1008 located on a first side of the spine and the ribs 1008 located on a second side of the spine, e.g. the size of the gap 1015, can be selected to vary the flexibility and stiffness of the support structure 1004, 1004A, 1004B, 1004C, 1004D, 1004E, 1004F or parts of it.

The stiffness of the support structure 11004, 1004A, 1004B, 1004C, 1004D, 1004E, 1004F can be selected according to forces the conduit 1000, 1000A, 1000B may be subjected to in use. This may assist in limiting or preventing occlusion or collapse of the conduit 1000, 1000A, 1000B. For example, the support structure 1004 incorporated in a conduit 1000, 1000A, 1000B for a patient that has a larger weight may require a greater stiffness in the first region compared to the support structure 1004, 1004A, 1004B, 1004C, 1004D, 1004E, 1004F in a conduit 1000, 1000A, 1000B used by a patient who has a lighter weight.

The conduit 1000A may comprise a cross-section which varies along the length of conduit 1000A. For example, the conduit 1000A may be configured to provide a larger cross-sectional area of the flow path through the second region compared to a cross-sectional area of the flow path through the first region.

As illustrated in Figs. 37A and 37B, the conduit 1000A can be formed with cross-section which tapers along the length of the conduit 1000A. The conduit 1000A can provide better flow dynamics when compared to a straight conduit, e.g. conduit 1000.

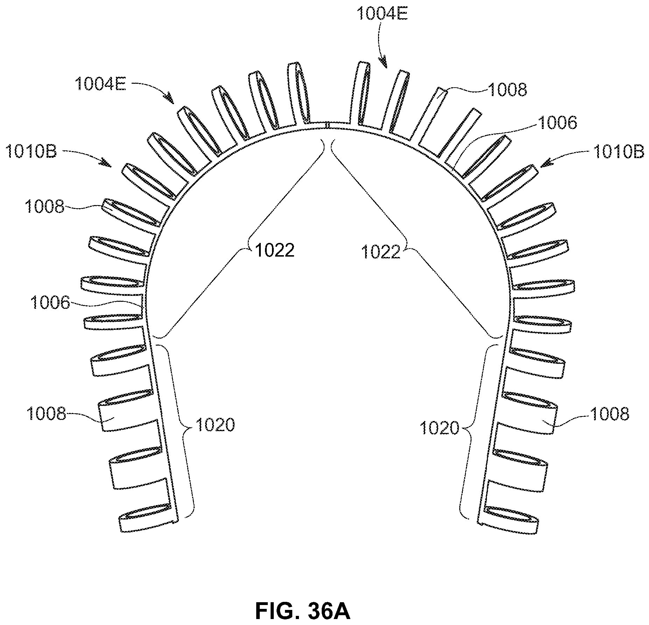

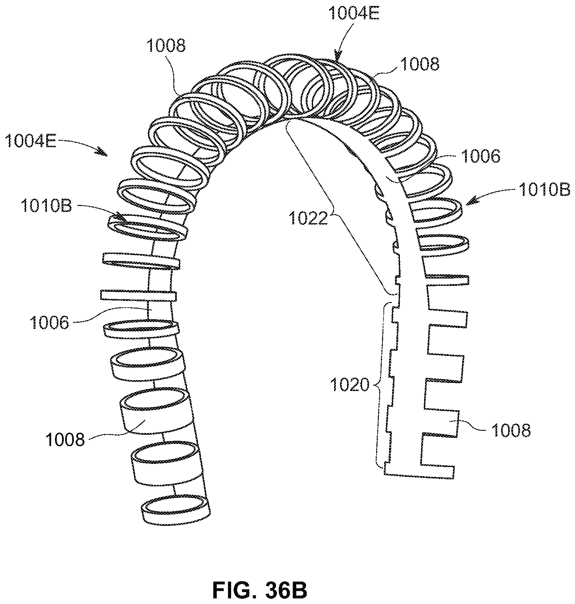

The support structure 1004C may be shaped and/or dimensioned to provide the varying cross-section described above. For example, referring again to Figs. 36A and 36B, ribs 1008 that are located in the first region may have dimensions which are smaller than the dimension of the ribs 1008 located in the second region. The ribs 1008 in the first region may define a perimeter which is smaller than a perimeter defined by the ribs 1008 in the second region. This creates a larger cross-sectional area of the flow path through the second region of the conduit 1000A compared to the cross-sectional area of the flow path through of the first region of the conduit 1000A.

The conduit 1000, 1000A, 1000B may be configured to contour the shape of the patient's face and/or head. The conduit 1000, 1000A, 1000B may be substantially flexible so that it can flex or bend to contour the shape of the patient's face and/or head. Alternatively, or in addition to this, the conduit 1000, 1000A, 1000B may be formed with a predefined shape that contours the shape of the patient's face and/or head.

The benefit of the above arrangement is that the conduit 1000, 1000A, 1000B can conform to the shape of the patient's head and/or face without kinking and/or collapsing which may impact the flow rate or impede gas flow through the conduit 1000, 1000A, 1000B.

The conduit 1000, 1000A, 1000B may be substantially flexible in one or more directions and may be configured to limit or prevent flexing in one or more other directions. For example, the spine 1006 may be substantially flexible in a direction towards or away from the patient's head and/or face in use. This may facilitate positioning the conduit 1000, 1000A, 1000B on or removing it from the patient's head and/or face in use. However, the flexibility of the spine 1006 may be limited or prevented in a direction that extends in use across the side of the patient's head and/or face. This can help limit or prevent headgear forces or other forces from disrupting the seal created between a patient interface (not shown in the figures) which comprising the conduit 1000, 1000A, 1000B and the patient's face (not shown in the figures).

As described, the first region may be stiffer than the second region. Therefore, the second region may be more flexible than the first region. For example, a portion of the spine 1006 in the side of the head region 1020 may be more flexible than a portion of the spine 1006 in the top of the head region 1022.

This arrangement may be achieved by varying the shape and/or dimensions of the spine 1006. The width and/or thickness of the spine 1006 may be varied along the length of the spine 1006. For example, as is perhaps best shown in Figs. 35B to 36B, a portion of the spine 1006 in the side of head region 1020 may have a greater width than a portion of the spine 1006 in the top of the head region 1022. In other forms, the material used to form the spine 1006 may be selected to achieve this arrangement.

This arrangement may allow the conduit 1000, 1000A to flex or bend more in the top of the head region 1022 compared to the side of head region 1020 in order for it to contour to the shape or curvature the respective regions of the patient's head. This may also facilitate the ease with which the conduit 1000, 1000A can be positioned over the person's head and/or face. As a result, comfort and fit may be improved.

In addition, this arrangement can help limit or prevent occlusion or collapse in the side of head region 1020 which is more prone to experience this, as previously explained.

The conduit 1000, 1000A, 1000B may be structured to provide a conduit with a desired size and weight. For example, the casing 1002 and the support structure 1004, 1004A, 1004B, 1004C, 1004D, 1004E, 10004F may be shaped and/or dimensioned to provide a substantially narrow, flattened and lightweight conduit 1000, 1000A, 1000B. This may limit or prevent obtrusiveness of the conduit 1000, 1000A, 1000B and provide a lighter conduit 1000, 1000A, 1000B compared to existing conduits known to one skilled in the art. This can help improve comfort and fit of the conduit 1000, 1000A, 1000B on the patient's face and/or head in use, and can improve treatment compliance.

In addition, the material used to form the conduit 1000, 1000A, 1000B may be selected to provide the conduit 1000, 1000A, 1000B with a desired size and weight. For example, a thermoplastic material, e.g. Hytrel or thermoplastic polyester material, may be selected over thermoset materials, e.g. silicone material, to form the support structure 1004, 1004A, 1004B, 1004C, 1004D, 1004E, 1004F. This may be because support structures formed from silicone require more material to provide sufficient stiffness to limit or prevent collapse and occlusion of the conduit. Therefore, such conduits may need to be heavier and thicker so that they can achieve the stiffness required to limit or prevent occlusion and/or collapse of the conduit caused by compression forces applied to the conduit. Bulky and heavy conduits are not desirable and may cause discomfort to the patient and reduce compliance. In addition, the use of more materials may increase manufacturing costs. Yet a further issue with using materials such as silicone to form support structures is that these need to be relatively bulky. As a result, the support structures can reduce the size of the flow path, increase flow impedance and / or turbulence.

Therefore, the conduits according to the present technology may be relatively unobtrusive, lightweight yet have a stiffness that allows flexing but can limit or prevent collapse or occlusion.

In addition, the conduits 1000, 1000A, 1000B can be provided with a substantially smooth outer surface 1024. For example, the conduit 1000, 1000A, 1000B may have a smoother outer surface 1024 compared to known helix or corrugated type conduits. The structure of the conduit 1000, 1000A, 1000B as described herein may help achieve this. For example, the casing 1002 may be formed as a sleeve. The sleeve may be a continuous tubular structure, as illustrated, without edges, joints or seams which can cause irritation and discomfort to a patient. In addition, the shape of support structure 1004, 1004A, 1004B, 1004C, 1004D, 1004E, 1004F, e.g. an flattened oval shape or a circular shape, may limit or prevent the creation of sharp edges or corners on the conduit 1000. This arrangement may help make the conduit 1000, 1000A, 1000B more comfortable for the patient to wear.

In some forms, the conduit 1000, 1000A, 1000B can be provided with a substantially smooth inner surface 1026. For example, the conduit 1000, 1000A, 1000B may have a smoother inner surface 1026 when compared to known helix or corrugated type conduits which may have ridges or protrusions extending from an inner surface. The structure of the conduit 1000, 1000A, 1000B as described herein may help achieve this. For example, the substantially thin spine 1006 and thin ribs 1008 provide a flatter, thinner structure which may create a smoother inner surface 1026 compared to the known helix or corrugated type conduits. This arrangement can help reduce air turbulence generated in use in the conduit 1000, 1000A, 1000B by the flow of gases. This may limit or prevent noise being generated because of the turbulence, which may improve patient comfort and treatment compliance.

It is to be appreciated that in other forms not illustrated, a lining formed from one or more layers may be provided to the inside of the conduit 1000, 1000A, 1000B e.g. to the inner surface 1026. This lining may provide a layer that is gas impermeable layer. In addition, the lining may help provide a smoother inner surface of the conduit which may improve air flow and reduce turbulence and noise.

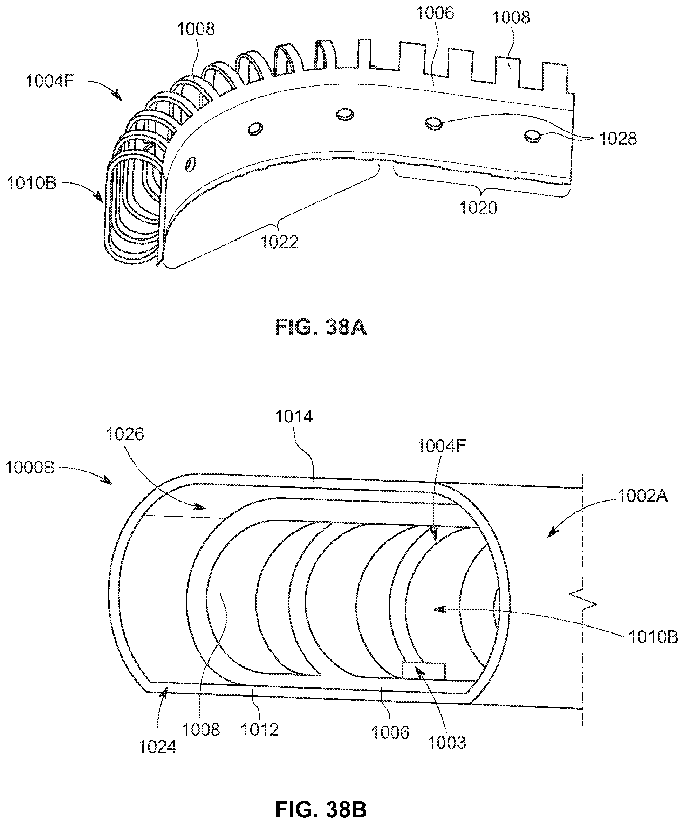

Referring now to Figs. 38A to 39B, which show a conduit 1000B that has an alternative support structure 1004F according to an aspect of the present technology.

The support structure 1004F may be attached to a casing 1002A, as described above. However, the support structure 1004F may comprise at least one connector configured to facilitate the attachment of the support structure 1004F and a casing 1002A, 1002B together. For example, the at least one connector may comprise a at least one aperture 1028, preferably a plurality of apertures 1028, as is perhaps best illustrated in Fig. 38A, and reference herein shall be made as such. The apertures 1028 may be through-holes as illustrated, or it may be a recess or cavity formed in an outer surface of the support structure 1004F, for example.

As illustrated, apertures 1028 may be located on the spine 1006. However, at least one connector e.g. a plurality of apertures, may be located on a rib(s) in other forms which are not illustrated.

The casing 1002A may be overmoulded or co-moulded to the support structure 1004F, as described above. As is perhaps best shown in Fig. 38B, during the moulding process, or a thermoforming process, a portion 1003 of the casing 1002A may enter into the aperture 1028 and set or cure here. As illustrated, the portion 1003 may extend through the aperture 1028. This may improve the attachment and bond between the casing 1002A and the support structure 1004F.

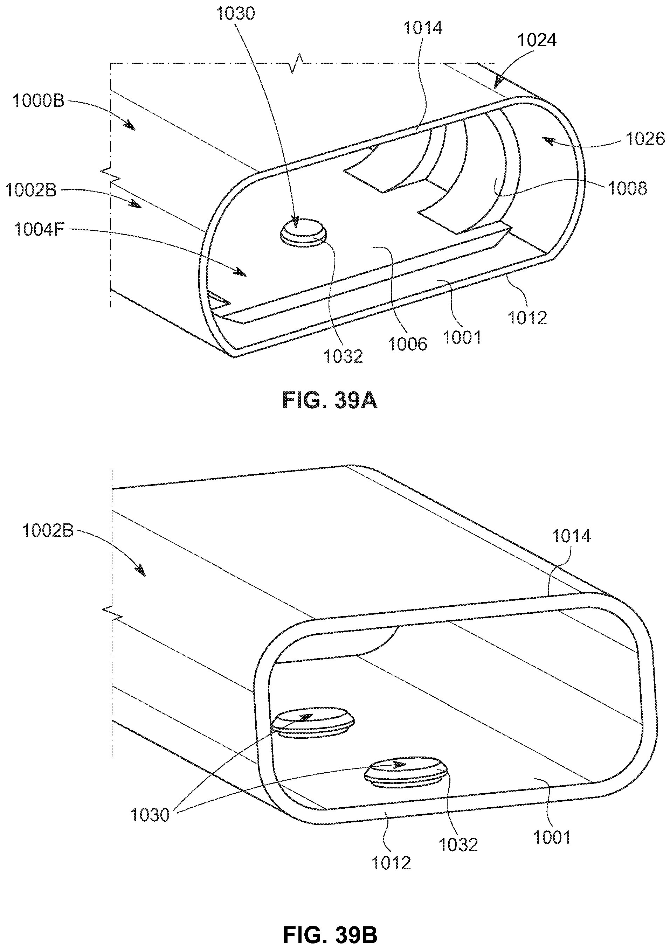

As is perhaps best shown in Figs. 39A and 39B, the casing 1002B may comprise at least one connector to facilitate attachment of the support structure 1004F and the casing 1002B to each other. For example, the at least one connector may comprise a plurality of preformed projecting members 1030, e.g. press studs or pushbuttons, and reference herein shall be made as such.

The casing connector(s) as described above, and the support structure connector(s) as described above may be structured to attach to each other. For example, the preformed projecting members 1030 may be formed on an inner surface 1001 of the casing 1002B. The preformed projecting members 1030 may then be inserted into the apertures 1028 to attach the casing 1002B to the support structure 1004F, as illustrated. Each preformed projecting member 1030 may be structured to engage with a portion of the support structure 1004F, such as the portion surrounding the aperture 1028, for example. The preformed projecting member 1030 may interference fit with the portion surrounding the aperture 1028 when the preformed projecting member 1030 is inserted through the aperture 1028. The preformed projecting member 1030 may comprise a lip or ridge 1032 which is configured to engage, for example in a snap-fit, with the portion of the support structure, e.g. a portion surrounding the aperture 1028, once the preformed projecting member 1030 is inserted through the aperture 1028, as illustrated.

It should be appreciated that in other forms (not illustrated), the connectors described above may take another suitable form, e.g. any suitable connector, fastener half arrangements, and/or attachment mechanism or arrangement known to one skilled in the art.

The provision of a conduit 1000, 1000A, 1000B with a support structure 1004, 1004A, 1004B, 1004C, 1004D, 1004E, 1004F according to the present technology may provide a number of advantages. These could include, amongst others:

- Providing a conduit 1000, 1000A, 1000B for use in a respiratory therapy system that is flexible and can adapt to the shape of the patient's head and/or face, and that is sufficiently stiff to limit or prevent collapse or occlusion during use;

- Reducing the weight of a conduit 1000, 1000A, 1000B for use in a respiratory therapy system compared to available conduits;
- Reducing the amount of materials used in constructing a conduit 1000, 1000A, 1000B compared to available conduits; and
- Optimizing the internal cross-section of a conduit 1000, 1000A, 1000B and therefore optimizing the available flow area for given external dimensions of the conduit 1000, 1000A, 1000B.

### 4.6.2 Patient Interface System

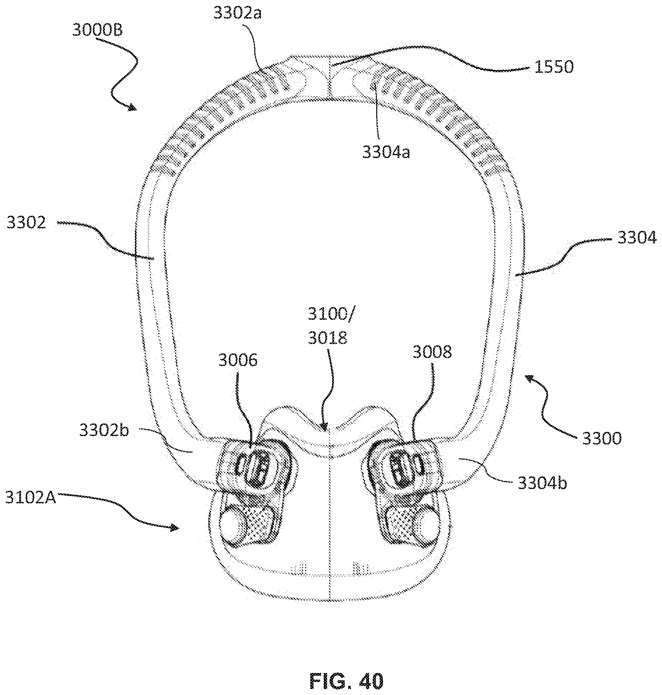

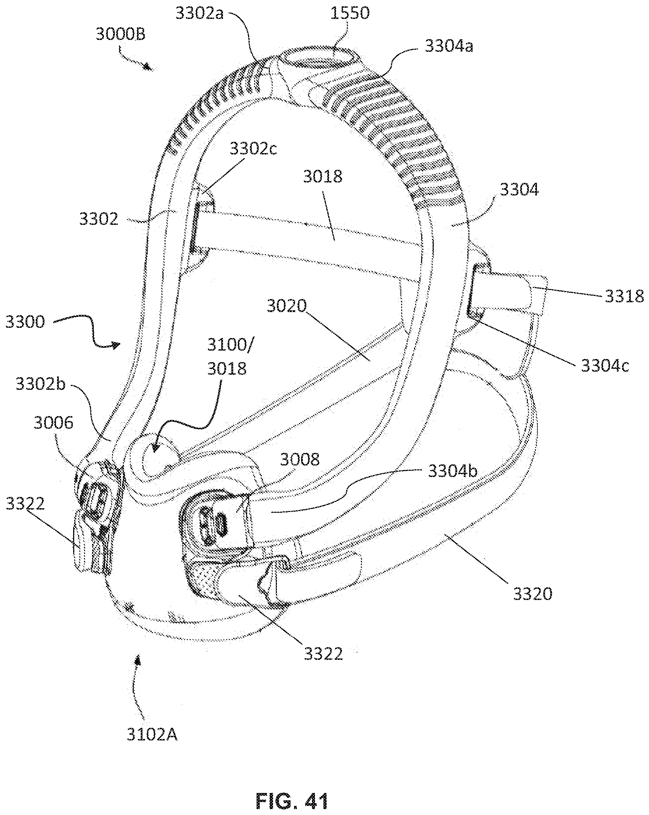

Referring now to Figs. 40 and 41 which shows views of a patient interface system 3000B including the conduit 1000, 1000A, 1000B and/or headgear conduit system 1500 according to the present technology.

The patient interface system 3000B comprises a patient interface indicated generally as 3102A and a positioning and stabilising structure 3300. The positioning and stabilising structure 3300 comprises at least one headgear tube, preferably a pair of headgear tubes, e.g. a first headgear tube 3302 and a second headgear tube 3304. At least one of the headgear tubes is a conduit 1000, 1000A, 1000B as described herein. Part of the positioning and stabilising structure 3300 may be provided by the headgear conduit system 1500, 1500A described herein.

The first headgear tube 3302 and the second headgear tube 3304 may fluidly connect to the patient interface 3102A and supply the flow of pressurised breathable gas to the patient interface 3102A in use. For example, the respective tubes 3302, 3304 may comprise inferior end regions 3302a, 3304a which may connect to the patient interface 3102A. The first headgear tube 3302 and the second headgear tube 3304 each have a connector 3006, 3008 respectively, which is configured to connect to the patient interface 3102A.

The first headgear tube 3302 and the second headgear tube 3304 may be attached or formed together. For example, the respective tubes respective tubes 3302, 3304 may comprise superior end regions 3302b, 3304b which are attached or formed together to provide the headgear conduit system 1500 as described herein.

As illustrated, a connection port, e.g. inlet 1550, which is configured to connect to a supply of pressurised respiratory gas in use, may be provided to at least one of the first headgear tube 3302 and the second headgear tube 3304.

However, in other forms, the superior end regions 3302a, 3304a may be configured to connect to a connector (not shown) which is configured to connect to a supply of pressurised respiratory gas in use. For example, the connector may include a connection port, e.g. an inlet, which connects to an air delivery conduit (not shown).

As illustrated, the patient interface system 3000B comprises a compact full-face or oro-nasal patient interface 3102 having a seal forming structure 3016 configured to create a seal with or around a patient's nose and mouth. However, other types of patient interfaces are envisaged, including a nasal mask comprising a seal-forming structure configured to create a seal with or around parts of a patient's nose, e.g. the patient's nares. An example of such a nasal mask is shown in Figs. 4A to 4D. Therefore, it is to be appreciated that the conduit 1000, 1000A, 1000B may be incorporated into other conduit headgear masks or mask arrangements, such as the patient interface system 3000 described elsewhere herein, and an example of which is shown in Figs 4A to 4D.

The seal-forming structure 3100 may have a nasal portion 3018 which seals with or around the patient's nose, and an oral portion (not shown) which seals with or around the patient's mouth. The seal-forming structure 3100 may include at least one opening (not shown). For example, the seal-forming structure 3100 may include a single opening and seals around the patient's nose and mouth. However, the seal-forming structure 3100 may include a plurality of openings. For example, the nasal portion 3018 may comprise one opening or a pair of openings and seals around the patient's nares, and the oral portion may comprise a single opening and seals around the patient's mouth. The seal-forming structure 3100 may have a nasal portion 3018 in the form of a nasal cradle. The seal-forming structure 3100 may be substantially as described in International Application No. PCT/AU2019/05027Σ

The first headgear tube 3302 and the second headgear tube 3304 may be releasably or permanently attached to the patient interface 3102A. In other forms not shown, the first headgear tube 3302 and the second headgear tube 3304 may be integrally formed with the patient interface 3102A, e.g. by co-moulding or moulding.

One or more headgear straps, e.g. a pair of lower headgear straps 3320, may be attached to the patient interface 3102A. For example, the headgear straps 3320 are provided with connectors 3322 to facilitate the attachment.

One or more headgear straps, e.g. a pair of upper headgear straps 3318, may be attached to the respective headgear tubes 3302, 3304, e.g. via a respective connector 3302c, 3304c as illustrated in Fig. 41.

However, the headgear straps 3318, 3320 may be attached to the respective headgear tubes 3302, 3304 and/or to the patient interface 3102A using one or more other fastener or connector arrangements.

### 4.6.3 Methods of Manufacture

It should be appreciated that various methods to construct conduits 1000, 1000A, 1000B and/or headgear conduit systems 1500, 1500A according to the present technology are envisaged. Representative methods are described herein.

In a first form, the support structure 1004, 1004A, 1004B, 1004C, 1004D, 1004E, 1004F may be formed as a separate component which is subsequently attached to the casing 1002, 1002A, 1002B. For example, the support structure 1004, 1004A, 1004B, 1004C, 1004D, 1004E, 1004F and the casing 1002, 1002A, 1002B may be formed in one of the following ways.

The support structure 1004, 1004A, 1004B, 1004C, 1004D, 1004E, 1004F may be formed by injection moulding.

At least one aperture 1028 may be formed in the support structure 1004F, preferably a plurality of aperture 1028.

The support structure 1004, 1004A, 1004B, 1004C, 1004D, 1004E, 1004F may be positioned on a flat sheet of material e.g. a multi-layer sheet comprising a layer of textile material and a layer of the coating material.

The flat sheet of material can subsequently be wrapped around the support structure 1004, 1004A, 1004B, 1004C, 1004D, 1004E, 1004F and secured in position to form the casing 1002, 1002A, 1002B. Options to secure the sheet of material include adhesive, tape or any other method that can form a gas-tight seal necessary for the conduit 1000 to function as a pathway in a respiratory therapy system 1500.

In another form, a material may be applied onto a flat sheet of material to form the support structure 1004, 1004, 1004A, 1004B, 1004C, 1004D, 1004E, 1004F integrally to the sheet. For instance, an additive layer manufacturing process may be used to apply bead(s) of a material onto the sheet.

In another form, a sheet of plastic material may be cut into a predefined shape. For example, the sheet of plastic material may be die-cut. The sheet of plastic material may then be heated to enable it to be deformed to a desired shape using for example a mould or mandrel, before being cured to harden.

The sheet of material can be subsequently deformed to provide the conduit 1000, 1000A, 1000B.

The conduit 1000, 1000A, 1000B can formed by co-moulding or overmoulding the casing 1002, 1002A, 1002B to the support structure 1004, 1004A, 1004B, 1004C, 1004D, 1004E, 1004F.

The casing 1002A, 1002B may enter the aperture(s) 1028 and set or cure in the aperture(s) 1028 during the moulding process, or a thermoforming process.

Projecting members 1030 may be formed on the casing, e.g. on the inner surface of the casing.

In yet another form, the casing 1002, 1002A, 1002B may be formed as a sleeve. The sleeve may be positioned over the support structure 1004, 1004A, 1004B, 1004C, 1004D, 1004E, 1004F. For example, the sleeve and support structure 1004, 1004A, 1004B, 1004C, 1004D, 1004E, 1004F may be permanently or removably attached to each other.

The sleeve may be formed from a thermoformable material, e.g. plastic or rubber, and thermoformed to attach to the support structure 1004, 1004A, 1004B, 1004C, 1004D, 1004E, 1004F. For example, heat may be applied to heat shrink the sleeve onto the support structure 1004, 1004A, 1004B, 1004C, 1004D, 1004E, 1004F to provide the conduit 1000, 1000A, 1000B.

The inlet 1550 may be formed in the conduit 1000, 1000A, 1000B and/or headgear conduit system 1500, 1500A.

The first connector 3010 and second connector 3012 can be subsequently attached to the conduit 1000, 1000A, 1000B using techniques as should be known to one skilled in the art.

## 4.7 RPT DEVICE

Referring now to Fig. 42 that shows an exploded view of a flow generator in the form of a RPT device 4000 according to an aspect of the present technology.

The RPT device 4000 in accordance with one aspect of the present technology comprises mechanical, pneumatic, and/or electrical components and is configured to execute one or more algorithms 4300, such as any of the methods, in whole or in part, described herein. The RPT device 4000 may be configured to generate a flow of air for delivery to a patient's airways, such as to treat one or more of the respiratory conditions described elsewhere in the present document.

In one form, the RPT device 4000 is constructed and arranged to be capable of delivering a flow of air in a range of -20 L/min to + 150 L/min while maintaining a positive pressure of at least 6 $cmH_2O$, or at least 10$cmH_2O$, or at least 20 $cmH_2O$.

The RPT device may have an external housing 4010, formed in two parts, an upper portion 4012 and a lower portion 4014. Furthermore, the external housing 4010 may include one or more panel(s) 4015. The RPT device 4000 comprises a chassis 4016 that supports one or more internal components of the RPT device 4000. The RPT device 4000 may include a handle 4018.

The pneumatic path of the RPT device 4000 may comprise one or more air path items, e.g., an inlet air filter 4112, an inlet muffler 4122, a pressure generator 4140 capable of supplying air at positive pressure (e.g., a blower 4142), an outlet muffler 4124 and one or more transducers 4270, such as pressure sensors 4272 and flow rate sensors 4274.

One or more of the respiratory gas path items may be located within a removable unitary structure which will be referred to as a pneumatic block 4020. The pneumatic block 4020 may be located within the external housing 4010. In one form a pneumatic block 4020 is supported by, or formed as part of the chassis 4016.

In one form particular, the first headgear tube 3302 and the second headgear tube 3304 of the positioning and stabilizing structure 3300 may be in fluid connection with the outlet of the pneumatic block 4020 and the patient interface 3102.

The RPT device 4000 may have an electrical power supply 4210, one or more input devices 4220, a RPT controller 4230, a therapy device controller 4240, a pressure generator 4140, one or more protection circuits 4250, memory 4260, transducers 4270, data communication interface 4280 and one or more output devices 4290. Electrical components 4200 may be mounted on a single Printed Circuit Board Assembly (PCBA) 4202. In an alternative form, the RPT device 4000 may include more than one PCBA 4202.

### 4.7.1 RPT device mechanical & pneumatic components

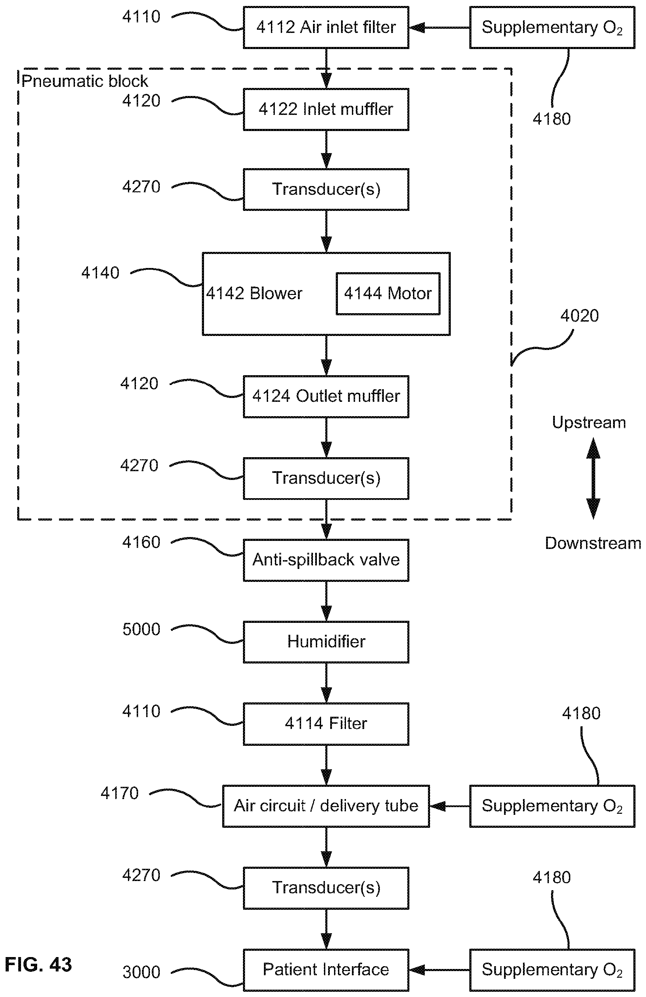

Referring now to Fig. 43 which shows a flow chart of the working of mechanical and pneumatic components of the RPT device 4000.

The RPT device 4000 may comprise one or more of the following components in an integral unit. In an alternative form, one or more of the following components may be located as respective separate units.

#### 4.7.1.1 Air filter(s)

The RPT device 4000 in accordance with one form of the present technology may include an air filter 4110, or a plurality of air filters 4110.

In one form, an inlet air filter 4112 is located at the beginning of the pneumatic path upstream of a pressure generator 4140.

In one form, an outlet air filter 4114, for example an antibacterial filter, is located between an outlet of the pneumatic block 4020 and a patient interface 3102.

#### 4.7.1.2 Muffler(s)

The RPT device 4000 in accordance with one form of the present technology may include a muffler 4120, or a plurality of mufflers 4120.

In one form of the present technology, an inlet muffler 4122 is located in the pneumatic path upstream of a pressure generator 4140.

In one form of the present technology, an outlet muffler 4124 is located in the pneumatic path between the pressure generator 4140 and a patient interface 3102.

#### 4.7.1.3 Pressure generator

In one form of the present technology, a pressure generator 4140 for producing a flow, or a supply, of air at positive pressure is a controllable blower 4142. For example the blower 4142 may include a brushless DC motor 4144 with one or more impellers. The impellers may be located in a volute. The blower may be capable of delivering a supply of air, for example at a rate of up to about 120 litres/minute, at a positive pressure in a range from about 4 $cmH_2O$ to about 20 $cmH_2O$, or in other forms up to about 30 $cmH_2O$ when delivering respiratory pressure therapy. The blower may be as described in any one of the following patents or patent applications: U.S. Patent No. 7,866,944; U.S. Patent No. 8,638,014; U.S. Patent No. 8,636,479; and PCT Patent Application Publication No. WO 2013/020167.

The pressure generator 4140 is under the control of the therapy device controller 4240.

In other forms, a pressure generator 4140 may be a piston-driven pump, a pressure regulator connected to a high pressure source (e.g. compressed air reservoir), or a bellows.

#### 4.7.1.4 Transducer(s)

Transducers may be internal of the RPT device 4000, or external of the RPT device 4000. External transducers may be located for example on or form part of the air circuit, e.g., the patient interface. External transducers may be in the form of non-contact sensors such as a Doppler radar movement sensor that transmit or transfer data to the RPT device 4000.

In one form of the present technology, one or more transducers 4270 are located upstream and/or downstream of the pressure generator 4140. The one or more transducers 4270 may be constructed and arranged to generate signals representing properties of the flow of air such as a flow rate, a pressure or a temperature at that point in the pneumatic path.

In one form of the present technology, one or more transducers 4270 may be located proximate to the patient interface 3102.

In one form, a signal from a transducer 4270 may be filtered, such as by low-pass, high-pass or band-pass filtering.

#### *4.7.1.4.1 Flow rate sensor*

A flow rate sensor 4274 in accordance with the present technology may be based on a differential pressure transducer, for example, an SDP600 Series differential pressure transducer from SENSIRION.

In one form, a signal generated by the flow rate sensor 4274 and representing a flow rate is received by the RPT controller 4230.

#### *4.7.1.4.2 Pressure sensor*

A pressure sensor 4272 in accordance with the present technology is located in fluid communication with the pneumatic path. An example of a suitable pressure sensor is a transducer from the HONEYWELL ASDX series. An alternative suitable pressure sensor is a transducer from the NPA Series from GENERAL ELECTRIC.

In one form, a signal generated by the pressure sensor 4272 is received by the RPT controller 4230.

#### *4.7.1.4.3 Motor speed transducer*

In one form of the present technology a motor speed transducer 4276 is used to determine a rotational velocity of the motor 4144 and/or the blower 4142. A motor speed signal from the motor speed transducer 4276 may be provided to the therapy device controller 4240. The motor speed transducer 4276 may, for example, be a speed sensor, such as a Hall effect sensor.

Alternatively, the RPT device 4000 typically also include an inlet filter, various sensors, and a microprocessor-based controller. A blower may include a servo-controlled motor, a blower housing, which may be a volute, and an impeller. In some cases a brake for the motor may be implemented to more rapidly reduce the speed of the blower so as to overcome the inertia of the motor and impeller. The braking can permit the blower to more rapidly achieve a lower pressure condition in time for synchronization with expiration despite the inertia. In some cases the pressure generator may also include a valve capable of discharging generated air to atmosphere as a means for altering the pressure delivered to the patient as an alternative to motor speed control. The sensors measure, amongst other things, motor speed, mass flow rate and outlet pressure, such as with a pressure transducer or the like. The controller may include data storage capacity with or without integrated data retrieval and display functions.

### 4.7.1.5 Humidifier and Anti-spill back valve

In one form of the present technology, the RPT device 4000 may include a humidifier 5000 which is configured to provide moisture to the respiratory gas supplied to the positioning and stabilizing structure 3300 and/or the patient interface 3102.

In one form of the present technology, air from the RPT device is humidified in the humidifier 5000, and passed along the duct 4050 to the positioning and stabilizing structure 3300 and/or the patient interface 3102.

In one form of the present technology, an anti-spill back valve 4160 is located between the humidifier 5000 and the pneumatic block 4020. The anti-spill back valve is constructed and arranged to reduce the risk that water will flow upstream from the humidifier 5000, for example to the motor 4144.

### 4.7.1.6 Heating Element

In some forms, the duct 4050 and/or the positioning and stabilizing structure 3300 may comprise one or more heating elements configured to heat the respiratory gas provided to the patient interface 3102, for example to maintain or raise the temperature of the respiratory gas.

In one form of the present technology, the heating element may be in a form of a heated wire circuit, and may comprise one or more transducers, such as temperature sensors.

In one form of the present technology, the heated wire circuit may be helically wound around the axis of the first headgear tube 3302 and the second headgear tube 3304. The heating element may be in communication with a controller such as a RPT controller 4230.

One example of an air circuit, such as a duct 4050 and/or the headgear tube(s) 3302, 3304 comprising a heated wire circuit is described in United States Patent 8,733,349.

**4.7.2 RPT** device electrical components

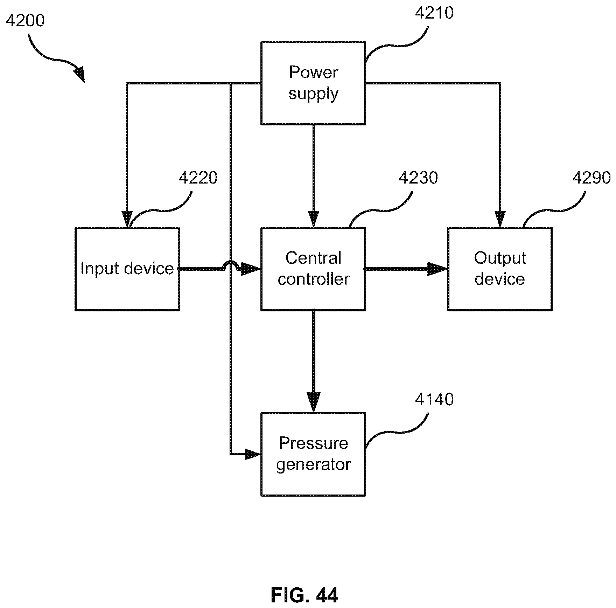

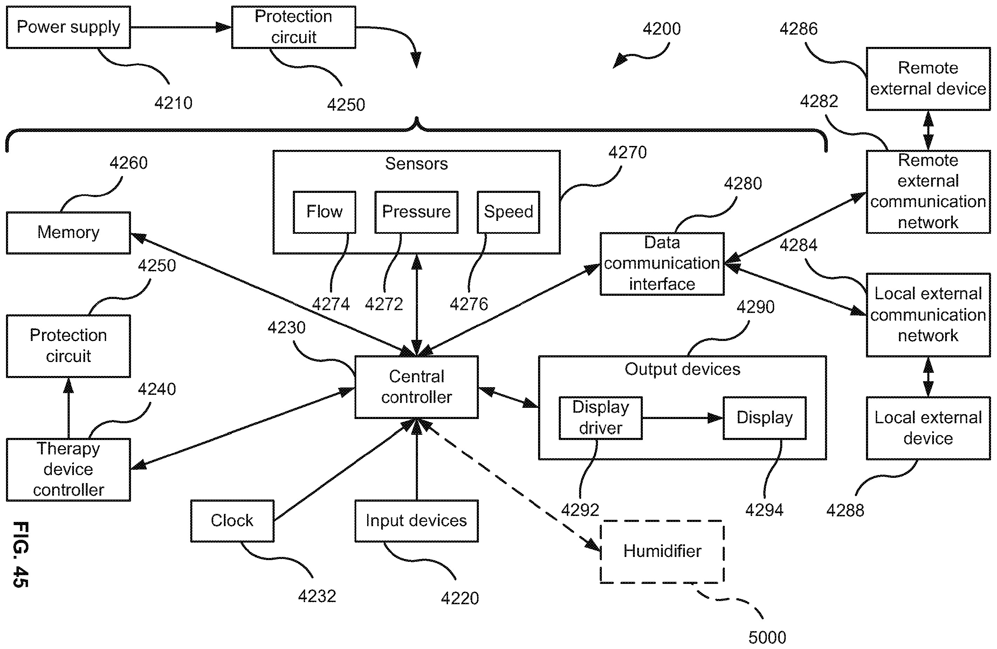

Referring now to Figs. 44 and 45 which are block diagrams to show the working of electrical components of the RPT device 4000.

4.7.2.1 Power supply

A power supply 4210 may be located internal or external of the external housing 4010 of the RPT device 4000.

In one form of the present technology, power supply 4210 provides electrical power to the RPT device 4000 only. In another form of the present technology, power supply 4210 provides electrical power to both RPT device 4000 and humidifier 5000.

**4.7.2.2 Input devices**

In one form of the present technology, the RPT device 4000 includes one or more input devices 4220 in the form of buttons, switches or dials to allow a person to interact with the device. The buttons, switches or dials may be physical devices, or software devices accessible via a touch screen. The buttons, switches or dials may, in one form, be physically connected to the external housing 4010, or may, in another form, be in wireless communication with a receiver that is in electrical connection to the RPT controller 4230.

In one form, the input device 4220 may be constructed and arranged to allow a person to select a value and/or a menu option.

**4.7.2.3 RPT controller**

In one form of the present technology, the RPT controller 4230 is one or a plurality of processors suitable to control the RPT device 4000.

Suitable processors may include an x86 INTEL processor, a processor based on ARM® Cortex®-M processor from ARM Holdings such as an STM32 series microcontroller from ST MICROELECTRONIC. In certain alternative forms of the present technology, a 32-bit RISC CPU, such as an STR9 series microcontroller from ST MICROELECTRONICS or a 16-bit RISC CPU such as a processor from the MSP430 family of microcontrollers, manufactured by TEXAS INSTRUMENTS may also be suitable.

In one form of the present technology, the RPT controller 4230 is a dedicated electronic circuit.

In one form, the RPT controller 4230 is an application-specific integrated circuit. In another form, the RPT controller 4230 comprises discrete electronic components.

The RPT controller 4230 may be configured to receive input signal(s) from one or more transducers 4270, one or more input devices 4220, and the humidifier 5000.

The RPT controller 4230 may be configured to provide output signal(s) to one or more of an output device 4290, a therapy device controller 4240, a data communication interface 4280, and the humidifier 5000.

In some forms of the present technology, the RPT controller 4230 is configured to implement the one or more methodologies described herein, such as the one or more algorithms 4300 expressed as computer programs stored in a non-transitory computer readable storage medium, such as memory 4260. In some forms of the present technology, the RPT controller 4230 may be integrated with an RPT device 4000. However, in some forms of the present technology, some methodologies may be performed by a remotely located device. For example, the remotely located device may determine control settings for a ventilator or detect respiratory related events by analysis of stored data such as from any of the sensors described herein.

**4.7.2.4 Clock**

The RPT device 4000 may include a clock 4232 that is connected to the RPT controller 4230.

**4.7.2.5 Therapy device controller**

In one form of the present technology, therapy device controller 4240 is a therapy control module 4330 that forms part of the algorithms executed by the RPT controller 4230.

In one form of the present technology, therapy device controller 4240 is a dedicated motor control integrated circuit. For example, in one form a MC33035 brushless DC motor controller, manufactured by ONSEMI is used.

#### 4.7.2.6 Protection circuits

The one or more protection circuits 4250 in accordance with the present technology may comprise an electrical protection circuit, a temperature and/or pressure safety circuit.

#### 4.7.2.7 Memory

In accordance with one form of the present technology the RPT device 4000 includes memory 4260, e.g., non-volatile memory. In some forms, memory 4260 may include battery powered static RAM. In some forms, memory 4260 may include volatile RAM.

Memory 4260 may be located on the PCBA 4202. Memory 4260 may be in the form of EEPROM, or NAND flash.

Additionally or alternatively, RPT device 4000 includes a removable form of memory 4260, for example a memory card made in accordance with the Secure Digital (SD) standard.

In one form of the present technology, the memory 4260 acts as a non-transitory computer readable storage medium on which is stored computer program instructions expressing the one or more methodologies described herein, such as the one or more algorithms 4300.

#### 4.7.2.8 Data communication systems

In one form of the present technology, a data communication interface 4280 is provided, and is connected to the RPT controller 4230. Data communication interface 4280 may be connectable to a remote external communication network 4282 and/or a local external communication network 4284. The remote external communication network 4282 may be connectable to a remote external device 4286. The local external communication network 4284 may be connectable to a local external device 4288.

In one form, data communication interface 4280 is part of the RPT controller 4230. In another form, data communication interface 4280 is separate from the RPT controller 4230, and may comprise an integrated circuit or a processor.

In one form, remote external communication network 4282 is the Internet. The data communication interface 4280 may use wired communication (e.g. via Ethernet, or optical fibre) or a wireless protocol (e.g. CDMA, GSM, LTE) to connect to the Internet.

In one form, local external communication network 4284 utilises one or more communication standards, such as Bluetooth, or a consumer infrared protocol.

In one form, remote external device 4286 is one or more computers, for example a cluster of networked computers. In one form, remote external device 4286 may be virtual computers, rather than physical computers. In either case, such a remote external device 4286 may be accessible to an appropriately authorised person such as a clinician.

The local external device 4288 may be a personal computer, mobile phone, tablet or remote control.

#### 4.7.2.9 Output devices including optional display, alarms

An output device 4290 in accordance with the present technology may take the form of one or more of a visual, audio and haptic unit. A visual display may be a Liquid Crystal Display (LCD) or Light Emitting Diode (LED) display.

##### *4.7.2.9.1 Display driver*

A display driver 4292 receives as an input the characters, symbols, or images intended for display on the display 4294, and converts them to commands that cause the display 4294 to display those characters, symbols, or images.

##### *4.7.2.9.2 Display*

A display 4294 is configured to visually display characters, symbols, or images in response to commands received from the display driver 4292. For example, the display 4294 may be an eight-segment display, in which case the display driver 4292 converts each character or symbol, such as the Fig "0", to eight logical signals indicating whether the eight respective segments are to be activated to display a particular character or symbol.

### 4.7.3 RPT device algorithms

As mentioned above, in some forms of the present technology, the RPT controller 4230 may be configured to implement one or more algorithms expressed as computer programs stored in a non-transitory computer readable storage medium, such as memory 4260. The algorithms are generally grouped into groups referred to as modules.

In other forms of the present technology, some portion or all of the algorithms may be implemented by a controller of an external device such as the local external device 4288 or the remote external device 4286. In such forms, data representing the input signals and / or intermediate algorithm outputs necessary for the portion of the algorithms to be executed at the external device may be communicated to the external device via the local external communication network 4284 or the remote external communication network 4282. In such forms, the portion of the algorithms to be executed at the external device may be expressed as computer programs stored in a non-transitory computer readable storage medium accessible to the controller of the external device. Such programs configure the controller of the external device to execute the portion of the algorithms.

In such forms, the therapy parameters generated by the external device via the therapy engine module 4320 (if such forms part of the portion of the algorithms executed by the external device) may be communicated to the RPT controller 4230 to be passed to the therapy control module 4330.

#### 4.7.3.1 Pre-processing module

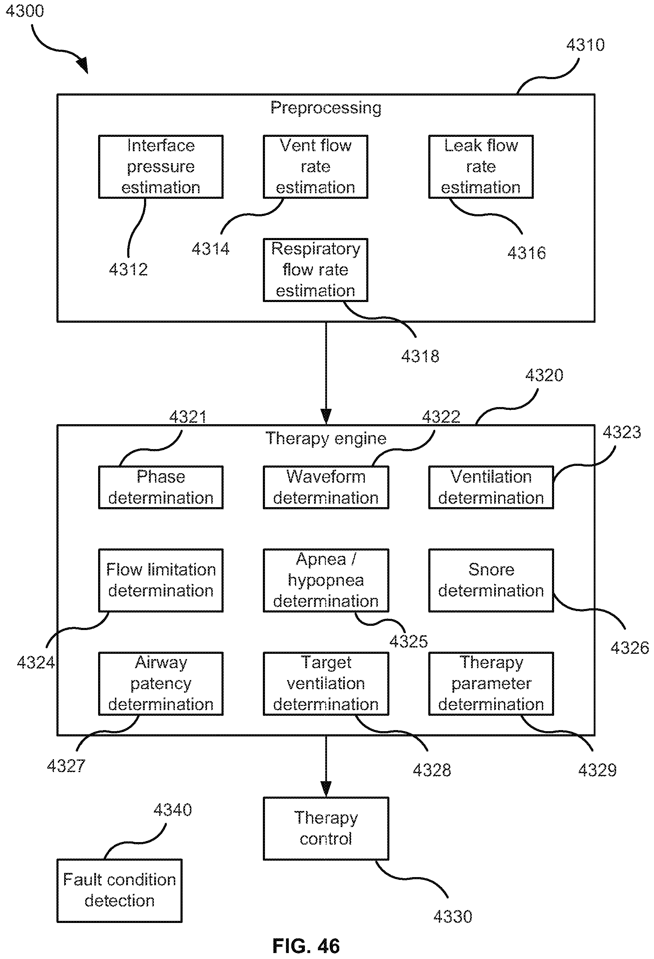

Referring now to Fig. 46 which is a flow chart that shows a schematic diagram of the algorithms implemented in an RPT device in accordance with one form of the present technology.

A pre-processing module 4310 in accordance with one form of the present technology receives as an input a signal from a transducer 4270, for example a flow rate sensor 4274 or pressure sensor 4272, and performs one or more process steps to calculate one or more output values that will be used as an input to another module, for example a therapy engine module 4320.

In one form of the present technology, the output values include the interface pressure *Pm,* the respiratory flow rate *Qr,* and the leak flow rate *Ql.*

In various forms of the present technology, the pre-processing module 4310 comprises one or more of the following algorithms: interface pressure estimation 4312, vent flow rate estimation 4314, leak flow rate estimation 4316, and respiratory flow rate estimation 4318.

##### *4.7.3.1.1 Interface pressure estimation*

In one form of the present technology, an interface pressure estimation algorithm 4312 receives as inputs a signal from the pressure sensor 4272 indicative of the pressure in the pneumatic path proximal to an outlet of the pneumatic block (the device pressure *Pd)* and a signal from the flow rate sensor 4274 representative of the flow rate of the airflow leaving the RPT device 4000 (the device flow rate *Qd).* The device flow rate *Qd,* absent any supplementary gas 4180, may be used as the total flow rate *Qt.* The interface pressure algorithm 4312 estimates the pressure drop $\Delta P$ through the duct 4050, the positioning and stabilizing structure 3300 and/or the patient interface 3102. The dependence of the pressure drop $\Delta P$ on the total flow rate *Qt* may be modelled for the particular duct 4050, the positioning and stabilizing structure 3300 and/or the patient interface 3102 by a pressure drop characteristic $\Delta P(Q)$. The interface pressure estimation algorithm, 4312 then provides as an output an estimated pressure, *Pm,* in the patient interface 3102. The pressure, *Pm,* in the patient interface 3102 may be estimated as the device pressure *Pd* minus the air circuit pressure drop $\Delta P$.

##### *4.7.3.1.2 Vent flow rate estimation*

In one form of the present technology, a vent flow rate estimation algorithm 4314 receives as an input an estimated pressure, *Pm,* in the patient interface 3012 from the interface pressure estimation algorithm 4312 and estimates a vent flow rate of air, *Qv,* from a vent 3400 in a patient interface 3102. The dependence of the vent flow rate *Qv* on the interface pressure *Pm* for the particular vent 3400 in use may be modelled by a vent characteristic *Qv(Pm).*

##### *4.7.3.1.3 Leak flow rate estimation*

In one form of the present technology, a leak flow rate estimation algorithm 4316 receives as an input a total flow rate, *Qt,* and a vent flow rate *Qv,* and provides as an output an estimate of the leak flow rate *Ql.* In one form, the leak flow rate estimation algorithm estimates the leak flow rate *Ql* by calculating an average of the difference between total flow rate *Qt* and vent flow rate *Qv* over a period sufficiently long to include several breathing cycles, e.g. about 10 seconds.

In one form, the leak flow rate estimation algorithm 4316 receives as an input a total flow rate *Qt,* a vent flow rate *Qv,* and an estimated pressure, *Pm,* in the patient interface 3102, and provides as an output a leak flow rate *Ql,* by calculating a leak conductance, and determining a leak flow rate *Ql* to be a function of leak conductance and pressure, *Pm.* Leak conductance is calculated as the quotient of low pass filtered non-vent flow rate equal to the difference between total flow rate *Qt* and vent flow rate *Qv,* and low pass filtered square root of pressure *Pm,* where the low pass filter time constant has a value sufficiently long to include several breathing cycles, e.g. about 10 seconds. The leak flow rate *Ql* may be estimated as the product of leak conductance and a function of pressure, *Pm.*

#### *4.7.3.1.4 Respiratory flow rate estimation*

In one form of the present technology, a respiratory flow rate estimation algorithm 4318 receives as an input a total flow rate, *Qt,* a vent flow rate, *Qv,* and a leak flow rate, *Ql,* and estimates a respiratory flow rate of *air, Qr,* to the patient, by subtracting the vent flow rate *Qv* and the leak flow rate *Ql* from the total flow rate *Qt.*

### 4.7.3.2 Therapy Engine Module

In one form of the present technology, a therapy engine module 4320 receives as inputs one or more of a pressure, *Pm,* in a patient interface 3102, and a respiratory flow rate of air to a patient, *Qr,* and provides as an output one or more therapy parameters.

In one form of the present technology, a therapy parameter is a treatment pressure *Pt.*

In one form of the present technology, therapy parameters are one or more of an amplitude of a pressure variation, a base pressure, and a target ventilation.

In various forms, the therapy engine module 4320 comprises one or more of the following algorithms: phase determination 4321, waveform determination 4322, ventilation determination 4323, inspiratory flow limitation determination 4324, apnea / hypopnea determination 4325, snore determination 4326, airway patency determination 4327, target ventilation determination 4328, and therapy parameter determination 4329.

#### *4.7.3.2.1 Phase determination*

In one form of the present technology, the RPT device 4000 does not determine phase.

In one form of the present technology, a phase determination algorithm 4321 receives as an input a signal indicative of respiratory flow rate, *Qr,* and provides as an output a phase $\Phi$ of a current breathing cycle of a patient 9000.

In some forms, known as discrete phase determination, the phase output $\Phi$ is a discrete variable. One implementation of discrete phase determination provides a bi-valued phase output $\Phi$ with values of either inhalation or exhalation, for example represented as values of 0 and 0.5 revolutions respectively, upon detecting the start of spontaneous inhalation and exhalation respectively. RPT devices 4000 that "trigger" and "cycle" effectively perform discrete phase determination, since the trigger and cycle points are the instants at which the phase changes from exhalation to inhalation and from inhalation to exhalation, respectively. In one implementation of bi-valued phase determination, the phase output $\Phi$ is determined to have a discrete value of 0 (thereby "triggering" the RPT device 4000) when the respiratory flow rate *Qr* has a value that exceeds a positive threshold, and a discrete value of 0.5 revolutions (thereby "cycling" the RPT device 4000) when a respiratory flow rate *Qr* has a value that is more negative than a negative threshold. The inhalation time *Ti* and the exhalation time *Te* may be estimated as typical values over many respiratory cycles of the time spent with phase $\Phi$ equal to 0 (indicating inspiration) and 0.5 (indicating expiration) respectively.

Another implementation of discrete phase determination provides a tri-valued phase output $\Phi$ with a value of one of inhalation, mid-inspiratory pause, and exhalation.

In other forms, known as continuous phase determination, the phase output $\Phi$ is a continuous variable, for example varying from 0 to 1 revolutions, or 0 to $2\pi$ radians. RPT devices 4000 that perform continuous phase determination may trigger and cycle when the continuous phase reaches 0 and 0.5 revolutions, respectively. In one implementation of continuous phase determination, a continuous value of phase $\Phi$ is determined using a fuzzy logic analysis of the respiratory flow rate *Qr.* A continuous value of phase determined in this implementation is often referred to as "fuzzy phase". In one implementation of a fuzzy phase determination algorithm 4321, the following rules are applied to the respiratory flow rate *Qr*:

1. If the respiratory flow rate is zero and increasing fast then the phase is 0 revolutions.

2. If the respiratory flow rate is large positive and steady then the phase is 0.25 revolutions.

3. If the respiratory flow rate is zero and falling fast, then the phase is 0.5 revolutions.

4. If the respiratory flow rate is large negative and steady then the phase is 0.75 revolutions.

5. If the respiratory flow rate is zero and steady and the 5-second low-pass filtered absolute value of the respiratory flow rate is large then the phase is 0.9 revolutions.

6. If the respiratory flow rate is positive and the phase is expiratory, then the phase is 0 revolutions.

7. If the respiratory flow rate is negative and the phase is inspiratory, then the phase is 0.5 revolutions.

8. If the 5-second low-pass filtered absolute value of the respiratory flow rate is large, the phase is increasing at a steady rate equal to the patient's breathing rate, low-pass filtered with a time constant of 20 seconds.

The output of each rule may be represented as a vector whose phase is the result of the rule and whose magnitude is the fuzzy extent to which the rule is true. The fuzzy extent to which the respiratory flow rate is "large", "steady", etc. is determined with suitable membership functions. The results of the rules, represented as vectors, are then combined by some function such as taking the centroid. In such a combination, the rules may be equally weighted, or differently weighted.

In another implementation of continuous phase determination, the phase $\Phi$ is first discretely estimated from the respiratory flow rate *Qr* as described above, as are the inhalation time *Ti* and the exhalation time *Te*. The continuous phase $\Phi$ at any instant may be determined as the half the proportion of the inhalation time *Ti* that has elapsed since the previous trigger instant, or 0.5 revolutions plus half the proportion of the exhalation time *Te* that has elapsed since the previous cycle instant (whichever instant was more recent).

#### *4.7.3.2.2 Waveform determination*

In one form of the present technology, the therapy parameter determination algorithm 4329 provides an approximately constant treatment pressure throughout a respiratory cycle of a patient.

In other forms of the present technology, the therapy control module 4330 controls the pressure generator 4140 to provide a treatment pressure *Pt* that varies as a function of phase $\Phi$ of a respiratory cycle of a patient according to a waveform template $\Pi(\Phi)$.

In one form of the present technology, a waveform determination algorithm 4322 provides a waveform template $\Pi(\Phi)$ with values in the range [0, 1] on the domain of phase values $\Phi$ provided by the phase determination algorithm 4321 to be used by the therapy parameter determination algorithm 4329.

In one form, suitable for either discrete or continuously-valued phase, the waveform template $\Pi(\Phi)$ is a square-wave template, having a value of 1 for values of phase up to and including 0.5 revolutions, and a value of 0 for values of phase above 0.5 revolutions. In one form, suitable for continuously-valued phase, the waveform template $\Pi(\Phi)$ comprises two smoothly curved portions, namely a smoothly curved (e.g. raised cosine) rise from 0 to 1 for values of phase up to 0.5 revolutions, and a smoothly curved (e.g. exponential) decay from 1 to 0 for values of phase above 0.5 revolutions. In one form, suitable for continuously-valued phase, the waveform template $\Pi(\Phi)$ is based on a square wave, but with a smooth rise from 0 to 1 for values of phase up to a "rise time" that is less than 0.5 revolutions, and a smooth fall from 1 to 0 for values of phase within a "fall time" after 0.5 revolutions, with a "fall time" that is less than 0.5 revolutions.

In some forms of the present technology, the waveform determination algorithm 4322 selects a waveform template $\Pi(\Phi)$ from a library of waveform templates, dependent on a setting of the RPT device. Each waveform template $\Pi(\Phi)$ in the library may be provided as a lookup table of values $\Pi$ against phase values $\Phi$. In other forms, the waveform determination algorithm 4322 computes a waveform template $\Pi(\Phi)$ "on the fly" using a predetermined functional form, possibly parametrised by one or more parameters (e.g. time constant of an exponentially curved portion). The parameters of the functional form may be predetermined or dependent on a current state of the patient 9000.

In some forms of the present technology, suitable for discrete bi-valued phase of either inhalation ($\Phi$ = 0 revolutions) or exhalation ($\Phi$ = 0.5 revolutions), the waveform determination algorithm 4322 computes a waveform template $\Pi$ "on the fly" as a function of both discrete phase $\Phi$ and time *t* measured since the most recent trigger instant. In one such form, the waveform determination algorithm 4322 computes the waveform template $\Pi(\Phi, t)$ in two portions (inspiratory and expiratory) as follows:

$$\Pi(\Phi,t)=\begin{cases}\Pi_i(t), & \Phi=0\\ \Pi_e(t-T_i), & \Phi=0.5\end{cases}$$

where $\Pi_i(t)$ and $\Pi_e(t)$ are inspiratory and expiratory portions of the waveform template $\Pi(\Phi, t)$. In one such form, the inspiratory portion $\Pi_i(t)$ of the waveform template is a smooth rise from 0 to 1 parametrised by a rise time, and the expiratory portion $\Pi_e(t)$ of the waveform template is a smooth fall from 1 to 0 parametrised by a fall time.

### *4.7.3.2.3 Ventilation determination*

In one form of the present technology, a ventilation determination algorithm 4323 receives an input a respiratory flow rate *Qr,* and determines a measure indicative of current patient ventilation, *Vent.*
In some implementations, the ventilation determination algorithm 4323 determines a measure of ventilation *Vent* that is an estimate of actual patient ventilation. One such implementation is to take half the absolute value of respiratory flow rate, *Qr,* optionally filtered by low-pass filter such as a second order Bessel low-pass filter with a corner frequency of 0.11 Hz.
In other implementations, the ventilation determination algorithm 4323 determines a measure of ventilation *Vent* that is broadly proportional to actual patient ventilation. One such implementation estimates peak respiratory flow rate *Qpeak* over the inspiratory portion of the cycle. This and many other procedures involving sampling the respiratory flow rate *Qr* produce measures which are broadly proportional to ventilation, provided the flow rate waveform shape does not vary very much (here, the shape of two breaths is taken to be similar when the flow rate waveforms of the breaths normalised in time and amplitude are similar). Some simple examples include the median positive respiratory flow rate, the median of the absolute value of respiratory flow rate, and the standard deviation of flow rate. Arbitrary linear combinations of arbitrary order statistics of the absolute value of respiratory flow rate using positive coefficients, and even some using both positive and negative coefficients, are approximately proportional to ventilation. Another example is the mean of the respiratory flow rate in the middle *K* proportion (by time) of the inspiratory portion, where $0 < K < 1$. There is an arbitrarily large number of measures that are exactly proportional to ventilation if the flow rate shape is constant.

### *4.7.3.2.4 Determination of Inspiratory Flow limitation*

In one form of the present technology, the RPT controller 4230 executes an inspiratory flow limitation determination algorithm 4324 for the determination of the extent of inspiratory flow limitation.
In one form, the inspiratory flow limitation determination algorithm 4324 receives as an input a respiratory flow rate signal *Qr* and provides as an output a metric of the extent to which the inspiratory portion of the breath exhibits inspiratory flow limitation.
In one form of the present technology, the inspiratory portion of each breath is identified by a zero-crossing detector. A number of evenly spaced points (for example, sixty-five), representing points in time, are interpolated by an interpolator along the inspiratory flow rate-time curve for each breath. The curve described by the points is then scaled by a scalar to have unity length (duration/period) and unity area to remove the effects of changing breathing rate and depth. The scaled breaths are then compared in a comparator with a pre-stored template representing a normal unobstructed breath, similar to the inspiratory portion of the breath. Breaths deviating by more than a specified threshold (typically 1 scaled unit) at any time during the inspiration from this template, such as those due to coughs, sighs, swallows and hiccups, as determined by a test element, are rejected. For non-rejected data, a moving average of the first such scaled point is calculated by the RPT controller 4230 for the preceding several inspiratory events. This is repeated over the same inspiratory events for the second such point, and so on. Thus, for example, sixty five scaled data points are generated by the RPT controller 4230, and represent a moving average of the preceding several inspiratory events, e.g., three events. The moving average of continuously updated values of the (e.g., sixty five) points are hereinafter called the "scaled flow rate ", designated as *Qs(t).* Alternatively, a single inspiratory event can be utilised rather than a moving average.
From the scaled flow rate, two shape factors relating to the determination of partial obstruction may be calculated.
Shape factor 1 is the ratio of the mean of the middle (e.g. thirty-two) scaled flow rate points to the mean overall (e.g. sixty-five) scaled flow rate points. Where this ratio is in excess of unity, the breath will be taken to be normal. Where the ratio is unity or less, the breath will be taken to be obstructed. A ratio of about 1.17 is taken as a threshold between partially obstructed and unobstructed breathing, and equates to a degree of obstruction that would permit maintenance of adequate oxygenation in a typical patient.
Shape factor 2 is calculated as the RMS deviation from unit scaled flow rate, taken over the middle (e.g. thirty two) points. An RMS deviation of about 0.2 units is taken to be normal. An RMS deviation of zero is taken to be a totally flow-limited breath. The closer the RMS deviation to zero, the breath will be taken to be more flow limited.
Shape factors 1 and 2 may be used as alternatives, or in combination. In other forms of the present technology, the number of sampled points, breaths and middle points may differ from those described above. Furthermore, the threshold values can be other than those described.

### *4.7.3.2.5 Determination of apneas and hypopneas*

In one form of the present technology, the RPT controller 4230 executes an apnea / hypopnea determination algorithm 4325 for the determination of the presence of apneas and/or hypopneas.
In one form, the apnea / hypopnea determination algorithm 4325 receives as an input a respiratory flow rate signal *Qr* and provides as an output a flag that indicates that an apnea or a hypopnea has been detected.

In one form, an apnea will be said to have been detected when a function of respiratory flow rate *Qr* falls below a flow rate threshold for a predetermined period of time. The function may determine a peak flow rate, a relatively short-term mean flow rate, or a flow rate intermediate of relatively short-term mean and peak flow rate, for example an RMS flow rate. The flow rate threshold may be a relatively long-term measure of flow rate.

In one form, a hypopnea will be said to have been detected when a function of respiratory flow rate *Qr* falls below a second flow rate threshold for a predetermined period of time. The function may determine a peak flow, a relatively short-term mean flow rate, or a flow rate intermediate of relatively short-term mean and peak flow rate, for example an RMS flow rate. The second flow rate threshold may be a relatively long-term measure of flow rate. The second flow rate threshold is greater than the flow rate threshold used to detect apneas.

#### *4.7.3.2.6 Determination of snore*

In one form of the present technology, the RPT controller 4230 executes one or more snore determination algorithms 4326 for the determination of the extent of snore.

In one form, the snore determination algorithm 4326 receives as an input a respiratory flow rate signal *Qr* and provides as an output a metric of the extent to which snoring is present.

The snore determination algorithm 4326 may comprise the step of determining the intensity of the flow rate signal in the range of 30-300 Hz. Further, the snore determination algorithm 4326 may comprise a step of filtering the respiratory flow rate signal *Qr* to reduce background noise, e.g., the sound of airflow in the system from the blower.

#### *4.7.3.2.7 Determination of airway patency*

In one form of the present technology, the RPT controller 4230 executes one or more airway patency determination algorithms 4327 for the determination of the extent of airway patency.

In one form, the airway patency determination algorithm 4327 receives as an input a respiratory flow rate signal *Qr,* and determines the power of the signal in the frequency range of about 0.75 Hz and about 3 Hz. The presence of a peak in this frequency range is taken to indicate an open airway. The absence of a peak is taken to be an indication of a closed airway.

In one form, the frequency range within which the peak is sought is the frequency of a small forced oscillation in the treatment pressure *Pt.* In one implementation, the forced oscillation is of frequency 2 Hz with amplitude about 1 $cmH_2O$.

In one form, airway patency determination algorithm 4327 receives as an input a respiratory flow rate signal *Qr,* and determines the presence or absence of a cardiogenic signal. The absence of a cardiogenic signal is taken to be an indication of a closed airway.

#### *4.7.3.2.8 Determination of target ventilation*

In one form of the present technology, the RPT controller 4230 takes as input the measure of current ventilation, *Vent,* and executes one or more target ventilation determination algorithms 4328 for the determination of a target value *Vtgt* for the measure of ventilation.

In some forms of the present technology, there is no target ventilation determination algorithm 4328, and the target value *Vtgt* is predetermined, for example by hard-coding during configuration of the RPT device 4000 or by manual entry through the input device 4220.

In other forms of the present technology, such as adaptive servo-ventilation (ASV), the target ventilation determination algorithm 4328 computes a target value *Vtgt* from a value *Vtyp* indicative of the typical recent ventilation of the patient.

In some forms of adaptive servo-ventilation, the target ventilation *Vtgt* is computed as a high proportion of, but less than, the typical recent ventilation *Vtyp.* The high proportion in such forms may be in the range (80%, 100%), or (85%, 95%), or (87%, 92%).

In other forms of adaptive servo-ventilation, the target ventilation *Vtgt* is computed as a slightly greater than unity multiple of the typical recent ventilation *Vtyp.*

The typical recent ventilation *Vtyp* is the value around which the distribution of the measure of current ventilation *Vent* over multiple time instants over some predetermined timescale tends to cluster, that is, a measure of the central tendency of the measure of current ventilation over recent history. In one implementation of the target ventilation determination algorithm 4328, the recent history is of the order of several minutes, but in any case should be longer than the timescale of Cheyne-Stokes waxing and waning cycles. The target ventilation determination algorithm 4328 may use any of the variety of well-known measures of central tendency to determine the typical recent ventilation *Vtyp* from the measure of current ventilation, *Vent.* One such measure is the output of a low-pass filter on the measure of current ventilation *Vent,* with time constant equal to one hundred seconds.

#### *4.7.3.2.9 Determination of therapy parameters*

In some forms of the present technology, the RPT controller 4230 executes one or more therapy parameter determination algorithms 4329 for the determination of one or more therapy parameters using the values returned by one or more of the other algorithms in the therapy engine module 4320.

In one form of the present technology, the therapy parameter is an instantaneous treatment pressure *Pt.* In one implementation of this form, the therapy parameter determination algorithm 4329 determines the treatment pressure *Pt* using the equation

$$Pt = A\Pi(\Phi, t) + P_0 \qquad (1)$$

where:

- A is the amplitude,
- $\Pi(\Phi, t)$ is the waveform template value (in the range 0 to 1) at the current value $\Phi$ of phase and t of time, and
- $P_0$ is a base pressure.

If the waveform determination algorithm 4322 provides the waveform template $\Pi(\Phi, t)$ as a lookup table of values $\Pi$ indexed by phase $\Phi$, the therapy parameter determination algorithm 4329 applies equation (1) by locating the nearest lookup table entry to the current value $\Phi$ of phase returned by the phase determination algorithm 4321, or by interpolation between the two entries straddling the current value $\Phi$ of phase.

The values of the amplitude *A* and the base pressure $P_0$ may be set by the therapy parameter determination algorithm 4329 depending on the chosen respiratory pressure therapy mode in the manner described below.

#### 4.7.3.3 Therapy Control module

The therapy control module 4330 in accordance with one aspect of the present technology receives as inputs the therapy parameters from the therapy parameter determination algorithm 4329 of the therapy engine module 4320, and controls the pressure generator 4140 to deliver a flow of air in accordance with the therapy parameters.

In one form of the present technology, the therapy parameter is a treatment pressure *Pt,* and the therapy control module 4330 controls the pressure generator 4140 to deliver a flow of air whose interface pressure *Pm* at the patient interface 3102 is equal to the treatment pressure *Pt.*

#### 4.7.3.4 Detection of fault conditions

In one form of the present technology, the RPT controller 4230 executes one or more methods 4340 for the detection of fault conditions. The fault conditions detected by the one or more methods 4340 may include at least one of the following:

- Power failure (no power, or insufficient power)
- Transducer fault detection
- Failure to detect the presence of a component
- Operating parameters outside recommended ranges (e.g. pressure, flow rate, temperature, $PaO_2$)
- Failure of a test alarm to generate a detectable alarm signal.

Upon detection of the fault condition, the corresponding algorithm 4340 signals the presence of the fault by one or more of the following:

- Initiation of an audible, visual &/or kinetic (e.g. vibrating) alarm
- Sending a message to an external device
- Logging of the incident

### 4.7.4 Supplementary gas delivery

In one form of the present technology, supplementary gas, e.g. oxygen, 4180 is delivered to one or more points in the pneumatic path, such as upstream of the pneumatic block 4020, to the air circuit 4170, and/or to the patient interface 3102.

## 4.8 HUMIDIFIER

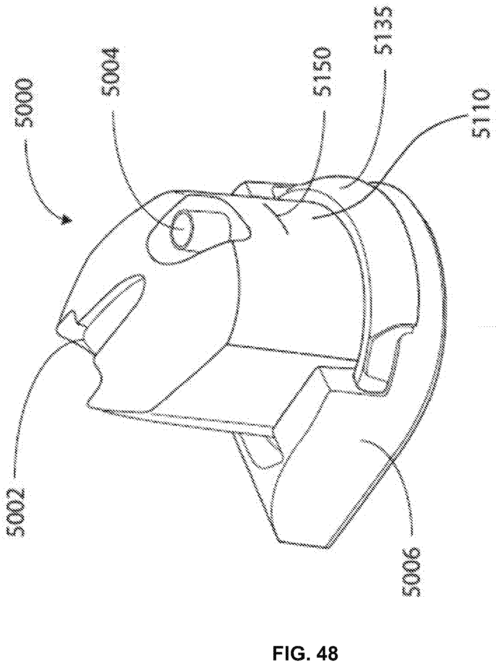

Referring now to Figs. 48 to 50 which show the components of the humidifier 5000.

### 4.8.1 Humidifier overview

In one form of the present technology there is provided a humidifier 5000 to change the absolute humidity of air or gas for delivery to a patient relative to ambient air. Typically, the humidifier 5000 is used to increase the absolute humidity and increase the temperature of the flow of respiratory gas (relative to ambient air) before delivery to the patient's airways.

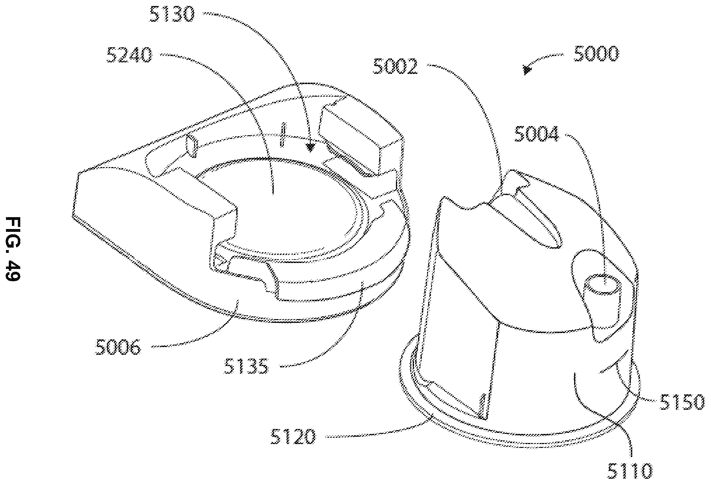

The humidifier 5000 may comprise a humidifier reservoir 5110, a humidifier inlet 5002 to receive a flow of air, and a humidifier outlet 5004 to deliver a humidified flow of air. In some forms, as shown in Fig. 48 and Fig. 49, an inlet and an outlet of the humidifier reservoir 5110 may be the humidifier inlet 5002 and the humidifier outlet 5004 respectively. The humidifier 5000 may further comprise a humidifier base 5006, which may be adapted to receive the humidifier reservoir 5110 and comprise a heating element 5240.

Respiratory humidifiers 5000 are available in many forms and may be a standalone device that is coupled to an RPT device 4000 via an air circuit, is integrated with the RPT device 4000 or configured to be directly coupled to the relevant RPT device 4000. While known passive humidifiers 5000 can provide some relief, generally a heated humidifier may be used to provide sufficient humidity and temperature to the air so that the patient 9000 will be comfortable.

Humidifiers 5000 typically comprise a water reservoir 5110 or tub having a capacity of several hundred milliliters (ml), a heating element 5240 for heating the water in the reservoir 5110, a control to enable the level of humidification to be varied, a gas inlet to receive gas from the flow generator or RPT device 4000, and a gas outlet adapted to be connected to the positioning and stabilizing structure 3300 that delivers the humidified gas to the patient interface 3102.

Heated passover humidification is one common form of humidification used with an RPT device 4000. In such humidifiers 5000 the heating element 5240 may be incorporated in a heater plate which sits under, and is in thermal contact with, the water tub 5110. Thus, heat is transferred from the heater plate to the water reservoir 5110 primarily by conduction. The air flow from the RPT device 4000 passes over the heated water in the water tub 5110 resulting in water vapour being taken up by the air flow. The ResMed H4i™ and H5i™ Humidifiers are examples of such heated passover humidifiers that are used in combination with ResMed S8 and S9 CPAP devices respectively.

Other humidifiers may also be used such as a bubble or diffuser humidifier, a jet humidifier or a wicking humidifier. In a bubble or diffuser humidifier the *air* is conducted below the surface of the water and allowed to bubble back to the top. A jet humidifier produces an aerosol of water and baffles or filters may be used so that the particles are either removed or evaporated before leaving the humidifier. A wicking humidifier uses a water absorbing material, such as sponge or paper, to absorb water by capillary action. The water absorbing material is placed within or adjacent at least a portion of the air flow path to allow evaporation of the water in the absorbing material to be taken up into the air flow.

An alternative form of humidification is provided by the ResMed HumiCare™ D900 humidifier that uses a CounterStream™ technology that directs the air flow over a large surface area in a first direction whilst supplying heated water to the large surface area in a second opposite direction. The ResMed HumiCare™ D900 humidifier may be used with a range of invasive and non-invasive ventilators.

### 4.8.2 Humidifier components

#### 4.8.2.1 Water reservoir

According to one arrangement, the humidifier 5000 may comprise a water reservoir 5110 configured to hold, or retain, a volume of liquid (e.g. water) to be evaporated for humidification of the flow of air. The water reservoir 5110 may be configured to hold a predetermined maximum volume of water in order to provide adequate humidification for at least the duration of a respiratory therapy session, such as one evening of sleep. Typically, the reservoir 5110 is configured to hold several hundred millilitres of water, e.g. 300 millilitres (ml), 325 ml, 350 ml or 400 ml. In other forms, the humidifier 5000 may be configured to receive a supply of water from an external water source such as a building's water supply system.

According to one aspect, the water reservoir 5110 is configured to add humidity to a flow of air from the RPT device 4000 as the flow of air travels therethrough. In one form, the water reservoir 5110 may be configured to encourage the flow of air to travel in a tortuous path through the reservoir 5110 while in contact with the volume of water therein.

According to one form, the reservoir 5110 may be removable from the humidifier 5000, for example in a lateral direction as shown in Fig. 48 and Fig. 49.

The reservoir 5110 may also be configured to discourage egress of liquid therefrom, such as when the reservoir 5110 is displaced and/or rotated from its normal, working orientation, such as through any apertures and/or in between its subcomponents. As the flow of air to be humidified by the humidifier 5000 is typically pressurised, the reservoir 5110 may also be configured to prevent losses in pneumatic pressure through leak and/or flow impedance.

#### 4.8.2.2 Conductive portion

According to one arrangement, the reservoir 5110 comprises a conductive portion 5120 configured to allow efficient transfer of heat from the heating element 5240 to the volume of liquid in the reservoir 5110. In one form, the conductive portion 5120 may be arranged as a plate, although other shapes may also be suitable. All or a part of the conductive portion 5120 may be made of a thermally conductive material such as aluminium (e.g. approximately 2 mm thick, such as 1 mm, 1.5 mm, 2.5 mm or 3 mm), another heat conducting metal or some plastics. In some cases, suitable heat conductivity may be achieved with less conductive materials of suitable geometry.

#### 4.8.2.3 Humidifier reservoir dock

In one form, the humidifier 5000 may comprise a humidifier reservoir dock 5130 (as shown in Fig. 49) configured to receive the humidifier reservoir 5110. In some arrangements, the humidifier reservoir dock 5130 may comprise a locking feature such as a locking lever 5135 configured to retain the reservoir 5110 in the humidifier reservoir dock 5130.

#### 4.8.2.4 Water level indicator

The humidifier reservoir 5110 may comprise a water level indicator 5150 as shown in Figs. 48 to 49. In some forms, the water level indicator 5150 may provide one or more indications to a user such as the patient 9000 or a care giver regarding a quantity of the volume of water in the humidifier reservoir 5110. The one or more indications provided by the water level indicator 5150 may include an indication of a maximum, predetermined volume of water, any portions thereof, such as 25%, 50% or 75% or volumes such as 200 ml, 300 ml or 400ml.

#### 4.8.2.5 Humidifier transducer(s)

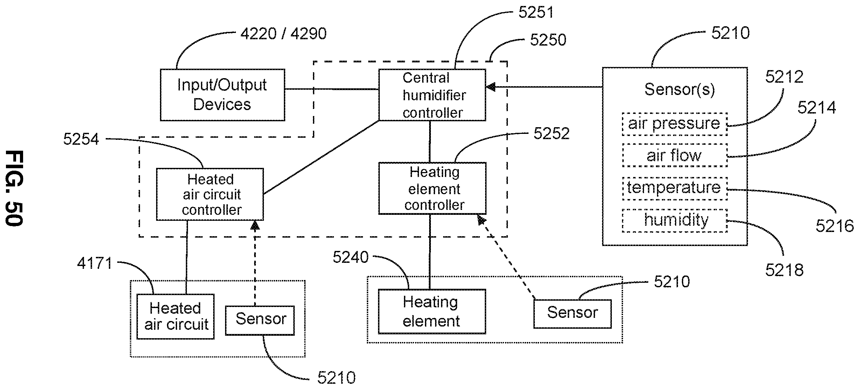

The humidifier 5000 may comprise one or more humidifier transducers (sensors) 5210 instead of, or in addition to, transducers 4270 described above. Humidifier transducers 5210 may include one or more of an air pressure sensor 5212, an *air* flow rate transducer 5214, a temperature sensor 5216, or a humidity sensor 5218 as shown in Fig. 50. A humidifier transducer 5210 may produce one or more output signals which may be communicated to a controller such as the RPT controller 4230 and/or the humidifier controller 5250. In some forms, a humidifier transducer may be located externally to the humidifier 5000 (such as in the duct 4050) while communicating the output signal to the controller.

##### *4.8.2.5.1 Pressure transducer*

One or more pressure transducers 5212 may be provided to the humidifier 5000 in addition to, or instead of, a pressure sensor 4272 provided in the RPT device 4000.

##### *4.8.2.5.2 Flow rate transducer*

One or more flow rate transducers 5214 may be provided to the humidifier 5000 in addition to, or instead of, a flow rate sensor 4274 provided in the RPT device 4000.

##### *4.8.2.5.3 Temperature transducer*

The humidifier 5000 may comprise one or more temperature transducers 5216. The one or more temperature transducers 5216 may be configured to measure one or more temperatures such as of the heating element 5240 and/or of the flow of *air* downstream of the humidifier outlet 5004. In some forms, the humidifier 5000 may further comprise a temperature sensor 5216 to detect the temperature of the ambient air.

##### *4.8.2.5.4 Humidity transducer*

In one form, the humidifier 5000 may comprise one or more humidity sensors 5218 to detect a humidity of a gas, such as the ambient air. The humidity sensor 5218 may be placed towards the humidifier outlet 5004 in some forms to measure a humidity of the gas delivered from the humidifier 5000. The humidity sensor may be an absolute humidity sensor or a relative humidity sensor.

#### 4.8.2.6 Heating element

A heating element 5240 may be provided to the humidifier 5000 in some cases to provide a heat input to one or more of the volume of water in the humidifier reservoir 5110 and/or to the flow of air. The heating element 5240 may comprise a heat generating component such as an electrically resistive heating track. One suitable example of a heating element 5240 is a layered heating element such as one described in the PCT Patent Application Publication No. WO 2012/171072.

In some forms, the heating element 5240 may be provided in the humidifier base 5006 where heat may be provided to the humidifier reservoir 5110 primarily by conduction as shown in Fig. 49.

#### 4.8.2.7 Humidifier controller

According to one arrangement of the present technology, a humidifier 5000 may comprise a humidifier controller 5250 as shown in Fig. 50. In one form, the humidifier controller 5250 may be a part of the RPT controller 4230. In another form, the humidifier controller 5250 may be a separate controller, which may be in communication with the RPT controller 4230.

In one form, the humidifier controller 5250 may receive as inputs measures of properties (such as temperature, humidity, pressure and/or flow rate), for example of the flow of air, the water in the reservoir 5110 and/or the humidifier 5000. The humidifier controller 5250 may also be configured to execute or implement humidifier algorithms and/or deliver one or more output signals.

As shown in Fig. 50, the humidifier controller 5250 may comprise one or more controllers, such as a central humidifier controller 5251, a heated air circuit controller 5254 configured to control the temperature of a heated air circuit 4171 and/or a heating element controller 5252 configured to control the temperature of a heating element 5240.

### 4.9 CENTRAL CONTROLLER

In certain forms of the present technology (not shown in the Figs), the respiratory treatment system 500 comprises a central controller which includes one or more of the flow controller 3040, the RPT controller 4230, the therapy device controller 4240, the humidifier controller 5250 and the heating element controller 5252.

In one form of the present technology, the central controller may include one or more processors which is/are configured to carry out the functions of one or more of the flow controller 3040, the RPT controller 4230, the therapy device controller 4240, the humidifier controller 5250 and the heating element controller 5252.

### 4.10 AIR CIRCUIT

An air circuit 4170 in accordance with an aspect of the present technology is a conduit or a tube constructed and arranged to allow, in use, a flow of air to travel between two components such as RPT device 4000 and the patient interface system 3000, 3000A, 3000B.

In particular, the air circuit 4170 may be in fluid connection with the outlet of the pneumatic block 4020 and the patient interface. The air circuit may be referred to as an air delivery tube. In some cases there may be separate limbs of the circuit for inhalation and exhalation. In other cases a single limb is used.

In some forms, the air circuit 4170 may comprise one or more heating elements configured to heat air in the air circuit, for example to maintain or raise the temperature of the air. The heating element may be in a form of a heated wire circuit, and may comprise one or more transducers, such as temperature sensors. In one form, the heated wire circuit may be helically wound around the axis of the air circuit 4170. The heating element may be in communication with a controller such as a central controller 4230. One example of an air circuit 4170 comprising a heated wire circuit is described in United States Patent 8,733,349.

#### 4.10.1 Supplementary gas delivery

In one form of the present technology, supplementary gas, e.g. oxygen, 4180 is delivered to one or more points in the pneumatic path, such as upstream of the pneumatic block 4020, to the air circuit 4170, and/or to a patient interface.

### 4.11 PORTABLE OXYGEN CONCENTRATOR

In certain forms of the present technology, the respiratory gas provided to the patient interface system 3000 comprises concentrated oxygen.

In one form of the present technology, the concentrated oxygen may be generated by a portable oxygen concentrator.

Oxygen concentrators may take advantage of pressure swing adsorption (PSA). Pressure swing adsorption may involve using a compressor to increase gas pressure inside a canister that contains particles of a gas separation adsorbent. As the pressure increases, certain molecules in the gas may become adsorbed onto the gas separation adsorbent. Removal of a portion of the gas in the canister under the pressurized conditions allows separation of the non-adsorbed molecules from the adsorbed molecules. The gas separation adsorbent may be regenerated by reducing the pressure, which reverses the adsorption of molecules from the adsorbent. Further details regarding oxygen concentrators may be found, for example, in U.S. Published Patent Application No. 2009-0065007, published March 12, 2009, and entitled "Oxygen Concentrator Apparatus and Method".

Ambient air usually includes approximately 78% nitrogen and 21% oxygen with the balance comprised of argon, carbon dioxide, water vapor and other trace gases. If a gas mixture such as air, for example, is passed under pressure through a vessel containing a gas separation adsorbent bed that attracts nitrogen more strongly than it does oxygen, part or all of the nitrogen will stay in the bed, and the gas coming out of the vessel will be enriched in oxygen. When the bed reaches the end of its capacity to adsorb nitrogen, it can be regenerated by reducing the pressure, thereby releasing the adsorbed nitrogen. It is then ready for another cycle of producing oxygen enriched gas. By alternating canisters in a two-canister system, one canister can be collecting oxygen while the other canister is being purged (resulting in a continuous separation of the oxygen from the nitrogen). In this manner, oxygen can be accumulated, such as in a storage container or other pressurizable vessel or conduit coupled to the canisters, from the ambient air for a variety of uses include providing supplemental oxygen to patients.

## 4.12 RESPIRATORY THERAPY MODES

Various respiratory therapy modes may be implemented by the disclosed respiratory treatment system 500.

### 4.12.1 CPAP therapy

In some implementations of respiratory pressure therapy, the RPT controller 4230 sets the treatment pressure *Pt* according to the treatment pressure equation (1) as part of the therapy parameter determination algorithm 4329. In one such implementation, the amplitude *A* is identically zero, so the treatment pressure *Pt* (which represents a target value to be achieved by the interface pressure *Pm* at the current instant of time) is identically equal to the base pressure $P_0$ throughout the respiratory cycle. Such implementations are generally grouped under the heading of CPAP therapy. In such implementations, there is no need for the therapy engine module 4320 to determine phase $\Phi$ or the waveform template $\Pi(\Phi)$.

In CPAP therapy, the base pressure $P_0$ may be a constant value that is hard-coded or manually entered to the RPT device 4000. Alternatively, the RPT controller 4230 may repeatedly compute the base pressure $P_0$ as a function of indices or measures of sleep disordered breathing returned by the respective algorithms in the therapy engine module 4320, such as one or more of flow limitation, apnea, hypopnea, patency, and snore. This alternative is sometimes referred to as APAP therapy.

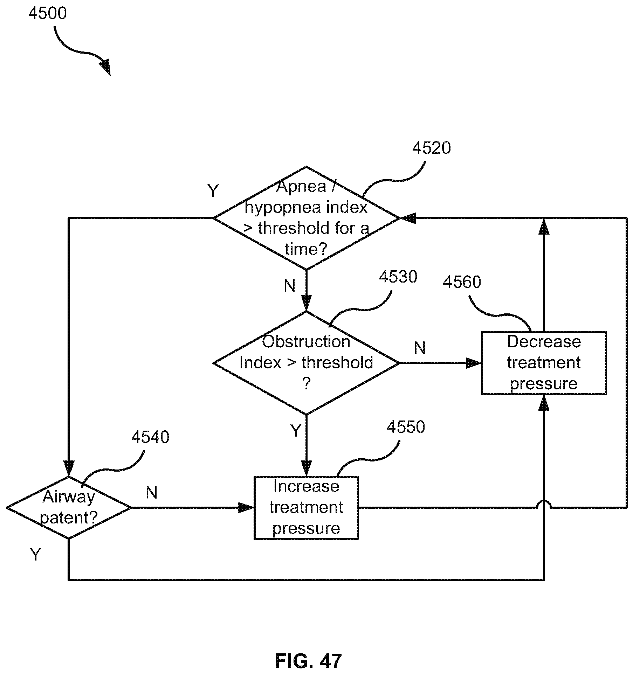

Fig. 47 is a flow chart illustrating a method 4500 carried out by the RPT controller 4230 to continuously compute the base pressure $P_0$ as part of an APAP therapy implementation of the therapy parameter determination algorithm 4329, when the pressure support *A* is identically zero.

The method 4500 starts at step 4520, at which the RPT controller 4230 compares the measure of the presence of apnea / hypopnea with a first threshold, and determines whether the measure of the presence of apnea / hypopnea has exceeded the first threshold for a predetermined period of time, indicating an apnea / hypopnea is occurring. If so, the method 4500 proceeds to step 4540; otherwise, the method 4500 proceeds to step 4530. At step 4540, the RPT controller 4230 compares the measure of airway patency with a second threshold. If the measure of airway patency exceeds the second threshold, indicating the airway is patent, the detected apnea / hypopnea is deemed central, and the method 4500 proceeds to step 4560; otherwise, the apnea / hypopnea is deemed obstructive, and the method 4500 proceeds to step 4550.

At step 4530, the RPT controller 4230 compares the measure of flow limitation with a third threshold. If the measure of flow limitation exceeds the third threshold, indicating inspiratory flow is limited, the method 4500 proceeds to step 4550; otherwise, the method 4500 proceeds to step 4560.

At step 4550, the RPT controller 4230 increases the base pressure $P_0$ by a predetermined pressure increment $\Delta P$, provided the resulting treatment pressure *Pt* would not exceed a maximum treatment pressure *Pmax.* In one implementation, the predetermined pressure increment $\Delta P$ and maximum treatment pressure *Pmax* are 1 $cmH_2O$ and 25 $cmH_2O$ respectively. In other implementations, the pressure increment $\Delta P$ can be as low as 0.1 $cmH_2O$ and

as high as 3 $cmH_2O$, or as low as 0.5 $cmH_2O$ and as high as 2 $cmH_2O$. In other implementations, the maximum treatment pressure *Pmax* can be as low as 15 $cmH_2O$ and as high as 35 $cmH_2O$, or as low as 20 $cmH_2O$ and as high as 30 $cmH_2O$. The method 4500 then returns to step 4520.

At step 4560, the RPT controller 4230 decreases the base pressure $P_0$ by a decrement, provided the decreased base pressure $P_0$ would not fall below a minimum treatment pressure *Pmin.* The method 4500 then returns to step 4520. In one implementation, the decrement is proportional to the value of $P_0$-*Pmin,* so that the decrease in $P_0$ to the minimum treatment pressure *Pmin* in the absence of any detected events is exponential. In one implementation, the constant of proportionality is set such that the time constant $\tau$ of the exponential decrease of $P_0$ is 60 minutes, and the minimum treatment pressure *Pmin* is 4 $cmH_2O$. In other implementations, the time constant $\tau$ could be as low as 1 minute and as high as 300 minutes, or as low as 5 minutes and as high as 180 minutes. In other implementations, the minimum treatment pressure *Pmin* can be as low as 0 $cmH_2O$ and as high as 8 $cmH_2O$, or as low as 2 $cmH_2O$ and as high as 6 $cmH_2O$. Alternatively, the decrement in $P_0$ could be predetermined, so the decrease in $P_0$ to the minimum treatment pressure *Pmin* in the absence of any detected events is linear.

### 4.12.2 Bi-level therapy

In other implementations of this form of the present technology, the value of amplitude *A* in equation (1) may be positive. Such implementations are known as bi-level therapy, because in determining the treatment pressure *Pt* using equation (1) with positive amplitude *A*, the therapy parameter determination algorithm 4329 oscillates the treatment pressure *Pt* between two values or levels in synchrony with the spontaneous respiratory effort of the patient 9000. That is, based on the typical waveform templates $\Pi(\Phi, t)$ described above, the therapy parameter determination algorithm 4329 increases the treatment pressure *Pt* to $P_0 + A$ (known as the IPAP) at the start of, or during, or inspiration and decreases the treatment pressure *Pt* to the base pressure $P_0$ (known as the EPAP) at the start of, or during, expiration.

In some forms of bi-level therapy, the IPAP is a treatment pressure that has the same purpose as the treatment pressure in CPAP therapy modes, and the EPAP is the IPAP minus the amplitude *A*, which has a "small" value (a few $cmH_2O$) sometimes referred to as the Expiratory Pressure Relief (EPR). Such forms are sometimes referred to as CPAP therapy with EPR, which is generally thought to be more comfortable than straight CPAP therapy. In CPAP therapy with EPR, either or both of the IPAP and the EPAP may be constant values that are hard-coded or manually entered to the RPT device 4000. Alternatively, the therapy parameter determination algorithm 4329 may repeatedly compute the IPAP and / or the EPAP during CPAP with EPR. In this alternative, the therapy parameter determination algorithm 4329 repeatedly computes the EPAP and / or the IPAP as a function of indices or measures of sleep disordered breathing returned by the respective algorithms in the therapy engine module 4320 in analogous fashion to the computation of the base pressure $P_0$ in APAP therapy described above.

In other forms of bi-level therapy, the amplitude A is large enough that the RPT device 4000 does some or all of the work of breathing of the patient 9000. In such forms, known as pressure support ventilation therapy, the amplitude *A* is referred to as the pressure support, or swing. In pressure support ventilation therapy, the IPAP is the base pressure $P_0$ plus the pressure support *A*, and the EPAP is the base pressure $P_0$.

In some forms of pressure support ventilation therapy, known as fixed pressure support ventilation therapy, the pressure support *A* is fixed at a predetermined value, e.g. 10 $cmH_2O$. The predetermined pressure support value is a setting of the RPT device 4000, and may be set for example by hard-coding during configuration of the RPT device 4000 or by manual entry through the input device 4220.

In other forms of pressure support ventilation therapy, broadly known as servo-ventilation, the therapy parameter determination algorithm 4329 takes as input some currently measured or estimated parameter of the respiratory cycle (e.g. the current measure *Vent* of ventilation) and a target value of that respiratory parameter (e.g. a target value *Vtgt* of ventilation) and repeatedly adjusts the parameters of equation (1) to bring the current measure of the respiratory parameter towards the target value. In a form of servo-ventilation known as adaptive servo-ventilation (ASV), which has been used to treat CSR, the respiratory parameter is ventilation, and the target ventilation value *Vtgt* is computed by the target ventilation determination algorithm 4328 from the typical recent ventilation *Vtyp,* as described above.

In some forms of servo-ventilation, the therapy parameter determination algorithm 4329 applies a control methodology to repeatedly compute the pressure support *A* so as to bring the current measure of the respiratory parameter towards the target value. One such control methodology is Proportional-Integral (PI) control. In one implementation of PI control, suitable for ASV modes in which a target ventilation *Vtgt* is set to slightly less than the typical recent ventilation *Vtyp,* the pressure support *A* is repeatedly computed as:

$$A = G\int (Vent - Vtgt)dt \qquad (2)$$

where *G* is the gain of the PI control. Larger values of gain *G* can result in positive feedback in the therapy engine module 4320. Smaller values of gain *G* may permit some residual untreated CSR or central sleep apnea. In some implementations, the gain *G* is fixed at a predetermined value, such as -0.4 $cmH_2O$/(L/min)/sec. Alternatively, the gain *G* may be varied between therapy sessions, starting small and increasing from session to session until a value that substantially eliminates CSR is reached. Conventional means for retrospectively analysing the parameters of a therapy session to assess the severity of CSR during the therapy session may be employed in such implementations In yet other implementations, the gain *G* may vary depending on the difference between the current measure *Vent* of ventilation and the target ventilation *Vtgt.*

Other servo-ventilation control methodologies that may be applied by the therapy parameter determination algorithm 4329 include proportional (P), proportional-differential (PD), and proportional-integral-differential (PID).

The value of the pressure support *A* computed via a known equation may be clipped to a range defined as [*Amin, Amax*]. In this implementation, the pressure support *A* sits by default at the minimum pressure support *Amin* until the measure of current ventilation *Vent* falls below the target ventilation *Vtgt,* at which point *A* starts increasing, only falling back to *Amin* when *Vent* exceeds *Vtgt* once again.

The pressure support limits *Amin* and *Amax* are settings of the RPT device 4000, set for example by hard-coding during configuration of the RPT device 4000 or by manual entry through the input device 4220.

In pressure support ventilation therapy modes, the EPAP is the base pressure $P_0$. As with the base pressure $P_0$ in CPAP therapy, the EPAP may be a constant value that is prescribed or determined during titration. Such a constant EPAP may be set for example by hard-coding during configuration of the RPT device 4000 or by manual entry through the input device 4220. This alternative is sometimes referred to as fixed-EPAP pressure support ventilation therapy. Titration of the EPAP for a given patient may be performed by a clinician during a titration session with the aid of PSG, with the aim of preventing obstructive apneas, thereby maintaining an open airway for the pressure support ventilation therapy, in similar fashion to titration of the base pressure $P_0$ in constant CPAP therapy.

Alternatively, the therapy parameter determination algorithm 4329 may repeatedly compute the base pressure $P_0$ during pressure support ventilation therapy. In such implementations, the therapy parameter determination algorithm 4329 repeatedly computes the EPAP as a function of indices or measures of sleep disordered breathing returned by the respective algorithms in the therapy engine module 4320, such as one or more of flow limitation, apnea, hypopnea, patency, and snore. Because the continuous computation of the EPAP resembles the manual adjustment of the EPAP by a clinician during titration of the EPAP, this process is also sometimes referred to as auto-titration of the EPAP, and the therapy mode is known as auto-titrating EPAP pressure support ventilation therapy, or auto-EPAP pressure support ventilation therapy.

### 4.12.3 High flow therapy

In other forms of respiratory therapy, the pressure of the flow of air is not controlled as it is for respiratory pressure therapy. Rather, the RPT controller 4230 controls the pressure generator 4140 to deliver a flow of air whose device flow rate *Qd* is controlled to a treatment or target flow rate *Qtgt* that is typically positive throughout the patient's breathing cycle. Such forms are generally grouped under the heading of flow therapy. In flow therapy, the treatment flow rate *Qtgt* may be a constant value that is hard-coded or manually entered to the RPT device 4000. If the treatment flow rate *Qtgt* is sufficient to exceed the patient's peak inspiratory flow rate, the therapy is generally referred to as high flow therapy (HFT). Alternatively, the treatment flow rate may be a profile *Qtgt(t)* that varies over the respiratory cycle.

## 4.13 GLOSSARY

For the purposes of the present technology disclosure, in certain forms of the present technology, one or more of the following definitions may apply. In other forms of the present technology, alternative definitions may apply.

### 4.13.1 General

*Respiratory Gas*: In certain forms of the present technology, respiratory gas may be taken to mean some other combination of breathable gases, e.g. atmospheric air enriched with oxygen. In other forms of the present technology, respiratory gas may be taken to mean atmospheric air.

*Ambient*: In certain forms of the present technology, the term ambient will be taken to mean (i) external of the patient interface system 3000, the positioning and stabilizing structure 3300 or patient 9000, and (ii) immediately surrounding the patient interface system 3000, the positioning and stabilizing structure 3300 or patient 9000.

For example, ambient humidity with respect to a humidifier 5000 may be the humidity of respiratory gas immediately surrounding the humidifier 5000, e.g. the humidity in the room where a patient is sleeping. Such ambient humidity may be different to the humidity outside the room where a patient 9000 is sleeping.

In another example, ambient pressure may be the pressure immediately surrounding or external to the body.

In certain forms, ambient (e.g., acoustic) noise may be considered to be the background noise level in the room where a patient is located, other than for example, noise generated by an RPT device 4000 or emanating from a mask or patient interface 3102. Ambient noise may be generated by sources outside the room.

*Automatic Positive Airway Pressure (APAP) therapy*: CPAP therapy in which the treatment pressure is automatically adjustable, e.g. from breath to breath, between minimum and maximum limits, depending on the presence or absence of indications of SDB (Sleep Disordered Breathing) events.

*Continuous Positive Airway Pressure (CPAP) therapy*: Respiratory pressure therapy in which the treatment pressure is approximately constant through a respiratory cycle of a patient. In some forms, the pressure at the entrance to the airways will be slightly higher during exhalation, and slightly lower during inhalation. In some forms, the pressure will vary between different respiratory cycles of the patient, for example, being increased in response to detection of indications of partial upper airway obstruction, and decreased in the absence of indications of partial upper airway obstruction.

*Flow rate*: The volume (or mass) of air delivered per unit time. Flow rate may refer to an instantaneous quantity. In some cases, a reference to flow rate will be a reference to a scalar quantity, namely a quantity having magnitude only. In other cases, a reference to flow rate will be a reference to a vector quantity, namely a quantity having both magnitude and direction. Flow rate may be given the symbol *Q*. 'Flow rate' is sometimes shortened to simply 'flow' or 'airflow'.

In the example of patient respiration, a flow rate may be nominally positive for the inspiratory portion of a breathing cycle of a patient, and hence negative for the expiratory portion of the breathing cycle of a patient. Device flow rate, *Qd*, is the flow rate of air leaving the RPT device. Total flow rate, *Qt*, is the flow rate of air and any supplementary gas reaching the patient interface via the air circuit. Vent flow rate, *Qv*, is the flow rate of air leaving a vent to allow washout of exhaled gases. Leak flow rate, *Ql*, is the flow rate of leak from a patient interface system or elsewhere. Respiratory flow rate, *Qr,* is the flow rate of air that is received into the patient's respiratory system.

*Flow therapy*: Respiratory therapy comprising the delivery of a flow of air to an entrance to the airways at a controlled flow rate referred to as the treatment flow rate that is typically positive throughout the patient's breathing cycle.

*Humidifier*: The word humidifier 5000 will be taken to mean a humidifying apparatus constructed and arranged, or configured with a physical structure to be capable of providing a therapeutically beneficial amount of water ($H_2O$) vapour to a flow of air to ameliorate a medical respiratory condition of a patient.

*Leak*: The word leak will be taken to be an unintended flow of air. In one example, leak may occur as the result of an incomplete seal between a mask and a patient's face. In another example leak may occur in a swivel elbow to the ambient.

*Noise, conducted (acoustic)*: Conducted noise in the present document refers to noise which is carried to the patient by the pneumatic path, such as the air circuit and the patient interface as well as the air therein. In one form, conducted noise may be quantified by measuring sound pressure levels at the end of an air circuit.

*Noise, radiated (acoustic)*: Radiated noise in the present document refers to noise which is carried to the patient 9000 by the ambient air. In one form, radiated noise may be quantified by measuring sound power/pressure levels of the object in question according to ISO 3744.

*Noise, vent (acoustic)*: Vent noise in the present document refers to noise which is generated by the flow of air through any vents such as vent holes of the patient interface.

*Patient*: A person, whether or not they are suffering from a respiratory condition.

*Pressure:* Force per unit area. Pressure may be expressed in a range of units, including $cmH_2O$, g-f/cm$^2$ and hectopascal. 1 $cmH_2O$ is equal to 1 g-f/cm$^2$ and is approximately 0.98 hectopascal (1 hectopascal = 100 Pa = 100 N/m$^2$ = 1 millibar ~ 0.001 atm). In this specification, unless otherwise stated, pressure is given in units of $cmH_2O$.

The pressure in the patient interface is given the symbol *Pm,* while the treatment pressure, which represents a target value to be achieved by the interface pressure *Pm* at the current instant of time, is given the symbol *Pt.*

*Respiratory Pressure Therapy (RPT)*: The application of a supply of air to an entrance to the airways at a treatment pressure that is typically positive with respect to atmosphere.

*Ventilator*: A mechanical device that provides pressure support to a patient to perform some or all of the work of breathing.

#### 4.13.1.1 Materials

*Silicone or Silicone Elastomer*: A synthetic rubber. In this specification, a reference to silicone is a reference to liquid silicone rubber (LSR) or a compression moulded silicone rubber (CMSR). One form of commercially available LSR is SILASTIC (included in the range of products sold under this trademark), manufactured by Dow Corning. Another manufacturer of LSR is Wacker. Unless otherwise specified to the contrary, an exemplary form of LSR has a Shore A (or Type A) indentation hardness in the range of about 35 to about 45 as measured using ASTM D2240. (Year? Required?)

*Polycarbonate*: a thermoplastic polymer of Bisphenol-A Carbonate.

#### 4.13.1.2 Mechanical properties

*Resilience*: Ability of a material to absorb energy when deformed elastically and to release the energy upon unloading.

*Resilient*: Will release substantially all of the energy when unloaded. Includes e.g. certain silicones, and thermoplastic elastomers.

*Hardness*: The ability of a material *per se* to resist deformation (e.g. described by a Young's Modulus, or an indentation *hardness* scale measured on a standardised sample size).

- 'Soft' materials may include silicone or thermo-plastic elastomer (TPE), and may, e.g. readily deform under finger pressure.
- 'Hard' materials may include polycarbonate, polypropylene, steel or aluminium, and may not e.g. readily deform under finger pressure.

*Stiffness (or rigidity) of a structure or component*: The ability of the structure or component to resist deformation in response to an applied load. The load may be a force or a moment, e.g. compression, tension, bending or torsion. The structure or component may offer different resistances in different directions. The inverse of stiffness is *flexibility.*

*Floppy structure or component:* A structure or component that will change shape, e.g. bend, when caused to support its own weight, within a relatively short period of time such as 1 second.

*Rigid structure or component*: A structure or component that will not substantially change shape when subject to the loads typically encountered in use. An example of such a use may be setting up and maintaining a patient interface in sealing relationship with an entrance to a patient's airways, e.g. at a load of approximately 20 to 30 $cmH_2O$ pressure.

As an example, an I-beam may comprise a different bending stiffness (resistance to a bending load) in a first direction in comparison to a second, orthogonal direction. In another example, a structure or component may be floppy in a first direction and rigid in a second direction.

#### 4.13.2 Respiratory cycle

*Apnea*: According to some definitions, an apnea is said to have occurred when flow falls below a predetermined threshold for a duration, e.g. 10 seconds. An obstructive apnea will be said to have occurred when, despite patient effort, some obstruction of the airway does not allow air to flow. A central apnea will be said to have occurred when an apnea is detected that is due to a reduction in breathing effort, or the absence of breathing effort, despite the airway being patent. A mixed apnea occurs when a reduction or absence of breathing effort coincides with an obstructed airway.

*Breathing rate*: The rate of spontaneous respiration of a patient, usually measured in breaths per minute.

*Duty cycle*: The ratio of inhalation time, *Ti* to total breath time, *Ttot.*

*Effort (breathing)*: The work done by a spontaneously breathing person attempting to breathe.

*Expiratory portion of a breathing cycle:* The period from the start of expiratory flow to the start of inspiratory flow.

*Flow limitation*: Flow limitation will be taken to be the state of affairs in a patient's respiration where an increase in effort by the patient does not give rise to a corresponding increase in flow. Where flow limitation occurs during an inspiratory portion of the breathing cycle it may be described as inspiratory flow limitation. Where flow limitation occurs during an expiratory portion of the breathing cycle it may be described as expiratory flow limitation.

*Hypopnea*: According to some definitions, a hypopnea is taken to be a reduction in flow, but not a cessation of flow. In one form, a hypopnea may be said to have occurred when there is a reduction in flow below a threshold rate for a duration. A central hypopnea will be said to have occurred when a hypopnea is detected that is due to a reduction in breathing effort. In one form in adults, either of the following may be regarded as being hypopneas:

(i) a 30% reduction in patient breathing for at least 10 seconds plus an associated 4% desaturation; or

(ii) a reduction in patient breathing (but less than 50%) for at least 10 seconds, with an associated desaturation of at least 3% or an arousal.

*Hyperpnea*: An increase in flow to a level higher than normal.

*Inspiratory portion of a breathing cycle*: The period from the start of inspiratory flow to the start of expiratory flow will be taken to be the inspiratory portion of a breathing cycle.

*Patency (airway)*: The degree of the airway being open, or the extent to which the airway is open. A *patent* airway is open. Airway patency may be quantified, for example with a value of one (1) being patent, and a value of zero (0), being closed (obstructed).

*Positive End-Expiratory Pressure (PEEP)*: The pressure above atmosphere in the lungs that exists at the end of expiration.

*Peak flow rate (Qpeak)*: The maximum value of flow rate during the inspiratory portion of the respiratory flow waveform.

*Respiratory flow rate*, patient *airflow rate, respiratory airflow rate (Qr)*: These terms may be understood to refer to the RPT device's estimate of respiratory flow rate, as opposed to "true respiratory flow rate" or "true respiratory flow rate", which is the actual respiratory flow rate experienced by the patient, usually expressed in litres per minute.

*Tidal volume (Vt)*: The volume of air inhaled or exhaled during normal breathing, when extra effort is not applied. In principle the inspiratory volume *Vi* (the volume of air inhaled) is equal to the expiratory volume *Ve* (the volume of air exhaled), and therefore a single tidal volume *Vt* may be defined as equal to either quantity. In practice the tidal volume *Vt* is estimated as some combination, e.g. the mean, of the inspiratory volume *Vi* and the expiratory volume *Ve.*

*(inhalation) Time (Ti)*: The duration of the inspiratory portion of the respiratory flow rate waveform.

*(exhalation) Time (Te)*: The duration of the expiratory portion of the respiratory flow rate waveform.

*(total) Time (Ttot)*: The total duration between the start of one inspiratory portion of a respiratory flow rate waveform and the start of the following inspiratory portion of the respiratory flow rate waveform.

*Typical recent ventilation*: The value of ventilation around which recent values of ventilation *Vent* over some predetermined timescale tend to cluster, that is, a measure of the central tendency of the recent values of ventilation.

*Upper airway obstruction (UAO)*: includes both partial and total upper airway obstruction. This may be associated with a state of flow limitation, in which the flow rate increases only slightly or may even decrease as the pressure difference across the upper airway increases (Starling resistor behaviour).

*Ventilation (Vent)*: A measure of a rate of gas being exchanged by the patient's respiratory system. Measures of ventilation may include one or both of inspiratory and expiratory flow, per unit time. When expressed as a volume per minute, this quantity is often referred to as "minute ventilation". Minute ventilation is sometimes given simply as a volume, understood to be the volume per minute.

**4.13.3 Ventilation**

*Adaptive Servo-Ventilator (ASV)*: A servo-ventilator that has a changeable, rather than fixed target ventilation. The changeable target ventilation may be learned from some characteristic of the patient, for example, a respiratory characteristic of the patient.

*Backup rate*: A parameter of a ventilator that establishes the minimum breathing rate (typically in number of breaths per minute) that the ventilator will deliver to the patient, if not triggered by spontaneous respiratory effort.

*Cycled*: The termination of a ventilator's inspiratory phase. When a ventilator delivers a breath to a spontaneously breathing patient, at the end of the inspiratory portion of the breathing cycle, the ventilator is said to be cycled to stop delivering the breath.

*Expiratory positive airway pressure (EPAP):* a base pressure, to which a pressure varying within the breath is added to produce the desired interface pressure which the ventilator will attempt to achieve at a given time.

*End expiratory pressure (EEP)*: Desired interface pressure which the ventilator will attempt to achieve at the end of the expiratory portion of the breath. If the pressure waveform template $\Pi(\Phi)$ is zero-valued at the end of expiration, i.e. $\Pi(\Phi) = 0$ when $\Phi = 1$, the EEP is equal to the EPAP.

*Inspiratory positive airway pressure (IPAP)*: Maximum desired interface pressure which the ventilator will attempt to achieve during the inspiratory portion of the breath.

*Pressure support*: A number that is indicative of the increase in pressure during ventilator inspiration over that during ventilator expiration, and generally means the difference in pressure between the maximum value during inspiration and the base pressure (e.g., *PS = IPAP - EPAP*). In some contexts pressure support means the difference which the ventilator aims to achieve, rather than what it actually achieves.

*Servo-ventilator:* A ventilator that measures patient ventilation, has a target ventilation, and which adjusts the level of pressure support to bring the patient ventilation towards the target ventilation.

*Spontaneous/Timed (S/T)*: A mode of a ventilator or other device that attempts to detect the initiation of a breath of a spontaneously breathing patient. If however, the device is unable to detect a breath within a predetermined period of time, the device will automatically initiate delivery of the breath.

*Swing*: Equivalent term to pressure support.

*Triggered*: When a ventilator delivers a breath of *air* to a spontaneously breathing patient, it is said to be triggered to do so at the initiation of the respiratory portion of the breathing cycle by the patient's efforts.

**4.13.4 Anatomy**

#### 4.13.4.1 Anatomy of the face

*Ala:* the external outer wall or "wing" of each nostril (plural: alar)

*Alare:* The most lateral point on the nasal *ala.*

*Alar curvature (or alar crest) point*: The most posterior point in the curved base line of each *ala,* found in the crease formed by the union of the *ala* with the cheek.

*Auricle:* The whole external visible part of the ear.

*(nose) Bony framework:* The bony framework of the nose comprises the nasal bones, the frontal process of the maxillae and the nasal part of the frontal bone.

*(nose) Cartilaginous framework*: The cartilaginous framework of the nose comprises the septal, lateral, major and minor cartilages.

*Columella:* the strip of skin that separates the nares and which runs from the pronasale to the upper lip.

*Columella angle:* The angle between the line drawn through the midpoint of the nostril aperture and a line drawn perpendicular to the Frankfort horizontal while intersecting subnasale.

*Frankfort horizontal plane*: A line extending from the most inferior point of the orbital margin to the left tragion. The tragion is the deepest point in the notch superior to the tragus of the auricle.

*Glabella:* Located on the soft tissue, the most prominent point in the midsagittal plane of the forehead.

*Lateral nasal cartilage*: A generally triangular plate of cartilage. Its superior margin is attached to the nasal bone and frontal process of the maxilla, and its inferior margin is connected to the greater alar cartilage.

*Greater alar cartilage*: A plate of cartilage lying below the lateral nasal cartilage. It is curved around the anterior part of the naris. Its posterior end is connected to the frontal process of the maxilla by a tough fibrous membrane containing three or four minor cartilages of the *ala.*

*Nares (Nostrils)*: Approximately ellipsoidal apertures forming the entrance to the nasal cavity. The singular form of nares 9002A, 9002B is naris (nostril). The nares are separated by the nasal septum.

*Naso-labial sulcus or Naso-labial fold*: The skin fold or groove that runs from each side of the nose to the corners of the mouth, separating the cheeks from the upper lip.

*Naso-labial angle*: The angle between the columella and the upper lip, while intersecting subnasale.

*Mouth*: Entrance to the buccal cavity which is situated below the nasal cavity.

*Otobasion inferior*: The lowest point of attachment of the auricle to the skin of the face.

*Otobasion superior*: The highest point of attachment of the auricle to the skin of the face.

*Pronasale:* the most protruded point or tip of the nose, which can be identified in lateral view of the rest of the portion of the head.

*Philtrum:* the midline groove that runs from lower border of the nasal septum to the top of the lip in the upper lip region.

*Pogonion:* Located on the soft tissue, the most anterior midpoint of the chin.

*Ridge (nasal)*: The nasal ridge is the midline prominence of the nose, extending from the Sellion to the Pronasale.

*Sagittal plane*: A vertical plane that passes from anterior (front) to posterior (rear). The midsagittal plane is a sagittal plane that divides the body into right and left halves.

*Sellion:* Located on the soft tissue, the most concave point overlying the area of the frontonasal suture.

*Septal cartilage (nasal)*: The nasal septal cartilage forms part of the septum and divides the front part of the nasal cavity.

*Subalare*: The point at the lower margin of the alar base, where the alar base joins with the skin of the superior (upper) lip.

*Subnasal point*: Located on the soft tissue, the point at which the columella merges with the upper lip in the midsagittal plane.

*Supramenton*: The point of greatest concavity in the midline of the lower lip between labrale inferius and soft tissue pogonion.

#### 4.13.4.2 Anatomy of the skull

*Frontal bone*: The frontal bone includes a large vertical portion, the *squama frontalis*, corresponding to the region known as the forehead.

*Mandible*: The mandible forms the lower jaw. The mental protuberance is the bony protuberance of the jaw that forms the chin.

*Maxilla*: The maxilla forms the upper jaw and is located above the mandible and below the orbits. The *frontal process of the maxilla* projects upwards by the side of the nose, and forms part of its lateral boundary.

*Nasal bones*: The nasal bones are two small oblong bones, varying in size and form in different individuals; they are placed side by side at the middle and upper part of the face, and form, by their junction, the "bridge" of the nose.

*Nasion:* The intersection of the frontal bone and the two nasal bones, a depressed area directly between the eyes and superior to the bridge of the nose.

*Occipital bone*: The occipital bone is situated at the back and lower part of the cranium. It includes an oval aperture, the *foramen magnum*, through which the cranial cavity communicates with the vertebral canal. The curved plate behind the foramen magnum is the *squama occipitalis.*

*Orbit*: The bony cavity in the skull to contain the eyeball.

*Parietal bones*: The parietal bones are the bones that, when joined together, form the roof and sides of the cranium.

*Temporal bones*: The temporal bones are situated on the bases and sides of the skull, and support that part of the face known as the temple.

*Zygomatic bones*: The face includes two zygomatic bones, located in the upper and lateral parts of the face and forming the prominence of the cheek.

#### 4.13.4.3 Anatomy of the respiratory system

*Diaphragm:* A sheet of muscle that extends across the bottom of the rib cage. The diaphragm separates the thoracic cavity, containing the heart, lungs and ribs, from the abdominal cavity. As the diaphragm contracts the volume of the thoracic cavity increases and air is drawn into the lungs.

*Larynx*: The larynx, or voice box houses the vocal folds and connects the inferior part of the pharynx (hypopharynx) with the trachea.

*Lungs*: The organs of respiration in humans. The conducting zone of the lungs contains the trachea, the bronchi, the bronchioles, and the terminal bronchioles. The respiratory zone contains the respiratory bronchioles, the alveolar ducts, and the alveoli.

*Nasal cavity*: The nasal cavity (or nasal fossa) is a large air filled space above and behind the nose in the middle of the face. The nasal cavity is divided in two by a vertical fin called the nasal septum. On the sides of the nasal cavity are three horizontal outgrowths called nasal conchae (singular "concha") or turbinates. To the front of the nasal cavity is the nose, while the back blends, via the choanae, into the nasopharynx.

*Pharynx:* The part of the throat situated immediately inferior to (below) the nasal cavity, and superior to the oesophagus and larynx. The pharynx is conventionally divided into three sections: the nasopharynx (epipharynx) (the nasal part of the pharynx), the oropharynx (mesopharynx) (the oral part of the pharynx), and the laryngopharynx (hypopharynx).

### 4.13.5 Patient interface

*Anti-asphyxia valve (AAV):* The component or sub-assembly of the patient interface system 3000 and/or the positioning and stabilizing structure 3300 that, by opening to atmosphere in a failsafe manner, reduces the risk of excessive $CO_2$ rebreathing by a patient 9000.

*Elbow:* An elbow is an example of a structure that directs an axis of flow of air travelling therethrough to change direction through an angle. In one form, the angle may be approximately 90 degrees. In another form, the angle may be more, or less than 90 degrees. The elbow may have an approximately circular cross-section. In another form the elbow may have an oval or a rectangular cross-section. In certain forms an elbow may be rotatable with respect to a mating component, e.g. about 360 degrees. In certain forms an elbow may be removable from a mating component, e.g. via a snap connection. In certain forms, an elbow may be assembled to a mating component via a one-time snap during manufacture, but not removable by a patient.

*Frame*: Frame will be taken to mean a mask structure that bears the load of tension between two or more points of connection with a headgear. A mask frame may be a non-airtight load bearing structure in the mask. However, some forms of mask frame may also be air-tight.

*Headgear:* Headgear will be taken to mean a form of positioning and stabilizing structure designed for use on a head. For example the headgear may comprise a collection of one or more struts, ties and stiffeners configured to locate and retain a patient interface in position on a patient's face for delivery of respiratory therapy. Some ties are formed of a soft, flexible, elastic material such as a laminated composite of foam and fabric.

*Membrane*: Membrane or seal-forming structure 3100 will be taken to mean a typically thin element that has, preferably, substantially no resistance to bending, but has resistance to being stretched.

*Plenum chamber*: a plenum chamber 2200 will be taken to mean a portion of a patient interface 3102 having walls at least partially enclosing a volume of space, the volume having air therein pressurised above atmospheric pressure in use. A shell may form part of the walls of a plenum chamber 2200.

*Seal*: May be a noun form ("a seal") which refers to a structure (the sea-forming structure 3100), or a verb form ("to seal") which refers to the effect. In an alternative form of the present technology, two elements may be constructed and/or arranged to 'seal' or to effect 'sealing' therebetween without requiring a separate 'seal' element *per se.*

*Shell*: A shell will be taken to mean a curved, relatively thin structure having bending, tensile and compressive stiffness. For example, a curved structural wall of a mask may be a shell. In some forms, a shell may be faceted. In some forms a shell may be airtight. In some forms a shell may not be airtight.

*Stiffener*: A stiffener will be taken to mean a structural component designed to increase the bending resistance of another component in at least one direction.

*Strut:* A strut will be taken to be a structural component designed to increase the compression resistance of another component in at least one direction.

*Swivel* (noun): A subassembly of components configured to rotate about a common axis, preferably independently, preferably under low torque. In one form, the swivel may be constructed to rotate through an angle of at least 360 degrees. In another form, the swivel may be constructed to rotate through an angle less than 360 degrees.

*Tie* (noun): A structure designed to resist tension.

*Vent*: (noun): A structure that allows a flow of *respiratory gas* from an interior of the patient interface 3102 and/or the positioning and stabilizing structure 3300, to ambient air for clinically effective washout of exhaled gases. For example, a clinically effective washout may involve a flow rate of about 10 litres per minute to about 100 litres per minute, depending on the mask design and treatment pressure.

4.14 OTHER REMARKS

A portion of the disclosure of this patent document contains material which is subject to copyright protection. The copyright owner has no objection to the facsimile reproduction by anyone of the patent document or the patent disclosure, as it appears in Patent Office patent files or records, but otherwise reserves all copyright rights whatsoever.

Unless the context clearly dictates otherwise and where a range of values is provided, it is understood that each intervening value, to the tenth of the unit of the lower limit, between the upper and lower limit of that range, and any other stated or intervening value in that stated range is encompassed within the technology. The upper and lower limits of these intervening ranges, which may be independently included in the intervening ranges, are also encompassed within the technology, subject to any specifically excluded limit in the stated range. Where the stated range includes one or both of the limits, ranges excluding either or both of those included limits are also included in the technology.

Furthermore, where a value or values are stated herein as being implemented as part of the technology, it is understood that such values may be approximated, unless otherwise stated, and such values may be utilized to any suitable significant digit to the extent that a practical technical implementation may permit or require it.

Unless defined otherwise, all technical and scientific terms used herein have the same meaning as commonly understood by one of ordinary skill in the art to which this technology belongs. Although any methods and materials similar or equivalent to those described herein can also be used in the practice or testing of the present technology, a limited number of the exemplary methods and materials are described herein.

When a particular material is identified as being used to construct a component, obvious alternative materials with similar properties may be used as a substitute. Furthermore, unless specified to the contrary, any and all components herein described are understood to be capable of being manufactured and, as such, may be manufactured together or separately.

It must be noted that as used herein and in the appended claims, the singular forms "a", "an", and "the" include their plural equivalents, unless the context clearly dictates otherwise.

All publications discussed herein are provided solely for their disclosure prior to the filing date of the present application. Nothing herein is to be construed as an admission that the present technology is not entitled to antedate such publication by virtue of prior invention. Further, the dates of publication provided may be different from the actual publication dates, which may need to be independently confirmed.

The terms "comprises" and "comprising" should be interpreted as referring to elements, components, or steps in a non-exclusive manner, indicating that the referenced elements, components, or steps may be present, or utilized, or combined with other elements, components, or steps that are not expressly referenced.

The subject headings used in the detailed description are included only for the ease of reference of the reader and should not be used to limit the subject matter found throughout the disclosure or the claims. The subject headings should not be used in construing the scope of the claims or the claim limitations.

Although the technology herein has been described with reference to particular examples, it is to be understood that these examples are merely illustrative of the principles and applications of the technology. In some instances, the terminology and symbols may imply specific details that are not required to practice the technology. For example, although the terms "first" and "second" may be used, unless otherwise specified, they are not intended to indicate any order but may be utilised to distinguish between distinct elements. Furthermore, although process steps in the methodologies may be described or illustrated in an order, such an ordering is not required. Those skilled in the art will recognize that such ordering may be modified and/or aspects thereof may be conducted concurrently or even synchronously.

It is therefore to be understood that numerous modifications may be made to the illustrative examples and that other arrangements may be devised without departing from the scope of the technology.

## Claims

1. A positioning and stabilizing structure (3300) of a respiratory therapy treatment system configured in use to maintain a patient interface (3102) in a position relative to a patient's airway(s), the positioning and stabilizing structure (3300) comprising a first headgear tube (3302), a second headgear tube (3304), and being **characterized by** a flow regulator (3010, 7010), wherein the flow regulator (3010, 7010) is configured to in use receive a flow of respiratory gas and to adjust the proportion of a flow of respiratory gas which flows from the flow regulator (3010, 7010) into the first headgear tube (3302) and the second headgear tube (3304), and wherein the flow regulator (3010, 7010) includes a barrier arrangement (3020, 7020) which is moveable between a first configuration and a second configuration so as to adjust the proportion of the flow of respiratory gas which flows from the flow regulator (3010, 7010) into the first headgear tube (3302) and the second headgear tube (3304), and wherein the barrier arrangement (3010, 7010) is configured to be moved between the first configuration and the second configuration by gravity.

2. The positioning and stabilizing structure (3300) of claim 1, wherein the flow regulator (3010, 7010) is configured to direct a relatively greater portion of the flow of respiratory gas into one of the first headgear tube (3302) and second headgear tube (3304) and a relatively less portion of the flow of respiratory gas into the other of the first headgear tube (3302) and second headgear tube (3304).

3. The positioning and stabilizing structure (3300) of claim 2, wherein the relatively greater portion of the flow of respiratory is all, or a substantial portion of, the flow.

4. The positioning and stabilizing structure (3300) of any one of claims 1 to 3,
wherein the flow regulator (3010, 7010) is configured to, in use, direct all, or a substantial portion of, the flow of respiratory gas to:

   the first headgear tube (3302) if/when the patient's head is tilted substantially to a first side; and/or
   the second headgear tube (3304) if/when the patient's head is tilted to a second side; and/or
   the ratio of the portion of the flow directed to first headgear tube (3302) and the second headgear tube (3304) is dependent on the orientation of the patient's head.

5. The positioning and stabilizing structure (3300) of any one of claims 1 to 4, wherein the flow regulator (3010, 7010) comprises a body which defines a chamber (3024, 7024), including an inlet (3012, 7012) configured to facilitate a flow of respiratory gas flowing into the chamber (3024, 7024).

6. The positioning and stabilizing structure (3300) of claim 5, wherein the flow regulator (3010, 7010) includes an inlet connector that is configured to in use connect a duct (4050) to the inlet.

7. The positioning and stabilizing structure (3300) of claim 5 or claim 6, wherein the chamber (3024, 7024) includes a first outlet (3014, 7014) configured to, in use, be in flow communication with the first headgear tube (3302) and a second outlet (3016, 7016) configured to in use be in flow communication with the second headgear tube (3304).

8. The positioning and stabilizing structure (3300) of claim 7, wherein when in the first configuration:

   the barrier arrangement (3020, 7020) completely or substantially blocks the second outlet (3016, 7016); and/or
   the barrier arrangement (3020, 7020) substantially or completely prevents the flow of respiratory gas flowing into the second headgear tube (3304); and/or
   the first outlet (3014, 7014) is substantially or completely open.

9. The positioning and stabilizing structure (3300) of claim 7, wherein when in the second configuration:

   the barrier arrangement (3020, 7020) completely or substantially blocks the first outlet (3014, 7014); and/or
   the barrier substantially or completely prevents the flow of respiratory gas flowing into the first headgear tube (3302); and or
   the second outlet (3016, 7016) is substantially or completely open.

**10.** The positioning and stabilizing structure (3300) of any one of claims 1 to 9, wherein the barrier arrangement (3020, 7020):
is a piston (3022) or a spherically shaped ball component (7022); and/or provided in a / the chamber (3024, 7024).

**11.** The positioning and stabilizing structure (3300) of any one of claims 1 to 10, wherein:

the first headgear tube (3302) comprises a first connector (3006) configured to connect to the patient interface (3102) and the second headgear tube (3304) comprises a second connector (3008) configured to connect to the patient interface (3102); and/or
the first headgear tube (3302) is configured to in use lie against a first cheek region of the patient, and the second headgear tube (3304) is configured to in use lie against a second cheek region of the patient; and/or one or more of the first headgear tube (3302) and the second headgear tube (3304) may be partially or completely compressible.

**12.** A respiratory treatment system, including

the positioning and stabilising structure of claims 10 or 11, and
a patient interface (3102).

**13.** The respiratory treatment system of claim 12, wherein the patient interface (3102) is configured to convey the respiratory gas to one or more of the patient's nasal airways and the patient's mouth, wherein the patient interface (3102) includes a sealing component which is configured to:

sealingly engage at least a portion of the patient's face; and/or
seal around at least one of the patient's nasal airways and the patient's mouth.

**14.** The respiratory treatment system of claim 13, wherein the sealing component is a cushion.

**Patentansprüche**

**1.** Positionierungs- und Stabilisierungsstruktur (3300) eines Atemtherapie-Behandlungssystems, die im Gebrauch zum Halten einer Patientenschnittstelle (3102) in einer Position relativ zu dem/den Atemweg(en) eines Patienten konfiguriert ist, wobei die Positionierungs- und Stabilisierungsstruktur (3300) ein erstes Kopfhalterungsrohr (3302), ein zweites Kopfhalterungsrohr (3304) aufweist und durch einen Durchflussregler (3010, 7010) gekennzeichnet ist, wobei der Durchflussregler (3010, 7010) im Gebrauch zum Aufnehmen eines Atemgasstroms und zum Einstellen des Anteils eines Atemgasstroms, der von dem Durchflussregler (3010, 7010) in das erste Kopfhalterungsrohr (3302) und das zweite Kopfhalterungsrohr (3304) strömt, konfiguriert ist und wobei der Durchflussregler (3010, 7010) eine Barrierenanordnung (3020, 7020) aufweist, die zwischen einer ersten Konfiguration und einer zweiten Konfiguration bewegbar ist, um den Anteil des Atemgasstroms, der vom Durchflussregler (3010, 7010) in das erste Kopfhalterungsrohr (3302) und das zweite Kopfhalterungsrohr (3304) strömt, einzustellen und wobei die Barrierenanordnung (3010, 7010) dazu konfiguriert ist, sich durch Schwerkraft zwischen der ersten Konfiguration und der zweiten Konfiguration zu bewegen.

**2.** Positionierungs- und Stabilisierungsstruktur (3300) nach Anspruch 1, wobei der Durchflussregler (3010, 7010) zum Leiten eines relativ größeren Teils des Atemgasstroms in das eine von erstem Kopfhalterungsrohr (3302) und zweitem Kopfhalterungsrohr (3304) und eines relativ geringeren Teils des Atemgasstroms in das andere von erstem Kopfhalterungsrohr (3302) und zweitem Kopfhalterungsrohr (3304) konfiguriert ist.

**3.** Positionierungs- und Stabilisierungsstruktur (3300) nach Anspruch 2, wobei der relativ größere Teil des Atemstroms der gesamte oder ein wesentlicher Teil des Stroms ist.

**4.** Positionierungs- und Stabilisierungsstruktur (3300) nach einem der Ansprüche 1 bis 3, wobei der Durchflussregler (3010, 7010) konfiguriert ist, im Gebrauch den gesamten oder einen wesentlichen Teils des Atemgasstroms zu leiten in:

das erste Kopfhalterungsrohr (3302), wenn der Kopf des Patienten im Wesentlichen zu einer ersten Seite geneigt ist; und/oder
das zweite Kopfhalterungsrohr (3304), wenn der Kopf des Patienten zu einer zweiten Seite geneigt ist; und/oder

so, dass das Verhältnis des zum ersten Kopfhalterungsrohr (3302) und zum zweiten Kopfhalterungsrohr (3304) geleiteten Teils des Stroms von der Ausrichtung des Kopfes des Patienten abhängt.

**5.** Positionierungs- und Stabilisierungsstruktur (3300) nach einem der Ansprüche 1 bis 4, wobei der Durchflussregler (3010, 7010) einen Körper aufweist, der eine Kammer (3024, 7024) definiert, die einen Einlass (3012, 7012) aufweist, der zum Ermöglichen eines Atemgasstroms in die Kammer (3024, 7024) konfiguriert ist.

**6.** Positionierungs- und Stabilisierungsstruktur (3300) nach Anspruch 5, wobei der Durchflussregler (3010, 7010) ein Einlassverbindungsstück aufweist, das im Gebrauch zum Verbinden einer Röhre (4050) mit dem Einlass konfiguriert ist.

**7.** Positionierungs- und Stabilisierungsstruktur (3300) nach Anspruch 5 oder Anspruch 6, wobei die Kammer (3024, 7024) einen ersten Auslass (3014, 7014), der dazu konfiguriert ist, im Gebrauch in Strömungsverbindung mit dem ersten Kopfhalterungsrohr (3302) zu stehen, und einen zweiten Auslass (3016, 7016), der dazu konfiguriert ist, im Gebrauch in Strömungsverbindung mit dem zweiten Kopfhalterungsrohr (3304) zu stehen, aufweist.

**8.** Positionierungs- und Stabilisierungsstruktur (3300) nach Anspruch 7, wobei in der ersten Konfiguration:

die Barrierenanordnung (3020, 7020) den zweiten Auslass (3016, 7016) vollständig oder im Wesentlichen blockiert; und/oder
die Barrierenanordnung (3020, 7020) im Wesentlichen oder vollständig verhindert, dass der Atemgasstrom in das zweite Kopfhalterungsrohr (3304) strömt; und/oder
der erste Auslass (3014, 7014) im Wesentlichen oder vollständig geöffnet ist.

**9.** Positionierungs- und Stabilisierungsstruktur (3300) nach Anspruch 7, wobei in der zweiten Konfiguration:

die Barrierenanordnung (3020, 7020) den ersten Auslass (3014, 7014) vollständig oder im Wesentlichen blockiert; und/oder
die Barriere im Wesentlichen oder vollständig verhindert, dass der Atemgasstrom in das erste Kopfhalterungsrohr (3302) strömt; und/oder
der zweite Auslass (3016, 7016) im Wesentlichen oder vollständig geöffnet ist.

**10.** Positionierungs- und Stabilisierungsstruktur (3300) nach einem der Ansprüche 1 bis 9, wobei die Barrierenanordnung (3020, 7020):
ein Kolben (3022) oder eine kugelförmig geformte Kugelkomponente (7022) ist; und/oder in einer/der Kammer (3024, 7024) vorgesehen ist.

**11.** Positionierungs- und Stabilisierungsstruktur (3300) nach einem der Ansprüche 1 bis 10, wobei:

das erste Kopfhalterungsrohr (3302) ein erstes Verbindungsstück (3006) aufweist, das zum Verbinden mit der Patientenschnittstelle (3102) konfiguriert ist, und das zweite Kopfhalterungsrohr (3304) ein zweites Verbindungsstück (3008) aufweist, das zum Verbinden mit der Patientenschnittstelle (3102) konfiguriert ist; und/oder
das erste Kopfhalterungsrohr (3302) dazu konfiguriert ist, im Gebrauch an einem ersten Wangenbereich des Patienten anzuliegen, und das zweite Kopfhalterungsrohr (3304) dazu konfiguriert ist, im Gebrauch an einem zweiten Wangenbereich des Patienten anzuliegen; und/oder das erste Kopfhalterungsrohr (3302) und/oder das zweite Kopfhalterungsrohr (3304) teilweise oder vollständig komprimierbar sein kann.

**12.** Atembehandlungssystem, aufweisend
die Positionierungs- und Stabilisierungsstruktur nach Anspruch 10 oder 11 und eine Patientenschnittstelle (3102).

**13.** Atmungsbehandlungssystem nach Anspruch 12, wobei die Patientenschnittstelle (3102) zum Leiten des Atemgases zu den nasalen Atemwegen des Patienten und/oder dem Mund des Patienten konfiguriert ist, wobei die Patientenschnittstelle (3102) eine Dichtungskomponente aufweist, die konfiguriert ist zum:

abdichtenden Eingreifen in mindestens einen Teil des Gesichts des Patienten und/oder
Abdichten um mindestens die nasalen Atemwege des Patienten oder um den Mund des Patienten.

**14.** Atmungsbehandlungssystem nach Anspruch 13, wobei die Dichtungskomponente ein Polster ist.

**Revendications**

1. Structure de positionnement et de stabilisation (3300) d'un système de traitement par thérapie respiratoire configuré en utilisation pour maintenir une interface patient (3102) dans une position par rapport à la voie ou aux voies aériennes d'un patient, la structure de positionnement et de stabilisation (3300) comprenant un premier tube de casque (3302), un second tube de casque (3304), et étant **caractérisée par** un régulateur de débit (3010, 7010), dans laquelle le régulateur de débit (3010, 7010) est configuré pour recevoir en utilisation un flux de gaz respiratoire et pour ajuster la proportion d'un flux de gaz respiratoire qui s'écoule depuis le régulateur de débit (3010, 7010) dans le premier tube de casque (3302) et le second tube de casque (3304), et dans laquelle le régulateur de débit (3010, 7010) comporte un agencement barrière (3020, 7020) qui est mobile entre une première configuration et une seconde configuration de manière à ajuster la proportion du flux de gaz respiratoire qui s'écoule depuis le régulateur de débit (3010, 7010) dans le premier tube de casque (3302) et le second tube de casque (3304), et dans laquelle l'agencement barrière (3010, 7010) est configuré pour être déplacé entre la première configuration et la seconde configuration par gravité.

2. Structure de positionnement et de stabilisation (3300) selon la revendication 1, dans laquelle le régulateur de débit (3010, 7010) est configuré pour diriger une partie relativement plus importante du flux de gaz respiratoire dans l'un parmi le premier tube de casque (3302) et le second tube de casque (3304) et une partie relativement moins importante du flux de gaz respiratoire dans l'autre parmi le premier tube de casque (3302) et le second tube de casque (3304).

3. Structure de positionnement et de stabilisation (3300) selon la revendication 2, dans laquelle la partie relativement plus importante du flux de gaz respiratoire est la totalité ou une partie substantielle du flux.

4. Structure de positionnement et de stabilisation (3300) selon l'une quelconque des revendications 1 à 3, dans laquelle le régulateur de débit (3010, 7010) est configuré pour, en utilisation, diriger la totalité ou une partie substantielle du flux de gaz respiratoire vers :

   le premier tube de casque (3302) si/lorsque la tête du patient est sensiblement inclinée vers un premier côté ; et/ou
   le second tube de casque (3304) si/lorsque la tête du patient est inclinée vers un second côté ; et/ou
   le rapport de la partie du flux dirigé vers le premier tube de casque (3302) et le second tube de casque (3304) dépend de l'orientation de la tête du patient.

5. Structure de positionnement et de stabilisation (3300) selon l'une quelconque des revendications 1 à 4, dans laquelle le régulateur de débit (3010, 7010) comprend un corps qui définit une chambre (3024, 7024), comportant une entrée (3012, 7012) configurée pour faciliter un flux de gaz respiratoire s'écoulant dans la chambre (3024, 7024).

6. Structure de positionnement et de stabilisation (3300) selon la revendication 5, dans laquelle le régulateur de débit (3010, 7010) comporte un connecteur d'entrée qui est configuré pour connecter, en utilisation, un conduit (4050) à l'entrée.

7. Structure de positionnement et de stabilisation (3300) selon la revendication 5 ou la revendication 6, dans laquelle la chambre (3024, 7024) comporte une première sortie (3014, 7014) configurée pour, en utilisation, être en communication d'écoulement avec le premier tube de casque (3302) et une seconde sortie (3016, 7016) configurée pour, en utilisation, être en communication d'écoulement avec le second tube de casque (3304).

8. Structure de positionnement et de stabilisation (3300) selon la revendication 7, dans laquelle, lorsqu'elle est dans la première configuration :

   l'agencement barrière (3020, 7020) bloque complètement ou sensiblement la seconde sortie (3016, 7016) ; et/ou l'agencement barrière (3020, 7020) empêche sensiblement ou complètement l'écoulement du flux de gaz respiratoire dans le second tube de casque (3304) ; et/ou la première sortie (3014, 7014) est sensiblement ou complètement ouverte.

9. Structure de positionnement et de stabilisation (3300) selon la revendication 7, dans laquelle, lorsqu'elle est dans la seconde configuration :

   l'agencement barrière (3020, 7020) bloque complètement ou sensiblement la première sortie (3014, 7014) ; et/ou

la barrière empêche sensiblement ou complètement l'écoulement du flux de gaz respiratoire dans le premier tube de casque (3302) ; et/ou
la seconde sortie (3016, 7016) est sensiblement ou complètement ouverte.

**10.** Structure de positionnement et de stabilisation (3300) selon l'une quelconque des revendications 1 à 9, dans laquelle l'agencement barrière (3020, 7020) :
est un piston (3022) ou un composant à bille de forme sphérique (7022) ; et/ou disposé dans une/la chambre (3024, 7024).

**11.** Structure de positionnement et de stabilisation (3300) selon l'une quelconque des revendications 1 à 10, dans laquelle ;

le premier tube de casque (3302) comprend un premier connecteur (3006) configuré pour se connecter à l'interface patient (3102) et le second tube de casque (3304) comprend un second connecteur (3008) configuré pour se connecter à l'interface patient (3102) ; et/ou
le premier tube de casque (3302) est configuré pour reposer, en utilisation, contre une première région de joue du patient, et le second tube de casque (3304) est configuré pour reposer, en utilisation, contre une seconde région de joue du patient ; et/ou
un ou plusieurs parmi le premier tube de casque (3302) et le second tube de casque (3304) peuvent être partiellement ou complètement compressibles.

**12.** Système de traitement respiratoire, comportant
la structure de positionnement et de stabilisation selon les revendications 10 ou 11, et une interface patient (3102).

**13.** Système de traitement respiratoire selon la revendication 12, dans lequel l'interface patient (3102) est configurée pour transporter le gaz respiratoire vers une ou plusieurs des voies respiratoires nasales du patient et la bouche du patient, dans lequel l'interface patient (3102) comporte un composant d'étanchéité qui est configuré pour :

mettre en prise de manière étanche au moins une partie du visage du patient ; et/ou
étanchéifier la zone autour d'au moins une parmi les voies respiratoires nasales du patient et la bouche du patient.

**14.** Système de traitement respiratoire selon la revendication 13, dans lequel le composant d'étanchéité est un coussinet.

500
3000
4000
5000
4170
9000

FIG. 1A

500
9000
3000
4170
5000
4000

FIG. 1B

FIG. 1C

9000
500
3000
4000
5000
4170

Nasal cavity
Oral cavity
Larynx
Vocal folds
Oesophagus
Trachea
Bronchus
Lung
Heart
Diaphragm
Alveolar sacs

FIG. 2A

Copyright 2012 ResMed Limited

Nasal cavity
Nasal bone
Lateral nasal cartilage
Greater alar cartilage
Nostril
Lip superior
Lip inferior
Hard palate
Soft palate
Oropharynx
Tongue
Epiglottis
Vocal folds
Esophagus
Trachea
Larynx

FIG. 2B

Copyright 2012 ResMed Limited

Superior
Inferior
Sagittal plane
Right
Left
Endocanthion
Nasal ala
Lip Superior
Nasolabial sulcus
Upper Vermillion
Lower Vermillion
Cheilion
Lip Inferior
Mouth width
radially outward
radially inward

FIG. 2C

Copyright 2012 ResMed Limited

Superior
Anterior
Posterior
Inferior
Otobasion superior
Otobasion inferior
Alar crest point
Glabella
Sellion
Ridge
Pronasale
Subnasale
Lip superior
Lip inferior
Supramenton

FIG. 2D

Copyright 2012 ResMed Limited

Coronal plane
Superior
Posterior
Anterior
Inferior
Frankfort horizontal
Nasolabial
angle

FIG. 2E

Copyright 2012 ResMed Limited

Sagittal plane
Pronasale
columella
Subnasale
Naris
Major axis
of naris
Upper vermilion
Lip inferior
Naso-labial sulcus

FIG. 2F

Copyright 2012 ResMed Limited

Epidermis
Adipose tissue
Nasal bone
Lateral cartilage
Septum cartilage
Greater alar cartilage
Frontal process of maxilla
Lesser alar cartilage
Fibrofatty tissue
Frontal sinus
Nasal bone
Septum cartilage
Medial crus of greater alar cartilage
Anterior nasal spine

FIG. 2G

FIG. 2H

FIG. 2I

Copyright 2012 ResMed Limited

Concha
Frontal bone
Sphenoid bone
Nasal bone
Zygomatic bone
Maxilla
Masseter m.
Mandible
Mental protuberance
Digastricus m.
Sternocleidomastoid m.
Parietal bone
Temporal bone
Occipital bone
Trapezius m.

FIG. 2J

FIG. 2K

Frontal bone
Supraorbital foramen
Nasal bones
Septal cartilage
Lateral cartilage
Sesamoid cartilage
Greater alar cartilage
Medial crus of greater alar cartilage
Anterior nasal spine
Infraorbital foramen
Lesser nasal cartilage
Alar fibrofatty tissue
Septal cartilage

FIG. 2L

Copyright 2012 ResMed Limited

2000
9000
3300
3004
2506B
9002A
9002B
2504
2506A
3002
2502


FIG. 3A

3300
3000
3700
3100
3102
3200
3400
3600

FIG. 3B

3106
3106B
3100
+ = Positive Curvature =
— = Negative Curvature =
= Outward Normal
3106A
Saddle Region
Edge of Surface
Dome Region
3104
Exterior Surface
Saddle Region
Dome Region

FIG. 3C

Dome Region
Saddle Region
+ = Positive Curvature =
— = Negative Curvature =
= Outward Normal
A Path on Surface
B
Edge of Surface
A
Straight Line Distance
3100
Exterior Surface
Saddle Region


FIG. 3D

4050
3309
3304
3010
3302
3006
3008
3000
3100
3102
3200

FIG. 4A

4050
3304
3309
3008
3200
3102
3006
2100
3010
3302
3000

FIG. 4B

3010
3304
3008
3200
3102
2100
3006
3000
3302
4050
3309

FIG. 4C

3309
4050
3304
3010
3302
3000
2100
3008
3102

FIG. 4D

3016
3012
3011
3014

FIG. 5

3016
3012
3011
3014
4050
3010

FIG. 6

3304
4050
3016
3012
3010
3014
3302

FIG. 7

3016
4050
3012
3020 (3022)
3024
3014
3010

FIG. 8

3016
3024
3012
3020 (3022)
4050
3014
3010

FIG. 9

9000
Fetus

FIG. 10A

9000
Log

FIG. 10B

9000
Yearner

FIG. 10C

9000
Soldier

FIG. 10D

9000
Starfish

FIG. 10E

9000
Freefall

FIG. 10F

3040
3042
Sensor(s)
Data
Flow
Controller
Command
Actuator
3030
3032

FIG. 11

7020 (7022)
7016
B
A
A
7012
B
A
A
B
B
7014
7011
7010

FIG. 12

7011
7016
7020 (7022)
7012
7014
7010

FIG. 13

7016
7024
7012
7020 (7022)
7011
7010

FIG. 14

7016
7024
7012
7011
7020 (7022)
7014
7010

FIG. 15

7016
7024
7012
7011
7020 (7022)
7014
7010

FIG. 16

7016
7011
7012
7020 (7022)
7024
7014
7010

FIG. 17

Textile conduit with 452 value
Current silicone conduit
Pressure drop differences when the conduit is occluded and not occluded
Pressure drop (cmH2)
3
2.5
2
1.5
1
0.5
0
30
50
100
Flow rate (L/min)

**FIG. 18**

Pressure
Contour 1
783.7
734.7
685.7
636.7
587.8
538.8
489.8
440.8
391.8
342.9
293.9
244.9
195.9
146.9
98.0
49.0
0.0
[Pa]
ANSYS

FIG. 19A

Pressure
Contour 1
783.7
734.7
685.7
636.7
587.8
538.8
489.8
440.8
391.8
342.9
293.9
244.9
195.9
146.9
98.0
49.0
0.0
[Pa]
ANSYS

FIG. 19B

valve 452 not occluded
valve 452 occluded
System impedance comparison on the valve 452
- with and without occluding the conduit
Impedance fluctuation is small
Pressure drop (cmH2O)
0
1
2
3
4
5
6
7
8
9
Flow rate (L/min)
20
30
40
50
60
70
80
90
100

FIG. 20

3000A
3306
3107
3110
3108
3105
3103
3120a
3306
3114
3120b
3400
3112
3118

FIG. 21A

3306
3000A
3107
3110
3108
3103
3103a
3105
3120a
3306
3400
3120b
3114b
3114a
3112
3400
3118

FIG. 21B

3110
3108
3107
3105
3103
3114a
3103a
3134a
3134
3122
3136a
3142a
3142b
3137
3136b
3114
3114a

**FIG. 22A**

3106
3107/
3107a
3110
3108
3103a
3105/
3105a
3114a
3103b
3114
3138
3122
3134a
3140
3134
3142a
3142b
3136
3122a
3137

FIG. 22B

3103
3106a
3146
3134a
3142b
3140
3134
3107
3110
3103b
3108
3137
3122
3122a
3105
3142a
3144
3138

FIG. 22C

3103
3105
3144
3138
3134a
3142a
3134
3108
3103b
3110
3137
3104
3122
3107
3146
3142b
3140
3122a
3103a

FIG. 22D

3103
3103a
3134a
3142a
3144
3105
3138
3122
3108
3105b
3110
3140
3122a
3137
3107
3134
3142b
3146

FIG. 22E

3103
3107
3122
3114
3110
3114a
3134
3142a
3136a
3103a
3122a
3137
3134a
3114a

FIG. 22F

3134
3144
3134a
3122
3138
3136a
3137
3140
3146
3142b

FIG. 23A

3134
3142a
3137
3122a
3136
3122
3144
3134a
3138
3142b
3140
3146

FIG. 23B

3122
3142b
3122a
3140
3146
3137
3144
3134
3142a
3134a
3138

FIG. 23C

3000A
3116
3400
3112
3106
3306
3107
3130
9002B
9000

FIG. 24A

3000A
3306
9000
3120a
3110
9002B
3400
3114
3116
3107
3103a
3105
9002A
3400
3108
3112
3103
3120b
3306

FIG. 24B

3306
3000A
9000
3120b
3114
3103
3108
3400
9002A
3114
3105
9002B
3116
3107
3110
3400
3120a
3306

**FIG. 24C**

3107
3000A
3110
3105
3306
3108
3103
3306
3112
3120c
3118

FIG. 25A

3306
3000A
3107
3110
3108
3105
3103
3103a/
3134
3306
3122
3312
3120c
3142a
3118

FIG. 25B

3000A
3306
3107/3140
3110
3103
3106a/3134
3105/
3138
3120d
3134a
3306
3122
3136
3108
3120c
3142a
3312
3122a
3116
3118


FIG. 25C

3103
3110
3107
3103a/
3134
3134a
3105
3108
3120d
3142b
3114d
3136
3114d
3142a
3120c

FIG. 25D

3312
3137
3114c
3116
3137
3114c

FIG. 25E

3000A
3103
3108
3105
3103a/3134
3134a
3112
3122
3122a
3137
3136
3306
3118
3116

FIG. 25F

3000A
3108
3108
3105
3306
3120c
3142a
3122a
3116
3136
3118
3137
3114d
3114c
3142b
3120d
3306
3107
3103
3110
3103a/
3134
3134a
3122

FIG. 25G

3000A
3120c
3306
3142a
3103
3114d
3134a
3114c
3107
3312
3110
3136
3103a/3134
3122
3108
3118
3116
3137
3122a
3142b
3120d
3105
3306

FIG. 25H

3000A
3306
3120c
3142b
3114d
3108
3103
3114c
3112
3105
3136
3116
3103a/
3134
3122a
3122
3110
3118
3137
3142a
3107
3306
3120d

**FIG. 25I**

3000A
3306
3120c
3142b
3114d
3108
3114c
3112
3105
3103
3116
3136
3103a/
3134
3122
3118
3110
3137
3142a
3122a
3120d
3107
3306

FIG. 25J

3000A
3308
3300
3306
3107
3110
3105
3108
3103
3306
3310
3112
3120c
3122
3142a
3118

FIG. 25K

6000
6002
6010
4000
6006
6004
6008

FIG. 26A

6004
6008
6002a
6002b
6004a

FIG. 26B

1006
1012
1008
1002
1026
1024
1000
1014

FIG. 27A

1002
1024
1008
1014
1010
1012
1006
1026
1000
1008

FIG. 27B

1006
1026
1012
1008
1002
1024
1008
1008
1000
1008
1008

FIG. 27C

1006
1002
1008
1010A
1008
1000
1008

FIG. 27D

1010A
1008
1008
1008
1008
1006
1004

FIG. 28A

1008
1004
1006
1008
1008
A
B

FIG. 28B

1006
1008
1010A
1008
1004
1008

FIG. 28C

1008
1010A
1016
1015
1006
1018
1004
1008

FIG. 28D

1500
1002
1000
1000
4000
1002
1024
1024

FIG. 29A

1500
1000
1000
1010A
1008
1002
4000
1006
1024
1006
1008
1026

FIG. 29B

1014
1016
1015
1018
1010A
1008
1500
1002
1008
1006
1012

FIG. 29C

1000
1500
1550
1002
1000

FIG. 30

1004
1008
1010A
1006

FIG. 31

1004A
1008
1010A
1006
1008

FIG. 32

1008
1010B
1008
1009
1004B
1006

FIG. 33

1004C
1004C
1008
1008
1006
1006
1022
1022
1008
1008
1020
1020

FIG. 34A

1010A
1010A
1008
1008
1006
1004C
1004C
1022
1022
1006
4000
1020
1020
1008
1008

FIG. 34B

1004D
1008
1006
1010B
1022
1022
1008
1006
1010B
1020
1020
1008
1008

FIG. 35A

1010B
1008
1008
1006
1004D
1020

FIG. 35B

1004E
1004E
1008
1006
1010B
1010B
1008
1022
1022
1006
1008
1020
1020
1008

FIG. 36A

1004E
1008
1008
1004E
1006
1010B
1010B
1022
1006
1020
1008
1008

FIG. 36B

1550
1500A
1024
1026

FIG. 37A

1002
1004E
1000A
1550
1500A
1008
1024
1006
1004E
1008
1000A
1006
1026

FIG. 37B

1008
1006
1008
1004F
1028
1010B
1020
1022

FIG. 38A

1014
1026
1004F
1000B
1002A
1010B
1024
1008
1012
1006
1003

FIG. 38B

1030
1014
1024
1000B
1026
1002B
1008
1004F
1012
1001
1006
1032

FIG. 39A

1002B
1014
1030
1012
1032
1001

FIG. 39B

3302a
3000B
1550
3304a
3302
3304
3100/
3018
3006
3008
3302b
3300
3304b
3102A

FIG. 40

3302a
1550
3000B
3304a
3302c
3018
3304
3302
3318
3300
3020
3100/
3018
3304c
3302b
3006
3008
3304b
3322
3320
3322
3102A

FIG. 41

4015
4000
4018
4112
4015
4220
Superior
Posterior
4012
Anterior
Inferior
4202
4142
4100, 4020
4200
4010
4016
4210
4014

FIG. 42

4110
4112 Air inlet filter
Supplementary $O_2$
4180
Pneumatic block
4120
4122 Inlet muffler
4270
Transducer(s)
4140
4142 Blower
4144 Motor
4020
4120
4124 Outlet muffler
Upstream
4270
Transducer(s)
Downstream
4160
Anti-spillback valve
5000
Humidifier
4110
4114 Filter
4180
4170
Air circuit / delivery tube
Supplementary $O_2$
4270
Transducer(s)
4180
3000
Patient Interface
Supplementary $O_2$

FIG. 43

4200
4210
Power supply
4220
4230
4290
Input device
Central controller
Output device
4140
Pressure generator

FIG. 44

Power supply
4210
Protection circuit
4250
4200
4286
Remote external device
4282
Remote external communication network
Memory
4260
Sensors
Flow
Pressure
Speed
4270
4274
4272
4276
4280
Data communication interface
4284
Local external communication network
Protection circuit
4250
4230
Central controller
4290
Output devices
Display driver
Display
Local external device
4240
Therapy device controller
Clock
Input devices
4292
4294
4288
Humidifier
4232
4220
5000

FIG. 45

4300
4310
Preprocessing
Interface pressure estimation
Vent flow rate estimation
Leak flow rate estimation
4312
4314
4316
Respiratory flow rate estimation
4318
4320
4321
4322
Therapy engine
4323
Phase determination
Waveform determination
Ventilation determination
Flow limitation determination
Apnea / hypopnea determination
Snore determination
4324
4325
4326
Airway patency determination
Target ventilation determination
Therapy parameter determination
4327
4328
4329
4340
Therapy control
Fault condition detection
4330

FIG. 46

4500

4520
Y
Apnea / hypopnea index > threshold for a time?
N
4530
4560
Obstruction Index > threshold ?
N
Decrease treatment pressure
Y
4550
4540
Airway patent?
N
Increase treatment pressure
Y

FIG. 47

5000
5004
5150
5135
5110
5002
5006

FIG. 48

5000
5004
5150
5110
5002
5120
5135
5130
5240
5006

FIG. 49

5210
Sensor(s)
air pressure
air flow
temperature
humidity
5212
5214
5216
5218
5251
5250
5252
Central humidifier controller
Heating element controller
Sensor
5210
Heating element
5240
4220 / 4290
Input/Output Devices
Heated air circuit controller
Sensor
5210
Heated air circuit
5254
4171

FIG. 50

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4944310 A, Sullivan
- US 6532959 B, Berthon-Jones
- WO 1998004310 A
- WO 2006074513 A
- WO 2010135785 A
- US 4782832 A, Trimble
- WO 2004073778 A
- US 20090044808 A
- WO 2005063328 A
- WO 2006130903 A
- WO 2009052560 A
- US 20100000534 A
- AU 2009000671 W
- US 2015367092 A1
- WO 2018065867 A1
- WO 2012107858 A1
- EP 2964300 A1
- AU 201905027 W
- US 7866944 B
- US 8638014 B
- US 8636479 B
- WO 2013020167 A
- US 8733349 B
- WO 2012171072 A
- US 20090065007 A


### Non-patent literature cited in the description


- **JOHN B. WEST**. Respiratory Physiology. Lippincott Williams & Wilkins, 2012
- **D. WHITE et al.** Why nasal airways experience drying during nasal-applied continuous positive airway pressure therapy. *Journal of Sleep Research, European Sleep Research Society*, 24 October 2017 (1)